# EUROPEAN PATENT APPLICATION

(11) **EP 1 548 445 A2**
(43) Date of publication of application: **29.06.2005**
(21) Application number: 04029641.0
(22) Date of filing: 15.12.2004
(51) Int. Cl.: G01N 33/68, C12Q 1/68

(54) **Novel targets for obesity from fat tissue**

(30) Priority: 22.12.2003 EP 03104902
(71) Applicant: F. Hoffmann-La Roche AG, 4070 Basel (CH); Claes-Goran Ostenson, 17164 Solna (SE)
(72) Inventor: Clerc, Roger G., 4102 Binningen (CH); Duchateau-Nguyen, Guillemette, 68400 Riedisheim (FR); Gardes, Christophe, 68870 Bartenheim (FR); Mizrahi, Jacques, 4103 Bottmingen (CH); Ostenson, Claes-Goran, 17176 Stockholm (SE)
(74) Representative: Klostermeyer, Doerte

(57) **Abstract**

The present invention relates to novel targets for identifying compounds that may be useful for the prevention and treatment of obesity.

SEQUENCE LISTING

<110>  F. Hoffmann-La Roche AG

<120>  Novel targets for obesity from subcutaneous fat

<130>  22304

<160>  146

<170>  PatentIn version 3.2

<210>  1

<211>  897

<212>  DNA

<213>  Homo sapiens

<220>

<221>  alpha-2-glycoprotein 1, zinc (AZGP1)

<222>  (1)..(897)

<223>  accession No.s: NM 001185, Hs.512643, Locus ID: 563

<400>  1

atggtaagaa tggtgcctgt cctgctgtct ctgctgctgc ttctgggtcc tgctgtcccc    60

caggagaacc aagatggtcg ttactctctg acctatatct acactgggct gtccaagcat   120

gttgaagacg tccccgcgtt tcaggccctt ggctcactca atgacctcca gttctttaga   180

tacaacagta aagacaggaa gtctcagccc atgggactct ggagacaggt ggaaggaatg   240

gaggattgga agcaggacag ccaacttcag aaggccaggg aggacatctt tatggagacc   300

ctgaaagaca ttgtggagta ttacaacgac agtaacgggt ctcacgtatt gcagggaagg   360

tttggttgtg agatcgagaa taacagaagc agcggagcat tctggaaata ttactatgat   420

ggaaaggact acattgaatt caacaaagaa atcccagcct gggtcccctt cgacccagca   480

gcccagataa ccaagcagaa gtgggaggca gaaccagtct acgtgcagcg ggccaaggct   540

tacctggagg aggagtgccc tgcgactctg cggaaatacc tgaaatacag caaaaatatc   600

ctggaccggc aagatcctcc ctctgtggtg gtcaccagcc accaggcccc aggagaaaag   660

aagaaactga agtgcctggc ctacgacttc tacccaggga aaattgatgt gcactggact   720

cgggccggcg aggtgcagga gcctgagtta cggggagatg ttcttcacaa tggaaatggc   780

acttaccagt cctgggtggt ggtggcagtg cccccgcagg acacagcccc ctactcctgc   840

cacgtgcagc acagcagcct ggcccagccc ctcgtggtgc cctgggaggc cagctag 897

<210> 2

<211> 5828

<212> DNA

<213> Homo sapiens

<220>

<221> insulin receptor substrate 1 (IRS1)

<222> (1)..(5828)

<223> accession No.s: NM005544, Hs.96063, LocusID: 3667

<400> 2

cggcggcgcg gtcggagggg gccggcgcgc agagccagac gccgccgctt gttttggttg 60

gggctctcgg caactctccg aggaggagga ggaggaggga ggaggggaga agtaactgca 120

gcggcagcgc cctcccgagg aacaggcgtc ttccccgaac ccttcccaaa cctcccccat 180

cccctctcgc ccttgtcccc tcccctcctc cccagccgcc tggagcgagg ggcagggatg 240

agtctgtccc tccggccggt ccccagctgc agtggctgcc cggtatcgtt tcgcatggaa 300

aagccacttt ctccacccgc cgagatgggc ccggatgggg ctgcagagga cgcgcccgcg 360

ggcggcggca gcagcagcag cagcagcagc agcaacagca acagccgcag cgccgcggtc 420

tctgcgactg agctggtatt tgggcggctg gtggcggctg ggacggttgg ggggtgggag 480

gaggcgaagg aggagggaga accccgtgca acgttgggac ttggcaaccc gcctccccct 540

gcccaaggat atttaatttg cctcgggaat cgctgcttcc agaggggaac tcaggaggga 600

aggcgcgcgc gcgcgcgcgc tcctggaggg gcaccgcagg gacccccgac tgtcgcctcc 660

ctgtgccgga ctccagccgg ggcgacgaga gatgcatctt cgctccttcc tggtggcggc 720

ggcggctgag aggagacttg gctctcggag gatcggggct gccctcaccc cggacgcact 780

gcctccccgc cggcgtgaag cgcccgaaaa ctccggtcgg gctctctcct gggctcagca 840

gctgcgtcct ccttcagctg cccctccccg gcgcgggggg cggcgtggat ttcagagtcg 900

gggtttctgc tgcctccagc cctgtttgca tgtgccgggc cgcggcgagg agcctccgcc 960

ccccacccgg ttgtttttcg gagcctccct ctgctcagcg ttggtggtgg cggtggcagc 1020

atggcgagcc ctccggagag cgatggcttc tcggacgtgc gcaaggtggg ctacctgcgc 1080

aaacccaaga gcatgcacaa acgcttcttc gtactgcgcg cggccagcga ggctgggggc 1140

ccggcgcgcc tcgagtacta cgagaacgag aagaagtggc ggcacaagtc gagcgccccc 1200

aaacgctcga tcccccttga gagctgcttc aacatcaaca agcgggctga ctccaagaac 1260

aagcacctgg tggctctcta cacccgggac gagcactttg ccatcgcggc ggacagcgag 1320

gccgagcaag acagctggta ccaggctctc ctacagctgc acaaccgtgc taagggccac 1380

cacgacggag ctgcggccct cggggcggga ggtggtgggg gcagctgcag cggcagctcc 1440

ggccttggtg aggctgggga ggacttgagc tacggtgacg tgcccccagg acccgcattc 1500

aaagaggtct ggcaagtgat cctgaagccc aagggcctgg gtcagacaaa gaacctgatt 1560

ggtatctacc gcctttgcct gaccagcaag accatcagct tcgtgaagct gaactcggag 1620

gcagcggccg tggtgctgca gctgatgaac atcaggcgct gtggccactc ggaaaacttc 1680

ttcttcatcg aggtgggccg ttctgccgtg acggggcccg gggagttctg gatgcaggtg 1740

gatgactctg tggtggccca gaacatgcac gagaccatcc tggaggccat gcgggccatg 1800

agtgatgagt tccgccctcg cagcaagagc cagtcctcgt ccaactgctc taaccccatc 1860

agcgtccccc tgcgccggca ccatctcaac aatcccccgc ccagccaggt ggggctgacc 1920

cgccgatcac gcactgagag catcaccgcc acctccccgg ccagcatggt gggcgggaag 1980

ccaggctcct tccgtgtccg cgcctccagt gacggcgaag gcaccatgtc ccgcccagcc 2040

tcggtggacg gcagccctgt gagtcccagc accaacagaa cccacgccca ccggcatcgg 2100

ggcagcgccc ggctgcaccc cccgctcaac cacagccgct ccatccccat gccggcttcc 2160

cgctgctcgc cttcggccac cagcccggtc agtctgtcgt ccagtagcac cagtggccat 2220

ggctccacct cggattgtct cttcccacgg cgatctagtg cttcggtgtc tggttccccc 2280

agcgatggcg gtttcatctc ctcggatgag tatggctcca gtccctgcga tttccggagt 2340

tccttccgca gtgtcactcc ggattccctg ggccacaccc caccagcccg cggtgaggag 2400

gagctaagca actatatctg catgggtggc aaggggccct ccaccctgac cgcccccaac 2460

ggtcactaca ttttgtctcg gggtggcaat ggccaccgct gcaccccagg aacaggcttg 2520

ggcacgagtc cagccttggc tggggatgaa gcagccagtg ctgcagatct ggataatcgg 2580

ttccgaaaga gaactcactc ggcaggcaca tcccctacca ttacccacca gaagaccccg 2640

tcccagtcct cagtggcttc cattgaggag tacacagaga tgatgcctgc ctacccacca 2700

ggaggtggca gtggaggccg actgccggga cacaggcact ccgccttcgt gcccacccgc 2760

tcctacccag aggagggtct ggaaatgcac cccttggagc gtcggggggg gcaccaccgc 2820

ccagacagct ccaccctcca cacggatgat ggctacatgc ccatgtcccc aggggtggcc 2880

ccagtgccca gtggccgaaa gggcagtgga gactatatgc ccatgagccc caagagcgta 2940

tctgccccac agcagatcat caatcccatc agacgccatc cccagagagt ggaccccaat 3000

ggctacatga tgatgtcccc cagcggtggc tgctctcctg acattggagg tggccccagc 3060

agcagcagca gcagcagcaa cgccgtccct tccgggacca gctatggaaa gctgtggaca 3120

aacggggtag ggggccacca ctctcatgtc ttgcctcacc ccaaaccccc agtggagagc 3180

agcggtggta agctcttacc ttgcacaggt gactacatga acatgtcacc agtgggggac 3240

tccaacacca gcagcccctc cgactgctac tacggccctg aggaccccca gcacaagcca 3300

gtcctctcct actactcatt gccaagatcc tttaagcaca cccagcgccc cggggagccg 3360

gaggagggtg cccggcatca gcacctccgc ctttccacta gctctggtcg ccttctctat 3420

gctgcaacag cagatgattc ttcctcttcc accagcagcg acagcctggg tgggggatac 3480

tgcggggcta ggctggagcc cagccttcca catccccacc atcaggttct gcagccccat 3540

ctgcctcgaa aggtggacac agctgctcag accaatagcc gcctggcccg gcccacgagg 3600

ctgtccctgg gggatcccaa ggccagcacc ttacctcggg cccgagagca gcagcagcag 3660

cagcagccct tgctgcaccc tccagagccc aagagcccgg gggaatatgt caatattgaa 3720

tttgggagtg atcagtctgg ctacttgtct ggcccggtgg ctttccacag ctcaccttct 3780

gtcaggtgtc catcccagct ccagccagct cccagagagg aagagactgg cactgaggag 3840

tacatgaaga tggacctggg gccgggccgg agggcagcct ggcaggagag cactggggtc 3900

gagatgggca gactgggccc tgcacctccc ggggctgcta gcatttgcag gcctacccgg 3960

gcagtgccca gcagccgggg tgactacatg accatgcaga tgagttgtcc ccgtcagagc 4020

tacgtggaca cctcgccagc tgcccctgta agctatgctg acatgcgaac aggcattgct 4080

gcagaggagg tgagcctgcc cagggccacc atggctgctg cctcctcatc ctcagcagcc 4140

tctgcttccc cgactgggcc tcaaggggca gcagagctgg ctgcccactc gtccctgctg 4200

gggggcccac aaggacctgg gggcatgagc gccttcaccc gggtgaacct cagtcctaac 4260

cgcaaccaga gtgccaaagt gatccgtgca gacccacaag ggtgccggcg gaggcatagc 4320

tccgagactt tctcctcaac acccagtgcc acccgggtgg gcaacacagt gccctttgga 4380

gcgggggcag cagtaggggg cggtggcggt agcagcagca gcagcgagga tgtgaaacgc 4440

cacagctctg cttcctttga gaatgtgtgg ctgaggcctg gggagcttgg gggagccccc 4500

aaggagccag ccaaactgtg tggggctgct gggggtttgg agaatggtct taactacata 4560

gacctggatt tggtcaagga cttcaaacag tgccctcagg agtgcacccc tgaaccgcag 4620

cctcccccac ccccaccccc tcatcaaccc ctgggcagcg gtgagagcag ctccacccgc 4680

cgctcaagtg aggatttaag cgcctatgcc agcatcagtt tccagaagca gccagaggac 4740

cgtcagtagc tcaactggac atcacagcag aatgaagacc taaatgacct cagcaaatcc 4800

tcttctaact catgggtacc cagactctaa atatttcatg attcacaact aggacctcat 4860

atcttcctca tcagtagatg gtacgatgca tccatttcag tttgtttact ttatccaatc 4920

ctcaggattt cattgactga actgcacgtt ctatattgtg ccaagcgaaa aaaaaaaatg 4980

cactgtgaca ccagaataat gagtctgcat aaacttcatc ttcaacctta aggacttagc 5040

tggccacagt gagctgatgt gcccaccacc gtgtcatgag agaatgggtt tactctcaat 5100

gcattttcaa gatacatttc atctgctgct gaaactgtgt acgacaaagc atcattgtaa 5160

attatttcat acaaaactgt tcacgttggg tggagagagt attaaatatt taacataggt 5220

tttgatttat atgtgtaatt ttttaaatga aaatgtaact tttcttacag cacatctttt 5280

ttttggatgt gggatggagg tatacaatgt tctgttgtaa agagtggagc aaatgcttaa 5340

aacaaggctt aaaagagtag aatagggtat gatccttgtt ttaagattgt aattcagaaa 5400

acataatata agaatcatag tgccatagat ggttctcaat tgtatagtta tatttgctga 5460

tactatctct tgtcatataa acctgatgtt gagctgagtt ccttataaga attaatctta 5520

attttgtatt ttttcctgta agacaatagg ccatgttaat taaactgaag aaggatatat 5580

ttggctgggt gttttcaaat gtcagcttaa aattggtaat tgaatggaag caaaattata 5640

agaagaggaa attaaagtct tccattgcat gtattgtaaa cagaaggaga tgggtgattc 5700

cttcaattca aaagctctct ttggaatgaa caatgtgggc gtttgtaaat tctggaaatg 5760

tctttctatt cataataaac tagatactgt tgatctttta aaaaaaaaaa aaaaaaaaaa 5820

aaaaaaaa 5828

<210> 3

<211> 878

<212> DNA

<213> Homo sapiens

<220>

<221> apolipoprotein a-i (APOA1)

<222> (1)..(878)

<223> accession No.s: M27875, Hs.93194, LocusID:897

<400> 3

tcccccacgg cccttcagga tgaaagctgc ggtgctgacc ttggccgtgc tcttcctgac 60

ggggagccag gctcggcatt tctggcagca agatgaaccc ccccagagcc cctgggatcg 120

agtgaaggac ctggccactg tgtacgtgga tgtgctcaaa gacagcggca gagactatgt 180

gtcccagttt gaaggctccg ccttgggaaa acagctaaac ctaaagctcc ttgacaactg 240

ggacagcgtg acctccacct tcagcaagct gcgcgaacag ctcggccctg tgacccagga 300

gttctgggat aacctggaaa aggagacaga gggcctgagg caggagatga gcaaggatct 360

ggaggaggtg aaggccaagg tgcagcccta cctggacgac ttccagaaga agtggcagga 420

ggagatggag ctctaccgcc agaaggtgga gccgctgcgc gcagagctcc aagagggcgc 480

gcgccagaag ctgcacgagc tgcaagagaa gctgagccca ctgggcgagg agatgcgcga 540

ccgcgcgcgc gcccatgtgg acgcgctgcg cacgcatctg gccccctaca gcgacgagct 600

gcgccagcgc ttggccgcgc gccttgaggc tctcaaggag aacggcggcg ccagactggc 660

cgagtaccac gccaaggcca ccgagcatct gagcacgctc agcgagaagg ccaagcccgc 720

gctcgaggac ctccgccaag gcctgctgcc cgtgctggag agcttcaagg tcagcttcct 780

gagcgctctc gaggagtaca ctaagaagct caacacccag tgaggcgccc gccgccgccc 840

cccttcccgg tgctcagaat aaacgtttcc aaagtggg 878

<210> 4

<211> 2616

<212> DNA

<213> Homo sapiens

<220>

<221> aryl hydrocarbon receptor nuclear translocator (ARNT)

<222> (1)..(2616)

<223> accession No.s: M69238.1, Hs.131494, LocusID:405

<400> 4

atggcggctc ctcccactgg ggggggggtg gcgcggcggc ggtggcatct gcggccatgg 60

cggcgactac tgccaacccc gaaatgacat cagatgtacc atcactgggt ccagccattg 120

cctctggaaa ctctggacct ggaattcaag gtggaggagc cattgtccag agggctatta 180

agcggcgacc agggctggat tttgatgatg atggagaagg gaacagtaaa tttttgaggt 240

gtgatgatga tcagatgtct aacgataagg agcggtttgc caggtcggat gatgagcaga 300

gctctgcgga taaagagaga cttgccaggg aaaatcacag tgaaattgaa cggcggcgac 360

ggaacaagat gacagcctac atcacagaac tgtcagatat ggtacccacc tgtagtgccc 420

tggctcgaaa accagacaag ctaaccatct tacgcatggc agtttctcac atgaagtcct 480

tgcggggaac tggcaacaca tccactgatg gctcctataa gccgtctttc ctcactgatc 540

aggaactgaa acatttgatc ttggaggcag cagatggctt tctgtttatt gtctcatgtg 600

agacaggcag ggtggtgtat gtgtctgact ccgtgactcc tgttttgaac cagccacagt 660

ctgaatggtt tggcagcaca ctctatgatc aggtgcaccc agatgatgtg gataaacttc 720

gtgagcagct ttccacttca gaaaatgccc tgacagggcg tatcctggat ctaaagactg 780

gaacagtgaa aaaggaaggt cagcagtctt ccatgagaat gtgtatgggc tcaaggagat 840
cgtttatttg ccgaatgagg tgtggcagta gctctgtgga cccagtttct gtgaataggc 900
tgagctttgt gaggaacaga tgcaggaatg gacttggctc tgtaaaggat ggggaacctc 960
acttcgtggt ggtccactgc acaggctaca tcaaggcctg gcccccagca ggtgtttccc 1020
tcccagatga tgacccagag gctggccagg gaagcaagtt ttgcctagtg gccattggca 1080
gattgcaggt aactagttct cccaactgta cagacatgag taatgtttgt caaccaacag 1140
agttcatctc ccgacacaac attgagggta tcttcacttt tgtggatcac cgctgtgtgg 1200
ctactgttgg ctaccagcca caggaactct taggaaagaa tattgtagaa ttctgtcatc 1260
ctgaagacca gcagcttcta agagacagct tccaacaggt agtgaaatta aaaggccaag 1320
tgctgtctgt catgttccgg ttccggtcta agaaccaaga atggctctgg atgagaacca 1380
gctcctttac tttccagaac ccttactcag atgaaattga gtacatcatc tgtaccaaca 1440
ccaatgtgaa gaactctagc caagaaccac ggcctacact ctccaacaca atccagaggc 1500
cacaactagg tcccacagct aatttacccc tggagatggg ctcaggacag ctggcaccca 1560
ggcagcagca acagcaaaca gaattggaca tggtaccagg aagagatgga ctggccagct 1620
acaatcattc ccaggtggtt cagcctgtga caaccacagg accagaacac agcaagcccc 1680
ttgagaagtc agatggttta tttgcccagg atagagatcc aagattttca gaaatctatc 1740
acaacatcaa tgcggatcag agtaaaggca tctcctccag cactgtccct gccacccaac 1800
agctattctc ccagggcaac acattccctc ctacccccgg gccggcagag aatttcagga 1860
atagtggcct agcccctcct gtaaccattg tccagccatc agcttctgca ggacagatgt 1920
tggcccagat ttcccgccac tccaacccca cccaaggagc aaccccaact tggaccccta 1980
ctacccgctc aggcttttct gcccagcagg tggctaccca ggctactgct aagactcgta 2040
cttcccagtt tggtgtgggc agctttcaga ctccatcctc cttcagctcc atgtccctcc 2100
ctggtgcccc aactgcatcg cctggtgctg ctgcctaccc tagtctcacc aatcgtggat 2160
ctaactttgc tcctgagact ggacagactg caggacaatt ccagacacgg acagcagagg 2220
gtgtgggtgt ctggccacag tggcagggcc agcagcctca tcatcgttca agttctagtg 2280
agcaacatgt tcaacaaccg ccagcacagc aacctggcca gcctgaggtc ttccaggaga 2340
tgctgtccat gctgggagat cagagcaaca gctacaacaa tgaagaattc cctgatctaa 2400
ctatgtttcc ccccttttca gaatagaact attggggtga ggataagggg tgggggagaa 2460
aaaatcactg tttgttttta aaaagcaaat ctttctgtaa acagaataaa agttcctctc 2520
ccttcccttc cctcacccct gacatgtacc ccctttccct tctggctgtt cccctgctct 2580
gttgcctcct aaggtaacat ttataaaaaa aaaaaa 2616

<210> 5

<211> 1431

<212> DNA

<213> Homo sapiens

<220>

<221> creatine kinase, brain (CKB)

<222> (1)..(1413)

<223> accession No.s: NM001823, Hs.173724, LocusID:6309

<400> 5

gctgttcgcc tgcgtcgctc cgggagctgc cgacggacgg agcgcccccg cccccgcccg 60

gccgcccgcc cgccgccgcc atgcccttct ccaacagcca caacgcactg aagctgcgct 120

tcccggccga ggacgagttc cccgacctga gcgcccacaa caaccacatg gccaaggtgc 180

tgacccccga gctgtacgcg gagctgcgcg ccaagagcac gccgagcggc ttcacgctgg 240

acgacgtcat ccagacaggc gtggacaacc cgggccaccc gtacatcatg accgtgggct 300

gcgtggcggg cgacgaggag tcctacgaag tgttcaagga tctcttcgac cccatcatcg 360

aggaccggca cggcggctac aagcccagcg atgagcacaa gaccgacctc aaccccgaca 420

acctgcaggg cggcgacgac ctggacccca actacgtgct gagctcgcgg gtgcgcacgg 480

gccgcagcat ccgtggcttc tgcctccccc cgcactgcag ccgcggggag cgccgcgcca 540

tcgagaagct cgcggtggaa gccctgtcca gcctggacgg cgacctggcg ggccgatact 600

acgcgctcaa gagcatgacg gaggcggagc agcagcagct catcgacgac cacttcctct 660

tcgacaagcc cgtgtcgccc ctgctgctgg cctcgggcat ggcccgcgac tggcccgacg 720

cccgcggtat ctggcacaat gacaataaga ccttcctggt gtgggtcaac gaggaggacc 780

acctgcgggt catctccatg cagaaggggg gcaacatgaa ggaggtgttc acccgcttct 840

gcaccggcct cacccagatt gaaactctct tcaagtctaa ggactatgag ttcatgtgga 900

accctcacct gggctacatc ctcacctgcc catccaacct gggcaccggg ctgcgggcag 960

gtgtgcatat caagctgccc aacctgggca agcatgagaa gttctcggag gtgcttaagc 1020

ggctgcgact tcagaagcga ggcacaggcg gtgtggacac ggctgcggtg ggcggggtct 1080

tcgacgtctc caacgctgac cgcctgggct tctcagaggt ggagctggtg cagatggtgg 1140

tggacggagt gaagctgctc atcgagatgg agcagcggct ggagcagggc caggccatcg 1200

acgacctcat gcctgcccag aaatgaagcc cggcccacac ccgacaccag ccctgctgct 1260

tcctaactta ttgcctgggc agtgcccacc atgcacccct gatgttcgcc gtctggcgag 1320

cccttagcct tgctgtagag acttccgtca cccttggtag agtttatttt tttgatggct 1380

aagatactgc tgatgctgaa ataaactagg gttttggcct gcctgcgtct g 1431

<210> 6

<211> 900

<212> DNA

<213> Homo sapiens

<220>

<221> delta7-sterol-C5-desaturase

<222> (1)..(900)

<223> accession No.s: AF187981.1, Hs.287749, LocusID:6309

<400> 6

atggatcttg tactccgtgt tgcagattac tattttttta caccatacgt gtatccagcc 60

acatggccag aagatgacat cttccgacaa gctattagtc ttctgattgt aacaaatgtt 120

ggtgcttaca tcctttattt cttctgtgca acactgagct attattttgt cttcgatcat 180

gcattaatga aacatccaca atttttaaag aatcaagtcc gtcgagagat taagtttact 240

gtccaggcat tgccatggat aagtattctt actgttgcac tgttcttgct ggagataaga 300

ggttacagca aattacatga tgacctagga gagtttccat atggattgtt tgaacttgtc 360

gttagtataa tatctttcct ctttttcact gacatgttca tctactggat tcacagaggc 420

cttcatcata gactggtata taagcgccta cataaacctc accatatttg gaagattcct 480

actccatttg caagtcatgc ttttcaccct attgatggct ttcttcagag tctaccttac 540

catatatacc cttttatctt tccattacac aaggtggttt atttaagtct gtacatcttg 600

gttaatatct ggacaatttc cattcatgac ggtgattttc gtgtccccca aatcttacag 660

ccatttatta atggctcagc tcatcataca gaccaccata tgttctttga ctataattat 720

ggacaatatt tcactttgtg ggataggatt ggcggctcat tcaaaaatcc ttcatccttt 780

gaggggaagg gaccgctcag ttatgtgaag gagatgacag agggaaagcg cagcagccat 840

tcaggaaatg gctgtaagaa tgaaaaatta ttcaatggag agtttacaaa gactgaatag 900

<210> 7

<211>  2263

<212>  DNA

<213>  Homo sapiens

<220>

<221>  osteomodulin (OMD)

<222>  (1)..(2263)

<223>  accession No.s: NM005014.1, Hs.94070

<400>  7

gcacgagaag aatttaggaa tttctgattc atttaaagga tttacaaatt catcaacccc    60

tgaaaactaa agcaaattga acaggaaaaa aaaaaagaag atgggttttt taagtccaat   120

atatgttatt ttcttctttt ttggagtcaa agtacattgc caatatgaaa cttatcagtg   180

ggatgaagac tatgaccaag agccagatga tgattaccaa acaggattcc catttcgtca   240

aaatgtagac tacggagttc cttttcatca gtatacttta ggctgtgtca gtgaatgctt   300

ctgtccaact aactttccat catcaatgta ctgtgataat cgcaaactca agactatccc   360

aaatattccg atgcacattc agcaactcta ccttcagttc aatgaaattg aggctgtgac   420

tgcaaattca ttcatcaatg caactcatct taaagaaatt aacctcagcc acaacaaaat   480

taaatctcaa aagattgatt atggtgtgtt tgctaagctt ccaaatctac tacaacttca   540

tctagagcat aataatttag aagaatttcc atttcctctt cctaaatctc tggaaagact   600

ccttcttggt tacaatgaaa tctccaaact gcagacaaat gctatggatg ggctagtaaa   660

cttgaccatg cttgatctct gttataatta tcttcatgat tctctgctaa aagacaaaat   720

ctttgccaaa atggaaaaac taatgcagct caacctctgc agtaacagat tagaatcaat   780

gcctcctggt ttgccttctt cacttatgta tctgtcttta gaaaataatt caatttcttc   840

tatacccgaa aaatacttcg acaaacttcc aaaacttcat actctaagaa tgtcacacaa   900

caaactacaa gacatcccat ataatatttt taatcttccc aacattgtag aactcagtgt   960

tggacacaac aaattgaagc aagcattcta tattccaaga aatttggaac acctatacct  1020

acaaaataat gaaatagaaa agatgaatct tacagtgatg tgtccttcta ttgacccact  1080

acattaccac catttaacat acattcgtgt ggaccaaaat aaactaaaag aaccaataag  1140

ctcatacatc ttcttctgct tccctcatat acacactatt tattatggtg aacaacgaag  1200

cactaatggt caaacaatac aactaaagac acaagttttc aggagatttc cagatgatga  1260

tgatgaaagt gaagatcacg atgatcctga caatgctcat gagagcccag aacaagaagg  1320

agcagaaggg cactttgacc ttcattatta tgaaaatcaa gaatagcaag aaactatata  1380

ggtatacact tacgacttca caaaacctat acttaatata gtaaatctaa gtaaacatgt  1440

attactcaaa gtaatatatt tagaattatg tattagtata agatcagaat tgaatttaag 1500

ttgttggtga catctgcatc atttcatagg attagaactt actcaaaata atgtaaatct 1560

ttaaaaatat aaattagaat gacaagtggg aatcataaat taaacgttaa tggtttctta 1620

tgctcttttt aaatatagaa atatcatgtt aaagaaagtg agtgtatcat ttctattaac 1680

agtaattttt ctaaaaatga ggaaggaagt agcattagca gtaaagaccc acaggccacg 1740

accctcctga tcactctgag gacaatattt aaatgacagg aaggtatatt aatgtaacaa 1800

gcattcattt aaggaataga ccattttctc tgacctcttc ttcaggaaag ctttcacact 1860

ggtatttgtg atctcaccat tatgacatcc atccccctag ctcaccacat agcacataga 1920

atgatatttt tgatttgtaa gaggccatcc aggtactaag gacccaaggc atacagattc 1980

acaaaattaa ctaatctttt tgcctcagaa taccaaaaca acaaaattat aaagctgtta 2040

tttggacaac taaaaaacac caaactatct cattgcaatt tgtattttag cagatttcag 2100

caactatcct aaacaatgtt attgtgttcc attttaactg ggataaatgt ttttgtaaaa 2160

atacaaccat agaaaggcct ctttgttaca aaatgatttg caaagaaata actgctttgt 2220

ttgcaagatt aaattagtgt tggcaaaata aagttctaaa gat 2263

<210> 8

<211> 3155

<212> DNA

<213> Homo sapiens

<220>

<221> very low density lipoprotein receptor (VLDLR)

<222> (1)..(3155)

<223> accession No.s: D16494.1, Hs.275215, LocusID: 7436

<400> 8

catgggcacg tccgcgctct gggcgctctg gctgctgctc gcgctgtgct gggcgccccg 60

ggagagcggc gccaccggaa ccgggagaaa agccaaatgt gaaccctccc aattccagtg 120

cacaaatggt cgctgtatta cgctgttgtg gaaatgtgat ggggatgaag actgtgttga 180

cggcagtgat gaaaagaact gtgtaaagaa gacgtgtgct gaatctgact tcgtgtgcaa 240

caatggccag tgtgttccca gccgatggaa gtgtgatgga gatcctgact gcgaagatgg 300

ttcagatgaa agcccagaac agtgccatat gagaacatgc cgcatacatg aaatcagctg 360

tggcgcccat tctactcagt gtatcccagt gtcctggaga tgtgatggtg aaaatgattg 420

tgacagtgga gaagatgaag aaaactgtgg caatataaca tgtagtcccg acgagttcac 480
ctgctccagt ggccgctgca tctccaggaa ctttgtatgc aatggccagg atgactgcag 540
cgatggcagt gatgagctgg actgtgcccc gccaacctgt ggcgcccatg agttccagtg 600
cagcacctcc tcctgcatcc ccatcagctg ggtatgcgac gatgatgcag actgctccga 660
ccaatctgat gagtccctgg agcagtgtgg ccgtcagcca gtcatacaca ccaagtgtcc 720
agccagcgaa atccagtgcg gctctggcga gtgcatccat aagaagtggc gatgtgatgg 780
ggaccctgac tgcaaggatg gcagtgatga ggtcaactgt ccctctcgaa cttgccgacc 840
tgaccaattt gaatgtgagg atggcagctg catccatggc agcaggcagt gtaatggtat 900
ccgagactgt gtcgatggtt ccgatgaagt caactgcaaa aatgtcaatc agtgcttggg 960
ccctggaaaa ttcaagtgca gaagtggaga atgcatagat atcagcaaag tatgtaacca 1020
ggagcaggac tgcagggact ggagtgatga gcccctgaaa gagtgtcata taaacgaatg 1080
cttggtaaat aatggtggat gttctcatat ctgcaaagac ctagttatag gctacgagtg 1140
tgactgtgca gctgggtttg aactgataga taggaaaacc tgtggagata ttgatgaatg 1200
ccaaaatcca ggaatctgca gtcaaatttg tatcaactta aaaggcggtt acaagtgtga 1260
atgtagtcgt ggctatcaaa tggatcttgc tactggcgtg tgcaaggcag taggcaaaga 1320
gccaagtctg atcttcacta atcgaagaga catcaggaag attggcttag agaggaaaga 1380
atatatccaa ctagttgaac agctaagaaa cactgtggct ctcgatgctg acattgctgc 1440
ccagaaacta ttctgggccg atctaagcca aaaggctatc ttcagtgcct caattgatga 1500
caaggttggt agacatgtta aaatgatcga caatgtctat aatcctgcag ccattgctgt 1560
tgattgggtg tacaagacca tctactggac tgatgcggct tctaagacta tttcagtagc 1620
taccctagat ggaaccaaga ggaagttcct gtttaactct gacttgcgag agcctgcctc 1680
catagctgtg gacccactgt ctggctttgt ttactggtca gactggggtg aaccagctaa 1740
aatagaaaaa gcaggaatga atggattcga tagacgtcca ctggtgacag cggatatcca 1800
gtggcctaac ggaattacac ttgaccttat aaaaagtcgc ctctattggc ttgattctaa 1860
gttgcacatg ttatccagcg tggacttgaa tggccaagat cgtaggatag tactaaagtc 1920
tctggagttc ctagctcatc ctcttgcact aacaatattt gaggatcgtg tctactggat 1980
agatggggaa aatgaagcag tctatggtgc caataaattc actggatcag agctagccac 2040
tctagtcaac aacctgaatg atgcccaaga catcattgtc tatcatgaac ttgtacagcc 2100
atcaggtaaa aattggtgtg aagaagacat ggagaatgga ggatgtgaat acctatgcct 2160
gccagcacca cagattaatg atcactctcc aaaatatacc tgttcctgtc ccagtgggta 2220
caatgtagag gaaaatggcc gagactgtca aaggatcaat gtgaccacag cagtatcaga 2280
ggtcagtgtt cccccaaaag ggacttctgc cgcatgggcc attcttcctc tcttgctctt 2340

agtgatggca gcagtaggtg gctacttgat gtggcggaat tggcaacaca agaacatgaa 2400

aagcatgaac tttgacaatc ctgtgtactt gaaaaccact gaagaggacc tctccataga 2460

cattggtaga cacagtgctt ctgttggaca cacgtaccca gcaatatcag ttgtaagcac 2520

agatgatgat ctagcttgac ttctgtgaca aatgttgacc tttgaggtct aaacaaataa 2580

tacccccgtc ggaatggaac cgagccagca gctgaagtct ctttttcttc ctctcggctg 2640

gaagaacatc aagatacctt tgcgtggatc aagcttgtgt acttgaccgt ttttatatta 2700

cttttgtaaa tattcttgtc cacattctac ttcagctttg gatgtggtta ccgagtatct 2760

gtaacccttg aatttctaga cagtattgcc acctctggcc aaatatgcac tttccctaga 2820

aagccatatt ccagcagtga aacttgtgct atagtgtata ccacctgtac atacattgta 2880

taggccatct gtaaatatcc cagagaacaa tcactattct taagcacttt gaaaatattt 2940

ctatgtaaat tattgtaaac tttttcaatg gttgggacaa tggcaatagg acaaaacggg 3000

ttactaagat gaaattgcca aaaaatttta taaactaatt ttgtacgtat gaatgatatc 3060

tttgacctca atggaggttt gcaaagactg agtgttcaaa ctactgtaca ttttttttca 3120

agtgctaaaa aattaaacca agcagcttaa ccatg 3155

<210> 9

<211> 1405

<212> DNA

<213> Homo sapiens

<220>

<221> hydroxysteroid (11-beta) dehydrogenase 1 (HSD11B1)

<222> (1)..(1405)

<223> accession No.s: NM005525.2, Hs.275215

<400> 9
   acaattcaga ggctgctgcc tgcttaggag gttgtagaaa gctctgtagg ttctctctgt 60
   gtgtcctaca ggagtcttca ggccagctcc ctgtcggatg gcttttatga aaaaatatct 120
   cctccccatt ctggggctct tcatggccta ctactactat tctgcaaacg aggaattcag 180
   accagagatg ctccaaggaa agaaagtgat tgtcacaggg gccagcaaag ggatcggaag 240
   agagatggct tatcatctgg cgaagatggg agcccatgtg gtggtgacag cgaggtcaaa 300
   agaaactcta cagaaggtgg tatcccactg cctggagctt ggagcagcct cagcacacta 360
   cattgctggc accatggaag acatgacctt cgcagagcaa tttgttgccc aagcaggaaa 420
   gctcatggga ggactagaca tgctcattct caaccacatc accaacactt ctttgaatct 480
   ttttcatgat gatattcacc atgtgcgcaa aagcatggaa gtcaacttcc tcagttacgt 540
   ggtcctgact gtagctgcct tgcccatgct gaagcagagc aatggaagca ttgttgtcgt 600
   ctcctctctg gctgggaaag tggcttatcc aatggttgct gcctattctg caagcaagtt 660
   tgctttggat gggttcttct cctccatcag aaaggaatat tcagtgtcca gggtcaatgt 720
   atcaatcact ctctgtgttc ttggcctcat agacacagaa acagccatga aggcagtttc 780
   tgggatagtc catatgcaag cagctccaaa ggaggaatgt gccctggaga tcatcaaagg 840
   gggagctctg cgccaagaag aagtgtatta tgacagctca ctctggacca ctcttctgat 900
   cagaaatcca tgcaggaaga tcctggaatt tctctactca acgagctata atatggacag 960
   attcataaac aagtaggaac tccctgaggg ctgggcatgc tgagggattt tgggactgtt 1020
   ctgtctcatg tttatctgag ctcttatcta tgaagacatc ttcccagagt gtccccagag 1080
   acatgcaagt catgggtcac acctgacaaa tggaaggagt tcctctaaca tttgcaaaat 1140
   ggaaatgtaa taataatgaa tgtcatgcac cgctgcagcc agcagttgta aaattgttag 1200
   taaacatagg tataattacc agatagttat attaaattta tatcttatat ataataatat 1260
   gtgatgatta atacaatatt aattataata aaggtcacat aaactttata aattcataac 1320
   tggtagctat aacttgagct tattcaggat ggtttcttta aaaccataaa ctgtacaaat 1380
   gaaatttttc aatatttgtt tctta 1405

<210> 10

<211> 11580

<212> DNA

<213> Homo sapiens

<220>

<221> reelin (RELN)

<222> (1)..(11580)

<223> accession No.s: U79716.1, Hs.431010, LocusID: 5649

<400> 10

cacgcgtggg ctcggcgggg gcccgctccc aggcccgctc ccgagcccgt tccgctcccg 60

tccgccttct tctcgccttc tctccgcgtg gctcctccgt cccggcgtct ccaaaactga 120

atgagcgagc ggcgcgtagg gcgscggcgg cggcggcggc ggcggcggcg gcggcatgga 180

gcgcagtggc tgggcccggc agactttcct cctagcgctg ttgctggggg cgacgctgag 240

ggcgcgcgcg gcggctggct attacccccg cttttcgccc ttctttttcc tgtgcaccca 300

ccacggggag ctggaagggg atggggagca gggcgaggtg ctcatttccc tgcatattgc 360

gggcaacccc acctactacg ttccgggaca agaataccat gtgacaattt caacaagcac 420

cttttttgac ggcttgctgg tgacaggact atacacatct acaagtgttc aggcatcaca 480

gagcattgga ggttccagtg ctttcggatt tgggatcatg tctgaccacc agtttggtaa 540

ccagtttatg tgcagtgtgg tagcctctca cgtgagtcac ctgcccacaa ccaacctcag 600

tttcatctgg attgctccac ctgcgggcac aggctgtgtg aatttcatgg ctacagcaac 660

acaccggggc caggttattt tcaaagatgc tttagcccag cagttgtgtg aacaaggagc 720

tccaacagat gtcactgtgc acccacatct agctgaaata catagtgaca gcattatcct 780

gagagatgac tttgactcct accaccaact gcaattaaat ccaaatatat gggttgaatg 840

taacaactgt gagactggag aacagtgtgg cgcgattatg catggcaatg ccgtcacctt 900

ctgtgaacca tatggcccac gagaactgat taccacaggc cttaatacaa caacagcttc 960

tgtcctccaa ttttccattg ggtcaggttc atgtcgcttt agttattcag accccagcat 1020

catcgtgtta tatgccaaga ataactctgc ggactggatt cagctagaga aaattagagc 1080

cccttccaat gtcagcacaa tcatccatat cctctacctt cctgaggacg ccaaagggga 1140

gaatgtccaa tttcagtgga agcaggaaaa tcttcgtgta ggtgaagtgt atgaagcctg 1200

ctgggcctta gataacatct tgatcatcaa ttcagctcac agacaagtcg ttttagaaga 1260

tagtctcgac ccagtggaca caggcaactg gcttttcttc ccaggagcta cagttaagca 1320

tagctgtcag tcagatggga actccattta tttccatgga aatgaaggca gcgagttcaa 1380

ttttgccacc accagggatg tagatctttc cacagaagat attcaagagc aatggtcaga 1440

agaatttgag agccagccta caggatggga tgtcttggga gctgtcattg gtacagaatg 1500

tggaacgata gaatcaggct tatcaatggt cttcctcaaa gatggagaga ggaaattatg 1560

cactccatcc atggacacta ccggttatgg gaacctgagg ttttactttg tgatgggagg 1620

aatttgtgac cctggaaatt ctcatgaaaa tgacataatc ctgtatgcaa aaattgaagg 1680

aagaaaagag catataacac tggataccct ttcctattcc tcatataagg ttccgtcttt 1740

ggtttctgtg gtcatcaatc ctgaacttca gactcctgct accaaatttt gtctcaggca 1800

aaagaaccat caaggacata ataggaatgt ctgggctgta gactttttcc atgtcttgcc 1860

tgttctccct tctacaatgt ctcacatgat acagttttcc atcaatctgg gatgtggaac 1920

gcatcagcct ggtaacagtg tcagcttgga attttctacc aaccatgggc gctcctggtc 1980

cctccttcac actgaatgct tacctgagat ctgtgctgga ccccacctcc cccacagcac 2040

tgtctactcc tctgaaaact acagtgggtg gaaccgaata acaattcccc ttcctaacgc 2100

agcactaacc cggaacacca ggattcgctg gagacaaaca ggaccaatcc ttggaaacat 2160

gtgggcaatt gataatgttt atattggccc gtcatgtctc aaattctgtt ctggcagagg 2220
acagtgcact agacatggtt gcaagtgtga ccctggattt tctggcccag cttgtgagat 2280
ggcatcccag acattcccaa tgtttatttc tgaaagcttt ggcagttcca ggctctcctc 2340
ttaccataac ttttactcta tccgtggtgc tgaagtcagc tttggttgtg gtgtcttggc 2400
cagtggtaag gccctggttt tcaacaaaga agggcggcgt cagctaatta catctttcct 2460
tgacagctca caatccaggt ttctccagtt cacactgaga ctggggagca aatctgttct 2520
gagcacgtgc agagcccctg atcagcctgg tgaaggagtt ttgctgcatt attcttatga 2580
taatgggata acttggaaac tcctggagca ttattcatat ctcagctatc atgagcccag 2640
aataatctcc gtagaactac caggtgatgc aaagcagttt ggaattcagt tcagatggtg 2700
gcaaccgtat cattcttccc agagagaaga tgtatgggct attgatgaga ttatcatgac 2760
atctgtgctt ttcaacagca ttagtcttga ctttaccaat cttgtggagg tcactcagtc 2820
tctgggattc taccttggaa atgttcagcc atactgtggc cacgactgga ccctttgttt 2880
tacaggagat tctaaacttg cctcaagtat gcgctatgtg gaaacacaat caatgcagat 2940
aggagcatcc tatatgattc agttcagttt ggtgatggga tgtggccaga aatacacccc 3000
acacatggac aaccaggtga agctggagta ctcaaccaac cacggcctta cctggcacct 3060
cgtccaagaa gaatgccttc caagtatgcc aagttgtcag gaatttacat cagcaagtat 3120
ttaccatgcc agtgagttta cacagtggag gagagtcata gtgcttcttc cccagaaaac 3180
ttggtccagt gctacccgtt tccgctggag ccagagctat tacacagctc aagacgagtg 3240
ggctttggac agcatttaca ttgggcagca gtgccccaac atgtgcagtg ggcatggctc 3300
atgcgatcat ggcatatgca ggtgtgacca ggggtaccaa ggcactgaat gccacccaga 3360
agctgccctt ccgtccacaa ttatgtcaga ttttgagaac cagaatggct gggagtctga 3420
ctggcaagaa gttattgggg gagaaattgt aaaaccagaa caagggtgtg gtgtcatctc 3480
ttctggatca tctctgtact tcagcaaggc tgggaaaaga cagctggtga gttgggacct 3540
ggatacttct tgggtggact ttgtccagtt ctacatccag ataggcggag agagtgcttc 3600
atgcaacaag cctgacagca gagaggaggg cgtcctcctt cagtacagca acaatggggg 3660
catccagtgg cacctgctag cagagatgta cttttcagac ttcagcaaac ccagatttgt 3720
ctatctggag cttccagctg ctgccaagac cccttgcacc aggttccgct ggtggcagcc 3780
cgtgttctca ggggaggact atgaccagtg ggcagtcgat gacatcatca ttctgtccga 3840
gaagcagaag cagatcatcc cagttatcaa tccaacttta cctcagaact tttatgagaa 3900
gccagctttt gattacccta tgaatcagat gagtgtgtgg ttgatgttgg ctaatgaagg 3960
aatggttaaa aatgaaacct tctgtgctgc cacaccatca gcaatgatat ttggaaaatc 4020
agatggagat cgatttgcag taactcgaga tttgaccctg aaacctggat atgtgctaca 4080

gttcaagcta aacataggtt gtgccaatca attcagcagt actgctccag ttcttcttca 4140
gtactctcat gatgctggta tgtcctggtt tctggtgaaa gaaggctgtt acccggcttc 4200
tgcaggcaaa ggatgcgaag gaaactccag agaactaagt gagcccacca tgtatcacac 4260
aggggacttt gaagaatgga caagaatcac cattgttatt ccaaggtctc ttgcatccag 4320
caagaccaga ttccgatgga tccaggagag cagctcacag aaaaacgtgc ctccatttgg 4380
tttagatgga gtgtacatat ccgagccttg tcccagttac tgcagtggcc atggggactg 4440
catttcagga gtgtgtttct gtgacctggg atatactgct gcacaaggaa cctgtgtgtc 4500
aaatgtcccc aatcacaatg agatgttcga taggtttgag gggaagctca gccctctgtg 4560
gtacaagata acaggtgccc aggttggaac tggctgtgga acacttaacg atggcaaatc 4620
tctctacttc aatggccctg ggaaaaggga agcccggacg gtccctctgg acaccaggaa 4680
tatcagactt gttcaatttt atatacaaat tggaagcaaa acttcaggca ttacctgcat 4740
caaaccaaga actagaaatg aagggcttat tgttcagtat tcaaatgaca atgggatact 4800
ctggcatttg cttcgagagt tggacttcat gtccttcctg gaaccacaga tcatttccat 4860
tgacctgcca caggacgcga agacacctgc aacggcattt cgatggtggc aaccgcaaca 4920
tgggaagcat tcagcccagt gggctttgga tgatgttctt ataggaatga atgacagctc 4980
tcaaactgga tttcaagaca aatttgatgg ctctatagat ttgcaagcca actggtatcg 5040
aatccaagga ggtcaagttg atattgactg tctctctatg gatactgctc tgatattcac 5100
tgaaaacata ggaaaacctc gttatgctga gacctgggat tttcatgtgt cagcatctac 5160
ctttttgcag tttgaaatga gcatgggctg tagcaagccc ttcagcaact cccacagtgt 5220
acagctccag tattctctga acaatggcaa ggactggcat cttgtcaccg aagagtgtgt 5280
tcctccaacc attggctgtc tgcattacac ggaaagttca atttacacct cggaaagatt 5340
ccagaattgg aagcggatca ctgtctacct tccactctcc accatttctc ccaggacccg 5400
gttcagatgg attcaggcca actacactgt gggggctgat tcctgggcga ttgataatgt 5460
tgtactggcc tcagggtgcc cttggatgtg ctcaggacga gggatttgtg atgctggacg 5520
ctgtgtgtgt gaccggggct ttggtggacc ctattgtgtt cctgttgttc ctctgccctc 5580
gattcttaaa gacgatttca atgggaattt acatcctgac ctttggcctg aagtgtatgg 5640
tgcagagagg gggaatctga atggtgaaac catcaaatct ggaacatctc taatttttaa 5700
aggggaagga ctaaggatgc ttatttcaag agatctagat tgtacaaata caatgtatgt 5760
ccagttttca cttagattta tagcaaaaag taccccagag agatctcact ctattctgtt 5820
acaattctcc atcagtggag gaatcacttg gcacctgatg gatgaatttt actttcctca 5880
aacaacgaat atacttttca tcaatgttcc cttgccatac actgcccaaa ccaatgctac 5940
aagattcaga ctctggcaac cttataataa cggtaagaaa gaagaaatct ggattgttga 6000

tgacttcatt atcgatggaa ataatgtaaa caaccctgtg atgctcttgg atacatttga 6060
ttttgggccc agagaagaca attggttttt ctatcctggt ggtaacatcg gtctttattg 6120
tccatattct tcaaaggggg cacctgaaga agattcagct atggtgtttg tttcaaatga 6180
agttggtgag cattccatta ccacccgtga cctaaatgtg aatgagaaca ccatcataca 6240
atttgagatc aacgttggct gttcgactga tagctcatcc gcggatccag tgagactgga 6300
attttcaagg gacttcgggg cgacctggca ccttctgctg cccctctgct accacagcag 6360
cagccacgtc agctctttat gctccaccga gcaccacccc agcagcacct actacgcagg 6420
aaccatgcag ggctggagga gggaggtcgt gcactttggg aagctgcacc tttgtggatc 6480
tgtccgtttc agatggtacc agggatttta ccctgccggc tctcagccag tgacatgggc 6540
cattgataat gtctacatcg gtccccagtg tgaggagatg tgtaatggac aggggagctg 6600
tatcaatgga accaaatgta tatgtgaccc tggctactca ggtccaacct gtaaaataag 6660
caccaaaaat cctgattttc tcaaagatga tttcgaaggt cagctagaat ctgatagatt 6720
cttattaatg agtggtggga aaccatctcg aaagtgtgga atcctttcta gtggaaacaa 6780
cctctttttc aatgaagatg gcttgcgcat gttgatgaca cgagacctgg atttatcaca 6840
tgctagattt gtgcagttct tcatgagact gggatgtggt aaaggcgttc ctgaccccag 6900
gagtcaaccc gtgctcctac agtattctct caacggtggc ctctcgtgga gtcttcttca 6960
ggagttcctt ttcagcaatt ccagcaatgt gggcaggtac attgccctgg agataccctt 7020
gaaagcccgt tctggttcta ctcgccttcg ctggtggcaa ccgtctgaga atgggcactt 7080
ctacagcccc tgggttatcg atcagattct tattggagga aatatttctg gtaatacggt 7140
cttggaagat gatttcacaa cccttgatag taggaaatgg ctgcttcacc caggaggcac 7200
caagatgccc gtgtgtggct ctactggtga tgccctggtc ttcattgaaa aggccagcac 7260
ccgttacgtg gtcagcacag acgttgccgt gaatgaggat tccttcctac agatagactt 7320
cgctgcctcc tgctcagtca cagactcttg ttatgcgatt gaattggaat actcagtaga 7380
tcttggattg tcatggcacc cattggtaag ggactgtctg cctaccaatg tggaatgcag 7440
tcgctatcat ctgcaacgga tcctggtgtc agacactttc aacaagtgga ctagaatcac 7500
tctgcctctc cctccttata ccaggtccca agccactcgt ttccgttggc atcaaccagc 7560
tccttttgac aagcagcaga catgggcaat agataatgtc tatatcgggg atggctgcat 7620
agacatgtgc agtggccatg ggagatgcat ccagggaaac tgcgtctgtg atgaacagtg 7680
gggtggcctg tactgtgatg accccgagac ctctcttcca acccaactca aagacaactt 7740
caatcgagct ccatccagtc agaactggct gactgtgaac ggagggaaat tgagtacagt 7800
gtgtggagcc gtggcgtcgg gaatggctct ccatttcagt gggggttgta gtcgattatt 7860
agtcactgtg gatctaaacc tcactaatgc tgagttcatc caattttact tcatgtatgg 7920

gtgcctgatt acaccaaaca accgtaacca aggtgttctc ttggaatatt ctgtcaatgg 7980
aggcattacc tggaacctgc tcatggagat tttctatgac cagtacagta agcccggatt 8040
tgtgaatatc cttctccctc ctgatgctaa agagattgcc actcgcttcc gctggtggca 8100
gccaagacat gacggcctgg atcagaacga ctgggccatt gacaatgtcc tcatctcagg 8160
ctctgctgac caaaggaccg ttatgctgga caccttcagc agcgccccag taccccagca 8220
cgagcgctcc cctgcagatg ccggccctgt cgggaggatc gcctttgaca tgtttatgga 8280
agacaaaact tcagtgaatg agcactggct attccatgat gattgtacag tagaaagatt 8340
ctgtgactcc cctgatggtg tgatgctctg tggcagtcat gatggacggg aggtgtatgc 8400
agtgacccat gacctgactc ccactgaagg ctggattatg caattcaaga tctcagttgg 8460
atgtaaggtg tctgaaaaaa ttgcccagaa tcaaattcat gtgcagtatt ctactgactt 8520
cggtgtgagt tggaattatc tggtccctca gtgcttgcct gctgacccaa aatgctctgg 8580
aagtgtttct cagccatctg tattctttcc aactaaaggg tggaaaagga tcacctaccc 8640
acttcctgaa agcttagtgg gaaatccggt aaggtttagg ttctatcaga agtactcaga 8700
catgcagtgg gcaatcgata atttctacct gggccctgga tgcttggaca actgcagggg 8760
ccatggagat tgcttaaggg aacagtgcat ctgtgatccg ggatactcag ggccaaactg 8820
ctacttgacc cacactctga agactttcct gaaggaacgc tttgacagtg aagaaatcaa 8880
acctgactta tggatgtcct tagaaggtgg aagtacttgc actgagtgtg gaattcttgc 8940
cgaggacact gcactctatt ttgggggatc cactgtgaga caagcggtta cacaagattt 9000
ggatcttcga ggtgcaaagt tcctgcaata ctgggggcgc atcggtagtg agaacaacat 9060
gacctcttgc catcgtccca tctgccggaa ggaaggcgtg ctgttggact actctaccga 9120
tggaggaatt acctggactt tgctccatga gatggattac cagaaataca tttctgttag 9180
acacgactac atacttcttc ctgaagatgc cctcaccaac acaactcgac ttcgctggtg 9240
gcagcctttt gtgatcagca atggaattgt ggtctctggg gtggagcgtg ctcagtgggc 9300
actggacaac attttgattg gtggagcaga aatcaatccc agccaattgg tggacacttt 9360
tgatgatgaa ggcacttccc atgaagaaaa ctggagtttt taccctaatg ctgtaaggac 9420
agcaggattt tgtggcaatc catcctttca cctctattgg ccaaataaaa agaaggacaa 9480
gactcacaat gctctctcct cccgagaact cattatacag ccaggataca tgatgcagtt 9540
taaaattgtg gtgggttgtg aagccacttc ttgtggtgac cttcattccg taatgctgga 9600
atacactaag gatgcaagat cggattcctg gcagctcgta cagacccagt gccttccttc 9660
ctcttctaac agcattggct gctccccttt ccagttccat gaagccacca tctacaactc 9720
tgtcaacagc tcaagctgga aaagaatcac catccagctg cctgaccatg tctcctctag 9780
tgcaacacag ttccgctgga tccagaaggg agaagaaact gagaagcaaa gctgggcaat 9840

tgaccacgtg tacattggag aggcttgccc caagctctgc agcgggcacg gatactgcac 9900
gaccggtgcc atctgcatct gcgacgagag cttccaaggt gatgactgct ctgttttcag 9960
tcacgacctt cccagttata ttaaagataa ttttgagtcc gcaagagtca ccgaggcaaa 10020
ctgggagacc attcaaggtg gagtcatagg aagtggctgt gggcagctgg ccccctacgc 10080
ccatggagac tcactgtact ttaatggctg tcagatcagg caagcagcta ccaagcctct 10140
ggatctcact cgagcaagca aaatcatgtt tgttttgcaa attgggagca tgtcgcagac 10200
ggacagctgc aacagtgacc tgagtggccc ccacgctgtg gacaaggcgg tgctgctgca 10260
atacagcgtc aacaacggga tcacctggca tgtcatcgcc cagcaccagc caaaggactt 10320
cacacaagct cagagagtgt cttacaatgt ccccctggag gcacggatga aaggagtctt 10380
actgcgctgg tggcaaccac gccacaatgg aacaggtcat gatcaatggg ctttggacca 10440
tgtggaggtc gtcctagtaa gcactcgcaa acaaaattac atgatgaatt tttcacgaca 10500
acatgggctc agacatttct acaacagaag acgaaggtca cttaggcgat acccatgaag 10560
aatcaaaaag tttatttttt ttcttccaac atgtgatgtg ttgctctcca ttcttttaaa 10620
tctcgcacta catctgatat caggaaatat ctgtgaagga cttggtgatt acctgaaagc 10680
ccttctcaag accgagtgta caccactttc ccacactgtg aactaatgac aagtgactta 10740
tttgctcata agtaaatgtc ttcatgttga tgtgtccgtg aaagttgtga tctgttgtaa 10800
tatcagttac agtggcagta ttgacaataa gaaacagttt aacagaaaaa tgaaatttaa 10860
gcacaaaaaa tttaagagat tttatgttta aaatggcatt tagcacagta tttaacattc 10920
ttggtcacaa agctatttaa gtggactgta tttcagctat gtctcatgtt ttatatgatt 10980
aaattatcat tgtttgtcct ttatgtattc tcttctacaa tacaacacat tgaaactgta 11040
tttacttgtt atgttgtaat attttgctgc tgaatttggg gctacttata ttctgcagaa 11100
aattaattga aatacctatt caagaagata gttgtaaaga tattgtatct cctttaatat 11160
actccttaaa aatgtatgtt ggtttagcgt tgttttgtgg ataagaaaaa tgcttgaccc 11220
tgaaatattt tctactttaa attgtggatg aagaccctat ctcccacaaa taagttccca 11280
tttccttgtc taaagatctt tttttaagtg ttctgtggct gatttactaa cagtaactgc 11340
cattttttgt ctgtgataac agagtgattt gtaaaacagt ggttgttttt tcattgtgtt 11400
ttcttcgtgg attgtttttt ctgcgggtca tattcatacc ttctgatgaa gttgtacaac 11460
accagcaaca ttataatggc cctgtagctc tgaatgctat ttgtgtaact gaaaggttgc 11520
actctagggt gaaccaagct ataaaagccc atgcttaaat aaaaattatg tccaaaagcc 11580

<210> 11

<211> 2472

<212> DNA

<213> Homo sapiens

<220>

<221> atp-binding cassette, sub-family c, member 10 (ABCC10)

<222> (1)..(2472)

<223> accession No.s: AL133613.1, Hs.55879

<400> 11

acgcggtgct gctgatggag gccgggcgcc tcatccgggc tggacctccc tctgagattc 60

tgccactggt acaagctgtc cccaaagcct gggctgagaa tggacaagag tctgactcag 120

ccacagccca gtcagtacag aacccagaga aaacaaagga ggggctggag gaggagcaga 180

gcacatctgg tcgcctgctg caggaagaaa gcaagaagga gggcgccgtg gccttgcacg 240

tgtaccaagc ttactggaag gccgtgggcc agggcttggc cttagccatc ctcttctctc 300

tgcttctcat gcaagccacg cggaacgctg ctgactggtg gctctcccac tggatctctc 360

agctgaaggc tgagaatagc tcccaggagg cgcaaccctc caccagccca gcttctatgg 420

ggctcttctc tccgcagctg ctcctctttt cccctggaaa cctctacatc ccagtgttcc 480

cactgcccaa agctgccccc aatggctcct cagacatccg tttctacctc accgtgtatg 540

cgaccattgc tggtgtaaat tccctctgca cccttctccg ggcagtgctc tttgcagcag 600

gcacccttca agcagctgcc actctgcatc gccgcctgct gcatcgagtc cttatggcac 660

cagtgacttt cttcaatgcc acacccacgg gccggatcct aaaccgcttc tcctctgatg 720

tggcctgtgc ggatgacagc ctgcccttca tcctcaacat cctcctggcc aacgcggcag 780

gcctgctggg gctcctggcc gtgctgggct ctggcctgcc ctggctgctg ctcctgctgc 840

cgcctttgag catcatgtac tatcacgtgc agcgccacta cagggcctcc tcacgggagc 900

tgcggcgcct gggcagcctc accctgtctc cactgtatag ccatctggcc gataccttgg 960

ctggcctctc tgtgctccgg gccacagggg ccacctacag gtttgaggag gagaacctgc 1020

gactccttga gctaaaccag aggtgccagt ttgccaccag tgccacaatg cagtggctgg 1080

acattcggct acagctcatg ggggcggcag tggtcagcgc tatcgcaggc atcgctctgg 1140

tgcagcacca gcagggcctc gctaacccag ggctggtggg cttgtcgctg tcttatgccc 1200

tgtccctgac gggcctgctc tcgggcctgg tgagcagctt cacacagaca gaggccatgc 1260

tggtgagcgt cgagcggctg gaagagtaca cctgtgacct gccccaggaa ccccagggcc 1320

agccactgca gctgggcacc ggctggctga cccagggggg cgtggagttc caggacgtgg 1380

tgttggcgta ccggccaggg ctgccgaatg ccctggatgg agtgaccttc tgcgtgcagc 1440

ctggagagaa gttgggcatc gtgggccgca caggctccgg caagtcttcc ctgttgttgg 1500

tgctcttccg gctgctagag cccagttcag ggcgagtgct gctggacggc gtggacacca 1560

gccagctgga gctggcccag ctcagatccc agttggctat catcccccag gagccctttt 1620

tgttcagtgg gactgttcgg gaaaacctgg acccccaggg cctacataag gacagggcct 1680

tgtggcaggc cctgaagcag tgccacctga gtgaggtgat tacatccatg ggtggtctgg 1740

atggtgagct gggtgagggg ggccggagct tatctcttgg gcagaggcag ctgttgtgtt 1800

tggccagggc tctcctcaca gatgccaaga tcctgtgtat cgatgaggcc acagcaagtg 1860

tggaccagaa gacagaccag ctgctccagc agaccatctg caaacgcttt gccaacaaga 1920

cagtgctgac cattgcccat aggctcaaca cgatcctgaa ctcagaccgg gtgctggtgc 1980

tacaagcggg gagagtggta gagctggact ccccggccac cctgcgcaac cagccccact 2040

ccctgttcca gcagctgctg cagagcagcc agcagggagt ccctgcctca ctcggaggtc 2100

cctgagccca atcccacacc ctgcagagtt ctcccctctc tctgatccag gccgggccta 2160

tacagaggtg ctggctgctt gtttacattc tcctctgggg ctctacctct ccacacttcc 2220

ccagaaggga aaagggcacc ctggattact ctttggaaat cactccttgg tgggcagcat 2280

cctgaggctt ccccagaacc aggcctctgc tctggccctc ttgcatctgg aacgccaggt 2340

gggtttttct ggcataggag cccacttgca ttttcatagt tttatttgat aaaattccat 2400

cttacattct gtgtattaaa aaaataatat ttctggaaaa aaaaaaaaaa aaaaaaaaaa 2460

aaaaaaaaaa aa 2472

<210> 12

<211> 17133

<212> DNA

<213> Homo sapiens

<220>

<221> cyclophilin 40

<222> (1)..(17133)

<223> accession No.s: D63861

<400> 12

attatgaaaa aatttaaatg tgcaaaataa agaagagtag tataataaac tcccatgcac 60

caatcatctg cctggccctt atgtttcatc cacaacccaa cccacttacc tgactccagg 120

ttattttaaa acaacttcag gcactatagc atctaatttt cagtaattta aaaaagagtg 180
tataagaatg ctttagagat tacaaggcaa atgcttacac attatctttt tccattctca 240
taccctgttc tttggcactt taccgaaaag gaaatgagaa gggttcagag aaattaaatg 300
acttttcttt ggtaacatgc ctagtataca tgtagtcaga acttgaagct tttgcttttt 360
ttttttcttt tttaacatca actttcgtgc ttagtccagt gtataacatc acaggcaaat 420
tcaaattgag aataggaatc agttgttctt taaccgtaac ccttctaact tttacttatc 480
ctaagaccgg gtagcaagta gggcaggagc cttcatctag agggatggtg acagctctga 540
taggggaaaa ttagcactgg tctttccttc caaaaagcca ctctggctgc tgctgttgac 600
acaaggctcc tggcttttga gacattattt gagtgctgga ttccaatact tttttacttc 660
tcataagaaa aaaaaatgtg aagcatctaa ggatatctcc tgccttattg ataaagtaag 720
cctcaggctg aatccaaatt gctgagctcc aatagaacta cagggcaaag catctacaca 780
gggaattgcc actcttctcc aaaattaggc tttattaaga ggcaaggcta tgttctcctc 840
atatatatgt gtgtgtgtgt gtgtgtgtgt gtgtgtgtgt gtgtgtgtgt aaaaagtaaa 900
tgttttacac agatccccat aagggaaaag tgggctatag taatacagta tttttttgtt 960
tttctttttt ttgggggggg gggacagttt cgctctgtca cccagtctgg agtacagtgg 1020
cgcgatctcg gttcactgca accgccgcct cccgggttca agcgattctc ctgcctcagc 1080
ctcccgagta gctgggacta caggcaagca ccaccacgcc cgggtgattt tttttttttt 1140
tatttttagt agagacgggt ttcatcatgt tggtcaggct ggtctcgaac tcctgacctc 1200
aaatgatccg cccgcctcgg cctcccaatg tgctgggact acaggcgtga gccaccgcgc 1260
cccgccaaat acagttttat caggaatcaa aactgtgctc tcttctggac tctcagcttt 1320
agctgtttgg agcgtttagt ctaagcctta ccacttacat tgtaatgtga agatcaaatt 1380
gtaacgacat ggcaataata aggctcctat tggttgggta ccaccagttt atgccaggca 1440
ttgtactgat tcatgtcttt aatcctcaaa acgaccttcc gaggtcagcg tttttattct 1500
ttttccagag gagcaaactg agtttctgag aagtcacacc caacttggtg gcagagctga 1560
cgctggcatg gggttgactc caaagctctc tacgccacga gggtgggaaa gacaacgaaa 1620
cacaagcgag aagaataatg agaactggga tcgcaacttc attaaatgag aagccaggaa 1680
caggtggcgg agggcacgcg gttcgctgca cggagtcgct caggctcttt gaaacaccgc 1740
gcgggggagc ggggcggtgc cggaagtcag gccatggctt ccggttcttg cgtcggaagt 1800
ggccggtcag cgtcgctgcc ggtctccggc ggagacggac tctggagttt gggcggcccg 1860
ggcggccact aggtactctg atattccgta ctaaacacgt ctgcaagtca agatgtcgca 1920
cccgtccccc caagccaagc cctccaaccc cagtaaccct cgagtcttct ttgacgtgga 1980
catcggaggg gagcgaggtg agcggagtct gacgctcgcg gaaaagcccc gaggaggcgg 2040

gggttcccgg gatttggggg attccaaggg cggaatgcgg ctccggccgg tgactgctca 2100
gtttcgcgga ggggatggct gcctaggctg gtccgccgga gcaggcccgg agatctaggg 2160
ctagatcgat ccagcgccct tgtatcaatg ccatcggtgg tgcggaagtt tctggaaatt 2220
tctagttcgg gaattgtggc gtcctttagg tttgcctcct gatttggtgg cacttccacg 2280
ccacctttgc actaccaagt tagcatgaaa tattttatgt ttgcaatggg acagttcgga 2340
aagcatatcc tgggatatgc gtctgctggg ccccaggaaa ctatatttgc cacatgagat 2400
aatatatcag cacgtgctgt gttatttata cgttaaaggg acttgtttgc tcattgcttt 2460
tgccttatta ctgattctta atttagaggt ccgttcagat gagcaaacat tttctcttaa 2520
tttgccattg ggaaaatgcg ctcaacgtag tgatggtgtt gcctaggtta catagccgct 2580
cagaagttcc taaggcctgg tgaattaggg agcgtgttgc tttgtaactg gattggcctt 2640
gttctggaac acgttggaat tgctggccag gcaatgtgtt tgaatgcatg tacaggacac 2700
acacacgttt acacgtacat tcagtttgtt gcaactagct agggctaggt ttaatctcac 2760
ctgtcttagc tgctatctag aggaggaatt tttacagctt ttgcttcaga ggttgcatga 2820
ggcagtttgc tcagagcaca aaggctgcag gtatacgtaa cacgtggcaa gtccgcccac 2880
ccaaagactg tttaattttc tcccgttggt tgcttggtga ttggaaattc tcagtacctc 2940
ttgaagaaat tactcaggat ttaaaactat ttgtatttaa tgatatctct tacatggtgc 3000
taatcttttg gctactataa ttggttatga tccttagtgt aatgtttcaa atctgtttat 3060
atacatacat gacagtaggt agactaaatt tgaatattag aataaaataa gattcactaa 3120
ttctagtgag tatattcaaa ggaaatgaag tttgtatgtc aaagaggtat ctgcactccc 3180
atgttcattg cagcattatt catgatagcc aagataatgg aatcaaacta ggtttccatc 3240
tacagatgga ttttttttaa aatttgatat atgtgcacag tggagtacta ttcacccata 3300
aaaaataatg aaatcttgtc acttctgaca acatgaatta atatggagga cattatgtta 3360
catgaaataa gctgggcaca ggaagacaag gcatgatctc acttacatgt ggtttctaaa 3420
aagttgacct tgaagtagag aatggaatgg tggatttcag gggttgggta gatgttgatc 3480
acgagagacc taatttcagt tagaaagggg gaataaattt aagagatcta ttgtataaca 3540
taatgactat agttagtagc aatgtgttgt gttcttgaaa ttgcaaaaaa gaaaaattac 3600
agtaaagggg aacttggtaa ttcatgattg atctgctacc ataattatac caaccttctt 3660
tttatcattt gtctatttgg ttgttttgca tactgacaag attttctttt cttttagttg 3720
gtcgaattgt cttagaattg tttgcagata tcgtacccaa aactgcggaa aattttcgtg 3780
cactgtgtac aggagaaaaa ggcattggac acacgactgg gaaacctctc catttcaaag 3840
gatgcccttt tcatcgaagt atgtgtaaat tttcttaatc ttgttattct tcatacagac 3900
aagtcctgtt ctttaggatt tggggaagaa aacactaagt aggaagtgaa gcttaaaaag 3960

tactattagc gtccatgtct gaggcagtaa cgcagtttcg taaaggttag gctgtgcgag 4020

gtggaattac ttatggtact aaggtttatc tatagagaaa tggactcccc tagctttacc 4080

catcctctca ttctccttca ctctaagatg atttgattga gaaaggaagg ccgtagtcaa 4140

ccaaccttag gtagcttcat tgcctggtga caaaaaagtt atgcatagac cgggcgcagt 4200

ggctcacgcc tgtaatccca gcactttggg aggctgaggc gggtgaatca cgaggtcagg 4260

agatcgagac catcttggct aacgcggtga aaccccgtct ctactaaaaa tacaaaaaat 4320

aagccgggtg tggtggtggg cgcctgtagt cccagctact cgggaggctg aggcagtaga 4380

atggcatgaa ctcagaaggt ggagcttgca gtgagccgag atcacgccac tgcactccat 4440

cctgggcgac agagtgagac tctgcctcaa acaaacaaac aaacaaacaa acaaaaaaat 4500

gcataatttt tgggcctgag tgcttgggaa ggcagcagat ttgaagattc agtgttttgg 4560

gcacatgggt gatagtgtgg tcaaaatatg ccatttcata ctgcttatta cactttgtaa 4620

tgaatcatac ttttgtttag tgataatggt tctcccaata gactgcaggc tctatgagtt 4680

caagaactgt atgttttcac cattttgttt ttggcatcta atacagttat tgagtaagaa 4740

ctcagtaagt atttgtttaa taaataggca aataagcaat aatcacaaga gcaacatttt 4800

ttaaacgatt tattaagaaa aaattaactt tttaaaaata tttaaaatgt ttactttcat 4860

gtttaataaa aacttgaaat atgctaataa actatatgca gaaaatattt aacatagcaa 4920

gtgtgagact gctatcttta gaaaggtctg cctgcaagag tagcacttgt ttggagtctg 4980

ggaacttgac acttgtacca tttcctaaat agtaatggtg atttactatg cctagcctat 5040

ttgtacaaac atgaatgtgt gctctctttt ggggagtctg gaattttggt acatgctagg 5100

tagagtgtgc ccaataaata ccttgggtgc tgagtctcta attggctctc ctgggcagaa 5160

atactgtgta catgttgctg catttttgtt gctgggggaa gtgtgtgttc tgtagcactt 5220

catgggacgg agaaagcata aggaagcctg cacatggatt tttccagact ctgcctgtgt 5280

ctttcccgtc atctcgctgt gtatccttac cactgtgatc caaccacata ctgatttgtg 5340

tgagctctag ggttctctga acatgaaagc aatcttggta acctttgaca caaagtgata 5400

tatttgctga aaattttcca ttagggtaga acaaatctac tgggaatcat tggtctgtat 5460

tcagcagaca ttattgagta actaatatgt actatgtgta catgcacata gttataagga 5520

tgtaaaacaa tacagttggt gtacttaagg agatcattgc tggaaggggc agatcataat 5580

gccatatgtt atgtaggcag agtcaagata ctatgggaga aaaattagga catacagctt 5640

agggatttca gtgacagttt tataggggag gtatcatttg aaccgatttt ctgaatgtta 5700

gcagggatca ggcagtgaaa ataggttgga ttcagatggt gaaaagcctg aagcgtttgg 5760

agttgcggag acgggtgggg agggacaaac aggagagaga catgactaga gtcatgcact 5820

acaaagttgt cttacgtacg tatccattag taagcattgc taaaggcatc catgtctcag 5880

tcactaatag ccattttcct tttttgtagt tattaagaaa tttatgattc agggtggaga 5940
cttctcaaat cagaatggga caggtggaga aagtatttat ggtgaaaaat ttgaagatga 6000
aaatttccat tacaaggtaa agtagagaaa atgcatgtta ttgctgataa aaagattgtt 6060
atatatctgg aatacttgtt ggattaagac accaaaaaat gtttcaagtg tgcttagatt 6120
ctccatttct tgtaatagga atcacttttg taaataatgg ttctttaata tggaaaagtt 6180
ttcattcagt aatgattaat tcagcattat ttcattgtga tccagttttt atatgcttca 6240
gttaagccag tgagttttta aatgcgacca gcatctggca aaattgtttc caggaaaaat 6300
gtttccattg ttggaaggat ggtaacttgt gcatccttat ccttgaatgt gaagagcaac 6360
aaaaccatca ctaatatcca ggatgtcagg ggatttgcac actagcagat atagttaaat 6420
gtccaagctc ctaaaggact tcagtggtgc ctgtgccttc ttgtcttcgt tgtcccaaga 6480
tgtaattggg gtatattgca tcattcaagt tataagaatg gacatgtaag tacaaaagta 6540
aagtgatatt tttggaaata aggaatatga gcaagggtat taggataatt tgaaaaaatg 6600
tcgtcagtct tccagtcagt gtttattgag ccactatcac tggcaccaaa gggtttataa 6660
aaatgaaagg ccttgatgac aaggaattta taatcctacc tcagagacaa gattaagata 6720
catgggacta ctggagaata atagtatata aataaatgct aaattgattg aatatccttg 6780
cttgtataac attgctaata ctgtctatat actgcaggag aaagatgtga tttatgcagt 6840
ctgaattatt atggaagtca ttgaggagga atggctactt gggctgtgca gactgggtct 6900
tgcaaggcgg caacatgtct gagatttggt taggcaggaa ctgtagtatt gatgcagaat 6960
ttaaaaaggg acgtcggtga ggaaagacaa aggcaagaga cgttgaggaa ataaagttct 7020
agttactgtt gtaccaaagg aagctaccag aaggatgaaa actaaaggtg atgtgatgat 7080
cacattgaca tagagaaaag gggcctatag acaagacaca tgattcttgc tttctataaa 7140
cgtggtggcc ttaaaatctg aatttgattt agactgaggg gagtaaaagg cattttggtg 7200
gggtgggggg tgtcatgaaa cattatataa catgagagtt acggtgcatg caccaacaag 7260
gaatacattt agaaagccta ggagataaag tgaaaaggtt aagaaattgg gtttcataga 7320
tcctttgaca ggattatgcc aaggtagaat tttgatttca agatgtatgc agcagtttct 7380
tttgagcact ctccgaagag gtgattcatc tgggatgaaa acctaatcca aatcctgtat 7440
gatttggatt cttttgagat taggaaagta ccaaactgaa tgtcttgggt aaattcagta 7500
tcccattatt gttccagttt attgatgttt tgtaaaggaa gacttatact gtaggcatat 7560
ttatgaattt ggaagaccgt tcaggtcttc ccagaaaaaa agagcattca tcataaagag 7620
ggcaaaatat attacaagag tttcataatg ctgcatcttc attttaaagg tgaagtgtaa 7680
gtattttctc atggccaatt tagaaataac cctccccccg actattgttt ctgttaccta 7740
tttactttga taaggttttt taaagtttaa agtgattaag tgccccatgt tttgtgaatt 7800

aaaggttgca gtttataaaa aaataaaaag taattaaaaa attttataag tgaaaagtaa 7860

aaaaaagtag tttgatctaa atggtagtat agtgcacccc agttttatgt tcagtaaaag 7920

aacagttcag actaacacac aattcagaaa agaacacatc taaccttggt tttcttttaa 7980

tttttatttc tacagcatga tcgggagggt ttactgagca tggcaaatgc aggccgcaac 8040

acaaacggtt ctcagttttt tatcacaaca gttccaactc ctcatttgga tgggaaacat 8100

gtggtgtttg gccaagtaat taaaggaata ggagtggcaa ggatattgga aaatgtggaa 8160

gtgaaaggtg aaaaacctgc taaagtaagt aaaaagttac agtgaaatac acttattatc 8220

ttagttgcta tctttttgag acggagtctc gatctgttcc aggctggagt gcagtggtgc 8280

ggtctcggct cactgcagcc tctgtctccc aggttcaagc tattctcctg cctcagcctt 8340

ctgagtagca gggattacag gcgcgcgcta caacacccgg ctaatttttt ctgtattttt 8400

aatagagacg gggtttcacc atgttggcca ggctggtctc aaactcctga ccttgtgatc 8460

cgcccacctc ggcctcccaa agtgctggga ttacaggcat gagccaccgc acccagcctg 8520

ttagttgcta tcttaaagaa atataagtaa aaggatcagg tgattattag ctggaaaaat 8580

gttataataa ttttgggtat aggagccagg atctgccact tttttttttt ttttttaact 8640

tttctacaat gggaaagaca attagggtag ttttaaagga gatttttatt atttcaactt 8700

tcatttggtg atctttgcta gtttacgtta agtcttccta tacctgcaaa actgttcagt 8760

tggtatgtat agtagtacat agtggaatga ggataataag cccaaaatct tgtctggatt 8820

gtagcattaa ctagaggctt ggccttgact ttgttactta acttttccac acgtgttttc 8880

tcctgtctaa attacttatg ggtttagact agattatctc tgaagaccct tgtgtactta 8940

atgtctgtga atcaaaatga ccttaatatt ggctgggcat ggtagctcat gcttgtaatc 9000

ccagcacttt gggaggctga ggtgggagga tcacttgaga ccaggagttg gaaaccagct 9060

ttggcaacat agtgagatgc tgtctctatt aaaaaaaaaa gaaaaaagaa aaacccctaa 9120

acgaccataa tactacccag cctaagtgac agggttgtga gtgggagttg gaagctcagt 9180

tacttgtagg aggtaggctg tcactcagtg tatgacaaga atagtagtaa accggaagct 9240

catgccccac ctaatgccac ttacatagac aatgataaaa ttgccaactt attctgtgtt 9300

tattgagctc ctgaatagca ataaaatgct tatgtaaata ctaacattgt cgtattagtt 9360

ggaatgtata aaaacagcat tatagtacat aaatgagcct tctcacataa atctgtgaat 9420

tggacagtgc tgcacatgac ttcagctgta tagcttttat ctctcattat tacgatagtg 9480

cacttctaca atttagggtt atttttacag ttgtgcgtta ttgcagaatg tggagaattg 9540

aaggaaggag atgacggggg aatattccca aaagatggct ctggcgacag tcatccagat 9600

ttccctgagg atgcggatat agatttaaaa gatgtgagta ctttcagatt agtcaaactt 9660

taattaattt agtaagtgaa aatatttagt actaaaagtt tgagcttttg tttcagtctc 9720

tcatatgata gaatctagca ttaaaccaca ttacttacta aaatccaaca tatttgtatt 9780
tttttataca ggtagataaa attttattaa taacagaaga cttaaaaaac attggaaata 9840
cttttttcaa atcccagaac tgggagatgg ctattaaaaa atatgcagaa gttttaaggt 9900
aatattattt ctaacaaatt atttttaaga aattataaat tgaattataa actaaaatta 9960
agaattgctc attatatttt tgcaaattac ttgtggaata accaggtgtg ttgaaatagt 10020
tgcatttcct tttttttccc tcagcttgtt ttgagaaatt tcaaaagaca gaagttgaac 10080
gagtagtaca taattactca taagttttac ttacattcac caatttttaa cttttgctgt 10140
ttacgttttt acagaactat tttgatagga agttgttgat actgtgatac tttaccctaa 10200
gtatttcagc acgtatttcc taagaaacaa agacattctg cataagttca ataccgttaa 10260
aacaccccaa agtaatattg atagataata tcaatccata tttaggctgg gcatagtggc 10320
tcatgcctat aatcccagca ctttgggagg ccaagctggg cggatcacct gaggtcggga 10380
gttcgagacc agcctggcca atatggtgaa accccatctc tactaaaaat acaaaaatta 10440
gctgggctgg tggcgcacgc ctgtaatccc agctacttgg gaggctgagg cacaagaatt 10500
acttgaaccc aggaggtgga ggttgcagca agccgagatt atgccactgc actccagcct 10560
gggcgacaga gtgagattct gtctcaaaaa aaaaaaaaaa agtaatccat attccaactt 10620
ccccagttgt ttcaatttct tagaaaaatc aagggtccaa actaagatta tgtgttatat 10680
ttagtggtag tatctcttac gtttcctacc tcccctggtt ttttattcat ttgcattttt 10740
gtggagtcca agtcagttgt ttttatagac ttccatctgt atgtgtttgt ttccttaaga 10800
ttagtttgag gtgaaatatt ttggcaagaa gacttaggtg ttgttttgta ttacctatga 10860
tatcacatcc tgaagacaca tttcgctttg gcctgtttga gttcacacac atggttaaag 10920
tagtgtcagc cacaagcatt tgttagtctg aggtggtatc gtgaaattgt atgaatttct 10980
agtccccaac aacttttcac acagtggatc ttagcatcat tgaaaatcca tgcctgaatc 11040
tgttatactg aacttctaag tttatcatcg tacagttatt aactagcatt cttctgtttg 11100
cgagagcctc ccgcatattt tttaagtatc actatagacc tgtggatatt tttcttttct 11160
ttttttttcc ttttatagaa acaaggcctc ctttatattg cccaggctgg tctggaactt 11220
ttgggcccaa gcaatcctcc ctcctcacct tcccaagtag cagggactag aggatatctt 11280
atgggtagtt ttcaattccg tttgttagta cgctattatt gtcattcttt ttcatgccca 11340
aagggttcca aatttgttca gtggaaggcc cctttccttt ggcatgttcc tattagtctg 11400
cgagcacttg cttgctctaa gctcatcttg ccttactttc agatctgata tggcagtttc 11460
tccaaggacc tctgatttct tttagagaaa gaaaacttta gaaaccaaat tctgggggcc 11520
aggtgtgctc agttttgggg cccttttcag ggacaatgca aaatcaacac aatagagttc 11580
tccttcatct ttcctctact gaattccctt ctcctattat gagaatcctg gttccaaaca 11640

taattatgtt ggaaagtaca tagtggggac ctgagtttta aaaatagaaa agtgttacta 11700

gtgcataatt agatttggga attgctctta aattggcctc tttgaacact ttggggaaca 11760

aaaacaattt tttttaagcc ttcttaaaaa ttttaaaaag tgtgatcatt aagattatta 11820

atagcatttt tagagctttt tatatcagaa atgacataac acttctggac atagagaaga 11880

gtaattcctg tttatgtggt ctttcatact gcttttatta atgcattctg cagatacgtg 11940

gacagttcaa aggctgttat tgagacagca gatagagcca agctgcaacc tatagcttta 12000

agctgtgtac tgaatattgg tgcttgtaaa ctgaagatgt caaattggca gggagcaatt 12060

gacagttgtt tagaggtaag tctgtttgat tttgaacttt ttgaataagt tggattaaga 12120

cttagtttga atagtagcaa cttttatata cattaatata tcttattgca taggacctaa 12180

tgaggcttaa gaaaacattt tgtttctaaa tattttggac cctcagttct ttctctacat 12240

tcattttgct tataactctg tgtcacttaa cagctatgaa gatccttgcc tttttttctc 12300

aaagcacata tatcagctca tgtattcact taagagcacc tattgtgtgt taggcattag 12360

aagacaaagt cagaaatgat taagatgtgg gttatggagt cagtcaggca tggctttttt 12420

cttcttcttt tttttttgag acggagtctt actctgttgc ccaggctgga gtgcagtggc 12480

gcaatctcgg cttactgcaa cctctgcctc ccgggttcaa gtgattctcc tgcctcatcc 12540

tcccaagtag ctgggattac aggcatgtgc caccatgccc agataatttt tgtattttta 12600

gtagagacag ggtttcacca tgttggccag gctggtctca aactcctgat ctcaggtgat 12660

ccacccgcct tggcctccca aagtgctagg attacaggtg tgagccaccg tgaagtagtt 12720

atagagagtt atataacaca atgtaaggca tttagtgtag tgcctggcac attttaagca 12780

cctaacagag acgagttatt ttaattaaaa gatccatacc ctcaagaagc ttaataagtg 12840

attcaggata ggagggggtc agtttcaatt taggttcatc aggacttgtg tatggagaat 12900

gggttctatt ggaacaggtc atttatcttt aggaggtcat ccaggtatgc taactgataa 12960

agctagaaag gtggcagttt taccttcctc aaatagagca attatgactc tcttccctgt 13020

tgtatagttt gaaaagtttt ccaaatgaaa ctttaagaat tttacatatt aagccaggca 13080

cctagactag ctatgctcag agcaagtaga actaaaatga gatcctaatt ttgtagggta 13140

gatggaataa gggataggaa tatatttaga agggatcata caacagagga aaaaacttat 13200

caaaagaaaa gatgggtctt cactctgggt ttttttttgt tctctgtcac ccatgctgga 13260

gtgcagtgat gcaatcaaag ctcactgcag cctcaacctc ctgggctcgg gtgatcctcc 13320

tacctcagcc tcctaagtag ctgggactac agacccttgc caccataccc agctgatttt 13380

taaatttttt gtagagatgg gtttttgacg tgttgcccag gctggtctct taactcctgg 13440

gctcaagctg tccacccgcc ttggcctcac aaagtgctgg tattatagtt aagagcctct 13500

ctgcccagcc tcgatctctt tttgacctgt gaggcaggta aaatctgagt attggccatt 13560

aaaagatgtt acataaacaa aagataattt taaaatgttt gttctagggg agagacctga 13620
tgcataaagt ctcttaggtt aaaagacctg ttgtaggtaa gaacctaggt tgggtatggt 13680
ggttcatgcc tgtaattcca gcacttaggg aggccacggc ggcgggcaga tcgcttaatt 13740
ccaggagttt gaaaccagcc tgggcaacat ggtgaaaccc tgtctctaaa aaaaaaaaaa 13800
aattagtctg gtgtggtggc acatacctgt agtcccagct acttggaggc tgaggtggga 13860
ggagcacttg agcctggggg gttgaggcag cagtgagctg tgatcaggcc actgcactcc 13920
agcctgggcc acagagtgag accttgtctt aaaaaaacaa aaaacaaaaa cttaagagtt 13980
tataacttaa gagttattaa gttattaatg ttcctaaaaa acttataatt caagtaaaaa 14040
gtgtatacaa atgtttcttc aaaatgattt tatcttaaaa gtgttagttc aggcccattc 14100
gggagattga aaccacagtg gggagcatat tggaaactag agaaatgtct ttctcctcca 14160
tgtccattca gtgccctctg ctgacaaaac ttagtgccca ctggcagagg agagctattt 14220
gcatgtttca gctccatttt cacagagcag tgaaggatgg gtttggagtt gataggcaat 14280
gaattgaaat ctggcatata agcttaatac tttgatataa atactagctt tatacttttt 14340
tgtaggctct tgaactagac ccatcaaata ccaaagcatt gtaccgcaga gctcaaggat 14400
ggcaaggatt aaaagaatat gatcaagcat tggtaaattt tgttccaaat gtttaatttt 14460
ttaaaataga caactacctt tataaatcat acacctaact taaatgtttt tttccaatta 14520
aaggctgatc ttaagaaagc tcaggggata gcaccagaag ataaaggtaa gttggcagct 14580
tttgtagtga aagttaattt tgttatttaa atacttatcc tcaggaacca ttgttcactt 14640
tgccagattt tagatgtttg ttcaacagac actacagaat gcctgctgtt gggccaggca 14700
ttatcatata gaatgaacaa gacagtcaaa gtccctgccc tcaaagagct tacattctac 14760
tcccattcaa gaatatagta gtttttcacg ttatttattc ttcaagttat catctctgta 14820
gttttttata tgtctaaata ctgtttgtct tatggtatcc tttgagactg tgttcctatt 14880
ctggttagtc tagtcgagag ctcagcattg taagacacag tattcatggt aaatttaaca 14940
tttgggacca ttcaagttga atgtggctat aaccttagta ttgatgtact ggttatatgt 15000
tttaggaaac tatgacaaaa ataagcttgt ttttcaaata aaccaactat gaatagtgac 15060
actagatctg ggtattacaa aatgtctttg gtctcttatt actaaaatga aacaaggaaa 15120
gcaattactg ttctctaatg tagaggcaga aggcaaatct tgatgtactt gagttctctc 15180
agttgacatc ctctaaaatt acgtttcatt tctgtgtttg tgtgattgct cagattttct 15240
gttactaggg cttagttact ttgtagagaa gttttttcaa ggggtagtta acattaaaat 15300
agtttgttag tcaccataat ttttggatat tttgagaaat aactcacatt gcatatttat 15360
tttacagcta tccaggcaga attgctgaaa gtcaaacaaa agataaaggc acagaaagat 15420
aaagagaagg cagtatatgc aaaaatgttt gcttagaaag gattcagttt tgcttattgt 15480

gtgttgattg tataaatgca ataagaaaat gtaaaggttt ttgtctatga atatgatccc 15540

taatgtgttt cttttgacac cttagttcct tactgtttac agtttaggag tactgatagg 15600

ggttcatgct taataaacat gtcacaatac agtaagtaaa gtggttttgt ttgtttcttt 15660

gagatggagt cttgctctgt cacccaggct ggagtgcggt ggcgcaatct cggctcactg 15720

catcctctgc ctcccgggtt caagcaattc tcctgcctca gcttcccaag tagctgggat 15780

tacaggcacg tgccaccacg cccagctaat ttttgtattt ttagtagaga tggggtttca 15840

ccatattggt cacgtcacgt tggtcttgaa ctcctgacct tgtgatccac cccgccttgg 15900

cctcccaaag tgctgggatt acaggtgtga gccaccgtgc ccggccaagt aaaatgtttt 15960

ttaaaatggt tatgtgcatt attcataaaa aataatggtg tccagtcttt ttaaacttgt 16020

aaagacacat cttattgaat aaagagatga gagcttaagt ttgtatattg tttccttcga 16080

ttctttgatg tatccatggt ggatgatgct aggttttcat acttatagat aatacacaca 16140

cacacacaca cacaatcttg ttcagggatg ggcatacttc ttctgcaagg gccagatcat 16200

aaagttttta ggctttgtag accatgtaat gtcacaccca ctcacctgca cagttgtaat 16260

tcaaaattag ccatagacaa tgtgtaacca atgggtattg ctgtgtttca ataaaacttt 16320

agtctagaat atatattcat ttttatattt gattacttaa ctttggcaag tcaaatgtaa 16380

agtccagtag tgaattttgc tcttggtact gaatctctga agaactgaag aggtaggaag 16440

atgctgcatt cttataaagg ttatttaaaa gcaatacccg taatttcttt atgggacaat 16500

cttacatata gtataaaatt tttatttgat aatcattttg tgactattct gttcagtaat 16560

atgcagaaag acattctgaa atctgttaat ctctaaacat tttaacgtta gcttgccata 16620

cttgaaagaa actggctagc tgcagtttac attccattgt aagcaggtcc tcctccacct 16680

tcaggtacca cccagttaat attctgactt ggatctttaa tatcacatgt tttacaatgt 16740

acacagttct gagcatttat ctgtaaccga aatccatcac cttgttccac aggtacaaat 16800

tcataaactc ctgaaaagta aaaataaata atattgaagt gcctaacttt aaaaaatttt 16860

taaattcaaa agtaaaaatt aaggaaagag tagccacaga aaccttaaag gttgctttct 16920

gaatgcttac ccatagagat tatgaagacc ttggtgcatt gctattgatg tgagtgtaaa 16980

ttacatgtac ataataaaag gaggtacagc ggtattcaac tgagttgtct cactatcaag 17040

ggtactcgac actgagtgtt tgagttccac acaacctcga agtcctccct tattcacagt 17100

catcctctca ttcacagggg acaagttcca aga 17133

<210> 13

<211> 298

<212>  PRT

<213>  Homo sapiens

<220>

<221>  Zn-alpha 2-glycoprotein

<222>  (1)..(298)

<223>  NM001185.1

<400>  13

Met Val Arg Met Val Pro Val Leu Leu Ser Leu Leu Leu Leu Leu Gly
1               5                   10                  15

Pro Ala Val Pro Gln Glu Asn Gln Asp Gly Arg Tyr Ser Leu Thr Tyr
            20                  25                  30

Ile Tyr Thr Gly Leu Ser Lys His Val Glu Asp Val Pro Ala Phe Gln
        35                  40                  45

Ala Leu Gly Ser Leu Asn Asp Leu Gln Phe Phe Arg Tyr Asn Ser Lys
    50                  55                  60

Asp Arg Lys Ser Gln Pro Met Gly Leu Trp Arg Gln Val Glu Gly Met
65                  70                  75                  80

Glu Asp Trp Lys Gln Asp Ser Gln Leu Gln Lys Ala Arg Glu Asp Ile
                85                  90                  95

Phe Met Glu Thr Leu Lys Asp Ile Val Glu Tyr Tyr Asn Asp Ser Asn
            100                 105                 110

Gly Ser His Val Leu Gln Gly Arg Phe Gly Cys Glu Ile Glu Asn Asn
        115                 120                 125

Arg Ser Ser Gly Ala Phe Trp Lys Tyr Tyr Tyr Asp Gly Lys Asp Tyr
    130                 135                 140

Ile Glu Phe Asn Lys Glu Ile Pro Ala Trp Val Pro Phe Asp Pro Ala
145                 150                 155                 160

Ala Gln Ile Thr Lys Gln Lys Trp Glu Ala Glu Pro Val Tyr Val Gln
                165                 170                 175

Arg Ala Lys Ala Tyr Leu Glu Glu Glu Cys Pro Ala Thr Leu Arg Lys
            180                 185                 190

Tyr Leu Lys Tyr Ser Lys Asn Ile Leu Asp Arg Gln Asp Pro Pro Ser
        195                 200                 205
Val Val Val Thr Ser His Gln Ala Pro Gly Glu Lys Lys Lys Leu Lys
    210                 215                 220
Cys Leu Ala Tyr Asp Phe Tyr Pro Gly Lys Ile Asp Val His Trp Thr
225                 230                 235                 240
Arg Ala Gly Glu Val Gln Glu Pro Glu Leu Arg Gly Asp Val Leu His
                245                 250                 255
Asn Gly Asn Gly Thr Tyr Gln Ser Trp Val Val Val Ala Val Pro Pro
            260                 265                 270
Gln Asp Thr Ala Pro Tyr Ser Cys His Val Gln His Ser Ser Leu Ala
        275                 280                 285
Gln Pro Leu Val Val Pro Trp Glu Ala Ser
    290                 295

<210>  14
<211>  1242
<212>  PRT
<213>  Homo sapiens
<220>
<221>  insulin receptor substrate 1 (IRS1)
<222>  (1)..(1242)
<223>  NM_005544.1
<400>  14

Met Ala Ser Pro Pro Glu Ser Asp Gly Phe Ser Asp Val Arg Lys Val
1               5                   10                  15
Gly Tyr Leu Arg Lys Pro Lys Ser Met His Lys Arg Phe Phe Val Leu
            20                  25                  30
Arg Ala Ala Ser Glu Ala Gly Gly Pro Ala Arg Leu Glu Tyr Tyr Glu
        35                  40                  45

Asn Glu Lys Lys Trp Arg His Lys Ser Ser Ala Pro Lys Arg Ser Ile
    50                  55                  60
Pro Leu Glu Ser Cys Phe Asn Ile Asn Lys Arg Ala Asp Ser Lys Asn
65                  70                  75                  80
Lys His Leu Val Ala Leu Tyr Thr Arg Asp Glu His Phe Ala Ile Ala
                85                  90                  95
Ala Asp Ser Glu Ala Glu Gln Asp Ser Trp Tyr Gln Ala Leu Leu Gln
            100                 105                 110
Leu His Asn Arg Ala Lys Gly His His Asp Gly Ala Ala Ala Leu Gly
        115                 120                 125
Ala Gly Gly Gly Gly Gly Ser Cys Ser Gly Ser Ser Gly Leu Gly Glu
    130                 135                 140
Ala Gly Glu Asp Leu Ser Tyr Gly Asp Val Pro Pro Gly Pro Ala Phe
145                 150                 155                 160
Lys Glu Val Trp Gln Val Ile Leu Lys Pro Lys Gly Leu Gly Gln Thr
                165                 170                 175
Lys Asn Leu Ile Gly Ile Tyr Arg Leu Cys Leu Thr Ser Lys Thr Ile
            180                 185                 190
Ser Phe Val Lys Leu Asn Ser Glu Ala Ala Ala Val Val Leu Gln Leu
        195                 200                 205
Met Asn Ile Arg Arg Cys Gly His Ser Glu Asn Phe Phe Phe Ile Glu
    210                 215                 220
Val Gly Arg Ser Ala Val Thr Gly Pro Gly Glu Phe Trp Met Gln Val
225                 230                 235                 240
Asp Asp Ser Val Val Ala Gln Asn Met His Glu Thr Ile Leu Glu Ala
                245                 250                 255
Met Arg Ala Met Ser Asp Glu Phe Arg Pro Arg Ser Lys Ser Gln Ser
            260                 265                 270
Ser Ser Asn Cys Ser Asn Pro Ile Ser Val Pro Leu Arg Arg His His
        275                 280                 285
Leu Asn Asn Pro Pro Pro Ser Gln Val Gly Leu Thr Arg Arg Ser Arg
    290                 295                 300

Thr Glu Ser Ile Thr Ala Thr Ser Pro Ala Ser Met Val Gly Gly Lys
305                 310                 315                 320
Pro Gly Ser Phe Arg Val Arg Ala Ser Ser Asp Gly Glu Gly Thr Met
                325                 330                 335
Ser Arg Pro Ala Ser Val Asp Gly Ser Pro Val Ser Pro Ser Thr Asn
            340                 345                 350
Arg Thr His Ala His Arg His Arg Gly Ser Ala Arg Leu His Pro Pro
        355                 360                 365
Leu Asn His Ser Arg Ser Ile Pro Met Pro Ala Ser Arg Cys Ser Pro
    370                 375                 380
Ser Ala Thr Ser Pro Val Ser Leu Ser Ser Ser Ser Thr Ser Gly His
385                 390                 395                 400
Gly Ser Thr Ser Asp Cys Leu Phe Pro Arg Arg Ser Ser Ala Ser Val
                405                 410                 415
Ser Gly Ser Pro Ser Asp Gly Gly Phe Ile Ser Ser Asp Glu Tyr Gly
            420                 425                 430
Ser Ser Pro Cys Asp Phe Arg Ser Ser Phe Arg Ser Val Thr Pro Asp
        435                 440                 445
Ser Leu Gly His Thr Pro Pro Ala Arg Gly Glu Glu Glu Leu Ser Asn
    450                 455                 460
Tyr Ile Cys Met Gly Gly Lys Gly Pro Ser Thr Leu Thr Ala Pro Asn
465                 470                 475                 480
Gly His Tyr Ile Leu Ser Arg Gly Gly Asn Gly His Arg Cys Thr Pro
                485                 490                 495
Gly Thr Gly Leu Gly Thr Ser Pro Ala Leu Ala Gly Asp Glu Ala Ala
            500                 505                 510
Ser Ala Ala Asp Leu Asp Asn Arg Phe Arg Lys Arg Thr His Ser Ala
        515                 520                 525
Gly Thr Ser Pro Thr Ile Thr His Gln Lys Thr Pro Ser Gln Ser Ser
    530                 535                 540
Val Ala Ser Ile Glu Glu Tyr Thr Glu Met Met Pro Ala Tyr Pro Pro
545                 550                 555                 560

Gly Gly Gly Ser Gly Gly Arg Leu Pro Gly His Arg His Ser Ala Phe
                565                 570                 575
Val Pro Thr Arg Ser Tyr Pro Glu Glu Gly Leu Glu Met His Pro Leu
            580                 585                 590
Glu Arg Arg Gly Gly His His Arg Pro Asp Ser Ser Thr Leu His Thr
        595                 600                 605
Asp Asp Gly Tyr Met Pro Met Ser Pro Gly Val Ala Pro Val Pro Ser
    610                 615                 620
Gly Arg Lys Gly Ser Gly Asp Tyr Met Pro Met Ser Pro Lys Ser Val
625                 630                 635                 640
Ser Ala Pro Gln Gln Ile Ile Asn Pro Ile Arg Arg His Pro Gln Arg
                645                 650                 655
Val Asp Pro Asn Gly Tyr Met Met Met Ser Pro Ser Gly Gly Cys Ser
            660                 665                 670
Pro Asp Ile Gly Gly Gly Pro Ser Ser Ser Ser Ser Ser Ser Asn Ala
        675                 680                 685
Val Pro Ser Gly Thr Ser Tyr Gly Lys Leu Trp Thr Asn Gly Val Gly
    690                 695                 700
Gly His His Ser His Val Leu Pro His Pro Lys Pro Pro Val Glu Ser
705                 710                 715                 720
Ser Gly Gly Lys Leu Leu Pro Cys Thr Gly Asp Tyr Met Asn Met Ser
                725                 730                 735
Pro Val Gly Asp Ser Asn Thr Ser Ser Pro Ser Asp Cys Tyr Tyr Gly
            740                 745                 750
Pro Glu Asp Pro Gln His Lys Pro Val Leu Ser Tyr Tyr Ser Leu Pro
        755                 760                 765
Arg Ser Phe Lys His Thr Gln Arg Pro Gly Glu Pro Glu Glu Gly Ala
    770                 775                 780
Arg His Gln His Leu Arg Leu Ser Thr Ser Ser Gly Arg Leu Leu Tyr
785                 790                 795                 800
Ala Ala Thr Ala Asp Asp Ser Ser Ser Ser Thr Ser Ser Asp Ser Leu
                805                 810                 815

Gly Gly Gly Tyr Cys Gly Ala Arg Leu Glu Pro Ser Leu Pro His Pro
            820                 825                 830
His His Gln Val Leu Gln Pro His Leu Pro Arg Lys Val Asp Thr Ala
        835                 840                 845
Ala Gln Thr Asn Ser Arg Leu Ala Arg Pro Thr Arg Leu Ser Leu Gly
    850                 855                 860
Asp Pro Lys Ala Ser Thr Leu Pro Arg Ala Arg Glu Gln Gln Gln Gln
865                 870                 875                 880
Gln Gln Pro Leu Leu His Pro Pro Glu Pro Lys Ser Pro Gly Glu Tyr
                885                 890                 895
Val Asn Ile Glu Phe Gly Ser Asp Gln Ser Gly Tyr Leu Ser Gly Pro
            900                 905                 910
Val Ala Phe His Ser Ser Pro Ser Val Arg Cys Pro Ser Gln Leu Gln
        915                 920                 925
Pro Ala Pro Arg Glu Glu Glu Thr Gly Thr Glu Glu Tyr Met Lys Met
    930                 935                 940
Asp Leu Gly Pro Gly Arg Arg Ala Ala Trp Gln Glu Ser Thr Gly Val
945                 950                 955                 960
Glu Met Gly Arg Leu Gly Pro Ala Pro Pro Gly Ala Ala Ser Ile Cys
                965                 970                 975
Arg Pro Thr Arg Ala Val Pro Ser Ser Arg Gly Asp Tyr Met Thr Met
            980                 985                 990
Gln Met Ser Cys Pro Arg Gln Ser Tyr Val Asp Thr Ser Pro Ala Ala
        995                 1000                1005
Pro Val Ser Tyr Ala Asp Met Arg Thr Gly Ile Ala Ala Glu Glu
    1010                1015                1020
Val Ser Leu Pro Arg Ala Thr Met Ala Ala Ala Ser Ser Ser Ser
    1025                1030                1035
Ala Ala Ser Ala Ser Pro Thr Gly Pro Gln Gly Ala Ala Glu Leu
    1040                1045                1050
Ala Ala His Ser Ser Leu Leu Gly Gly Pro Gln Gly Pro Gly Gly
    1055                1060                1065

Met Ser Ala Phe Thr Arg Val Asn Leu Ser Pro Asn Arg Asn Gln
    1070                1075                1080
Ser Ala Lys Val Ile Arg Ala Asp Pro Gln Gly Cys Arg Arg Arg
    1085                1090                1095
His Ser Ser Glu Thr Phe Ser Ser Thr Pro Ser Ala Thr Arg Val
    1100                1105                1110
Gly Asn Thr Val Pro Phe Gly Ala Gly Ala Ala Val Gly Gly Gly
    1115                1120                1125
Gly Gly Ser Ser Ser Ser Ser Glu Asp Val Lys Arg His Ser Ser
    1130                1135                1140
Ala Ser Phe Glu Asn Val Trp Leu Arg Pro Gly Glu Leu Gly Gly
    1145                1150                1155
Ala Pro Lys Glu Pro Ala Lys Leu Cys Gly Ala Ala Gly Gly Leu
    1160                1165                1170
Glu Asn Gly Leu Asn Tyr Ile Asp Leu Asp Leu Val Lys Asp Phe
    1175                1180                1185
Lys Gln Cys Pro Gln Glu Cys Thr Pro Glu Pro Gln Pro Pro Pro
    1190                1195                1200
Pro Pro Pro Pro His Gln Pro Leu Gly Ser Gly Glu Ser Ser Ser
    1205                1210                1215
Thr Arg Arg Ser Ser Glu Asp Leu Ser Ala Tyr Ala Ser Ile Ser
    1220                1225                1230
Phe Gln Lys Gln Pro Glu Asp Arg Gln
    1235                1240

<210> 15
<211> 267
<212> PRT
<213> Homo sapiens
<220>
<221> apolipoprotein A-I

<222>  (1)..(267)

<223>  M27875.1

<400>  15

<210>  16
<211>  789
<212>  PRT
<213>  Homo sapiens
<220>
<221>  aryl hydrocarbon receptor nuclear translocator (ARNT)
<222>  (1)..(789)
<223>  M69238.1
<400>  16

Met Ala Ala Thr Thr Ala Asn Pro Glu Met Thr Ser Asp Val Pro Ser
1               5                   10                  15
Leu Gly Pro Ala Ile Ala Ser Gly Asn Ser Gly Pro Gly Ile Gln Gly
            20                  25                  30
Gly Gly Ala Ile Val Gln Arg Ala Ile Lys Arg Arg Pro Gly Leu Asp
        35                  40                  45
Phe Asp Asp Asp Gly Glu Gly Asn Ser Lys Phe Leu Arg Cys Asp Asp
    50                  55                  60
Asp Gln Met Ser Asn Asp Lys Glu Arg Phe Ala Arg Ser Asp Asp Glu
65                  70                  75                  80
Gln Ser Ser Ala Asp Lys Glu Arg Leu Ala Arg Glu Asn His Ser Glu
                85                  90                  95
Ile Glu Arg Arg Arg Arg Asn Lys Met Thr Ala Tyr Ile Thr Glu Leu
            100                 105                 110

Ser Asp Met Val Pro Thr Cys Ser Ala Leu Ala Arg Lys Pro Asp Lys
        115                 120                 125
Leu Thr Ile Leu Arg Met Ala Val Ser His Met Lys Ser Leu Arg Gly
    130                 135                 140
Thr Gly Asn Thr Ser Thr Asp Gly Ser Tyr Lys Pro Ser Phe Leu Thr
145                 150                 155                 160
Asp Gln Glu Leu Lys His Leu Ile Leu Glu Ala Ala Asp Gly Phe Leu
                165                 170                 175
Phe Ile Val Ser Cys Glu Thr Gly Arg Val Val Tyr Val Ser Asp Ser
            180                 185                 190
Val Thr Pro Val Leu Asn Gln Pro Gln Ser Glu Trp Phe Gly Ser Thr
        195                 200                 205
Leu Tyr Asp Gln Val His Pro Asp Asp Val Asp Lys Leu Arg Glu Gln
    210                 215                 220
Leu Ser Thr Ser Glu Asn Ala Leu Thr Gly Arg Ile Leu Asp Leu Lys
225                 230                 235                 240
Thr Gly Thr Val Lys Lys Glu Gly Gln Gln Ser Ser Met Arg Met Cys
                245                 250                 255
Met Gly Ser Arg Arg Ser Phe Ile Cys Arg Met Arg Cys Gly Ser Ser
            260                 265                 270
Ser Val Asp Pro Val Ser Val Asn Arg Leu Ser Phe Val Arg Asn Arg
        275                 280                 285
Cys Arg Asn Gly Leu Gly Ser Val Lys Asp Gly Glu Pro His Phe Val
    290                 295                 300
Val Val His Cys Thr Gly Tyr Ile Lys Ala Trp Pro Pro Ala Gly Val
305                 310                 315                 320
Ser Leu Pro Asp Asp Asp Pro Glu Ala Gly Gln Gly Ser Lys Phe Cys
                325                 330                 335
Leu Val Ala Ile Gly Arg Leu Gln Val Thr Ser Ser Pro Asn Cys Thr
            340                 345                 350
Asp Met Ser Asn Val Cys Gln Pro Thr Glu Phe Ile Ser Arg His Asn
        355                 360                 365

Ile Glu Gly Ile Phe Thr Phe Val Asp His Arg Cys Val Ala Thr Val
    370                 375                 380
Gly Tyr Gln Pro Gln Glu Leu Leu Gly Lys Asn Ile Val Glu Phe Cys
385                 390                 395                 400
His Pro Glu Asp Gln Gln Leu Leu Arg Asp Ser Phe Gln Gln Val Val
                405                 410                 415
Lys Leu Lys Gly Gln Val Leu Ser Val Met Phe Arg Phe Arg Ser Lys
            420                 425                 430
Asn Gln Glu Trp Leu Trp Met Arg Thr Ser Ser Phe Thr Phe Gln Asn
        435                 440                 445
Pro Tyr Ser Asp Glu Ile Glu Tyr Ile Ile Cys Thr Asn Thr Asn Val
    450                 455                 460
Lys Asn Ser Ser Gln Glu Pro Arg Pro Thr Leu Ser Asn Thr Ile Gln
465                 470                 475                 480
Arg Pro Gln Leu Gly Pro Thr Ala Asn Leu Pro Leu Glu Met Gly Ser
                485                 490                 495
Gly Gln Leu Ala Pro Arg Gln Gln Gln Gln Gln Thr Glu Leu Asp Met
            500                 505                 510
Val Pro Gly Arg Asp Gly Leu Ala Ser Tyr Asn His Ser Gln Val Val
        515                 520                 525
Gln Pro Val Thr Thr Thr Gly Pro Glu His Ser Lys Pro Leu Glu Lys
    530                 535                 540
Ser Asp Gly Leu Phe Ala Gln Asp Arg Asp Pro Arg Phe Ser Glu Ile
545                 550                 555                 560
Tyr His Asn Ile Asn Ala Asp Gln Ser Lys Gly Ile Ser Ser Ser Thr
                565                 570                 575
Val Pro Ala Thr Gln Gln Leu Phe Ser Gln Gly Asn Thr Phe Pro Pro
            580                 585                 590
Thr Pro Arg Pro Ala Glu Asn Phe Arg Asn Ser Gly Leu Ala Pro Pro
        595                 600                 605
Val Thr Ile Val Gln Pro Ser Ala Ser Ala Gly Gln Met Leu Ala Gln
    610                 615                 620

Ile Ser Arg His Ser Asn Pro Thr Gln Gly Ala Thr Pro Thr Trp Thr
625                 630                 635                 640
Pro Thr Thr Arg Ser Gly Phe Ser Ala Gln Gln Val Ala Thr Gln Ala
                645                 650                 655
Thr Ala Lys Thr Arg Thr Ser Gln Phe Gly Val Gly Ser Phe Gln Thr
            660                 665                 670
Pro Ser Ser Phe Ser Ser Met Ser Leu Pro Gly Ala Pro Thr Ala Ser
        675                 680                 685
Pro Gly Ala Ala Ala Tyr Pro Ser Leu Thr Asn Arg Gly Ser Asn Phe
    690                 695                 700
Ala Pro Glu Thr Gly Gln Thr Ala Gly Gln Phe Gln Thr Arg Thr Ala
705                 710                 715                 720
Glu Gly Val Gly Val Trp Pro Gln Trp Gln Gly Gln Gln Pro His His
                725                 730                 735
Arg Ser Ser Ser Ser Glu Gln His Val Gln Gln Pro Pro Ala Gln Gln
            740                 745                 750
Pro Gly Gln Pro Glu Val Phe Gln Glu Met Leu Ser Met Leu Gly Asp
        755                 760                 765
Gln Ser Asn Ser Tyr Asn Asn Glu Glu Phe Pro Asp Leu Thr Met Phe
    770                 775                 780
Pro Pro Phe Ser Glu
785

<210>  17
<211>  381
<212>  PRT
<213>  Homo sapiens
<220>
<221>  creatine kinase, brain (CKB)
<222>  (1)..(381)
<223>  NM_001823.3

<400> 17

Met Pro Phe Ser Asn Ser His Asn Ala Leu Lys Leu Arg Phe Pro Ala
1               5                   10                  15

Glu Asp Glu Phe Pro Asp Leu Ser Ala His Asn Asn His Met Ala Lys
            20                  25                  30

Val Leu Thr Pro Glu Leu Tyr Ala Glu Leu Arg Ala Lys Ser Thr Pro
        35                  40                  45

Ser Gly Phe Thr Leu Asp Asp Val Ile Gln Thr Gly Val Asp Asn Pro
    50                  55                  60

Gly His Pro Tyr Ile Met Thr Val Gly Cys Val Ala Gly Asp Glu Glu
65                  70                  75                  80

Ser Tyr Glu Val Phe Lys Asp Leu Phe Asp Pro Ile Ile Glu Asp Arg
                85                  90                  95

His Gly Gly Tyr Lys Pro Ser Asp Glu His Lys Thr Asp Leu Asn Pro
            100                 105                 110

Asp Asn Leu Gln Gly Gly Asp Asp Leu Asp Pro Asn Tyr Val Leu Ser
        115                 120                 125

Ser Arg Val Arg Thr Gly Arg Ser Ile Arg Gly Phe Cys Leu Pro Pro
    130                 135                 140

His Cys Ser Arg Gly Glu Arg Arg Ala Ile Glu Lys Leu Ala Val Glu
145                 150                 155                 160

Ala Leu Ser Ser Leu Asp Gly Asp Leu Ala Gly Arg Tyr Tyr Ala Leu
                165                 170                 175

Lys Ser Met Thr Glu Ala Glu Gln Gln Gln Leu Ile Asp Asp His Phe
            180                 185                 190

Leu Phe Asp Lys Pro Val Ser Pro Leu Leu Leu Ala Ser Gly Met Ala
        195                 200                 205

Arg Asp Trp Pro Asp Ala Arg Gly Ile Trp His Asn Asp Asn Lys Thr
    210                 215                 220

Phe Leu Val Trp Val Asn Glu Glu Asp His Leu Arg Val Ile Ser Met

225                 230                 235                 240

Gln Lys Gly Gly Asn Met Lys Glu Val Phe Thr Arg Phe Cys Thr Gly

                245                 250                 255

Leu Thr Gln Ile Glu Thr Leu Phe Lys Ser Lys Asp Tyr Glu Phe Met

            260                 265                 270

Trp Asn Pro His Leu Gly Tyr Ile Leu Thr Cys Pro Ser Asn Leu Gly

        275                 280                 285

Thr Gly Leu Arg Ala Gly Val His Ile Lys Leu Pro Asn Leu Gly Lys

    290                 295                 300

His Glu Lys Phe Ser Glu Val Leu Lys Arg Leu Arg Leu Gln Lys Arg

305                 310                 315                 320

Gly Thr Gly Gly Val Asp Thr Ala Ala Val Gly Gly Val Phe Asp Val

                325                 330                 335

Ser Asn Ala Asp Arg Leu Gly Phe Ser Glu Val Glu Leu Val Gln Met

            340                 345                 350

Val Val Asp Gly Val Lys Leu Leu Ile Glu Met Glu Gln Arg Leu Glu

        355                 360                 365

Gln Gly Gln Ala Ile Asp Asp Leu Met Pro Ala Gln Lys

    370                 375                 380

<210>  18

<211>  299

<212>  PRT

<213>  Homo sapiens

<220>

<221>  delta7-sterol-C5-desaturase (S5DES)

<222>  (1)..(299)

<223>  AF187981.1

<400>  18

Met Asp Leu Val Leu Arg Val Ala Asp Tyr Tyr Phe Phe Thr Pro Tyr

1                   5                   10                  15

Val Tyr Pro Ala Thr Trp Pro Glu Asp Asp Ile Phe Arg Gln Ala Ile

                20                  25                  30

Ser Leu Leu Ile Val Thr Asn Val Gly Ala Tyr Ile Leu Tyr Phe Phe

            35                  40                  45

Cys Ala Thr Leu Ser Tyr Tyr Phe Val Phe Asp His Ala Leu Met Lys

    50                  55                  60

His Pro Gln Phe Leu Lys Asn Gln Val Arg Arg Glu Ile Lys Phe Thr

65                  70                  75                  80

Val Gln Ala Leu Pro Trp Ile Ser Ile Leu Thr Val Ala Leu Phe Leu

                85                  90                  95

Leu Glu Ile Arg Gly Tyr Ser Lys Leu His Asp Asp Leu Gly Glu Phe

            100                 105                 110

Pro Tyr Gly Leu Phe Glu Leu Val Val Ser Ile Ile Ser Phe Leu Phe

        115                 120                 125

Phe Thr Asp Met Phe Ile Tyr Trp Ile His Arg Gly Leu His His Arg

    130                 135                 140

Leu Val Tyr Lys Arg Leu His Lys Pro His His Ile Trp Lys Ile Pro

145                 150                 155                 160

Thr Pro Phe Ala Ser His Ala Phe His Pro Ile Asp Gly Phe Leu Gln

                165                 170                 175

Ser Leu Pro Tyr His Ile Tyr Pro Phe Ile Phe Pro Leu His Lys Val

            180                 185                 190

Val Tyr Leu Ser Leu Tyr Ile Leu Val Asn Ile Trp Thr Ile Ser Ile

        195                 200                 205

His Asp Gly Asp Phe Arg Val Pro Gln Ile Leu Gln Pro Phe Ile Asn

    210                 215                 220

Gly Ser Ala His His Thr Asp His His Met Phe Phe Asp Tyr Asn Tyr

225                 230                 235                 240

Gly Gln Tyr Phe Thr Leu Trp Asp Arg Ile Gly Gly Ser Phe Lys Asn

                245                 250                 255

Pro Ser Ser Phe Glu Gly Lys Gly Pro Leu Ser Tyr Val Lys Glu Met

            260                 265                 270

Thr Glu Gly Lys Arg Ser Ser His Ser Gly Asn Gly Cys Lys Asn Glu

        275                 280                 285

Lys Leu Phe Asn Gly Glu Phe Thr Lys Thr Glu

    290                 295

<210> 19

<211> 421

<212> PRT

<213> Homo sapiens

<220>

<221> osteomodulin (OMD)

<222> (1)..(421)

<223> NM_005014.1

<400> 19

Met Gly Phe Leu Ser Pro Ile Tyr Val Ile Phe Phe Phe Phe Gly Val

1               5                   10                  15

Lys Val His Cys Gln Tyr Glu Thr Tyr Gln Trp Asp Glu Asp Tyr Asp

            20                  25                  30

Gln Glu Pro Asp Asp Asp Tyr Gln Thr Gly Phe Pro Phe Arg Gln Asn

        35                  40                  45

Val Asp Tyr Gly Val Pro Phe His Gln Tyr Thr Leu Gly Cys Val Ser

    50                  55                  60

Glu Cys Phe Cys Pro Thr Asn Phe Pro Ser Ser Met Tyr Cys Asp Asn

65                  70                  75                  80

Arg Lys Leu Lys Thr Ile Pro Asn Ile Pro Met His Ile Gln Gln Leu

                85                  90                  95

Tyr Leu Gln Phe Asn Glu Ile Glu Ala Val Thr Ala Asn Ser Phe Ile

            100                 105                 110

Asn Ala Thr His Leu Lys Glu Ile Asn Leu Ser His Asn Lys Ile Lys

        115                 120                 125
Ser Gln Lys Ile Asp Tyr Gly Val Phe Ala Lys Leu Pro Asn Leu Leu
    130                 135                 140
Gln Leu His Leu Glu His Asn Asn Leu Glu Glu Phe Pro Phe Pro Leu
145                 150                 155                 160
Pro Lys Ser Leu Glu Arg Leu Leu Leu Gly Tyr Asn Glu Ile Ser Lys
                165                 170                 175
Leu Gln Thr Asn Ala Met Asp Gly Leu Val Asn Leu Thr Met Leu Asp
            180                 185                 190
Leu Cys Tyr Asn Tyr Leu His Asp Ser Leu Leu Lys Asp Lys Ile Phe
        195                 200                 205
Ala Lys Met Glu Lys Leu Met Gln Leu Asn Leu Cys Ser Asn Arg Leu
    210                 215                 220
Glu Ser Met Pro Pro Gly Leu Pro Ser Ser Leu Met Tyr Leu Ser Leu
225                 230                 235                 240
Glu Asn Asn Ser Ile Ser Ser Ile Pro Glu Lys Tyr Phe Asp Lys Leu
                245                 250                 255
Pro Lys Leu His Thr Leu Arg Met Ser His Asn Lys Leu Gln Asp Ile
            260                 265                 270
Pro Tyr Asn Ile Phe Asn Leu Pro Asn Ile Val Glu Leu Ser Val Gly
        275                 280                 285
His Asn Lys Leu Lys Gln Ala Phe Tyr Ile Pro Arg Asn Leu Glu His
    290                 295                 300
Leu Tyr Leu Gln Asn Asn Glu Ile Glu Lys Met Asn Leu Thr Val Met
305                 310                 315                 320
Cys Pro Ser Ile Asp Pro Leu His Tyr His His Leu Thr Tyr Ile Arg
                325                 330                 335
Val Asp Gln Asn Lys Leu Lys Glu Pro Ile Ser Ser Tyr Ile Phe Phe
            340                 345                 350
Cys Phe Pro His Ile His Thr Ile Tyr Tyr Gly Glu Gln Arg Ser Thr
        355                 360                 365
Asn Gly Gln Thr Ile Gln Leu Lys Thr Gln Val Phe Arg Arg Phe Pro

370                 375                 380

Asp Asp Asp Asp Glu Ser Glu Asp His Asp Asp Pro Asp Asn Ala His

385                 390                 395                 400

Glu Ser Pro Glu Gln Glu Gly Ala Glu Gly His Phe Asp Leu His Tyr

                405                 410                 415

Tyr Glu Asn Gln Glu

            420

<210>  20

<211>  845

<212>  PRT

<213>  Homo sapiens

<220>

<221>  very low density lipoprotein receptor

<222>  (1)..(845)

<223>  D16494.1

<400>  20

Met Gly Thr Ser Ala Leu Trp Ala Leu Trp Leu Leu Leu Ala Leu Cys

1               5                   10                  15

Trp Ala Pro Arg Glu Ser Gly Ala Thr Gly Thr Gly Arg Lys Ala Lys

            20                  25                  30

Cys Glu Pro Ser Gln Phe Gln Cys Thr Asn Gly Arg Cys Ile Thr Leu

        35                  40                  45

Leu Trp Lys Cys Asp Gly Asp Glu Asp Cys Val Asp Gly Ser Asp Glu

    50                  55                  60

Lys Asn Cys Val Lys Lys Thr Cys Ala Glu Ser Asp Phe Val Cys Asn

65                  70                  75                  80

Asn Gly Gln Cys Val Pro Ser Arg Trp Lys Cys Asp Gly Asp Pro Asp

                85                  90                  95

Cys Glu Asp Gly Ser Asp Glu Ser Pro Glu Gln Cys His Met Arg Thr

            100                 105                 110
Cys Arg Ile His Glu Ile Ser Cys Gly Ala His Ser Thr Gln Cys Ile
        115                 120                 125
Pro Val Ser Trp Arg Cys Asp Gly Glu Asn Asp Cys Asp Ser Gly Glu
    130                 135                 140
Asp Glu Glu Asn Cys Gly Asn Ile Thr Cys Ser Pro Asp Glu Phe Thr
145                 150                 155                 160
Cys Ser Ser Gly Arg Cys Ile Ser Arg Asn Phe Val Cys Asn Gly Gln
                165                 170                 175
Asp Asp Cys Ser Asp Gly Ser Asp Glu Leu Asp Cys Ala Pro Pro Thr
            180                 185                 190
Cys Gly Ala His Glu Phe Gln Cys Ser Thr Ser Ser Cys Ile Pro Ile
        195                 200                 205
Ser Trp Val Cys Asp Asp Asp Ala Asp Cys Ser Asp Gln Ser Asp Glu
    210                 215                 220
Ser Leu Glu Gln Cys Gly Arg Gln Pro Val Ile His Thr Lys Cys Pro
225                 230                 235                 240
Ala Ser Glu Ile Gln Cys Gly Ser Gly Glu Cys Ile His Lys Lys Trp
                245                 250                 255
Arg Cys Asp Gly Asp Pro Asp Cys Lys Asp Gly Ser Asp Glu Val Asn
            260                 265                 270
Cys Pro Ser Arg Thr Cys Arg Pro Asp Gln Phe Glu Cys Glu Asp Gly
        275                 280                 285
Ser Cys Ile His Gly Ser Arg Gln Cys Asn Gly Ile Arg Asp Cys Val
    290                 295                 300
Asp Gly Ser Asp Glu Val Asn Cys Lys Asn Val Asn Gln Cys Leu Gly
305                 310                 315                 320
Pro Gly Lys Phe Lys Cys Arg Ser Gly Glu Cys Ile Asp Ile Ser Lys
                325                 330                 335
Val Cys Asn Gln Glu Gln Asp Cys Arg Asp Trp Ser Asp Glu Pro Leu
            340                 345                 350
Lys Glu Cys His Ile Asn Glu Cys Leu Val Asn Asn Gly Gly Cys Ser

        355                 360                 365
His Ile Cys Lys Asp Leu Val Ile Gly Tyr Glu Cys Asp Cys Ala Ala
    370                 375                 380
Gly Phe Glu Leu Ile Asp Arg Lys Thr Cys Gly Asp Ile Asp Glu Cys
385                 390                 395                 400
Gln Asn Pro Gly Ile Cys Ser Gln Ile Cys Ile Asn Leu Lys Gly Gly
                405                 410                 415
Tyr Lys Cys Glu Cys Ser Arg Gly Tyr Gln Met Asp Leu Ala Thr Gly
            420                 425                 430
Val Cys Lys Ala Val Gly Lys Glu Pro Ser Leu Ile Phe Thr Asn Arg
        435                 440                 445
Arg Asp Ile Arg Lys Ile Gly Leu Glu Arg Lys Glu Tyr Ile Gln Leu
    450                 455                 460
Val Glu Gln Leu Arg Asn Thr Val Ala Leu Asp Ala Asp Ile Ala Ala
465                 470                 475                 480
Gln Lys Leu Phe Trp Ala Asp Leu Ser Gln Lys Ala Ile Phe Ser Ala
                485                 490                 495
Ser Ile Asp Asp Lys Val Gly Arg His Val Lys Met Ile Asp Asn Val
            500                 505                 510
Tyr Asn Pro Ala Ala Ile Ala Val Asp Trp Val Tyr Lys Thr Ile Tyr
        515                 520                 525
Trp Thr Asp Ala Ala Ser Lys Thr Ile Ser Val Ala Thr Leu Asp Gly
    530                 535                 540
Thr Lys Arg Lys Phe Leu Phe Asn Ser Asp Leu Arg Glu Pro Ala Ser
545                 550                 555                 560
Ile Ala Val Asp Pro Leu Ser Gly Phe Val Tyr Trp Ser Asp Trp Gly
                565                 570                 575
Glu Pro Ala Lys Ile Glu Lys Ala Gly Met Asn Gly Phe Asp Arg Arg
            580                 585                 590
Pro Leu Val Thr Ala Asp Ile Gln Trp Pro Asn Gly Ile Thr Leu Asp
        595                 600                 605

Leu Ile Lys Ser Arg Leu Tyr Trp Leu Asp Ser Lys Leu His Met Leu
    610                 615                 620
Ser Ser Val Asp Leu Asn Gly Gln Asp Arg Arg Ile Val Leu Lys Ser
625                 630                 635                 640
Leu Glu Phe Leu Ala His Pro Leu Ala Leu Thr Ile Phe Glu Asp Arg
                645                 650                 655
Val Tyr Trp Ile Asp Gly Glu Asn Glu Ala Val Tyr Gly Ala Asn Lys
            660                 665                 670
Phe Thr Gly Ser Glu Leu Ala Thr Leu Val Asn Asn Leu Asn Asp Ala
        675                 680                 685
Gln Asp Ile Ile Val Tyr His Glu Leu Val Gln Pro Ser Gly Lys Asn
    690                 695                 700
Trp Cys Glu Glu Asp Met Glu Asn Gly Gly Cys Glu Tyr Leu Cys Leu
705                 710                 715                 720
Pro Ala Pro Gln Ile Asn Asp His Ser Pro Lys Tyr Thr Cys Ser Cys
                725                 730                 735
Pro Ser Gly Tyr Asn Val Glu Glu Asn Gly Arg Asp Cys Gln Arg Ile
            740                 745                 750
Asn Val Thr Thr Ala Val Ser Glu Val Ser Val Pro Pro Lys Gly Thr
        755                 760                 765
Ser Ala Ala Trp Ala Ile Leu Pro Leu Leu Leu Leu Val Met Ala Ala
    770                 775                 780
Val Gly Gly Tyr Leu Met Trp Arg Asn Trp Gln His Lys Asn Met Lys
785                 790                 795                 800
Ser Met Asn Phe Asp Asn Pro Val Tyr Leu Lys Thr Thr Glu Glu Asp
                805                 810                 815
Leu Ser Ile Asp Ile Gly Arg His Ser Ala Ser Val Gly His Thr Tyr
            820                 825                 830
Pro Ala Ile Ser Val Val Ser Thr Asp Asp Asp Leu Ala
        835                 840                 845

<210>  21

<211>  297

<212>  PRT

<213>  Homo sapiens

<220>

<221>  hydroxysteroid (11-beta) dehydrogenase 1 (HSD11B1)

<222>  (1)..(297)

<223>  NM_005525.2

<400>  21

Met Ala Phe Met Lys Lys Tyr Leu Leu Pro Ile Leu Gly Leu Phe Met
1               5                   10                  15

Ala Tyr Tyr Tyr Tyr Ser Ala Asn Glu Glu Phe Arg Pro Glu Met Leu
            20                  25                  30

Gln Gly Lys Lys Val Ile Val Thr Gly Ala Ser Lys Gly Ile Gly Arg
        35                  40                  45

Glu Met Ala Tyr His Leu Ala Lys Met Gly Ala His Val Val Val Thr
    50                  55                  60

Ala Arg Ser Lys Glu Thr Leu Gln Lys Val Val Ser His Cys Leu Glu
65                  70                  75                  80

Leu Gly Ala Ala Ser Ala His Tyr Ile Ala Gly Thr Met Glu Asp Met
                85                  90                  95

Thr Phe Ala Glu Gln Phe Val Ala Gln Ala Gly Lys Leu Met Gly Gly
            100                 105                 110

Leu Asp Met Leu Ile Leu Asn His Ile Thr Asn Thr Ser Leu Asn Leu
        115                 120                 125

Phe His Asp Asp Ile His His Val Arg Lys Ser Met Glu Val Asn Phe
    130                 135                 140

Leu Ser Tyr Val Val Leu Thr Val Ala Ala Leu Pro Met Leu Lys Gln
145                 150                 155                 160

Ser Asn Gly Ser Ile Val Val Val Ser Ser Leu Ala Gly Lys Val Ala
                165                 170                 175

Tyr Pro Met Val Ala Ala Tyr Ser Ala Ser Lys Phe Ala Leu Asp Gly
            180                 185                 190
Phe Phe Ser Ser Ile Arg Lys Glu Tyr Ser Val Ser Arg Val Asn Val
        195                 200                 205
Ser Ile Thr Leu Cys Val Leu Gly Leu Ile Asp Thr Glu Thr Ala Met
    210                 215                 220
Lys Ala Val Ser Gly Ile Val His Met Gln Ala Ala Pro Lys Glu Glu
225                 230                 235                 240
Cys Ala Leu Glu Ile Ile Lys Gly Gly Ala Leu Arg Gln Glu Glu Val
                245                 250                 255
Tyr Tyr Asp Ser Ser Leu Trp Thr Thr Leu Leu Ile Arg Asn Pro Cys
            260                 265                 270
Arg Lys Ile Leu Glu Phe Leu Tyr Ser Thr Ser Tyr Asn Met Asp Arg
        275                 280                 285
Phe Ile Asn Lys Asp Asp Asp Leu Ala
    290                 295

<210> 22
<211> 3460
<212> PRT
<213> Homo sapiens
<220>
<221> reelin (RELN)
<222> (1)..(3460)
<223> U79716
<400> 22

<210> 23
<211> 700
<212> PRT
<213> Homo sapiens
<220>
<221> atp-binding cassette, sub-family c (cftr/mrp), member 10
(ABCC10)atp-binding
<222> (1)..(700)
<223> AL133613.1
<400> 23

Ala Val Leu Leu Met Glu Ala Gly Arg Leu Ile Arg Ala Gly Pro Pro
1               5                   10                  15
Ser Glu Ile Leu Pro Leu Val Gln Ala Val Pro Lys Ala Trp Ala Glu
            20                  25                  30
Asn Gly Gln Glu Ser Asp Ser Ala Thr Ala Gln Ser Val Gln Asn Pro
        35                  40                  45
Glu Lys Thr Lys Glu Gly Leu Glu Glu Glu Gln Ser Thr Ser Gly Arg
    50                  55                  60
Leu Leu Gln Glu Glu Ser Lys Lys Glu Gly Ala Val Ala Leu His Val
65                  70                  75                  80
Tyr Gln Ala Tyr Trp Lys Ala Val Gly Gln Gly Leu Ala Leu Ala Ile
                85                  90                  95
Leu Phe Ser Leu Leu Leu Met Gln Ala Thr Arg Asn Ala Ala Asp Trp
            100                 105                 110
Trp Leu Ser His Trp Ile Ser Gln Leu Lys Ala Glu Asn Ser Ser Gln
        115                 120                 125
Glu Ala Gln Pro Ser Thr Ser Pro Ala Ser Met Gly Leu Phe Ser Pro

130                 135                 140

Gln Leu Leu Leu Phe Ser Pro Gly Asn Leu Tyr Ile Pro Val Phe Pro

145                 150                 155                 160

Leu Pro Lys Ala Ala Pro Asn Gly Ser Ser Asp Ile Arg Phe Tyr Leu

                165                 170                 175

Thr Val Tyr Ala Thr Ile Ala Gly Val Asn Ser Leu Cys Thr Leu Leu

            180                 185                 190

Arg Ala Val Leu Phe Ala Ala Gly Thr Leu Gln Ala Ala Ala Thr Leu

        195                 200                 205

His Arg Arg Leu Leu His Arg Val Leu Met Ala Pro Val Thr Phe Phe

    210                 215                 220

Asn Ala Thr Pro Thr Gly Arg Ile Leu Asn Arg Phe Ser Ser Asp Val

225                 230                 235                 240

Ala Cys Ala Asp Asp Ser Leu Pro Phe Ile Leu Asn Ile Leu Leu Ala

                245                 250                 255

Asn Ala Ala Gly Leu Leu Gly Leu Leu Ala Val Leu Gly Ser Gly Leu

            260                 265                 270

Pro Trp Leu Leu Leu Leu Leu Pro Pro Leu Ser Ile Met Tyr Tyr His

        275                 280                 285

Val Gln Arg His Tyr Arg Ala Ser Ser Arg Glu Leu Arg Arg Leu Gly

    290                 295                 300

Ser Leu Thr Leu Ser Pro Leu Tyr Ser His Leu Ala Asp Thr Leu Ala

305                 310                 315                 320

Gly Leu Ser Val Leu Arg Ala Thr Gly Ala Thr Tyr Arg Phe Glu Glu

                325                 330                 335

Glu Asn Leu Arg Leu Leu Glu Leu Asn Gln Arg Cys Gln Phe Ala Thr

            340                 345                 350

Ser Ala Thr Met Gln Trp Leu Asp Ile Arg Leu Gln Leu Met Gly Ala

        355                 360                 365

Ala Val Val Ser Ala Ile Ala Gly Ile Ala Leu Val Gln His Gln Gln

    370                 375                 380

Gly Leu Ala Asn Pro Gly Leu Val Gly Leu Ser Leu Ser Tyr Ala Leu

385                 390                 395                 400

Ser Leu Thr Gly Leu Leu Ser Gly Leu Val Ser Ser Phe Thr Gln Thr

                405                 410                 415

Glu Ala Met Leu Val Ser Val Glu Arg Leu Glu Glu Tyr Thr Cys Asp

            420                 425                 430

Leu Pro Gln Glu Pro Gln Gly Gln Pro Leu Gln Leu Gly Thr Gly Trp

        435                 440                 445

Leu Thr Gln Gly Gly Val Glu Phe Gln Asp Val Val Leu Ala Tyr Arg

    450                 455                 460

Pro Gly Leu Pro Asn Ala Leu Asp Gly Val Thr Phe Cys Val Gln Pro

465                 470                 475                 480

Gly Glu Lys Leu Gly Ile Val Gly Arg Thr Gly Ser Gly Lys Ser Ser

                485                 490                 495

Leu Leu Leu Val Leu Phe Arg Leu Leu Glu Pro Ser Ser Gly Arg Val

            500                 505                 510

Leu Leu Asp Gly Val Asp Thr Ser Gln Leu Glu Leu Ala Gln Leu Arg

        515                 520                 525

Ser Gln Leu Ala Ile Ile Pro Gln Glu Pro Phe Leu Phe Ser Gly Thr

    530                 535                 540

Val Arg Glu Asn Leu Asp Pro Gln Gly Leu His Lys Asp Arg Ala Leu

545                 550                 555                 560

Trp Gln Ala Leu Lys Gln Cys His Leu Ser Glu Val Ile Thr Ser Met

                565                 570                 575

Gly Gly Leu Asp Gly Glu Leu Gly Glu Gly Gly Arg Ser Leu Ser Leu

            580                 585                 590

Gly Gln Arg Gln Leu Leu Cys Leu Ala Arg Ala Leu Leu Thr Asp Ala

        595                 600                 605

Lys Ile Leu Cys Ile Asp Glu Ala Thr Ala Ser Val Asp Gln Lys Thr

    610                 615                 620

Asp Gln Leu Leu Gln Gln Thr Ile Cys Lys Arg Phe Ala Asn Lys Thr

625                 630                 635                 640

Val Leu Thr Ile Ala His Arg Leu Asn Thr Ile Leu Asn Ser Asp Arg

                645                 650                 655
Val Leu Val Leu Gln Ala Gly Arg Val Val Glu Leu Asp Ser Pro Ala
            660                 665                 670
Thr Leu Arg Asn Gln Pro His Ser Leu Phe Gln Gln Leu Leu Gln Ser
        675                 680                 685
Ser Gln Gln Gly Val Pro Ala Ser Leu Gly Gly Pro
    690                 695                 700

<210> 24
<211> 370
<212> PRT
<213> Homo sapiens
<220>
<221> cyclophilin 40
<222> (1)..(370)
<223> D63861.1
<400> 24

Met Ser His Pro Ser Pro Gln Ala Lys Pro Ser Asn Pro Ser Asn Pro
1               5                   10                  15

Arg Val Phe Phe Asp Val Asp Ile Gly Gly Glu Arg Val Gly Arg Ile
            20                  25                  30
Val Leu Glu Leu Phe Ala Asp Ile Val Pro Lys Thr Ala Glu Asn Phe
        35                  40                  45
Arg Ala Leu Cys Thr Gly Glu Lys Gly Ile Gly His Thr Thr Gly Lys
    50                  55                  60
Pro Leu His Phe Lys Gly Cys Pro Phe His Arg Ile Ile Lys Lys Phe
65                  70                  75                  80
Met Ile Gln Gly Gly Asp Phe Ser Asn Gln Asn Gly Thr Gly Gly Glu
                85                  90                  95

Ser Ile Tyr Gly Glu Lys Phe Glu Asp Glu Asn Phe His Tyr Lys His
            100                 105                 110
Asp Arg Glu Gly Leu Leu Ser Met Ala Asn Ala Gly Arg Asn Thr Asn
        115                 120                 125
Gly Ser Gln Phe Phe Ile Thr Thr Val Pro Thr Pro His Leu Asp Gly
    130                 135                 140
Lys His Val Val Phe Gly Gln Val Ile Lys Gly Ile Gly Val Ala Arg
145                 150                 155                 160
Ile Leu Glu Asn Val Glu Val Lys Gly Glu Lys Pro Ala Lys Leu Cys
                165                 170                 175
Val Ile Ala Glu Cys Gly Glu Leu Lys Glu Gly Asp Asp Gly Gly Ile
            180                 185                 190
Phe Pro Lys Asp Gly Ser Gly Asp Ser His Pro Asp Phe Pro Glu Asp
        195                 200                 205
Ala Asp Ile Asp Leu Lys Asp Val Asp Lys Ile Leu Leu Ile Thr Glu
    210                 215                 220
Asp Leu Lys Asn Ile Gly Asn Thr Phe Phe Lys Ser Gln Asn Trp Glu
225                 230                 235                 240
Met Ala Ile Lys Lys Tyr Ala Glu Val Leu Arg Tyr Val Asp Ser Ser
                245                 250                 255
Lys Ala Val Ile Glu Thr Ala Asp Arg Ala Lys Leu Gln Pro Ile Ala
            260                 265                 270
Leu Ser Cys Val Leu Asn Ile Gly Ala Cys Lys Leu Lys Met Ser Asn
        275                 280                 285
Trp Gln Gly Ala Ile Asp Ser Cys Leu Glu Ala Leu Glu Leu Asp Pro
    290                 295                 300
Ser Asn Thr Lys Ala Leu Tyr Arg Arg Ala Gln Gly Trp Gln Gly Leu
305                 310                 315                 320
Lys Glu Tyr Asp Gln Ala Leu Ala Asp Leu Lys Lys Ala Gln Gly Ile
                325                 330                 335
Ala Pro Glu Asp Lys Ala Ile Gln Ala Glu Leu Leu Lys Val Lys Gln
            340                 345                 350

Lys Ile Lys Ala Gln Lys Asp Lys Glu Lys Ala Val Tyr Ala Lys Met
        355                 360                 365
Phe Ala
    370

<210> 25
<211> 1730
<212> DNA
<213> Homo sapiens
<220>
<221> retinoid X receptor, gamma
<222> (1)..(1730)
<223> LocusID: 6258; NM_006917
<400> 25
ctccgatcta gaggcagatt cctgactaat cccagagggc tggcccagcc tgtgctcccc 60
gggctgctag gaagcgatga ccactcttgt tagcccaagt tgaagaaagc cgggctgtgc 120
ctgggagccg agagaggcgg taatatttag aagctgcaca ggagaggaac atgaactgac 180
gagtaaacat gtatggaaat tattctcact tcatgaagtt tcccgcaggc tatggaggct 240
cccctggcca cactggctct acatccatga gcccatcagc agccttgtcc acagggaagc 300
caatggacag ccaccccagc tacacagata ccccagtgag tgccccacgg actctgagtg 360
cagtggggac ccccctcaat gccctgggct ctccatatcg agtcatcacc tctgccatgg 420
gcccaccctc aggagcactt gcagcgcctc caggaatcaa cttggttgcc ccacccagct 480
ctcagctaaa tgtggtcaac agtgtcagca gttcagagga catcaagccc ttaccagggc 540
ttcccgggat tggaaacatg aactacccat ccaccagccc cggatctctg gttaaacaca 600
tctgtgccat ctgtggagac agatcctcag gaaagcacta cggggtatac agttgtgaag 660
gctgcaaagg gttcttcaag aggacgataa ggaaggacct catctacacg tgtcgggata 720
ataaagactg cctcattgac aagcgtcagc gcaaccgctg ccagtactgt cgctatcaga 780
agtgccttgt catgggcatg aagagggaag ctgtgcaaga agaaagacag aggagccgag 840
agcgagctga gagtgaggca gaatgtgcta ccagtggtca tgaagacatg cctgtggaga 900
ggattctaga agctgaactt gctgttgaac caaagacaga atcctatggt gacatgaata 960
tggagaactc gacaaatgac cctgttacca acatatgtca tgctgctgac aagcagcttt 1020

tcaccctcgt tgaatgggcc aagcgtattc cccacttctc tgacctcacc ttggaggacc 1080

aggtcatttt gcttcgggca gggtggaatg aattgctgat tgcctctttc tcccaccgct 1140

cagtttccgt gcaggatggc atccttctgg ccacgggttt acatgtccac cggagcagtg 1200

cccacagtgc tggggtcggc tccatctttg acagagtcct aactgagctg gtttccaaaa 1260

tgaaagacat gcagatggac aagtcggaac tgggatgcct gcgagccatt gtactcttta 1320

acccagatgc caagggcctg tccaacccct ctgaggtgga gactctgcga gagaaggttt 1380

atgccaccct tgaggcctac accaagcaga agtatccgga acagccaggc aggtttgcca 1440

agctgctgct gcgcctccca gctctgcgtt ccattggctt gaaatgcctg gagcacctct 1500

tcttcttcaa gctcatcggg gacaccccca ttgacacctt cctcatggag atgttggaga 1560

ccccgctgca gatcacctga gccccaccag ccacagcctc cccacccagg atgacccctg 1620

ggcaggtgtg tgtggacccc caccctgcac tttcctccac ctcccaccct gacccccttc 1680

ctgtccccaa aatgtgatgc ttataataaa gaaaaccttt ctacaaaaaa 1730

<210> 26

<211> 1723

<212> DNA

<213> Homo sapiens

<220>

<221> vascular endothelial growth factor

<222> (1)..(1723)

<223> Locus ID: 7422; NM_003376

<400> 26

tcgcggaggc ttggggcagc cgggtagctc ggaggtcgtg gcgctggggg ctagcaccag 60

cgctctgtcg ggaggcgcag cggttaggtg gaccggtcag cggactcacc ggccagggcg 120

ctcggtgctg gaatttgata ttcattgatc cgggttttat ccctcttctt ttttcttaaa 180

catttttttt taaaactgta ttgtttctcg ttttaattta tttttgcttg ccattcccca 240

cttgaatcgg gccgacggct tggggagatt gctctacttc cccaaatcac tgtggatttt 300

ggaaaccagc agaaagagga aagaggtagc aagagctcca gagagaagtc gaggaagaga 360

gagacggggt cagagagagc gcgcgggcgt gcgagcagcg aaagcgacag gggcaaagtg 420

agtgacctgc ttttgggggt gaccgccgga gcgcggcgtg agccctcccc cttgggatcc 480

cgcagctgac cagtcgcgct gacggacaga cagacagaca ccgcccccag ccccagctac 540

cacctcctcc ccggccggcg gcggacagtg gacgcggcgg cgagccgcgg gcaggggccg 600

gagcccgcgc ccggaggcgg ggtggagggg gtcggggctc gcggcgtcgc actgaaactt 660

ttcgtccaac ttctgggctg ttctcgcttc ggaggagccg tggtccgcgc gggggaagcc 720

gagccgagcg gagccgcgag aagtgctagc tcgggccggg aggagccgca gccggaggag 780

ggggaggagg aagaagagaa ggaagaggag agggggccgc agtggcgact cggcgctcgg 840

aagccgggct catggacggg tgaggcggcg gtgtgcgcag acagtgctcc agccgcgcgc 900

gctccccagg ccctggcccg ggcctcgggc cggggaggaa gagtagctcg ccgaggcgcc 960

gaggagagcg ggccgcccca cagcccgagc cggagaggga gcgcgagccg cgccggcccc 1020

ggtcgggcct ccgaaaccat gaactttctg ctgtcttggg tgcattggag ccttgccttg 1080

ctgctctacc tccaccatgc caagtggtcc caggctgcac ccatggcaga aggaggaggg 1140

cagaatcatc acgaagtggt gaagttcatg gatgtctatc agcgcagcta ctgccatcca 1200

atcgagaccc tggtggacat cttccaggag taccctgatg agatcgagta catcttcaag 1260

ccatcctgtg tgcccctgat gcgatgcggg ggctgctgca atgacgaggg cctggagtgt 1320

gtgcccactg aggagtccaa catcaccatg cagattatgc ggatcaaacc tcaccaaggc 1380

cagcacatag gagagatgag cttcctacag cacaacaaat gtgaatgcag accaaagaaa 1440

gatagagcaa gacaagaaaa aaaatcagtt cgaggaaagg gaaaggggca aaaacgaaag 1500

cgcaagaaat cccggtataa gtcctggagc gttccctgtg ggccttgctc agagcggaga 1560

aagcatttgt ttgtacaaga tccgcagacg tgtaaatgtt cctgcaaaaa cacagactcg 1620

cgttgcaagg cgaggcagct tgagttaaac gaacgtactt gcagatgtga caagccgagg 1680

cggtgagccg ggcaggagga aggagcctcc ctcagggttt cgg 1723

<210> 27

<211> 5480

<212> DNA

<213> Homo sapiens

<220>

<221> cannabinoid receptor 1 (brain) variant 1

<222> (1)..(5480)

<223> LocusID: 1268; NM_016083

<400> 27

gattgccccc tgtgggtcac tttctcagtc attttgagct cagcctaatc aaagactgag 60

gttatgaagt cgatcctaga tggccttgca gataccacct tccgcaccat caccactgac 120
ctcctgtacg tgggctcaaa tgacattcag tacgaagaca tcaaaggtga catggcatcc 180
aaattagggt acttcccaca gaaattccct ttaacttcct ttaggggaag tcccttccaa 240
gagaagatga ctgcgggaga caacccccag ctagtcccag cagaccaggt gaacattaca 300
gaattttaca acaagtctct ctcgtccttc aaggagaatg aggagaacat ccagtgtggg 360
gagaacttca tggacataga gtgtttcatg gtcctgaacc ccagccagca gctggccatt 420
gcagtcctgt ccctcacgct gggcaccttc acggtcctgg agaacctcct ggtgctgtgc 480
gtcatcctcc actcccgcag cctccgctgc aggccttcct accacttcat cggcagcctg 540
gcggtggcag acctcctggg gagtgtcatt tttgtctaca gcttcattga cttccacgtg 600
ttccaccgca aagatagccg caacgtgttt ctgttcaaac tgggtggggt cacggcctcc 660
ttcactgcct ccgtgggcag cctgttcctc acagccatcg acaggtacat atccattcac 720
aggcccctgg cctataagag gattgtcacc aggcccaagg ccgtggtggc gttttgcctg 780
atgtggacca tagccattgt gatcgccgtg ctgcctctcc tgggctggaa ctgcgagaaa 840
ctgcaatctg tttgctcaga cattttccca cacattgatg aaacctacct gatgttctgg 900
atcggggtca ccagcgtact gcttctgttc atcgtgtatg cgtacatgta tattctctgg 960
aaggctcaca gccacgccgt ccgcatgatt cagcgtggca cccagaagag catcatcatc 1020
cacacgtctg aggatgggaa ggtacaggtg acccggccag accaagcccg catggacatt 1080
aggttagcca agaccctggt cctgatcctg gtggtgttga tcatctgctg gggccctctg 1140
cttgcaatca tggtgtatga tgtctttggg aagatgaaca agctcattaa gacggtgttt 1200
gcattctgca gtatgctctg cctgctgaac tccaccgtga accccatcat ctatgctctg 1260
aggagtaagg acctgcgaca cgctttccgg agcatgtttc cctcttgtga aggcactgcg 1320
cagcctctgg ataacagcat gggggactcg gactgcctgc acaaacacgc aaacaatgca 1380
gccagtgttc acagggccgc agaaagctgc atcaagagca cggtcaagat tgccaaggta 1440
accatgtctg tgtccacaga cacgtctgcc gaggctctgt gagcctgatg cctccctggc 1500
agcacaggaa aagaattttt ttttttaagc tcaaaatcta gaagagtcta ttgtctcctt 1560
ggttatattt ttttaacttt accatgctca atgaaaaggt gattgtcacc atgatcactt 1620
atcagtttgc taatgtttcc atagtttagg tactcaaact ccattctcca ggggtttaca 1680
gtgaagaaag cctgttgttt aagtgactga acgatccttc aaagtctcaa tgaaatagga 1740
gggaaacctt tggctacaca attggaagtc taagaaccca tggaaaaatg ccatcaaatg 1800
aataatgcct ttgtaaccac aactttcact ataatgtgaa atgtaactgt ccgtagtatc 1860
agagatgtcc atttttacaa gttatagtac tagagatatt ttgtaaaatg tattatgtcc 1920
tgtgagatgt gtatcagtgt ttatgtgcta ttaatatttg tttagttcag caaaactgaa 1980

aggtagactt ttatgagaac aatggacaag cagtggatac gtgtcaatgt gtgcactttt 2040
tttctatatt attgcccatg atataacttt agaaataaac cttaatattt cttcaaatat 2100
ctctatttaa ttttgacact gaaataaccg taaaggttta tttttctgtt acctcaacaa 2160
gaagaatttg aagacttcaa aatattgagc agaattcatt catacttaaa aatttattag 2220
ccctgcattt tcataggaag acacattatc ttctggacta tagctgttct aatggattat 2280
aatcagaatg gaagagagaa agcatattga ctttttttga gcgacatctc tgactttctt 2340
tagtctttag ctattactgg atctcttaag acagcatgtg ttaatcttaa tgtatatcgt 2400
tatcactgtg cagttgctgt ttacttgaat agtattgtgt tcctatattc caggtttaag 2460
tagatttcat gcctgggtgg ccaaacaaca gtcttcattt tttttaattg aaaagaagta 2520
gtgtctggat cagtaaaatt atactgtgtg tgagtgtgaa tataaatgtg tgtatgtgtg 2580
tttctgtcct gtaactgtta cagtaatgtc ataaagtgag aaaactgtga ccaagtataa 2640
acttttacca cttgctgcac tcttgcacat ggattcagtt tctaaaattg agttcttcct 2700
gtaatcttgt tgataaaaat actgactcca accattcaaa aatttcaccc catccctcct 2760
taagagattg gatcaagtat tactaaattg acctttaggt attacacaag accagtgctt 2820
agcaaaaaat aatgacaggc atccaaggaa gggatgtatt tgtagtgtta ttgccaggaa 2880
aggagagtac tttggtttct gagcaccgaa tattgagcaa tatgtcagtc actaaaagga 2940
agacagttct acagaaaaac aatggtaaca tttttcaata gcgtgtgtag atagtatgca 3000
ctatatacat cacgttaaag taggactatc acacccagcc catgtggcta aaaaagctga 3060
atcagacagt ggatgagaca cacaacggca gtgaagaacc gatacacttg gcattgacgt 3120
ctagctatgc tgtatctgtg ctttgcccac atgcccttgg tgacagctga gcacccagct 3180
ctgtcttggt aggtttgggc taaggaacaa atctctcctt tgctcgtggt tagcaagata 3240
cactcaagca tgaagataaa cacagctgct ttcttcttac accccggtct catgctcctt 3300
aatggcgcca tgggtgcttg ttgggccttt ttccagtaag gaatgatatt gctgaagaat 3360
ctacttaacc ctgacaaatt ttaattataa tctcttctta tacagataaa acatgactcc 3420
tacaaggccc caaggtttac atagtctgaa gtgaagtaca gagctggcat ctatctggtg 3480
atttctagct ctcgagatac ccaagcagcc tgatggggca gttccccttc ttacggttca 3540
cgctctaagg caggatgtgg cttatgagat actttgcatt gtctgtctgc acaccttgaa 3600
tctgcctgct ggctccctta ctttacctct ctgtcatgtg cagatgaagg ctcagggtgc 3660
tagaggatta gtaagatctc tttctaaaga caggagagat tatttacaag aagaactcac 3720
cagggtttag tttgcattta agaattgcca gtcttttgtc ctgcatcatc ttgaacatta 3780
atccacatgt ttcagagctc accaggcagt accaatgctc ttttcacagc tatgaagagc 3840
tagagaaatt cttgttatgg tagaaaaatt tcacgattca tttttgaaac tgcatttgtg 3900

cgtatgcagt gtagatttta tagtgtgttg tgctttcaag atctaaatca tatataataa 3960

attaagggac aatggggctg acagcactaa acttggtgct tattgatatt ctaagaaata 4020

tctgtgaaat atcatcacgt atgttataca accttcattt aaaaaggttt aaaactagtt 4080

agattcactt tgacactttt catatcattt cttaacccaa gtgacgaaaa cattgtcccc 4140

aatgaatata ctcattagaa ttaccatttg ttaatatcac tcattaatta accccataat 4200

tagatccatt aatttaaatg atttaaattt aagtaagttt tataaggtct gacatcagag 4260

gtatcttact ttcctctgag gatgatgtac ttgccctgac catgcatttt accatcacac 4320

atgttcagaa agggccaaat tcccaacctg ctcatttttt ttttatcaga gtcatgatga 4380

atcagtccta gaatgtttca tttgcacaag tagggctgcc tccaagagga acctctgatt 4440

tattttgtat gaaatatatg tgaaaggata tgaatctgag agatgctgta gacatctgtc 4500

ctacacttga gatgatttcc aagcctctct ggcactttga gttaagtcta tctggtatta 4560

aatgccaagg accttttgct gcctaaatcc actctgcagg aaataggccc aaccaccaga 4620

tgagaattag gccctggatg agtagcgcta tagttactgt cctgttgatt aatttctgcc 4680

atttcatgtc cataaaagag accacccata tcatgcacac aattagattt ctcacactct 4740

aactgtatat ttgtatgata ttttaaaatc tcctaaatgc tgggcaatgg ctattaacaa 4800

ttaattgtct tgcactggcc ttctgatgaa atgttaacaa tgcctattgt aatatagaaa 4860

aaaacattct atctactgat ttgggctgaa tgtatgtaaa taggtttcta aaaagtcaga 4920

tgtttgagca gtggcctaca aatcagtaat tttcggatgg gagagtttct ttacattgcc 4980

gtggcatctt aaaagctatc ttcatgtaaa ttgactgtac taggcctact ggggatcaga 5040

gttcccaaga aaggaaacct tttcttgtat ctggattcaa atttatttcc aatgtttcaa 5100

gcgggaaaca tgactcttta ttgtctgtaa atctaacatt attacttttc ctcttagaag 5160

aatattgtat tgttagatgt ttgttgagct ggtaacatcg ttgcaaccac tgcaatatct 5220

tcgttagtaa tctgtataat actttgtata caagtactgg taagattgtt attaaatgta 5280

gcttcagtca ttaaattact atagcaaagt agtacttctt ctgtaatatt tacaatgtat 5340

taagcccaca gtatatttta tttcaatgta attaaactgt taacttattc aaagagaaaa 5400

catctcatca tgtctattgt ccaaagttac ctggaatcaa ataaaaattc tagattacca 5460

tgaagaacat aaaaaaaaaa 5480

<210> 28

<211> 1252

<212> DNA

<213>  Homo sapiens

<220>

<221>  cannabinoid receptor 1 (brain) variant 2

<222>  (1)..(1252)

<223>  LocusID: 1268; NM_033181

<400>  28

atgaagtcga tcctagatgg ccttgcagat accaccttcc gcaccatcac cactgacctc    60

ctgtacgtgg gctcaaatga cattcagtac gaagacatca aaggagaatg aggagaacat   120

ccagtgtggg gagaacttca tggacataga gtgtttcatg gtcctgaacc ccagccagca   180

gctggccatt gcagtcctgt ccctcacgct gggcaccttc acggtcctgg agaacctcct   240

ggtgctgtgc gtcatcctcc actcccgcag cctccgctgc aggccttcct accacttcat   300

cggcagcctg gcggtggcag acctcctggg gagtgtcatt tttgtctaca gcttcattga   360

cttccacgtg ttccaccgca aagatagccg caacgtgttt ctgttcaaac tgggtggggt   420

cacggcctcc ttcactgcct ccgtgggcag cctgttcctc acagccatcg acaggtacat   480

atccattcac aggcccctgg cctataagag gattgtcacc aggcccaagg ccgtggtggc   540

gttttgcctg atgtggacca tagccattgt gatcgccgtg ctgcctctcc tgggctggaa   600

ctgcgagaaa ctgcaatctg tttgctcaga cattttccca cacattgatg aaacctacct   660

gatgttctgg atcggggtca ccagcgtact gcttctgttc atcgtgtatg cgtacatgta   720

tattctctgg aaggctcaca gccacgccgt ccgcatgatt cagcgtggca cccagaagag   780

catcatcatc cacacgtctg aggatgggaa ggtacaggtg acccggccag accaagcccg   840

catggacatt aggttagcca agaccctggt cctgatcctg gtggtgttga tcatctgctg   900

gggccctctg cttgcaatca tggtgtatga tgtctttggg aagatgaaca agctcattaa   960

gacggtgttt gcattctgca gtatgctctg cctgctgaac tccaccgtga accccatcat  1020

ctatgctctg aggagtaagg acctgcgaca cgctttccgg agcatgtttc cctcttgtga  1080

aggcactgcg cagcctctgg ataacagcat gggggactcg gactgcctgc acaaacacgc  1140

aaacaatgca gccagtgttc acagggccgc agaaagctgc atcaagagca cggtcaagat  1200

tgccaaggta accatgtctg tgtccacaga cacgtctgcc gaggctctgt ga          1252

<210>  29

<211>  2930

<212>  DNA

<213>  Homo sapiens

<220>

<221>  cathepsin O

<222>  (1)..(2930)

<223>  LocusID: 1519; NM_001334

<400>  29

ccgaaagaga aaacaggccg cgcgggcggc agaggagccg ggcgccgcaa tggacgtgcg    60

ggcgctgccg tggctgccgt ggctgctgtg gctgctgtgc cggggcggcg gcgatgcgga   120

ctcccgcgcc cccttcaccc cgacctggcc gcggagccgc gagcgtgaag ccgccgcctt   180

ccgggaaagt cttaatagac atcgatactt gaattcttta tttcccagtg aaaactccac   240

cgccttctat ggaataaatc agttttccta tttgtttcct gaagagttta aagccattta   300

tttaagaagc aaaccttcca agtttcccag atactcagca gaagtacata tgtccatccc   360

caatgtgtct ttgccgttaa gatttgactg gagggacaag caggttgtga cacaagtgag   420

aaaccagcag atgtgtggag gatgctgggc cttcagcgtg gtgggggcag tggaatctgc   480

ttatgcaata aaggggaagc ccctggaaga cctaagtgtc cagcaggtca ttgactgttc   540

gtataataat tatggctgca atggaggctc tactctcaat gctttgaact ggttaaacaa   600

gatgcaagta aaactggtga aagattcaga atatcctttt aaagcacaaa atggtctgtg   660

ccattacttt tctggttcac attctggatt ttcaatcaaa ggttattctg catatgactt   720

cagtgaccaa gaagatgaaa tggcaaaagc acttcttacc tttggccctt tggtagtcat   780

agtagatgca gtgagctggc aagattatct gggaggcatt atacagcatc actgctctag   840

tggagaagca aatcatgcag ttctcataac tgggtttgat aaaacaggaa gcactccata   900

ttggattgtg cggaattcct ggggaagttc ttggggagta gatggttatg cccatgtcaa   960

aatgggaagt aatgtttgtg gtattgcaga ttccgtttct tctatatttg tgtgacatgt  1020

tgggcagatc aagagacagc tacaaaaatg aaggttttca taatgcaatg taacatagta  1080

cttcaaagta ttattcaact tcaagtttca gcaactacct acaaaagatt ctaaggccta  1140

gtagtattta aactaagttt cagaatgttc ccttcttgta gagagatgga caaccaaagt  1200

cagtgggaca aactccagca cagaagcctg cgaggaagcc tatggaatag tttcctgtcc  1260

tgagacgaaa ttcagattag gagatatttt aggcccctgc aactggggaa ggctactgtt  1320

tgtttttgtt tgcttattat ttatttgttt gtttattgtg agatatttca ggtgggatca  1380

aagaggtcat aagaatttat tttcttttgt ggggtgtaac tactagcttt agattacccc  1440

tatacacaag aatggccaac ctaaaattat gtgtgtcttg tacagttagt tatattagca  1500

gccctctgag atggcgtatc tatcggaagg atttcaaaca ccaattgctt tacctgaaca  1560

aatggtgctt accctttgaa cagcagagtg accacgtaga aggaaggaaa agggcaaaat 1620

cgcttcagtt aaactgaaat taaatgaaca ataaggcaac tatataagta acttctagta 1680

gcattgcctg agagacaaat tattgtttga taattttcat tgtgaatagg aatccaatag 1740

atcatattgc ttactttgtt ctttttatac tatagaataa tattttgttc tctagtatat 1800

caaaatacca aaatattatc tcatattttc tccctctttc tcttactctt taccaagttt 1860

tcctggtggc ttggcttccc tgactaaaga attaagtctc atttttactt tccatttcta 1920

ttttcttacc acttggttgg ctccctttgt ctctgtacct ttaccaacat taggatctca 1980

cctctttctt cctcccttaa ttcataagca ccactcctat caaagtccca tctcttaacc 2040

ctgggtatca aacaaactgt gagttttcca gaatctgttt cccagttttc ccctcagctt 2100

tcctggtctc ccatccgaac tgcttctttg tgcacctctt gttctttctc ttggctccca 2160

gtcttgattc ctgtgatcac tcttgcatca ctaattgcac aagtgatttc aggtgcaatt 2220

ctgattagcc tgcgtccaca cagtgatcga tgatcctatg tgcctagaaa ggacactgtg 2280

tgctgctcat gacctgcaac aggaaaaaag ccatttcttg ttagcagtgt aagaacctta 2340

gagcaaagga gttgaccttc tgattgaata taagcacaac catattaaat gaatcaatac 2400

aagaaaatta tttctgatac tatgtatgta catatttctt ctctaaaatg tatcattctt 2460

ttctaatgta tatgatctaa caaaaatgaa acatgaaatg cagtagcaac cactaaaaaa 2520

aaaaattcaa ggacatctaa ctttttctct cacttttgcc ctttgtttat ccttccctgt 2580

gattagataa acaaaataaa aaacaaaatg ctgtatttct cttcttacgc cagtcagaac 2640

caatccgaaa agaatgtgtg ttgactcagg tttggagtta tttcaggaag acagatattg 2700

accttttaat tgataaatat tcttattatc ctggaatgcc agaaaagaac tatgtcctgc 2760

ttgtctagtt tgtattcgct gactttctat gtgatataga tgcatttgta atactctttt 2820

tcaagtgcta aaggatttct aaaatttcaa actgattaat atgtttctgc tgttctggat 2880

tttgatgaca tttacaataa aacaacctac atttgacttt ggtttaaaaa 2930

<210> 30

<211> 1326

<212> DNA

<213> Homo sapiens

<220>

<221> enoyl Coenzyme A hydratase, short chain, 1 mitochondrial

<222> (1)..(1326)

<223>  LocusID: 1892; NM_004092

<400>  30

gcggagcgag ccctgaaagg ctataggcga gggggcggct ccgcggggcc gggcgaggag    60

tccagagagc catggccgcc ctgcgtgtcc tgctgtcctg cgcccgcggc ccgctgaggc   120

ccccggttcg ctgtcccgcc tggcgtccct tcgcctcggg tgctaacttt gagtacatca   180

tcgcagaaaa aagagggaag aataacaccg tggggttgat ccaactgaac cgccccaagg   240

ccctcaatgc actttgcgat ggcctgattg acgagctcaa ccaggccctg aagatcttcg   300

aggaggaccc ggccgtgggg gccattgtcc tcaccggcgg ggataaggcc tttgcagctg   360

gagctgatat caaggaaatg cagaacctga gtttccagga ctgttactcc agcaagttct   420

tgaagcactg ggaccacctc acccaggtca agaagccagt catcgctgct gtcaatggct   480

atgcctttgg cgggggctgt gagcttgcca tgatgtgtga tatcatctat gccggtgaga   540

aggcccagtt tgcacagccg gagatcttaa taggaaccat cccaggtgcg ggcggcaccc   600

agagactcac ccgtgctgtt gggaagtcgc tggcgatgga gatggtcctc accggtgacc   660

ggatctcagc ccaggacgcc aagcaagcag gtcttgtcag caagatttgt cctgttgaga   720

cactggtgga agaagccatc cagtgtgcag aaaaaattgc cagcaattct aaaattgtag   780

tagcgatggc caaagaatca gtgaatgcag cttttgaaat gacattaaca gaaggaagta   840

agttggagaa gaaactcttt tattcaacct ttgccactga tgaccggaaa gaagggatga   900

ccgcgtttgt ggaaaagaga aaggccaact tcaaagacca gtgagaacca gctgcccctg   960

cttcacacct ctgcttggag aggacaagtg cagcctgtca gttttagaag caagtaaatc  1020

atcctctttt caagagcagt gtccgtggtg tgcagttcct ctccaattgc tgcgtggtcg  1080

tggcccgacc tctcacggca tgacagcctt cgtcacccag cctgtgaggg tcctgactgg  1140

agcaccttct aaatctaaga ttctgctgag gagcccccgc tggtccctct gggcatgctg  1200

tgctcggacg gaaagcgggg cctgcgggtc cttgtgtccc tgccgctgaa gaatggggct  1260

gctctgaggg aaacgctgtc tgctgccttc atacagatgc tgattaaagt gatagcgatt  1320

cagatt                                                              1326

<210>  31

<211>  1120

<212>  DNA

<213>  Homo sapiens

<220>

<221>  vascular endothelial growth factor B

<222>  (1)..(1120)

<223>  LocusID: 7423; NM_003377

<400>  31

gcgatgcggg cgcccccggc gggcggcccc ggcgggcacc atgagccctc tgctccgccg    60

cctgctgctc gccgcactcc tgcagctggc ccccgcccag gcccctgtct cccagcctga   120

tgcccctggc caccagagga aagtggtgtc atggatagat gtgtatactc gcgctacctg   180

ccagccccgg gaggtggtgg tgcccttgac tgtggagctc atgggcaccg tggccaaaca   240

gctggtgccc agctgcgtga ctgtgcagcg ctgtggtggc tgctgccctg acgatggcct   300

ggagtgtgtg cccactgggc agcaccaagt ccggatgcag atcctcatga tccggtaccc   360

gagcagtcag ctgggggaga tgtccctgga agaacacagc cagtgtgaat gcagacctaa   420

aaaaaaggac agtgctgtga agccagacag ggctgccact ccccaccacc gtccccagcc   480

ccgttctgtt ccgggctggg actctgcccc cggagcaccc tccccagctg acatcaccca   540

tcccactcca gccccaggcc cctctgccca cgctgcaccc agcaccacca gcgccctgac   600

ccccggacct gccgctgccg ctgccgacgc cgcagcttcc tccgttgcca agggcggggc   660

ttagagctca acccagacac ctgcaggtgc cggaagctgc gaaggtgaca catggctttt   720

cagactcagc agggtgactt gcctcagagg ctatatccca gtgggggaac aaagaggagc   780

ctggtaaaaa acagccaagc ccccaagacc tcagcccagg cagaagctgc tctaggacct   840

gggcctctca gagggctctt ctgccatccc ttgtctccct gaggccatca tcaaacagga   900

cagagttgga agaggagact gggaggcagc aagaggggtc acataccagc tcaggggaga   960

atggagtact gtctcagttt ctaaccactc tgtgcaagta agcatcttac aactggctct  1020

tcctcccctc actaagaaga cccaaacctc tgcataatgg gatttgggct ttggtacaag  1080

aactgtgacc cccaaccctg ataaaagaga tggaaggaaa                        1120

<210>  32

<211>  4111

<212>  DNA

<213>  Homo sapiens

<220>

<221>  pyruvate carboxylase transcript variant A

<222>  (1)..(4111)

<223>  Locus ID: 5091; NM_000920

<400>  32

gtttgccaaa ggagtcgggc ctgacggtgg aagatgggaa atggccaaaa gaataaacca    60

tttcctctta cttaccgaat gaaaagcttg cttcatcttg gcgttcaaga ctctctaata   120

tgattccacc tgttgggctc tttcttccat ttgcctccgc agatagtgtc tgccttctgg   180

agagctgacc aaacactaag gatgctgaag ttccgaacag tccatggggg cctgaggctc   240

ctgggaatcc gccgaacctc caccgccccc gctgcctccc caaatgtccg gcgcctggag   300

tataagccca tcaagaaagt catggtggcc aacagaggtg agattgccat ccgtgtgttc   360

cgggcctgca cggagctggg catccgcacc gtagccatct actctgagca ggacacgggc   420

cagatgcacc ggcagaaagc agatgaagcc tatctcatcg gccgcggcct ggcccccgtg   480

caggcctacc tgcacatccc agacatcatc aaggtggcca aggagaacaa cgtagatgca   540

gtgcaccctg gctacgggtt cctctctgag cgagcggact tcgcccaggc ctgccaggat   600

gcaggggtcc ggtttattgg gccaagccca gaagtggtcc gcaagatggg agacaaggtg   660

gaggcccggg ccatcgccat tgctgcgggt gttcccgttg tccctggcac agatgccccc   720

atcacgtccc tgcatgaggc ccacgagttc tccaacacct acggcttccc catcatcttc   780

aaggcggcct atgggggtgg agggcgtggc atgagggtgg tgcacagcta cgaggagctg   840

gaggagaatt acacccgggc ctactcagag gctctggccg cctttgggaa tggggcgctg   900

tttgtggaga agttcatcga gaagccacgg cacatcgagg tgcagatctt gggggaccag   960

tatgggaaca tcctgcacct gtacgagcga gactgctcca tccagcggcg gcaccagaag  1020

gtggtcgaga ttgcccccgc cgcccacctg gacccgcagc ttcggactcg gctcaccagc  1080

gactctgtga aactcgctaa acaggtgggc tacgagaacg caggcaccgt ggagttcctg  1140

gtggacaggc acggcaagca ctacttcatc gaggtcaact cccgcctgca ggtggagcac  1200

acggtcacag aggagatcac cgacgtagac ctggtccatg ctcagatcca cgtgtctgag  1260

ggcaggagcc tacccgacct gggcctgcgg caggagaaca tccgcatcaa cgggtgtgcc  1320

atccagtgcc gggtcaccac cgaggacccc gcgcgcagct tccagccgga caccggccgc  1380

attgaggtgt tccggagcgg agagggcatg ggcatccgcc tggataatgc ttccgccttc  1440

caaggagccg tcatctcgcc ccactacgac tccctgctgg tcaaagtcat tgcccacggc  1500

aaagaccacc ccacggccgc caccaagatg agcagggccc ttgcggagtt ccgcgtccga  1560

ggtgtgaaga ccaacatcgc cttcctgcag aatgtgctca acaaccagca gttcctggca  1620

ggcactgtgg acacccagtt catcgacgag aacccagagc tgttccagct gcggcctgca  1680

cagaaccggg cccaaaagct gttgcactac ctcggccatg tcatggtaaa cggtccaacc  1740

accccgattc ccgtcaaggc cagccccagc cccacggacc ccgttgtccc tgcagtgccc  1800

ataggcccgc ccccggctgg tttcagagac atcctgctgc gagaggggcc tgagggcttt 1860
gctcgagctg tgcggaacca cccggggctg ctgctgatgg acacgacctt cagggacgcc 1920
caccagtcac tgctggccac tcgtgtgcgc acccacgatc tcaaaaagat cgccccctat 1980
gttgcccaca acttcagcaa gctcttcagc atggagaact ggggaggagc cacgtttgac 2040
gtcgccatgc gcttcctgta tgagtgcccc tggcggcggc tgcaggagct ccgggagctc 2100
atccccaaca tccctttcca gatgctgctg cggggggcca atgctgtggg ctacaccaac 2160
tacccagaca acgtggtctt caagttctgt gaagtggcca aagagaatgg catggatgtc 2220
ttccgtgtgt ttgactccct caactacttg cccaacatgc tgctgggcat ggaggcggca 2280
ggaagtgccg gaggcgtggt ggaggctgcc atctcataca cgggcgacgt ggccgacccc 2340
agccgcacca agtactcact gcagtactac atgggcttgg ccgaagagct ggtgcgagct 2400
ggcacccaca tcctgtgcat caaggacatg gccgggctgc tgaagcccac ggcctgcacc 2460
atgctggtca gctccctccg ggaccggttc cccgacctcc cactgcacat ccacacccac 2520
gacacgtcag gggcaggcgt ggcagccatg ctggcctgtg cccaggctgg agctgatgtg 2580
gtggatgtgg cagctgattc catgtctggg atgacttcac agcccagcat gggggccctg 2640
gtggcctgta ccagagggac tcccctggac acagaggtgc ccatggagcg cgtgtttgac 2700
tacagtgagt actgggaggg ggctcgggga ctgtacgcgg ccttcgactg cacggccacc 2760
atgaagtctg gcaactcgga cgtgtatgaa aatgagatcc cagggggcca gtacaccaac 2820
ctgcacttcc aggcccacag catggggctt ggctccaagt tcaaggaggt caagaaggcc 2880
tatgtggagg ccaaccagat gctgggcgat ctcatcaagg tgacgccctc ctccaagatc 2940
gtgggggacc tggcccagtt tatggtgcag aatggattga gccgggcaga ggccgaagct 3000
caggcggaag agctgtcctt tccccgctcc gtggtggagt tcctgcaggg ctacatcggt 3060
gtcccccatg gggggttccc cgaacccttt cgctctaagg tactgaagga cctgccaagg 3120
gtggaggggc ggcctggagc ctccctccct cccctggatc tgcaggcact ggagaaggag 3180
ctggtagacc ggcatgggga ggaggtgacg ccggaagatg tgctctcagc agctatgtac 3240
cccgatgtgt ttgcccactt caaggacttc actgccacct ttggccccct ggatagcctg 3300
aatactcgcc tcttcctgca gggacccaag atcgcagagg agtttgaggt ggagctggag 3360
cggggcaaga cgctgcacat caaagccctg gccgtgagcg acctgaaccg ggccggccag 3420
aggcaggtct tctttgagct caatgggcag ctgcggtcca tcttggtcaa ggacacccag 3480
gccatgaagg agatgcactt ccaccccaag gccctaaagg acgtgaaggg ccagatcggg 3540
gcgcccatgc ctgggaaggt gatagacatc aaagtggtgg caggggccaa ggtggccaag 3600
ggccagcccc tgtgtgtgct cagtgccatg aagatggaga ctgtggtgac ctcacccatg 3660
gagggtactg tccgcaaggt tcatgtgacc aaggacatga cactggaagg tgacgacctc 3720

atcctggaga tcgagtgatc ttgccccaga ccggcagcct ggccatcccc aagccttcaa 3780
cagaagctgt gctgccacgg caggcccagg ccagccagtg cccgaggcca ggaaggccgg 3840
gccgtggagg tcctgtccac agctggacag gagagacacc gcctgcggtg gttcattcct 3900
ttcagccatc gtcctttcct ccggcggaca gctgcttaca tgttcatctc ttgccaaata 3960
agggtcccct cctcactgga gactacaagt ggtgggtcag gtggtcctag gacccagggg 4020
aggtttaggg gtcctatctc ctgggggaag gggagatcta agatgtccca ggtcctggga 4080
agtttactca ataaagctgg ctttcccctg c 4111

<210> 33
<211> 4041
<212> DNA
<213> Homo sapiens
<220>
<221> pyruvate carboxylase transcript variant 2
<222> (1)..(4041)
<223> LocusID: 5091; NM_022172
<400> 33
agttctggaa agtcattgac aggggattaa cctagaacat caattataaa caggcagcag 60
gcctcaggcc cagacttggg tgttacagga acagtgtggc ctctctggaa ctctgcagac 120
cacctgccgt gatgctgaag ttccgaacag tccatggggg cctgaggctc ctgggaatcc 180
gccgaacctc caccgccccc gctgcctccc caaatgtccg gcgcctggag tataagccca 240
tcaagaaagt catggtggcc aacagaggtg agattgccat ccgtgtgttc cgggcctgca 300
cggagctggg catccgcacc gtagccatct actctgagca ggacacgggc cagatgcacc 360
ggcagaaagc agatgaagcc tatctcatcg gccgcggcct ggcccccgtg caggcctacc 420
tgcacatccc agacatcatc aaggtggcca aggagaacaa cgtagatgca gtgcaccctg 480
gctacgggtt cctctctgag cgagcggact tcgcccaggc ctgccaggat gcaggggtcc 540
ggtttattgg gccaagccca gaagtggtcc gcaagatggg agacaaggtg gaggcccggg 600
ccatcgccat tgctgcgggt gttcccgttg tccctggcac agatgccccc atcacgtccc 660
tgcatgaggc ccacgagttc tccaacacct acggcttccc catcatcttc aaggcggcct 720
atgggggtgg agggcgtggc atgagggtgg tgcacagcta cgaggagctg gaggagaatt 780
acacccgggc ctactcagag gctctggccg cctttgggaa tggggcgctg tttgtggaga 840

agttcatcga gaagccacgg cacatcgagg tgcagatctt gggggaccag tatgggaaca 900
tcctgcacct gtacgagcga gactgctcca tccagcggcg gcaccagaag gtggtcgaga 960
ttgcccccgc cgcccacctg gacccgcagc ttcggactcg gctcaccagc gactctgtga 1020
aactcgctaa acaggtgggc tacgagaacg caggcaccgt ggagttcctg gtggacaggc 1080
acggcaagca ctacttcatc gaggtcaact cccgcctgca ggtggagcac acggtcacag 1140
aggagatcac cgacgtagac ctggtccatg ctcagatcca cgtgtctgag ggcaggagcc 1200
tacccgacct gggcctgcgg caggagaaca tccgcatcaa cgggtgtgcc atccagtgcc 1260
gggtcaccac cgaggacccc gcgcgcagct tccagccgga caccggccgc attgaggtgt 1320
tccggagcgg agagggcatg ggcatccgcc tggataatgc ttccgccttc caaggagccg 1380
tcatctcgcc ccactacgac tccctgctgg tcaaagtcat tgcccacggc aaagaccacc 1440
ccacggccgc caccaagatg agcagggccc ttgcggagtt ccgcgtccga ggtgtgaaga 1500
ccaacatcgc cttcctgcag aatgtgctca acaaccagca gttcctggca ggcactgtgg 1560
acacccagtt catcgacgag aacccagagc tgttccagct gcggcctgca cagaaccggg 1620
cccaaaagct gttgcactac ctcggccatg tcatggtaaa cggtccaacc accccgattc 1680
ccgtcaaggc cagccccagc cccacggacc ccgttgtccc tgcagtgccc ataggcccgc 1740
ccccggctgg tttcagagac atcctgctgc gagaggggcc tgagggcttt gctcgagctg 1800
tgcggaacca cccggggctg ctgctgatgg acacgacctt cagggacgcc caccagtcac 1860
tgctggccac tcgtgtgcgc acccacgatc tcaaaaagat cgccccctat gttgcccaca 1920
acttcagcaa gctcttcagc atggagaact ggggaggagc cacgtttgac gtcgccatgc 1980
gcttcctgta tgagtgcccc tggcggcggc tgcaggagct ccgggagctc atccccaaca 2040
tccctttcca gatgctgctg cggggggcca atgctgtggg ctacaccaac tacccagaca 2100
acgtggtctt caagttctgt gaagtggcca aagagaatgg catggatgtc ttccgtgtgt 2160
ttgactccct caactacttg cccaacatgc tgctgggcat ggaggcggca ggaagtgccg 2220
gaggcgtggt ggaggctgcc atctcataca cgggcgacgt ggccgacccc agccgcacca 2280
agtactcact gcagtactac atgggcttgg ccgaagagct ggtgcgagct ggcacccaca 2340
tcctgtgcat caaggacatg gccgggctgc tgaagcccac ggcctgcacc atgctggtca 2400
gctccctccg ggaccggttc cccgacctcc cactgcacat ccacacccac gacacgtcag 2460
gggcaggcgt ggcagccatg ctggcctgtg cccaggctgg agctgatgtg gtggatgtgg 2520
cagctgattc catgtctggg atgacttcac agcccagcat gggggccctg gtggcctgta 2580
ccagagggac tcccctggac acagaggtgc ccatggagcg cgtgtttgac tacagtgagt 2640
actgggaggg ggctcgggga ctgtacgcgg ccttcgactg cacggccacc atgaagtctg 2700
gcaactcgga cgtgtatgaa aatgagatcc cagggggcca gtacaccaac ctgcacttcc 2760

aggcccacag catggggctt ggctccaagt tcaaggaggt caagaaggcc tatgtggagg 2820

ccaaccagat gctgggcgat ctcatcaagg tgacgccctc ctccaagatc gtgggggacc 2880

tggcccagtt tatggtgcag aatggattga gccgggcaga ggccgaagct caggcggaag 2940

agctgtcctt tccccgctcc gtggtggagt tcctgcaggg ctacatcggt gtcccccatg 3000

gggggttccc cgaacccttt cgctctaagg tactgaagga cctgccaagg gtggaggggc 3060

ggcctggagc ctccctccct cccctggatc tgcaggcact ggagaaggag ctggtagacc 3120

ggcatgggga ggaggtgacg ccggaagatg tgctctcagc agctatgtac cccgatgtgt 3180

ttgcccactt caaggacttc actgccacct ttggccccct ggatagcctg aatactcgcc 3240

tcttcctgca gggacccaag atcgcagagg agtttgaggt ggagctggag cggggcaaga 3300

cgctgcacat caaagccctg gccgtgagcg acctgaaccg ggccggccag aggcaggtct 3360

tctttgagct caatgggcag ctgcggtcca tcttggtcaa ggacacccag gccatgaagg 3420

agatgcactt ccaccccaag gccctaaagg acgtgaaggg ccagatcggg gcgcccatgc 3480

ctgggaaggt gatagacatc aaagtggtgg caggggccaa ggtggccaag ggccagcccc 3540

tgtgtgtgct cagtgccatg aagatggaga ctgtggtgac ctcacccatg gagggtactg 3600

tccgcaaggt tcatgtgacc aaggacatga cactggaagg tgacgacctc atcctggaga 3660

tcgagtgatc ttgccccaga ccggcagcct ggccatcccc aagccttcaa cagaagctgt 3720

gctgccacgg caggcccagg ccagccagtg cccgaggcca ggaaggccgg gccgtggagg 3780

tcctgtccac agctggacag gagagacacc gcctgcggtg gttcattcct ttcagccatc 3840

gtcctttcct ccggcggaca gctgcttaca tgttcatctc ttgccaaata agggtcccct 3900

cctcactgga gactacaagt ggtgggtcag gtggtcctag gacccagggg aggtttaggg 3960

gtcctatctc ctgggggaag gggagatcta agatgtccca ggtcctggga agtttactca 4020

ataaagctgg ctttcccctg c 4041

<210> 34

<211> 1850

<212> DNA

<213> Homo sapiens

<220>

<221> L-3-hydroxyacyl-Coenzyme A dehydrogenase, short chain

<222> (1)..(1850)

<223> LocusID: 3033; NM_005327

<400> 34

cgcccccaga gtctggcttt ccgcggctgc ccgcctcgcg cgtcttccct gcccgggtct 60

cctcgctgtc gccgccgctg ccacaccatg gccttcgtca ccaggcagtt catgcgttcc 120

gtgtcctcct cgtccaccgc ctcggcctcg gccaagaaga taatcgtcaa gcacgtgacg 180

gtcatcggcg gcgggctgat gggcgccggc attgcccagg ttgctgcagc aactggtcac 240

acagtagtgt tggtagacca gacagaggac atcctggcaa aatccaaaaa gggaattgag 300

gaaagcctta ggaaagtggc aaagaagaag tttgcagaaa accctaaggc cggcgatgaa 360

tttgtggaga agaccctgag caccatagcg accagcacgg atgcagcctc cgttgtccac 420

agcacagact tggtggtgga agccatcgtg gagaatctga aggtgaaaaa cgagctcttc 480

aaaaggctgg acaagtttgc tgctgaacat acaatctttg ccagcaacac ttcctccttg 540

catattacaa gcatagctaa tgccaccacc agacaagacc gattcgctgg cctccatttc 600

ttcaacccag tgcctgtcat gaaacttgtg gaggtcatta aaacaccaat gaccagccag 660

aagacatttg aatctttggt agactttagc aaagccctag gaaagcatcc tgtttcttgc 720

aaggacactc ctgggtttat tgtgaaccgc ctcctggttc catacctcat ggaagcaatc 780

aggctgtatg aacgaggtga cgcatccaaa gaagacattg acactgctat gaaattagga 840

gccggttacc ccatgggccc atttgagctt ctagattatg tcggactgga tactacgaag 900

ttcatcgtgg atgggtggca tgaaatggat gcagagaacc cattacatca gcccagccca 960

tccttaaata agctggtagc agagaacaag ttcggcaaga agactggaga aggattttac 1020

aaatacaagt gatgtgcagc ttctccggct ctgagaagaa cacctgagag cgctttccag 1080

ccagtgcccc gagtgcctgt gggaatgctc tttggtcaga cattccctca cacagtacag 1140

tttaataaat gtgcattttg attgtaatct atcgaagtga ttattacacc agttacagca 1200

gtaatagatt ctccattaag aaataattcc cttttttagt ctgttcattt ctgtgtattt 1260

tctaaacagc tttacaccct tggtgccttg gagcaaacat gttttttgaa ccttgtcatt 1320

tttgtgaaga attgcctaga ttccttctct catcaacggg aaagtacttc ctctgagagt 1380

gcgagtgcac catgctcact gttgctgcgt gggagagtca caagccactg gcaagcaagt 1440

ggtatagtct gtgaagcact gcagcgagca gcacctggat cttgccttta taagaacatt 1500

ttactacctg cagctttgag tcttgcccta cattttgggc atgacataag atgtgtcttt 1560

attcagctcg tcgtgaagat gctgctgctg aatgggtcag catatctctg tttgcatggt 1620

ttgcaggagg tcggttttca tggtcattca gttccacaga tctgaatgat tactgtctgt 1680

ctgtgtcttt tttccatgag aaatcactgt tgcaaattgc ctataaattg actctactaa 1740

aataacaatg tttcagtctg aaaatttgaa ttgaaaaaaa tgtataatat aaaattgtaa 1800

tacactcaaa tgattataaa agtaaaagtt ggtaatttag gcaaaaaaaa 1850

<210> 35

<211> 2300

<212> DNA

<213> Homo sapiens

<220>

<221> pyruvate dehydrogenase kinase, isoenzyme 2

<222> (1)..(2300)

<223> LocusID: 5164; NM_002611

<400> 35
   agggcaaggg tagggaggag gcggccgaac cgcgtcgctg ggccgaaagg tgcgcgagcg 60
   ctgcccgcgc ggggaccaca acccaagtcg cggccgccgc agccatgcgc tgggtgtggg 120
   cgctgctgaa gaatgcgtcc ctggcagggg cgcccaagta catagagcac ttcagcaagt 180
   tctccccgtc cccgctgtcc atgaagcagt ttctggactt cggatccagc aatgcctgtg 240
   agaaaacctc cttcaccttc ctcaggcagg agctgcctgt gcgcctggcc aacatcatga 300
   aagagatcaa cctgcttccc gaccgagtgc tgagcacacc ctccgtgcag ctggtgcaga 360
   gctggtatgt ccagagcctc ctggacatca tggagttcct ggacaaggat cccgaggacc 420
   atcgcaccct gagccagttc actgacgccc tggtcaccat ccggaaccgg cacaacgacg 480
   tggtgcccac catggcacaa ggcgtgcttg agtacaagga cacctacggc gatgaccccg 540
   tctccaacca gaacatccag tacttcctgg accgcttcta cctcagccgc atctccatcc 600
   gcatgctcat caaccagcac accctcatct ttgatggcag caccaaccca gcccatccca 660
   aacacatcgg cagcatcgac cccaactgca acgtctctga ggtggtcaaa gatgcctacg 720
   acatggctaa gctcctgtgt gacaagtatt acatggcctc acctgacctg gagatccagg 780
   agatcaatgc agccaactcc aaacagccga ttcacatggt ctacgtcccc tcccacctct 840
   accacatgct ctttgagctc ttcaagaatg ccatgagggc gactgtggaa agccatgagt 900
   ccagcctcat tctcccaccc atcaaggtca tggtggcctt gggtgaggaa gatctgtcca 960
   tcaagatgag tgaccgaggt gggggtgttc ccttgaggaa gattgagcga ctcttcagct 1020
   acatgtactc cacagcaccc accccccagc ctggcaccgg gggaacgccg ctggctggct 1080
   ttggttatgg gctccccatt tcccgcctct acgccaagta cttccaggga gacctgcagc 1140
   tcttctccat ggaaggcttt gggaccgatg ctgtcatcta tctcaaggcc ctgtccacgg 1200
   actcggtgga gcgcctgcct gtctacaaca agtcagcctg gcgccactac cagaccatcc 1260
   aggaggccgg cgactggtgt gtgcccagca cggagcccaa gaacacgtcc acgtaccgcg 1320
   tcagctaagg gccgccgtgc atctgcacct gagaggacgg actgccgcct ctgggtcccc 1380
   ccaccgtggt gcccctcacc atcctcctgg gggagcaggg ggtgggttct ccctgatgac 1440
   caggttctgt ctctatggaa gtcactgcgg tgataggtct gtgatggtcc ctaagtgcca 1500
   gtccatctct gtggagaccc ctcggtggcc tccctatctc tgtgggcgat gcctgagggt 1560
   tagggatgtc tccaccctga tggggtgtcc cagagacatt ttcccatggc agtcctcctc 1620
   tctgagacca gggctgtcac ttttctgcca ggggtactgg gtccccctca gcaccctcca 1680
   cagcacaggc cttccaagtg gatgtcccgt tgccttattc ccccagccca caaaggcacc 1740
   ctggccttgg tctgctgaag tgttaggaag agggtgggtg ccctccagac ctggggactg 1800
   agtggggaaa ggagttacac ccgtgagtgg ggaatgaggc tggtcctgca gcctctccct 1860
   ccgctcaggg cttgaaggtc ggtggcggag ggggtggctc tcacagggcc caactctaaa 1920
   gtggaagaac cttgttagac cgagagcttg ccatccagcc aagctgctcg aggccctgca 1980
   gtggccttgg caatgtctgt gccacctcct gagccctccc agcatgtcct cacatgctca 2040
   tgcccacccg ctcctccaca agcctagtcc atcctgcctg agctccagcc cccagccccc 2100
   actgtgccca gacatgtgtg ctcagggtgg ctttctccct aggaccttct gtgtatatag 2160
   ttagttttat aaccctgaat gcccccaccc ttcccctaag cacacagggg ttaaagctgt 2220
   gtgtccctcc cagtggctgt ggcagtgaca gtgacaccca cacccacagt aaagaggaga 2280
   ctgaatgaga aaaaaaaaaa 2300

<210> 36

<211> 2990

<212> DNA

<213> Homo sapiens

<220>

<221> citrate synthase transcript variant 1

<222> (1)..(2990)

<223> LocusID: 1431; NM_004077

<400> 36

agtgggcggg gcctccttga ggaccccggg ctgggcgccg ccgccggttc gtctactctt 60

tccttcagcc gcctcctttc aaccttgtca acccgtcggc gcggcctctg gtgcagcggc 120

ggcggctcct gttcctgccg cagctctctc cctttcttac ctccccacca gatcccggag 180

atcgcccgcc atggctttac ttactgcggc cgcccggctc ttgggaacca agaatgcatc 240
ttgtcttgtt cttgcagccc ggcatgccag tgcttcctcc acgaatttga aagacatatt 300
ggctgacctg atacctaagg agcaggccag aattaagact ttcaggcagc aacatggcaa 360
gacggtggtg ggccaaatca ctgtggacat gatgtatggt ggcatgagag gcatgaaggg 420
attggtctat gaaacatcag ttcttgatcc tgatgagggc atccgtttcc gaggctttag 480
tatccctgaa tgccagaaac tgctacccaa ggctaagggt ggggaagaac ccctgcctga 540
gggcttattt tggctgctgg taactggaca tatcccaaca gaggaacagg tatcttggct 600
ctcaaaagag tgggcaaaga gggcagctct gccttcccat gtggtcacca tgctggacaa 660
ctttcccacc aatctacacc ccatgtctca gctcagtgca gctgttacag ccctcaacag 720
tgaaagtaac tttgcccgag catatgcaca gggtatcagc cgaaccaagt actgggagtt 780
gatttatgaa gactctatgg atctaatcgc aaagctacct tgtgttgcag caaagatcta 840
ccgaaatctc tacagagaag gcagcggtat tggggccatt gactctaacc tggactggtc 900
tcacaatttc accaacatgt taggctatac tgatcatcag ttcactgagc tcacgcgcct 960
gtacctcacc atccacagtg accatgaggg tggcaatgta agtgcccata ccagccattt 1020
ggtgggcagt gccctttccg acccttacct gtcctttgca gcagccatga acgggctggc 1080
agggcctctc catggactgg caaatcagga agtgcttgtc tggctaacac agctgcagaa 1140
ggaagttggc aaagatgtgt cagatgagaa gttacgagac tacatctgga acacactcaa 1200
ctcaggacgg gttgttccag gctatggcca tgcagtacta aggaagactg atccgcgata 1260
tacctgtcag cgagagtttg ctctgaaaca cctgcctaat gaccccatgt ttaagttggt 1320
tgctcagctg tacaagattg tgcccaatgt cctcttagag cagggtaaag ccaagaatcc 1380
ttggcccaat gtagatgctc acagtggggt gctgctccag tattatggca tgacggagat 1440
gaattactac acggtcctgt ttggggtgtc acgagcattg ggtgtactgg cacagctcat 1500
ctggagccga gccttaggct tccctctaga aaggcccaag tccatgagca cagagggtct 1560
gatgaagttt gtggactcta agtcagggta aaactggaga ctgggtgaaa gtgactacca 1620
gaaagtgagg aagcctaaat aaaaagtata cttttgtttc agggggcctt taaagactta 1680
agattaaatt atatctgagg cactgataat atgtttgagg ttaaaatata aattaagact 1740
ttaaaagatg aaaaatggtc ccttcttccc taatcagctc ccttcccctg cctggtatga 1800
gttgcccatc atacgcatgg tcctggagga tgaccaggac taatgcatgt ggtatgagta 1860
ggtttggccc cctcactatc tctagagtga gaatctggct cctgtttcca tgggtcaaag 1920
ccggttgcag agaatctgta gtcactttgg agctttagct tctctgccaa gccctcaata 1980
agccagcaaa ccaggactct gccccttctg tttccatagg aatcatgttg gatagtcagc 2040
tgtaccaagc cccttggccc tctcccatgc acacaaacac ctcctagcaa gacctgttgg 2100

ttagctggac atgctttggc aattttttta tactaccaag tgaccataaa ggcatggcat 2160

ttgttgtgac tggcacccaa tgtttgattt tttttttaaa actatccaat taaaattaag 2220

gtctgggagt gttctgtttc ccattacttt aatactcacc tcctcccaga ctttctacac 2280

ctgttgcacc tcaggcagag gatgttctgg acctccccct cttggtccct actagagacc 2340

tctcaacaga tctgtgggcc cagtcattgg gttttatcag tgcttaatgt gaactaagtt 2400

ttttacttcc acagaataca agccactacc ttctgacctc cccacccccc accaaccccc 2460

atcttttaat atgctgtggg gcatagaact ccggaatgac cagcatgata ttttcagagt 2520

cttgtccccg gggtattagc acctcttttt gaacagggaa ttgattcaag attggacatg 2580

gtctcctctg attatcaggt actggggctg agggcattaa aaatagtaag cctccctcct 2640

cgtcccctgc ctcaagaaat tgcctcctta tttatcaaca tctttttcct ccctttccct 2700

gagagctcac agtacaatgt ttcagaagcc ccatttgcac aggttttcag caactcagaa 2760

tgctctactt ctttttcttt gagaaaggat taagatacac tcctgctgtg cccccatctt 2820

tcctccaaac tcctgcctgt gtttgtgtgg atacccagtc ccagaaccac actgttgagt 2880

tggacacact gtaaacccct gggtaactgt caagtcatga tggagacttc aggttgttct 2940

gtataaaatg caaaataaat gtttttatta acaatgaaaa aaaaaaaaaa 2990

<210> 37

<211> 3070

<212> DNA

<213> Homo sapiens

<220>

<221> citrate synthase transcript variant 2

<222> (1)..(3070)

<223> LocusID: 1431; NM_198324

<400> 37

agtgggcggg gcctccttga ggaccccggg ctgggcgccg ccgccggttc gtctactctt 60

tccttcagcc gcctcctttc aaccttgtca acccgtcggc gcggcctctg gtgcagcggc 120

ggcggctcct gttcctgccg cagctctctc cctttcttac ctccccacca gatcccggag 180

atcgcccgcc atggctttac ttactgcggc cgcccggctc ttgggaacca aggcacccag 240

tggcaagtac tagctgagca tttgggagat gcttgtctta cttggctgtt gcttctcctg 300

ctgctgggga aaaggaatgc atcttgtctt gttcttgcag cccggcatgc cagtgcttcc 360

tccacgaatt tgaaagacat attggctgac ctgataccta aggagcaggc cagaattaag 420
actttcaggc agcaacatgg caagacggtg gtgggccaaa tcactgtgga catgatgtat 480
ggtggcatga gaggcatgaa gggattggtc tatgaaacat cagttcttga tcctgatgag 540
ggcatccgtt tccgaggctt tagtatccct gaatgccaga aactgctacc caaggctaag 600
ggtggggaag aacccctgcc tgagggctta ttttggctgc tggtaactgg acatatccca 660
acagaggaac aggtatcttg gctctcaaaa gagtgggcaa agagggcagc tctgccttcc 720
catgtggtca ccatgctgga caactttccc accaatctac accccatgtc tcagctcagt 780
gcagctgtta cagccctcaa cagtgaaagt aactttgccc gagcatatgc acagggtatc 840
agccgaacca agtactggga gttgatttat gaagactcta tggatctaat cgcaaagcta 900
ccttgtgttg cagcaaagat ctaccgaaat ctctacagag aaggcagcgg tattggggcc 960
attgactcta acctggactg gtctcacaat ttcaccaaca tgttaggcta tactgatcat 1020
cagttcactg agctcacgcg cctgtacctc accatccaca gtgaccatga gggtggcaat 1080
gtaagtgccc ataccagcca tttggtgggc agtgcccttt ccgaccctta cctgtccttt 1140
gcagcagcca tgaacgggct ggcagggcct ctccatggac tggcaaatca ggaagtgctt 1200
gtctggctaa cacagctgca gaaggaagtt ggcaaagatg tgtcagatga gaagttacga 1260
gactacatct ggaacacact caactcagga cgggttgttc caggctatgg ccatgcagta 1320
ctaaggaaga ctgatccgcg atatacctgt cagcgagagt ttgctctgaa acacctgcct 1380
aatgacccca tgtttaagtt ggttgctcag ctgtacaaga ttgtgcccaa tgtcctctta 1440
gagcagggta aagccaagaa tccttggccc aatgtagatg ctcacagtgg ggtgctgctc 1500
cagtattatg gcatgacgga gatgaattac tacacggtcc tgtttggggt gtcacgagca 1560
ttgggtgtac tggcacagct catctggagc cgagccttag gcttccctct agaaaggccc 1620
aagtccatga gcacagaggg tctgatgaag tttgtggact ctaagtcagg gtaaaactgg 1680
agactgggtg aaagtgacta ccagaaagtg aggaagccta aataaaaagt atacttttgt 1740
ttcagggggc ctttaaagac ttaagattaa attatatctg aggcactgat aatatgtttg 1800
aggttaaaat ataaattaag actttaaaag atgaaaaatg gtcccttctt ccctaatcag 1860
ctcccttccc ctgcctggta tgagttgccc atcatacgca tggtcctgga ggatgaccag 1920
gactaatgca tgtggtatga gtaggtttgg ccccctcact atctctagag tgagaatctg 1980
gctcctgttt ccatgggtca aagccggttg cagagaatct gtagtcactt tggagcttta 2040
gcttctctgc caagccctca ataagccagc aaaccaggac tctgcccctt ctgtttccat 2100
aggaatcatg ttggatagtc agctgtacca agccccttgg ccctctccca tgcacacaaa 2160
cacctcctag caagacctgt tggttagctg gacatgcttt ggcaattttt ttatactacc 2220
aagtgaccat aaaggcatgg catttgttgt gactggcacc caatgtttga tttttttttt 2280

aaaactatcc aattaaaatt aaggtctggg agtgttctgt ttcccattac tttaatactc 2340

acctcctccc agactttcta cacctgttgc acctcaggca gaggatgttc tggacctccc 2400

cctcttggtc cctactagag acctctcaac agatctgtgg gcccagtcat tgggttttat 2460

cagtgcttaa tgtgaactaa gttttttact tccacagaat acaagccact accttctgac 2520

ctccccaccc cccaccaacc cccatctttt aatatgctgt ggggcataga actccggaat 2580

gaccagcatg atattttcag agtcttgtcc ccggggtatt agcacctctt tttgaacagg 2640

gaattgattc aagattggac atggtctcct ctgattatca ggtactgggg ctgagggcat 2700

taaaaatagt aagcctccct cctcgtcccc tgcctcaaga aattgcctcc ttatttatca 2760

acatcttttt cctccctttc cctgagagct cacagtacaa tgtttcagaa gccccatttg 2820

cacaggtttt cagcaactca gaatgctcta cttctttttc tttgagaaag gattaagata 2880

cactcctgct gtgcccccat ctttcctcca aactcctgcc tgtgtttgtg tggataccca 2940

gtcccagaac cacactgttg agttggacac actgtaaacc cctgggtaac tgtcaagtca 3000

tgatggagac ttcaggttgt tctgtataaa atgcaaaata aatgttttta ttaacaatga 3060

aaaaaaaaaa 3070

<210> 38

<211> 4587

<212> DNA

<213> Homo sapiens

<220>

<221> fibroblast growth factor receptor 2 transcript variant 1

<222> (1)..(4587)

<223> LocusID: 2263; NM_000141

<400> 38

gagcgggcga gggagcgcgc gcggccgcca caaagctcgg gcgccgcggg gctgcatgcg 60

gcgtacctgg cccggcgcgg cgactgctct ccgggctggc gggggccggc cgcgagcccc 120

gggggccccg aggccgcagc ttgcctgcgc gctctgagcc ttcgcaactc gcgagcaaag 180

tttggtggag gcaacgccaa gcctgagtcc tttcttcctc tcgttcccca aatccgaggg 240

cagcccgcgg gcgtcatgcc cgcgctcctc cgcagcctgg ggtacgcgtg aagcccggga 300

ggcttggcgc cggcgaagac ccaaggacca ctcttctgcg tttggagttg ctccccacaa 360

ccccgggctc gtcgctttct ccatcccgac ccacgcgggg cgcggggaca acacaggtcg 420

cggaggagcg ttgccattca agtgactgca gcagcagcgg cagcgcctcg gttcctgagc 480
ccaccgcagg ctgaaggcat tgcgcgtagt ccatgcccgt agaggaagtg tgcagatggg 540
attaacgtcc acatggagat atggaagagg accggggatt ggtaccgtaa ccatggtcag 600
ctggggtcgt ttcatctgcc tggtcgtggt caccatggca accttgtccc tggcccggcc 660
ctccttcagt ttagttgagg ataccacatt agagccagaa gagccaccaa ccaaatacca 720
aatctctcaa ccagaagtgt acgtggctgc gccaggggag tcgctagagg tgcgctgcct 780
gttgaaagat gccgccgtga tcagttggac taaggatggg gtgcacttgg ggcccaacaa 840
taggacagtg cttattgggg agtacttgca gataaagggc gccacgccta gagactccgg 900
cctctatgct tgtactgcca gtaggactgt agacagtgaa acttggtact tcatggtgaa 960
tgtcacagat gccatctcat ccggagatga tgaggatgac accgatggtg cggaagattt 1020
tgtcagtgag aacagtaaca acaagagagc accatactgg accaacacag aaaagatgga 1080
aaagcggctc catgctgtgc ctgcggccaa cactgtcaag tttcgctgcc cagccggggg 1140
gaacccaatg ccaaccatgc ggtggctgaa aaaacggaag gagtttaagc aggagcatcg 1200
cattggaggc tacaaggtac gaaaccagca ctggagcctc attatggaaa gtgtggtccc 1260
atctgacaag ggaaattata cctgtgtggt ggagaatgaa tacgggtcca tcaatcacac 1320
gtaccacctg gatgttgtgg agcgatcgcc tcaccggccc atcctccaag ccggactgcc 1380
ggcaaatgcc tccacagtgg tcggaggaga cgtagagttt gtctgcaagg tttacagtga 1440
tgcccagccc cacatccagt ggatcaagca cgtggaaaag aacggcagta aatacgggcc 1500
cgacgggctg ccctacctca aggttctcaa ggccgccggt gttaacacca cggacaaaga 1560
gattgaggtt ctctatattc ggaatgtaac ttttgaggac gctggggaat atacgtgctt 1620
ggcgggtaat tctattggga tatcctttca ctctgcatgg ttgacagttc tgccagcgcc 1680
tggaagagaa aaggagatta cagcttcccc agactacctg gagatagcca tttactgcat 1740
aggggtcttc ttaatcgcct gtatggtggt aacagtcatc ctgtgccgaa tgaagaacac 1800
gaccaagaag ccagacttca gcagccagcc ggctgtgcac aagctgacca aacgtatccc 1860
cctgcggaga caggtaacag tttcggctga gtccagctcc tccatgaact ccaacacccc 1920
gctggtgagg ataacaacac gcctctcttc aacggcagac acccccatgc tggcaggggt 1980
ctccgagtat gaacttccag aggacccaaa atgggagttt ccaagagata agctgacact 2040
gggcaagccc ctgggagaag gttgctttgg gcaagtggtc atggcggaag cagtgggaat 2100
tgacaaagac aagcccaagg aggcggtcac cgtggccgtg aagatgttga aagatgatgc 2160
cacagagaaa gacctttctg atctggtgtc agagatggag atgatgaaga tgattgggaa 2220
acacaagaat atcataaatc ttcttggagc ctgcacacag gatgggcctc tctatgtcat 2280
agttgagtat gcctctaaag gcaacctccg agaatacctc cgagcccgga ggccacccgg 2340

gatggagtac tcctatgaca ttaaccgtgt tcctgaggag cagatgacct tcaaggactt 2400
ggtgtcatgc acctaccagc tggccagagg catggagtac ttggcttccc aaaaatgtat 2460
tcatcgagat ttagcagcca gaaatgtttt ggtaacagaa aacaatgtga tgaaaatagc 2520
agactttgga ctcgccagag atatcaacaa tatagactat tacaaaaaga ccaccaatgg 2580
gcggcttcca gtcaagtgga tggctccaga agccctgttt gatagagtat acactcatca 2640
gagtgatgtc tggtccttcg gggtgttaat gtgggagatc ttcactttag ggggctcgcc 2700
ctacccaggg attcccgtgg aggaactttt taagctgctg aaggaaggac acagaatgga 2760
taagccagcc aactgcacca acgaactgta catgatgatg agggactgtt ggcatgcagt 2820
gccctcccag agaccaacgt tcaagcagtt ggtagaagac ttggatcgaa ttctcactct 2880
cacaaccaat gaggaatact tggacctcag ccaacctctc gaacagtatt cacctagtta 2940
ccctgacaca agaagttctt gttcttcagg agatgattct gtttttctcc cagaccccat 3000
gccttacgaa ccatgccttc ctcagtatcc acacataaac ggcagtgtta aaacatgaat 3060
gactgtgtct gcctgtcccc aaacaggaca gcactgggaa cctagctaca ctgagcaggg 3120
agaccatgcc tcccagagct tgttgtctcc acttgtatat atggatcaga ggagtaaata 3180
attggaaaag taatcagcat atgtgtaaag atttatacag ttgaaaactt gtaatcttcc 3240
ccaggaggag aagaaggttt ctggagcagt ggactgccac aagccaccat gtaacccctc 3300
tcacctgccg tgcgtactgg ctgtggacca gtaggactca aggtggacgt gcgttctgcc 3360
ttccttgtta attttgtaat aattggagaa gatttatgtc agcacacact tacagagcac 3420
aaatgcagta tataggtgct ggatgtatgt aaatatattc aaattatgta taaatatata 3480
ttatatattt acaaggagtt attttttgta ttgattttaa atggatgtcc caatgcacct 3540
agaaaattgg tctctctttt tttaatagct atttgctaaa tgctgttctt acacataatt 3600
tcttaatttt caccgagcag aggtggaaaa atacttttgc tttcagggaa aatggtataa 3660
cgttaattta ttaataaatt ggtaatatac aaaacaatta atcatttata gttttttttg 3720
taatttaagt ggcatttcta tgcaggcagc acagcagact agttaatcta ttgcttggac 3780
ttaactagtt atcagatcct ttgaaaagag aatatttaca atatatgact aatttgggga 3840
aaatgaagtt ttgatttatt tgtgtttaaa tgctgctgtc agacgattgt tcttagacct 3900
cctaaatgcc ccatattaaa agaactcatt cataggaagg tgtttcattt tggtgtgcaa 3960
ccctgtcatt acgtcaacgc aacgtctaac tggacttccc aagataaatg gtaccagcgt 4020
cctcttaaaa gatgccttaa tccattcctt gaggacagac cttagttgaa atgatagcag 4080
aatgtgcttc tctctggcag ctggccttct gcttctgagt tgcacattaa tcagattagc 4140
ctgattctct tcagtgaatt ttgataatgg cttccagact ctttgcgttg gagacgcctg 4200
ttaggatctt caagtcccat catagaaaat tgaaacacag agttgttctg ctgatagttt 4260

tggggatacg tccatctttt taagggattg ctttcatcta attctggcag gacctcacca 4320

aaagatccag cctcatacct acatcagaca aaatatcgcc gttgttcctt ctgtactaaa 4380

gtattgtgtt ttgctttgga aacacccact cactttgcaa tagccgtgca agatgaatgc 4440

agattacact gatcttatgt gttacaaaat tggagaaagt atttaataaa acctgttaat 4500

ttttatactg acaataaaaa tgtttctaca gatattaatg ttaacaagac aaaataaatg 4560

tcacgcaact taaaaaaaaa aaaaaaa 4587

<210> 39

<211> 4574

<212> DNA

<213> Homo sapiens

<220>

<221> fibroblast growth factor receptor 2 transcript variant 2

<222> (1)..(4574)

<223> LocusID: 2263; NM_022969

<400> 39

gagcgggcga gggagcgcgc gcggccgcca caaagctcgg gcgccgcggg gctgcatgcg 60

gcgtacctgg cccggcgcgg cgactgctct ccgggctggc gggggccggc cgcgagcccc 120

gggggccccg aggccgcagc ttgcctgcgc gctctgagcc ttcgcaactc gcgagcaaag 180

tttggtggag gcaacgccaa gcctgagtcc tttcttcctc tcgttcccca aatccgaggc 240

agcccgcggg cgtcatgccc gcgctcctcc gcagcctggg gtacgcgctg aagcccggga 300

ggcttggcgc cggcgaagac ccaaggacca ctcttctgcg tttggagttg ctccccacaa 360

ccccgggctc gtcgctttct ccatcccgac ccagccgggg cgcggggaca acacaggtcg 420

cggaggagcg ttgccattca agtgactgca gcagcagcgg cagcgcctcg gttcctgagc 480

ccaccgcagg ctgaaggcat tgcgcgtagt ccatgcccgt agaggaagtg tgcagatggg 540

attaacgtcc acatggagat atggaagagg accggggatt ggtaccgtaa ccatggtcag 600

ctggggtcgt ttcatctgcc tggtcgtggt caccatggca accttgtccc tggcccggcc 660

ctccttcagt ttagttgagg ataccacatt agagccagaa gagccaccaa ccaaatacca 720

aatctctcaa ccagaagtgt acgtggctgc gccaggggag tcgctagagg tgcgctgcct 780

gttgaaagat gccgccgtga tcagttggac taaggatggg gtgcacttgg ggcccaacaa 840

taggacagtg cttattgggg agtacttgca gataaagggc gccacgccta gagactccgg 900

cctctatgct tgtactgcca gtaggactgt agacagtgaa acttggtact tcatggtgaa 960
tgtcacagat gccatctcat ccggagatga tgaggatgac accgatggtg cggaagattt 1020
tgtcagtgag aacagtaaca acaagagagc accatactgg accaacacag aaaagatgga 1080
aaagcggctc catgctgtgc ctgcggccaa cactgtcaag tttcgctgcc cagccggggg 1140
gaacccaatg ccaaccatgc ggtggctgaa aaacgggaag gagtttaagc aggagcatcg 1200
cattggaggc tacaaggtac gaaaccagca ctggagcctc attatggaaa gtgtggtccc 1260
atctgacaag ggaaattata cctgtgtggt ggagaatgaa tacgggtcca tcaatcacac 1320
gtaccacctg gatgttgtgg agcgatcgcc tcaccggccc atcctccaag ccggactgcc 1380
ggcaaatgcc tccacagtgg tcggaggaga cgtagagttt gtctgcaagg tttacagtga 1440
tgcccagccc cacatccagt ggatcaagca cgtggaaaag aacggcagta aatacgggcc 1500
cgacgggctg ccctacctca aggttctcaa gcactcgggg ataaatagtt ccaatgcaga 1560
agtgctggct ctgttcaatg tgaccgaggc ggatgctggg gaatatatat gtaaggtctc 1620
caattatata gggcaggcca accagtctgc ctggctcact gtcctgccaa aacagcaagc 1680
gcctggaaga gaaaaggaga ttacagcttc cccagactac ctggagatag ccatttactg 1740
cataggggtc ttcttaatcg cctgtatggt ggtaacagtc atcctgtgcc gaatgaagaa 1800
cacgaccaag aagccagact tcagcagcca gccggctgtg cacaagctga ccaaacgtat 1860
ccccctgcgg agacaggtaa cagtttcggc tgagtccagc tcctccatga actccaacac 1920
cccgctggtg aggataacaa cacgcctctc ttcaacggca gacaccccca tgctggcagg 1980
ggtctccgag tatgaacttc cagaggaccc aaaatgggag tttccaagag ataagctgac 2040
actgggcaag cccctgggag aaggttgctt tgggcaagtg gtcatggcgg aagcagtggg 2100
aattgacaaa gacaagccca aggaggcggt caccgtggcc gtgaagatgt tgaaagatga 2160
tgccacagag aaagaccttt ctgatctggt gtcagagatg gagatgatga agatgattgg 2220
gaaacacaag aatatcataa atcttcttgg agcctgcaca caggatgggc ctctctatgt 2280
catagttgag tatgcctcta aaggcaacct ccgagaatac ctccgagccc ggaggccacc 2340
cgggatggag tactcctatg acattaaccg tgttcctgag gagcagatga ccttcaagga 2400
cttggtgtca tgcacctacc agctggccag aggcatggag tacttggctt cccaaaaatg 2460
tattcatcga gatttagcag ccagaaatgt tttggtaaca gaaaacaatg tgatgaaaat 2520
agcagacttt ggactcgcca gagatatcaa caatatagac tattacaaaa agaccaccaa 2580
tgggcggctt ccagtcaagt ggatggctcc agaagccctg tttgatagag tatacactca 2640
tcagagtgat gtctggtcct tcggggtgtt aatgtgggag atcttcactt tagggggctc 2700
gccctaccca gggattcccg tggaggaact ttttaagctg ctgaaggaag gacacagaat 2760
ggataagcca gccaactgca ccaacgaact gtacatgatg atgagggact gttggcatgc 2820

agtgccctcc cagagaccaa cgttcaagca gttggtagaa gacttggatc gaattctcac 2880

tctcacaacc aatgaggaat acttggacct cagccaacct ctcgaacagt attcacctag 2940

ttaccctgac acaagaagtt cttgttcttc aggagatgat tctgtttttt ctccagaccc 3000

catgccttac gaaccatgcc ttcctcagta tccacacata aacggcagtg ttaaaacatg 3060

aatgactgtg tctgcctgtc cccaaacagg acagcactgg gaacctagct acactgagca 3120

gggagaccat gcctcccaga gcttgttgtc tccacttgta tatatggatc agaggagtaa 3180

ataattggaa aagtaatcag catatgtgta aagatttata cagttgaaaa cttgtaatct 3240

tccccaggag gagaagaagg tttctggagc agtggactgc cacaagccac catgtaaccc 3300

ctctcacctg ccgtgcgtac tggctgtgga ccagtaggac tcaaggtgga cgtgcgttct 3360

gccttccttg ttaattttgt aataattgga gaagatttat gtcagcacac acttacagag 3420

cacaaatgca gtatataggt gctggatgta tgtaaatata ttcaaattat gtataaatat 3480

atattatata tttacaagga gttatttttt gtattgattt taaatggatg tcccaatgca 3540

cctagaaaat tggtctctct ttttttaata gctatttgct aaatgctgtt cttacacata 3600

atttcttaat tttcaccgag cagaggtgga aaaatacttt tgctttcagg gaaaatggta 3660

taacgttaat ttattaataa attggtaata tacaaaacaa ttaatcattt atagtttttt 3720

ttgtaattta agtggcattt ctatgcaggc agcacagcag actagttaat ctattgcttg 3780

gacttaacta gttatcagat cctttgaaaa gagaatattt acaatatatg actaatttgg 3840

ggaaaatgaa gttttgattt atttgtgttt aaatgctgct gtcagacgat tgttcttaga 3900

cctcctaaat gccccatatt aaaagaactc attcatagga aggtgtttca ttttggtgtg 3960

caaccctgtc attacgtcaa cgcaacgtct aactggactt cccaagataa atggtaccag 4020

cgtcctctta aaagatgcct taatccattc cttgaggaca gaccttagtt gaaatgatag 4080

cagaatgtgc ttctctctgg cagctggcct tctgcttctg agttgcacat taatcagatt 4140

agcctgattc tcttcagtga attttgataa tggcttccag actctttgcg ttggagacgc 4200

ctgttaggat cttcaagtcc catcatagaa aattgaaaca cagagttgtt ctgctgatag 4260

ttttggggat acgtccatct ttttaaggga ttgctttcat ctaattctgg caggacctca 4320

ccaaaagatc cagcctcata cctacatcag acaaaatatc gccgttgttc cttctgtact 4380

aaagtattgt gttttgcttt ggaaacaccc actcactttg caatagccgt gcaagatgaa 4440

tgcagattac actgatctta tgtgttacaa aattggagaa agtatttaat aaaacctgtt 4500

aatttttata ctgacaataa aaatgtttct acagatatta atgttaacaa gacaaaataa 4560

atgtcacgca actt 4574

<210> 40

<211> 4223

<212> DNA

<213> Homo sapiens

<220>

<221> fibroblast growth factor receptor 2 transcript variant 3

<222> (1)..(4223)

<223> LocusID: 2263; NM_022970

<400> 40

gagcgggcga gggagcgcgc gcggccgcca caaagctcgg gcgccgcggg gctgcatgcg 60

gcgtacctgg cccggcgcgg cgactgctct ccgggctggc gggggccggc cgcgagcccc 120

gggggccccg aggccgcagc ttgcctgcgc gctctgagcc ttcgcaactc gcgagcaaag 180

tttggtggag gcaacgccaa gcctgagtcc tttcttcctc tcgttcccca aatccgaggg 240

cagcccgcgg gcgtcatgcc cgcgctcctc cgcagcctgg ggtacgcgtg aagcccggga 300

ggcttggcgc cggcgaagac ccaaggacca ctcttctgcg tttggagttg ctccccccaa 360

ccccgggctc gtcgctttct ccatcccgac ccacgcgggg cgcggggaca acacaggtcg 420

cggaggagcg ttgccattca agtgactgca gcagcagcgg cagcgcctcg gttcctgagc 480

ccaccgcagg ctgaaggcat tgcgcgtagt ccatgcccgt agaggaagtg tgcagatggg 540

attaacgtcc acatggagat atggaagagg accggggatt ggtaccgtaa ccatggtcag 600

ctggggtcgt ttcatctgcc tggtcgtggt caccatggca accttgtccc tggcccggcc 660

ctccttcagt ttagttgagg ataccacatt agagccagaa gagccaccaa ccaaatacca 720

aatctctcaa ccagaagtgt acgtggctgc gccaggggag tcgctagagg tgcgctgcct 780

gttgaaagat gccgccgtga tcagttggac taaggatggg gtgcacttgg ggcccaacaa 840

taggacagtg cttattgggg agtacttgca gataaagggc gccacgccta gagactccgg 900

cctctatgct tgtactgcca gtaggactgt agacagtgaa acttggtact tcatggtgaa 960

tgtcacagat gccatctcat ccggagatga tgaggatgac accgatggtg cggaagattt 1020

tgtcagtgag aacagtaaca acaagagagc accatactgg accaacacag aaaagatgga 1080

aaagcggctc catgctgtgc ctgcggccaa cactgtcaag tttcgctgcc cagccggggg 1140

gaacccaatg ccaaccatgc ggtggctgaa aaacgggaag gagtttaagc aggagcatcg 1200

cattggaggc tacaaggtac gaaaccagca ctggagcctc attatggaaa gtgtggtccc 1260

atctgacaag ggaaattata cctgtgtggt ggagaatgaa tacgggtcca tcaatcacac 1320

gtaccacctg gatgttgtgg agcgatcgcc tcaccggccc atcctccaag ccggactgcc 1380

ggcaaatgcc tccacagtgg tcggaggaga cgtagagttt gtctgcaagg tttacagtga 1440

tgcccagccc cacatccagt ggatcaagca cgtggaaaag aacggcagta aatacgggcc 1500

cgacgggctg ccctacctca aggttctcaa ggtttcggct gagtccagct cctccatgaa 1560

ctccaacacc ccgctggtga ggataacaac acgcctctct tcaacggcag acacccccat 1620

gctggcaggg gtctccgagt atgaacttcc agaggaccca aaatgggagt ttccaagaga 1680

taagctgaca ctgggcaagc ccctgggaga aggttgcttt gggcaagtgg tcatggcgga 1740

agcagtggga attgacaaag acaagcccaa ggaggcggtc accgtggccg tgaagatgtt 1800

gaaagatgat gccacagaga aagacctttc tgatctggtg tcagagatgg agatgatgaa 1860

gatgattggg aaacacaaga atatcataaa tcttcttgga gcctgcacac aggatgggcc 1920

tctctatgtc atagttgagt atgcctctaa aggcaacctc cgagaatacc tccgagcccg 1980

gaggccaccc gggatggagt actcctatga cattaaccgt gttcctgagg agcagatgac 2040

cttcaaggac ttggtgtcat gcacctacca gctggccaga ggcatggagt acttggcttc 2100

ccaaaaatgt attcatcgag atttagcagc cagaaatgtt ttggtaacag aaaacaatgt 2160

gatgaaaata gcagactttg gactcgccag agatatcaac aatatagact attacaaaaa 2220

gaccaccaat gggcggcttc cagtcaagtg gatggctcca gaagccctgt ttgatagagt 2280

atacactcat cagagtgatg tctggtcctt cggggtgtta atgtgggaga tcttcacttt 2340

agggggctcg ccctacccag ggattcccgt ggaggaactt tttaagctgc tgaaggaagg 2400

acacagaatg gataagccag ccaactgcac caacgaactg tacatgatga tgagggactg 2460

ttggcatgca gtgccctccc agagaccaac gttcaagcag ttggtagaag acttggatcg 2520

aattctcact ctcacaacca atgaggaata cttggacctc agccaacctc tcgaacagta 2580

ttcacctagt taccctgaca caagaagttc ttgttcttca ggagatgatt ctgttttttc 2640

tccagacccc atgccttacg aaccatgcct tcctcagtat ccacacataa acggcagtgt 2700

taaaacatga atgactgtgt ctgcctgtcc ccaaacagga cagcactggg aacctagcta 2760

cactgagcag ggagaccatg cctcccagag cttgttgtct ccacttgtat atatggatca 2820

gaggagtaaa taattggaaa agtaatcagc atatgtgtaa agatttatac agttgaaaac 2880

ttgtaatctt ccccaggagg agaagaaggt ttctggagca gtggactgcc acaagccacc 2940

atgtaacccc tctcacctgc cgtgcgtact ggctgtggac cagtaggact caaggtggac 3000

gtgcgttctg ccttccttgt taattttgta ataattggag aagatttatg tcagcacaca 3060

cttacagagc acaaatgcag tatataggtg ctggatgtat gtaaatatat tcaaattatg 3120

tataaatata tattatatat ttacaaggag ttattttttg tattgatttt aaatggatgt 3180

cccaatgcac ctagaaaatt ggtctctctt tttttaatag ctatttgcta aatgctgttc 3240

ttacacataa tttcttaatt ttcaccgagc agaggtggaa aaatactttt gctttcaggg 3300

aaaatggtat aacgttaatt tattaataaa ttggtaatat acaaaacaat taatcattta 3360

tagttttttt tgtaatttaa gtggcatttc tatgcaggca gcacagcaga ctagttaatc 3420

tattgcttgg acttaactag ttatcagatc ctttgaaaag agaatattta caatatatga 3480

ctaatttggg gaaaatgaag ttttgattta tttgtgttta aatgctgctg tcagacgatt 3540

gttcttagac ctcctaaatg ccccatatta aaagaactca ttcataggaa ggtgtttcat 3600

tttggtgtgc aaccctgtca ttacgtcaac gcaacgtcta actggacttc ccaagataaa 3660

tggtaccagc gtcctcttaa aagatgcctt aatccattcc ttgaggacag accttagttg 3720

aaatgatagc agaatgtgct tctctctggc agctggcctt ctgcttctga gttgcacatt 3780

aatcagatta gcctgattct cttcagtgaa ttttgataat ggcttccaga ctctttgcgt 3840

tggagacgcc tgttaggatc ttcaagtccc atcatagaaa attgaaacac agagttgttc 3900

tgctgatagt tttggggata cgtccatctt tttaagggat tgctttcatc taattctggc 3960

aggacctcac caaaagatcc agcctcatac ctacatcaga caaaatatcg ccgttgttcc 4020

ttctgtacta aagtattgtg ttttgctttg gaaacaccca ctcactttgc aatagccgtg 4080

caagatgaat gcagattaca ctgatcttat gtgttacaaa attggagaaa gtatttaata 4140

aaacctgtta atttttatac tgacaataaa aatgtttcta cagatattaa tgttaacaag 4200

acaaaataaa tgtcacgcaa ctt 4223

<210> 41

<211> 1800

<212> DNA

<213> Homo sapiens

<220>

<221> fibroblast growth factor receptor 2 transcript variant 4

<222> (1)..(1800)

<223> LocusID: 2263; NM_022971

<400> 41

gagcgggcga gggagcgcgc gcggccgcca caaagctcgg gcgccgcggg gctgcatgcg 60

gcgtacctgg cccggcgcgg cgactgctct ccgggctggc gggggccggc cgcgagcccc 120

gggggccccg aggccgcagc ttgcctgcgc gctctgagcc ttcgcaactc gcgagcaaag 180
tttggtggag gcaacgccaa gcctgagtcc tttcttcctc tcgttcccca aatccgaggg 240
cagcccgcgg gcgtcatgcc cgcgctcctc cgcagcctgg ggtacgcgct gaagcccggg 300
aggcttggcg ccggcgaaga cccaaggacc actcttctgc gtttggagtt gctccccaca 360
accccgggct cgtcgctttc tccatcccga cccacgcggg gcgcggggac aacacaggtc 420
gcggaggagc gttgccattc aagtgactgc agcagcagcg gcagcgcctc ggttcctgag 480
cccaccgcag gctgaaggca ttgcgcgtag tccatgcccg tagaggaagt gtgcagatgg 540
gattaacgtc cacatggaga tatggaagag gaccggggat tggtaccgta accatggtca 600
gctggggtcg tttcatctgc ctggtcgtgg tcaccatggc aaccttgtcc ctggcccggc 660
cctccttcag tttagttgag gataccacat tagagccaga agagccacca accaaatacc 720
aaatctctca accagaagtg tacgtggctg cgccagggga gtcgctagag gtgcgctgcc 780
tgttgaaaga tgccgccgtg atcagttgga ctaaggatgg ggtgcacttg gggcccaaca 840
ataggacagt gcttattggg gagtacttgc agataaaggg cgccacgcct agagactccg 900
gcctctatgc ttgtactgcc agtaggactg tagacagtga aacttggtac ttcatggtga 960
atgtcacaga tgccatctca tccggagatg atgaggatga caccgatggt gcggaagatt 1020
ttgtcagtga gaacagtaac aacaagagag caccatactg gaccaacaca gaaaagatgg 1080
aaaagcggct ccatgctgtg cctgcggcca acactgtcaa gtttcgctgc ccagccgggg 1140
ggaacccaat gccaaccatg cggtggctga aaaacgggaa ggagtttaag caggagcatc 1200
gcattggagg ctacaaggta cgaaaccagc actggagcct cattatggaa agtgtggtcc 1260
catctgacaa gggaaattat acctgtgtgg tggagaatga atacgggtcc atcaatcaca 1320
cgtaccacct ggatgttgtg ggcagccagg gtttatgagc tttgcatgat cctcatggtt 1380
cccaagcgtc atctgtgtaa agtggacgtg gtatgaaatg tctgacattt tggaagctga 1440
gattactctg aaaatgttaa ttgggcaggt gaaaagggta cagatgtgct gtagcagacc 1500
tttggtttta aaagagaagc atcatttccc caacagggca actgtagaag gccagctgaa 1560
gagtaaagga aaaggtctga ggactgagcc tgtggctggc tggaaaaagg tgaatgttga 1620
gggcccttca cttccatcac aagaaagtca ttagacggta ccaattcagt gtctgttcct 1680
ggcatctatt tcctctgtgc aaagggaacc atgtatatga gcttataaat acatttttgt 1740
cagagtgcac agataagtag gccattttaa ttaaacattg aagaccctgt ctcaaaaaaa 1800

<210> 42

<211> 4575

<212> DNA

<213> Homo sapiens

<220>

<221> fibroblast growth factor receptor 2 transcript variant 5

<222> (1)..(4575)

<223> LocusID: 2263; NM_022972

<400> 42

gagcgggcga gggagcgcgc gcggccgcca caaagctcgg gcgccgcggg gctgcatgcg 60

gcgtacctgg cccggcgcgg cgactgctct ccgggctggc gggggccggc cgcgagcccc 120

gggggccccg aggccgcagc ttgcctgcgc gctctgagcc ttcgcaactc gcgagcaaag 180

tttggtggag gcaacgccaa gcctgagtcc tttcttcctc tcgttcccca aatccgaggg 240

cagcccgcgg gcgtcatgcc cgcgctcctc cgcagcctgg ggtacgcgct gaagcccggg 300

aggcttggcg ccggcgaaga cccaaggacc actcttctgc gtttggagtt gctccccaca 360

accccgggct cgtcgctttc tccatcccga cccagccggg gcgcggggac aacacaggtc 420

gcggaggagc gttgccattc aagtgactgc agcagcagcg gcagcgcctc ggttcctgag 480

cccaccgcag gctgaaggca ttgcgcgtag tccatgcccg tagaggaagt gtgcagatgg 540

gattaacgtc cacatggaga tatggaagag gaccggggat tggtaccgta accatggtca 600

gctggggtcg tttcatctgc ctggtcgtgg tcaccatggc aaccttgtcc ctggcccggc 660

cctccttcag tttagttgag gataccacat tagagccaga agagccacca accaaatacc 720

aaatctctca accagaagtg tacgtggctg cgccagggga gtcgctagag gtgcgctgcc 780

tgttgaaaga tgccgccgtg atcagttgga ctaaggatgg ggtgcacttg gggcccaaca 840

ataggacagt gcttattggg gagtacttgc agataaaggg cgccacgcct agagactccg 900

gcctctatgc ttgtactgcc agtaggactg tagacagtga aacttggtac ttcatggtga 960

atgtcacaga tgccatctca tccggagatg atgaggatga caccgatggt gcggaagatt 1020

ttgtcagtga gaacagtaac aacaagagag caccatactg gaccaacaca gaaaagatgg 1080

aaaagcggct ccatgctgtg cctgcggcca acactgtcaa gtttcgctgc ccagccgggg 1140

ggaacccaat gccaaccatg cggtggctga aaaacgggaa ggagtttaag caggagcatc 1200

gcattggagg ctacaaggta cgaaaccagc actggagcct cattatggaa agtgtggtcc 1260

catctgacaa gggaaattat acctgtgtgg tggagaatga atacgggtcc atcaatcaca 1320

cgtaccacct ggatgttgtg gagcgatcgc ctcaccggcc catcctccaa gccggactgc 1380
cggcaaatgc ctccacagtg gtcggaggag acgtagagtt tgtctgcaag gtttacagtg 1440
atgcccagcc ccacatccag tggatcaagc acgtggaaaa gaacggcagt aaatacgggc 1500
ccgacgggct gccctacctc aaggttctca aggttctcaa ggccgccggt gttaacacca 1560
cggacaaaga gattgaggtt ctctatattc ggaatgtaac ttttgaggac gctggggaat 1620
atacgtgctt ggcgggtaat tctattggga tatcctttca ctctgcatgg ttgacagttc 1680
tgccagcgcc tggaagagaa aaggagatta cagcttcccc agactacctg gagatagcca 1740
tttactgcat aggggtcttc ttaatcgcct gtatggtggt aacagtcatc ctgtgccgaa 1800
tgaagaacac gaccaagaag ccagacttca gcagccagcc ggctgtgcac aagctgacca 1860
aacgtatccc cctgcggaga caggtttcgg ctgagtccag ctcctccatg aactccaaca 1920
ccccgctggt gaggataaca acacgcctct cttcaacggc agacaccccc atgctggcag 1980
gggtctccga gtatgaactt ccagaggacc caaaatggga gtttccaaga gataagctga 2040
cactgggcaa gcccctggga gaaggttgct ttgggcaagt ggtcatggcg gaagcagtgg 2100
gaattgacaa agacaagccc aaggaggcgg tcaccgtggc cgtgaagatg ttgaaagatg 2160
atgccacaga gaaagacctt tctgatctgg tgtcagagat ggagatgatg aagatgattg 2220
ggaaacacaa gaatatcata aatcttcttg gagcctgcac acaggatggg cctctctatg 2280
tcatagttga gtatgcctct aaaggcaacc tccgagaata cctccgagcc cggaggccac 2340
ccgggatgga gtactcctat gacattaacc gtgttcctga ggagcagatg accttcaagg 2400
acttggtgtc atgcacctac cagctggcca gaggcatgga gtacttggct tcccaaaaat 2460
gtattcatcg agatttagca gccagaaatg ttttggtaac agaaaacaat gtgatgaaaa 2520
tagcagactt tggactcgcc agagatatca acaatataga ctattacaaa aagaccacca 2580
atgggcggct tccagtcaag tggatggctc cagaagccct gtttgataga gtatacactc 2640
atcagagtga tgtctggtcc ttcggggtgt taatgtggga gatcttcact ttagggggct 2700
cgccctaccc agggattccc gtggaggaac tttttaagct gctgaaggaa ggacacagaa 2760
tggataagcc agccaactgc accaacgaac tgtacatgat gatgagggac tgttggcatg 2820
cagtgccctc ccagagacca acgttcaagc agttggtaga agacttggat cgaattctca 2880
ctctcacaac caatgaggaa tacttggacc tcagccaacc tctcgaacag tattcaccta 2940
gttaccctga cacaagaagt tcttgttctt caggagatga ttctgttttt tctccagacc 3000
ccatgcctta cgaaccatgc cttcctcagt atccacacat aaacggcagt gttaaaacat 3060
gaatgactgt gtctgcctgt ccccaaacag gacagcactg ggaacctagc tacactgagc 3120
agggagacca tgcctcccag agcttgttgt ctccacttgt atatatggat cagaggagta 3180

aataattgga aaagtaatca gcatatgtgt aaagatttat acagttgaaa acttgtaatc 3240

ttccccagga ggagaagaag gtttctggag cagtggactg ccacaagcca ccatgtaacc 3300

cctctcacct gccgtgcgta ctggctgtgg accagtagga ctcaaggtgg acgtgcgttc 3360

tgccttcctt gttaattttg taataattgg agaagattta tgtcagcaca cacttacaga 3420

gcacaaatgc agtatatagg tgctggatgt atgtaaatat attcaaatta tgtataaata 3480

tatattatat atttacaagg agttattttt tgtattgatt ttaaatggat gtcccaatgc 3540

acctagaaaa ttggtctctc tttttttaat agctatttgc taaatgctgt tcttacacat 3600

aatttcttaa ttttcaccga gcagaggtgg aaaaatactt ttgctttcag ggaaaatggt 3660

ataacgttaa tttattaata aattggtaat atacaaaaca attaatcatt tatagttttt 3720

tttgtaattt aagtggcatt tctatgcagg cagcacagca gactagttaa tctattgctt 3780

ggacttaact agttatcaga tcctttgaaa agagaatatt tacaatatat gactaatttg 3840

gggaaaatga agttttgatt tatttgtgtt taaatgctgc tgtcagacga ttgttcttag 3900

acctcctaaa tgccccatat taaaagaact cattcatagg aaggtgtttc attttggtgt 3960

gcaaccctgt cattacgtca acgcaacgtc taactggact tcccaagata aatggtacca 4020

gcgtcctctt aaaagatgcc ttaatccatt ccttgaggac agaccttagt tgaaatgata 4080

gcagaatgtg cttctctctg gcagctggcc ttctgcttct gagttgcaca ttaatcagat 4140

tagcctgatt ctcttcagtg aattttgata atggcttcca gactctttgc gttggagacg 4200

cctgttagga tcttcaagtc ccatcataga aaattgaaac acagagttgt tctgctgata 4260

gttttgggga tacgtccatc tttttaaggg attgctttca tctaattctg gcaggacctc 4320

accaaaagat ccagcctcat acctacatca gacaaaatat cgccgttgtt ccttctgtac 4380

taaagtattg tgttttgctt tggaaacacc cactcacttt gcaatagccg tgcaagatga 4440

atgcagatta cactgatctt atgtgttaca aaattggaga aagtatttaa taaaacctgt 4500

taatttttat actgacaata aaaatgtttc tacagatatt aatgttaaca agacaaaata 4560

aatgtcacgc aactt 4575

<210> 43

<211> 3216

<212> DNA

<213> Homo sapiens

<220>

<221>  fibroblast growth factor receptor 2 transcript variant 6

<222>  (1)..(3216)

<223>  LocusID: 2263; NM_022973

<400>  43

gagcgggcga gggagcgcgc gcggccgcca caaagctcgg gcgccgcggg gctgcatgcg    60

gcgtacctgg cccggcgcgg cgactgctct ccgggctggc gggggccggc cgcgagcccc   120

gggggccccg aggccgcagc ttgcctgcgc gctctgagcc ttcgcaactc gcgagcaaag   180

tttggtggag gcaacgccaa gcctgagtcc tttcttcctc tcgttcccca aatccgaggg   240

cagcccgcgg gcgtcatgcc cgcgctcctc cgcagcctgg ggtacgcgct gaagcccggg   300

aggcttggcg ccggcgaaga cccaaggacc actcttctgc gtttggagtt gctccccaca   360

accccgggct cgtcgctttc tccatcccga cccagccggg gcgcggggac aacacaggtc   420

gcggaggagc gttgccattc aagtgactgc agcagcagcg gcagcgcctc ggttcctgag   480

cccaccgcag gctgaaggca ttgcgcgtag tccatgcccg tagaggaagt gtgcagatgg   540

gattaacgtc cacatggaga tatggaagag gaccggggat tggtaccgta accatggtca   600

gctggggtcg tttcatctgc ctggtcgtgg tcaccatggc aaccttgtcc ctggcccggc   660

cctccttcag tttagttgag gataccacat tagagccaga agagccacca accaaatacc   720

aaatctctca accagaagtg tacgtggctg cgccagggga gtcgctagag gtgcgctgcc   780

tgttgaaaga tgccgccgtg atcagttgga ctaaggatgg ggtgcacttg gggcccaaca   840

ataggacagt gcttattggg gagtacttgc agataaaggg cgccacgcct agagactccg   900

gcctctatgc ttgtactgcc agtaggactg tagacagtga aacttggtac ttcatggtga   960

atgtcacaga tgccatctca tccggagatg atgaggatga caccgatggt gcggaagatt  1020

ttgtcagtga gaacagtaac aacaagagag caccatactg gaccaacaca gaaaagatgg  1080

aaaagcggct ccatgctgtg cctgcggcca acactgtcaa gtttcgctgc ccagccgggg  1140

ggaacccaat gccaaccatg cggtggctga aaaacgggaa ggagtttaag caggagcatc  1200

gcattggagg ctacaaggta cgaaaccagc actggagcct cattatggaa agtgtggtcc  1260

catctgacaa gggaaattat acctgtgtgg tggagaatga atacgggtcc atcaatcaca  1320

cgtaccacct ggatgttgtg gagcgatcgc ctcaccggcc catcctccaa gccggactgc  1380

cggcaaatgc ctccacagtg gtcggaggag acgtagagtt tgtctgcaag gtttacagtg  1440

atgcccagcc ccacatccag tggatcaagc acgtggaaaa gaacggcagt aaatacgggc  1500

ccgacgggct gccctacctc aaggttctca aggccgccgg tgttaacacc acggacaaag  1560

agattgaggt tctctatatt cggaatgtaa cttttgagga cgctggggaa tatacgtgct 1620

tggcgggtaa ttctattggg atatcctttc actctgcatg gttgacagtt ctgccagcgc 1680

ctggaagaga aaaggagatt acagcttccc cagactacct ggagatagcc atttactgca 1740

taggggtctt cttaatcgcc tgtatggtgg taacagtcat cctgtgccga atgaagaaca 1800

cgaccaagaa gccagacttc agcagccagc cggctgtgca caagctgacc aaacgtatcc 1860

ccctgcggag acaggtaaca gtttcggctg agtccagctc ctccatgaac tccaacaccc 1920

cgctggtgag gataacaaca cgcctctctt caacggcaga cacccccatg ctggcagggg 1980

tctccgagta tgaacttcca gaggacccaa aatgggagtt tccaagagat aagctgacac 2040

tgggcaagcc cctgggagaa ggttgctttg ggcaagtggt catggcggaa gcagtgggaa 2100

ttgacaaaga caagcccaag gaggcggtca ccgtggccgt gaagatgttg aaagatgatg 2160

ccacagagaa agacctttct gatctggtgt cagagatgga gatgatgaag atgattggga 2220

aacacaagaa tatcataaat cttcttggag cctgcacaca ggatgggcct ctctatgtca 2280

tagttgagta tgcctctaaa ggcaacctcc gagaatacct ccgagcccgg aggccacccg 2340

ggatggagta ctcctatgac attaaccgtg ttcctgagga gcagatgacc ttcaaggact 2400

tggtgtcatg cacctaccag ctggccagag gcatggagta cttggcttcc caaaaatgta 2460

ttcatcgaga tttagcagcc agaaatgttt tggtaacaga aaacaatgtg atgaaaatag 2520

cagactttgg actcgccaga gatatcaaca atatagacta ttacaaaaag accaccaatg 2580

ggcggcttcc agtcaagtgg atggctccag aagccctgtt tgatagagta tacactcatc 2640

agagtgatgt ctggtccttc ggggtgttaa tgtgggagat cttcacttta gggggctcgc 2700

cctacccagg gattcccgtg gaggaacttt ttaagctgct gaaggaagga cacagaatgg 2760

ataagccagc caactgcacc aacgaactgt acatgatgat gagggactgt tggcatgcag 2820

tgccctccca gagaccaacg ttcaagcagt tggtagaaga cttggatcga attctcactc 2880

tcacaaccaa tgagatctga aagtttatgg cttcattgag aaactgggaa aagttggtca 2940

ggcgcagtgg ctcatgcctg taatcccagc actttgggag gccgaggcag gcggatcatg 3000

aggtcaggag ttccagacca gcctggccaa catggtgaaa ccctgtctct actaaagata 3060

caaaaaatta gccgggcgtg ttggtgtgcg cctgtaatcc cagctactcc gggaggctga 3120

ggcaggagag tcacttgaac cggggaggcg gaggttacag tgagccgaga tcatgccatt 3180

gcattccagc cttggcgaca gagcgagact ccatct 3216

<210> 44

<211> 3219

<212>  DNA

<213>  Homo sapiens

<220>

<221>  fibroblast growth factor receptor 2 transcript variant 7

<222>  (1)..(3219)

<223>  LocusID: 2263; NM_022974

<400>  44

gagcgggcga gggagcgcgc gcggccgcca caaagctcgg gcgccgcggg gctgcatgcg    60

gcgtacctgg cccggcgcgg cgactgctct ccgggctggc gggggccggc cgcgagcccc   120

gggggccccg aggccgcagc ttgcctgcgc gctctgagcc ttcgcaactc gcgagcaaag   180

tttggtggag gcaacgccaa gcctgagtcc tttcttcctc tcgttcccca aatccgaggg   240

cagcccgcgg gcgtcatgcc cgcgctcctc cgcagcctgg ggtacgcgct gaagcccggg   300

aggcttggcg ccggcgaaga cccaaggacc actcttctgc gtttggagtt gctccccaca   360

accccgggct cgtcgctttc tccatcccga cccagccggg gcgcggggac aacacaggtc   420

gcggaggagc gttgccattc aagtgactgc agcagcagcg gcagcgcctc ggttcctgag   480

cccaccgcag gctgaaggca ttgcgcgtag tccatgcccg tagaggaagt gtgcagatgg   540

gattaacgtc cacatggaga tatggaagag gaccggggat tggtaccgta accatggtca   600

gctggggtcg tttcatctgc ctggtcgtgg tcaccatggc aaccttgtcc ctggcccggc   660

cctccttcag tttagttgag gataccacat tagagccaga agagccacca accaaatacc   720

aaatctctca accagaagtg tacgtggctg cgccagggga gtcgctagag gtgcgctgcc   780

tgttgaaaga tgccgccgtg atcagttgga ctaaggatgg ggtgcacttg gggcccaaca   840

ataggacagt gcttattggg gagtacttgc agataaaggg cgccacgcct agagactccg   900

gcctctatgc ttgtactgcc agtaggactg tagacagtga aacttggtac ttcatggtga   960

atgtcacaga tgccatctca tccggagatg atgaggatga caccgatggt gcggaagatt  1020

ttgtcagtga gaacagtaac aacaagagag caccatactg gaccaacaca gaaaagatgg  1080

aaaagcggct ccatgctgtg cctgcggcca acactgtcaa gtttcgctgc ccagccgggg  1140

ggaacccaat gccaaccatg cggtggctga aaaacgggaa ggagtttaag caggagcatc  1200

gcattggagg ctacaaggta cgaaaccagc actggagcct cattatggaa agtgtggtcc  1260

catctgacaa gggaaattat acctgtgtgg tggagaatga atacgggtcc atcaatcaca  1320

cgtaccacct ggatgttgtg gagcgatcgc ctcaccggcc catcctccaa gccggactgc  1380

cggcaaatgc ctccacagtg gtcggaggag acgtagagtt tgtctgcaag gtttacagtg  1440

atgcccagcc ccacatccag tggatcaagc acgtggaaaa gaacggcagt aaatacgggc 1500

ccgacgggct gccctacctc aaggttctca agcactcggg gataaatagt tccaatgcag 1560

aagtgctggc tctgttcaat gtgaccgagg cggatgcggg ggaatatata tgtaaggtct 1620

ccaattatat agggcaggcc aaccagtctg cctggctcac tgtcctgcca aaacagcaag 1680

cgcctggaag agaaaaggag attacagctt ccccagacta cctggagata gccatttact 1740

gcataggggt cttcttaatc gcctgtatgg tggtaacagt catcctgtgc cgaatgaaga 1800

acacgaccaa gaagccagac ttcagcagcc agccggctgt gcacaagctg accaaacgta 1860

tccccctgcg gagacaggta acagtttcgg ctgagtccag ctcctccatg aactccaaca 1920

ccccgctggt gaggataaca acacgcctct cttcaacggc agacaccccc atgctggcag 1980

gggtctccga gtatgaactt ccagaggacc caaaatggga gtttccaaga gataagctga 2040

cactgggcaa gcccctggga gaaggttgct ttgggcaagt ggtcatggcg gaagcagtgg 2100

gaattgacaa agacaagccc aaggaggcgg tcaccgtggc cgtgaagatg ttgaaagatg 2160

atgccacaga gaaagacctt tctgatctgg tgtcagagat ggagatgatg aagatgattg 2220

ggaaacacaa gaatatcata aatcttcttg gagcctgcac acaggatggg cctctctatg 2280

tcatagttga gtatgcctct aaaggcaacc tccgagaata cctccgagcc cggaggccac 2340

ccgggatgga gtactcctat gacattaacc gtgttcctga ggagcagatg accttcaagg 2400

acttggtgtc atgcacctac cagctggcca gaggcatgga gtacttggct tcccaaaaat 2460

gtattcatcg agatttagca gccagaaatg ttttggtaac agaaaacaat gtgatgaaaa 2520

tagcagactt tggactcgcc agagatatca acaatataga ctattacaaa aagaccacca 2580

atgggcggct tccagtcaag tggatggctc cagaagccct gtttgataga gtatacactc 2640

atcagagtga tgtctggtcc ttcggggtgt taatgtggga gatcttcact ttagggggct 2700

cgccctaccc agggattccc gtggaggaac tttttaagct gctgaaggaa ggacacagaa 2760

tggataagcc agccaactgc accaacgaac tgtacatgat gatgagggac tgttggcatg 2820

cagtgccctc ccagagacca acgttcaagc agttggtaga agacttggat cgaattctca 2880

ctctcacaac caatgagatc tgaaagttta tggcttcatt gagaaactgg gaaaagttgg 2940

tcaggcgcag tggctcatgc ctgtaatccc agcactttgg gaggccgagg caggcggatc 3000

atgaggtcag gagttccaga ccagcctggc caacatggtg aaaccctgtc tctactaaag 3060

atacaaaaaa ttagccgggc gtgttggtgt gcgcctgtaa tcccagctac tccgggaggc 3120

tgaggcagga gagtcacttg aaccggggag gcggaggtta cagtgagccg agatcatgcc 3180

attgcattcc agccttggcg acagagcgag actccatct 3219

<210> 45

<211> 4310

<212> DNA

<213> Homo sapiens

<220>

<221> fibroblast growth factor receptor 2 transcript variant 8

<222> (1)..(4310)

<223> LocusID: 2263; NM_022975

<400> 45

gagcgggcga gggagcgcgc gcggccgcca caaagctcgg gcgccgcggg gctgcatgcg 60

gcgtacctgg cccggcgcgg cgactgctct ccgggctggc gggggccggc cgcgagcccc 120

gggggccccg aggccgcagc ttgcctgcgc gctctgagcc ttcgcaactc gcgagcaaag 180

tttggtggag gcaacgccaa gcctgagtcc tttcttcctc tcgttcccca aatccgaggg 240

cagcccgcgg gcgtcatgcc cgcgctcctc cgcagcctgg ggtacgcgtg aagcccggga 300

ggcttggcgc cggcgaagac ccaaggacca ctcttctgcg tttggagttg ctccccacaa 360

ccccgggctc gtcgctttct ccatcccgac ccacgcgggg cgcggggaca acacaggtcg 420

cggaggagcg ttgccattca agtgactgca gcagcagcgg cagcgcctcg gttcctgagc 480

ccaccgcagg ctgaaggcat tgcgcgtagt ccatgcccgt agaggaagtg tgcagatggg 540

attaacgtcc acatggagat atggaagagg accggggatt ggtaccgtaa ccatggtcag 600

ctggggtcgt ttcatctgcc tggtcgtggt caccatggca accttgtccc tggcccggcc 660

ctccttcagt ttagttgagg ataccacatt agagccagaa gatgccatct catccggaga 720

tgatgaggat gacaccgatg gtgcggaaga ttttgtcagt gagaacagta acaacaagag 780

agcaccatac tggaccaaca cagaaaagat ggaaaagcgg ctccatgctg tgcctgcggc 840

caacactgtc aagtttcgct gcccagccgg ggggaaccca atgccaacca tgcggtggct 900

gaaaaacggg aaggagttta agcaggagca tcgcattgga ggctacaagg tacgaaacca 960

gcactggagc ctcattatgg aaagtgtggt cccatctgac aagggaaatt atacctgtgt 1020

ggtggagaat gaatacgggt ccatcaatca cacgtaccac ctggatgttg tggagcgatc 1080

gcctcaccgg cccatcctcc aagccggact gccggcaaat gcctccacag tggtcggagg 1140

agacgtagag tttgtctgca aggtttacag tgatgcccag ccccacatcc agtggatcaa 1200

gcacgtggaa aagaacggca gtaaatacgg gcccgacggg ctgccctacc tcaaggttct 1260

caagcactcg gggataaata gttccaatgc agaagtgctg gctctgttca atgtgaccga 1320

ggcggatgct ggggaatata tatgtaaggt ctccaattat atagggcagg ccaaccagtc 1380
tgcctggctc actgtcctgc caaaacagca agcgcctgga agagaaaagg agattacagc 1440
ttccccagac tacctggaga tagccattta ctgcataggg gtcttcttaa tcgcctgtat 1500
ggtggtaaca gtcatcctgt gccgaatgaa gaacacgacc aagaagccag acttcagcag 1560
ccagccggct gtgcacaagc tgaccaaacg tatccccctg cggagacagg tttcggctga 1620
gtccagctcc tccatgaact ccaacacccc gctggtgagg ataacaacac gcctctcttc 1680
aacggcagac acccccatgc tggcaggggt ctccgagtat gaacttccag aggacccaaa 1740
atgggagttt ccaagagata agctgacact gggcaagccc ctgggagaag gttgctttgg 1800
gcaagtggtc atggcggaag cagtgggaat tgacaaagac aagcccaagg aggcggtcac 1860
cgtggccgtg aagatgttga aagatgatgc cacagagaaa gacctttctg atctggtgtc 1920
agagatggag atgatgaaga tgattgggaa acacaagaat atcataaatc ttcttggagc 1980
ctgcacacag gatgggcctc tctatgtcat agttgagtat gcctctaaag gcaacctccg 2040
agaatacctc cgagcccgga ggccacccgg gatggagtac tcctatgaca ttaaccgtgt 2100
tcctgaggag cagatgacct tcaaggactt ggtgtcatgc acctaccagc tggccagagg 2160
catggagtac ttggcttccc aaaaatgtat tcatcgagat ttagcagcca gaaatgtttt 2220
ggtaacagaa aacaatgtga tgaaaatagc agactttgga ctcgccagag atatcaacaa 2280
tatagactat tacaaaaaga ccaccaatgg gcggcttcca gtcaagtgga tggctccaga 2340
agccctgttt gatagagtat acactcatca gagtgatgtc tggtccttcg gggtgttaat 2400
gtgggagatc ttcactttag gggctcgccc ctacccaggg attcccgtgg aggaactttt 2460
taagctgctg aaggaaggac acagaatgga taagccagcc aactgcacca acgaactgta 2520
catgatgatg agggactgtt ggcatgcagt gccctcccag agaccaacgt tcaagcagtt 2580
ggtagaagac ttggatcgaa ttctcactct cacaaccaat gaggaatact tggacctcag 2640
ccaacctctc gaacagtatt cacctagtta ccctgacaca agaagttctt gttcttcagg 2700
agatgattct gttttttctc cagaccccat gccttacgaa ccatgccttc ctcagtatcc 2760
acacataaac ggcagtgtta aaacatgaat gactgtgtct gcctgtcccc aaacaggaca 2820
gcactgggaa cctagctaca ctgagcaggg agaccatgcc tcccagagct tgttgtctcc 2880
acttgtatat atggatcaga ggagtaaata attggaaaag taatcagcat atgtgtaaag 2940
atttatacag ttgaaaactt gtaatcttcc ccaggaggag aagaaggttt ctggagcagt 3000
ggactgccac aagccaccat gtaacccctc tcacctgccg tgcgtactgg ctgtggacca 3060
gtaggactca aggtggacgt gcgttctgcc ttccttgtta attttgtaat aattggagaa 3120
gatttatgtc agcacacact tacagagcac aaatgcagta tataggtgct ggatgtatgt 3180

aaatatattc aaattatgta taaatatata ttatatattt acaaggagtt attttttgta 3240

ttgattttaa atggatgtcc caatgcacct agaaaattgg tctctctttt tttaatagct 3300

atttgctaaa tgctgttctt acacataatt tcttaatttt caccgagcag aggtggaaaa 3360

atacttttgc tttcagggaa aatggtataa cgttaattta ttaataaatt ggtaatatac 3420

aaaacaatta atcatttata gttttttttg taatttaagt ggcatttcta tgcaggcagc 3480

acagcagact agttaatcta ttgcttggac ttaactagtt atcagatcct ttgaaaagag 3540

aatatttaca atatatgact aatttgggga aaatgaagtt ttgatttatt tgtgtttaaa 3600

tgctgctgtc agacgattgt tcttagacct cctaaatgcc ccatattaaa agaactcatt 3660

cataggaagg tgtttcattt tggtgtgcaa ccctgtcatt acgtcaacgc aacgtctaac 3720

tggacttccc aagataaatg gtaccagcgt cctcttaaaa gatgccttaa tccattcctt 3780

gaggacagac cttagttgaa atgatagcag aatgtgcttc tctctggcag ctggccttct 3840

gcttctgagt tgcacattaa tcagattagc ctgtattctc ttcagtgaat tttgataatg 3900

gcttccagac tctttggcgt tggagacgcc tgttaggatc ttcaagtccc atcatagaaa 3960

attgaaacac agagttgttc tgctgatagt tttggggata cgtccatctt tttaagggat 4020

tgctttcatc taattctggc aggacctcac caaaagatcc agcctcatac ctacatcaga 4080

caaaatatcg ccgttgttcc ttctgtacta aagtattgtg ttttgctttg gaaacaccca 4140

ctcactttgc aatagccgtg caagatgaat gcagattaca ctgatcttat gtgttacaaa 4200

attggagaaa gtatttaata aaacctgtta atttttatac tgacaataaa aatgtttcta 4260

cagatattaa tgttaacaag acaaaataaa tgtcacgcaa cttaaaaaaa 4310

<210> 46

<211> 1807

<212> DNA

<213> Homo sapiens

<220>

<221> fibroblast growth factor receptor 2 transcript variant 9

<222> (1)..(1807)

<223> LocusID: 2263; NM_022976

<400> 46

gagcgggcga gggagcgcgc gcggccgcca caaagctcgg gcgccgcggg gctgcatgcg 60

gcgtacctgg cccggcgcgg cgactgctct ccgggctggc gggggccggc cgcgagcccc 120

gggggccccg aggccgcagc ttgcctgcgc gctctgagcc ttcgcaactc gcgagcaaag 180

tttggtggag gcaacgccaa gcctgagtcc tttcttcctc tcgttcccca aatccgaggg 240

cagcccgcgg gcgtcatgcc cgcgctcctc cgcagcctgg ggtacgcgct gaagcccggg 300

aggcttggcg ccggcgaaga cccaaggacc actcttctgc gtttggagtt gctccccgca 360

accccgggct cgtcgctttc tccatcccga cccacgcggg gccggggaca acacaggtcg 420

cggaggagcg ttgccattca agtgactgca gcagcagcgc agcgcctcgg ttcctgagcc 480

caccgcagct gaaggcattg cgcgtagtcc atgcccgtag aggaagtgtg cagatgggat 540

taacgtccac atggagatat ggaagaggac cggggattgg taccgtaacc atggtcagct 600

ggggtcgttt catctgcctg gtcgtggtca ccatggcaac cttgtccctg gcccggccct 660

ccttcagttt agttgaggat accacattag agccagaaga gccaccaacc aaataccaaa 720

tctctcaacc agaagtgtac gtggctgcgc caggggagtc gctagaggtg cgctgcctgt 780

tgaaagatgc cgccgtgatc agttggacta aggatggggt gcacttgggg cccaacaata 840

ggacagtgct tattggggag tacttgcaga taaagggcgc cacacctaga gactccggcc 900

tctatgcttg tactgccagt aggactgtag acagtgaaac ttggtacttc atggtgaatg 960

tcacagatgc catctcatcc ggagatgatg aggatgacac cgatggtgcg gaagattttg 1020

tcagtgagaa cagtaacaac aagagagcac catactggac caacacagaa aagatggaaa 1080

agcggctcca tgctgtgcct gcggccaaca ctgtcaagtt tcgctgccca gccgggggga 1140

acccaatgcc aaccatgcgg tggctgaaaa acgggaagga gtttaagcag gagcatcgca 1200

ttggaggcta caaggtacga aaccagcact ggagcctcat tatggaaagt gtggtcccat 1260

ctgacaaggg aaattatacc tgtgtagtgg agaatgaata cgggtccatc aatcacacgt 1320

accacctgga tgttgtggag cgatcgcctc accggcccat cctccaagcc ggactgccgg 1380

caaatgcctc cacagtggtc ggaggagacg tagagtttgt ctgcaaggtt tacagtgatg 1440

cccagcccca catccagtgg atcaagcacg tggaaaagaa cggcagtaaa tacgggcccg 1500

acgggctgcc ctacctcaag gttctcaagc actcggggat aaatagttcc aatgcagaag 1560

tgctggctct gttcaatgtg accgaggcgg atgctgggga atatatatgt aaggtctcca 1620

attatatagg gcaggccaac cagtctgcct ggctcactgt cctgccaaaa cagcaaggcc 1680

gccggtgtta acaccacgga caaagagatt gaggttctct atattcggaa tgtaactttt 1740

gaggacgctg gggaatatac gtgcttggcg ggtaattcta ttgggatatc ctttcactct 1800

gcatggt 1807

<210> 47

<211> 4667

<212> DNA

<213> Homo sapiens

<220>

<221> fibroblast growth factor receptor 2 transcript variant 10

<222> (1)..(4667)

<223> LocusID: 2263; NM_023028

<400> 47

gagcgggcga gggagcgcgc gcggccgcca caaagctcgg gcgccgcggg gctgcatgcg 60

gcgtacctgg cccggcgcgg cgactgctct ccgggctggc gggggccggc cgcgagcccc 120

gggggccccg aggccgcagc ttgcctgcgc gctctgagcc ttcgcaactc gcgagcaaag 180

tttggtggag gcaacgccaa gcctgagtcc tttcttcctc tcgttcccca aatccgaggg 240

cagcccgcgg gcgtcatgcc cgcgctcctc cgcagcctgg ggtacgcgct gaagcccggg 300

aggcttggcg ccggcgaaga cccaaggacc actcttctgc gtttggagtt gctccccaca 360

accccgggct cgtcgctttc tccatcccga cccagccggg gcgcggggac aacacaggtc 420

gcggaggagc gttgccattc aagtgactgc agcagcagcg gcagcgcctc ggttcctgag 480

cccaccgcag gctgaaggca ttgcgcgtag tccatgcccg tagaggaagt gtgcagatgg 540

gattaacgtc cacatggaga tatggaagag gaccggggat tggtaccgta accatggtca 600

gctggggtcg tttcatctgc ctggtcgtgg tcaccatggc aaccttgtcc ctggcccggc 660

cctccttcag tttagttgag gataccacat tagagccaga agagccacca accaaatacc 720

aaatctctca accagaagtg tacgtggctg cgccagggga gtcgctagag gtgcgctgcc 780

tgttgaaaga tgccgccgtg atcagttgga ctaaggatgg ggtgcacttg gggcccaaca 840

ataggacagt gcttattggg gagtacttgc agataaaggg cgccacgcct agagactccg 900

gcctctatgc ttgtactgcc agtaggactg tagacagtga aacttggtac ttcatggtga 960

atgtcacaga tgccatctca tccggagatg atgaggatga caccgatggt gcggaagatt 1020

ttgtcagtga gaacagtaac aacaagagag caccatactg gaccaacaca gaaaagatgg 1080

aaaagcggct ccatgctgtg cctgcggcca acactgtcaa gtttcgctgc ccagccgggg 1140

ggaacccaat gccaaccatg cggtggctga aaaacgggaa ggagtttaag caggagcatc 1200

gcattggagg ctacaaggta cgaaaccagc actggagcct cattatggaa agtgtggtcc 1260

catctgacaa gggaaattat acctgtgtgg tggagaatga atacgggtcc atcaatcaca 1320

cgtaccacct ggatgttgtg gagcgatcgc ctcaccggcc catcctccaa gccggactgc 1380
cggcaaatgc ctccacagtg gtcggaggag acgtagagtt tgtctgcaag gtttacagtg 1440
atgcccagcc ccacatccag tggatcaagc acgtggaaaa gaacggcagt aaatacgggc 1500
ccgacgggct gccctacctc aaggttctca aggttctcaa ggccgccggt gttaacacca 1560
cggacaaaga gattgaggtt ctctatattc ggaatgtaac ttttgaggac gctggggaat 1620
atacgtgctt ggcgggtaat tctattggga tatcctttca ctctgcatgg ttgacagttc 1680
tgccagcgcc tggaagagaa aaggagatta cagcttcccc agactacctg gagatagcca 1740
tttactgcat aggggtcttc ttaatcgcct gtatggtggt aacagtcatc ctgtgccgaa 1800
tgaagaacac gaccaagaag ccagacttca gcagccagcc ggctgtgcac aagctgacca 1860
aacgtatccc cctgcggaga caggtttcgg ctgagtccag ctcctccatg aactccaaca 1920
ccccgctggt gaggataaca acacgcctct cttcaacggc agacaccccc atgctggcag 1980
gggtctccga gtatgaactt ccagaggacc caaaatggga gtttccaaga gataagctga 2040
cactgggcaa gcccctggga gaaggttgct ttgggcaagt ggtcatggcg gaagcagtgg 2100
gaattgacaa agacaagccc aaggaggcgg tcaccgtggc cgtgaagatg ttgaaagatg 2160
atgccacaga gaaagacctt tctgatctgg tgtcagagat ggagatgatg aagatgattg 2220
ggaaacacaa gaatatcata aatcttcttg gagcctgcac acaggatggg cctctctatg 2280
tcatagttga gtatgcctct aaaggcaacc tccgagaata cctccgagcc cggaggccac 2340
ccgggatgga gtactcctat gacattaacc gtgttcctga ggagcagatg accttcaagg 2400
acttggtgtc atgcacctac cagctggcca gaggcatgga gtacttggct tcccaaaaat 2460
gtattcatcg agatttagca gccagaaatg ttttggtaac agaaaacaat gtgatgaaaa 2520
tagcagactt tggactcgcc agagatatca acaatataga ctattacaaa aagaccacca 2580
atgggcggct tccagtcaag tggatggctc cagaagccct gtttgataga gtatacactc 2640
atcagagtga tgtctggtcc ttcggggtgt taatgtggga gatcttcact ttagggggct 2700
cgccctaccc agggattccc gtggaggaac tttttaagct gctgaaggaa ggacacagaa 2760
tggataagcc agccaactgc accaacgaac tgtacatgat gatgagggac tgttggcatg 2820
cagtgccctc ccagagacca acgttcaagc agttggtaga agacttggat cgaattctca 2880
ctctcacaac caatgaggaa tacttggacc tcagtcagcc tctcgaaccg tattcacctt 2940
gttatcctga cccaagatga aataaaacgt ctctcttccc ttctttcagg aatacttgga 3000
cctcagccaa cctctcgaac agtattcacc tagttaccct gacacaagaa gttcttgttc 3060
ttcaggagat gattctgttt tttctccaga ccccatgcct tacgaaccat gccttcctca 3120
gtatccacac ataaacggca gtgttaaaac atgaatgact gtgtctgcct gtccccaaac 3180
aggacagcac tgggaaccta gctacactga gcagggagac catgcctccc agagcttgtt 3240

gtctccactt gtatatatgg atcagaggag taaataattg gaaaagtaat cagcatatgt 3300

gtaaagattt atacagttga aaacttgtaa tcttccccag gaggagaaga aggtttctgg 3360

agcagtggac tgccacaagc caccatgtaa cccctctcac ctgccgtgcg ttctggctgt 3420

ggaccagtag gactcaaggt ggacgtgcgt tctgccttcc ttgttaattt tgtaataatt 3480

ggagaagatt tatgtcagca cacacttaca gagcacaaat gcagtatata ggtgctggat 3540

gtatgtaaat atattcaaat tatgtataaa tatatattat atatttacaa ggagttattt 3600

tttgtattga ttttaaatgg atgtcccaat gcacctagaa aattggtctc tcttttttta 3660

atagctattt gctaaatgct gttcttacac ataatttctt aattttcacc gagcagaggt 3720

ggaaaaatac ttttgctttc agggaaaatg gtataacgtt aatttattaa taaattggta 3780

atatacaaaa caattaatca tttatagttt tttttgtaat ttaagtggca tttctatgca 3840

ggcagcacag cagactagtt aatctattgc ttggacttaa ctagttatca gatcctttga 3900

aaagagaata tttacaatat atgactaatt tggggaaaat gaagttttga tttatttgtg 3960

tttaaatgct gctgtcagac gattgttctt agacctccta aatgccccat attaaaagaa 4020

ctcattcata ggaaggtgtt tcattttggt gtgcaaccct gtcattacgt caacgcaacg 4080

tctaactgga cttcccaaga taaatggtac cagcgtcctc ttaaaagatg ccttaatcca 4140

ttccttgagg acagacctta gttgaaatga tagcagaatg tgcttctctc tggcagctgg 4200

ccttctgctt ctgagttgca cattaatcag attagcctga ttctcttcag tgaattttga 4260

taatggcttc cagactcttt gcgttggaga cgcctgttag gatcttcaag tcccatcata 432

gaaaattgaa acacagagtt gttctgctga tagttttggg gatacgtcca tctttttaag 4380

ggattgcttt catctaattc tggcaggacc tcaccaaaag atccagcctc atacctacat 4440

cagacaaaat atcgccgttg ttccttctgt actaaagtat tgtgttttgc tttggaaaca 4500

cccactcact ttgcaatagc cgtgcaagat gaatgcagat tacactgatc ttatgtgtta 4560

caaaattgga gaaagtattt aataaaacct gttaattttt atactgacaa taaaaatgtt 4620

tctacagata ttaatgttaa caagacaaaa taaatgtcac gcaactt 4667

<210> 48

<211> 4305

<212> DNA

<213> Homo sapiens

<220>

<221>  fibroblast growth factor receptor 2 transcript variant 11

<222>  (1)..(4305)

<223>  LocusID: 2263; NM_023029

<400>  48

gagcgggcga gggagcgcgc gcggccgcca caaagctcgg gcgccgcggg gctgcatgcg    60

gcgtacctgg cccggcgcgg cgactgctct ccgggctggc gggggccggc cgcgagcccc   120

gggggccccg aggccgcagc ttgcctgcgc gctctgagcc ttcgcaactc gcgagcaaag   180

tttggtggag gcaacgccaa gcctgagtcc tttcttcctc tcgttcccca aatccgaggg   240

cagcccgcgg gcgtcatgcc cgcgctcctc cgcagcctgg ggtacgcgct gaagcccggg   300

aggcttggcg ccggcgaaga cccaaggacc actcttctgc gtttggagtt gctccccaca   360

accccgggct cgtcgctttc tccatcccga cccacgcggg gcgcggggac aacacaggtc   420

gcggaggagc gttgccattc aagtgactgc agcagcagcg gcagcgcctc ggttcctgag   480

cccaccgcag gctgaaggca ttgcgcgtag tccatgcccg tagaggaagt gtgcagatgg   540

gattaacgtc cacatggaga tatggaagag gaccggggat tggtaccgta accatggtca   600

gctggggtcg tttcatctgc ctggtcgtgg tcaccatggc aaccttgtcc ctggcccggc   660

cctccttcag tttagttgag gataccacat tagagccaga agatgccatc tcatccggag   720

atgatgagga tgacaccgat ggtgcggaag attttgtcag tgagaacagt aacaacaaga   780

gagcaccata ctggaccaac acagaaaaga tggaaaagcg gctccatgct gtgcctgcgg   840

ccaacactgt caagtttcgc tgcccagccg gggggaaccc aatgccaacc atgcggtggc   900

tgaaaaacgg gaaggagttt aagcaggagc atcgcattgg aggctacaag gtacgaaacc   960

agcactggag cctcattatg gaaagtgtgg tcccatctga caagggaaat tatacctgtg  1020

tggtggagaa tgaatacggg tccatcaatc acacgtacca cctggatgtt gtggagcgat  1080

cgcctcaccg gcccatcctc caagccggac tgccggcaaa tgcctccaca gtggtcggag  1140

gagacgtaga gtttgtctgc aaggtttaca gtgatgccca gccccacatc cagtggatca  1200

agcacgtgga aaagaacggc agtaaatacg ggcccgacgg gctgccctac ctcaaggttc  1260

tcaaggccgc cggtgttaac accacggaca aagagattga ggttctctat attcggaatg  1320

taacttttga ggacgctggg gaatatacgt gcttggcggg taattctatt gggatatcct  1380

ttcactctgc atggttgaca gttctgccag cgcctggaag agaaaaggag attacagctt  1440

ccccagacta cctggagata gccatttact gcataggggt cttcttaatc gcctgtatgg  1500

tggtaacagt catcctgtgc cgaatgaaga acacgaccaa gaagccagac ttcagcagcc  1560

agccggctgt gcacaagctg accaaacgta tccccctgcg gagacaggta acagtttcgg  1620

ctgagtccag ctcctccatg aactccaaca ccccgctggt gaggataaca acacgcctct 1680
cttcaacggc agacaccccc atgctggcag gggtctccga gtatgaactt ccagaggacc 1740
caaaatggga gtttccaaga gataagctga cactgggcaa gcccctggga gaaggttgct 1800
ttgggcaagt ggtcatggcg gaagcagtgg gaattgacaa agacaagccc aaggaggcgg 1860
tcaccgtggc cgtgaagatg ttgaaagatg atgccacaga gaaagacctt tctgatctgg 1920
tgtcagagat ggagatgatg aagatgattg ggaaacacaa gaatatcata aatcttcttg 1980
gagcctgcac acaggatggg cctctctatg tcatagttga gtatgcctct aaaggcaacc 2040
tccgagaata cctccgagcc cggaggccac ccgggatgga gtactcctat gacattaacc 2100
gtgttcctga ggagcagatg accttcaagg acttggtgtc atgcacctac cagctggcca 2160
gaggcatgga gtacttggct tcccaaaaat gtattcatcg agatttagca gccagaaatg 2220
ttttggtaac agaaaacaat gtgatgaaaa tagcagactt tggactcgcc agagatatca 2280
acaatataga ctattacaaa aagaccacca atgggcggct tccagtcaag tggatggctc 2340
cagaagccct gtttgataga gtatacactc atcagagtga tgtctggtcc ttcggggtgt 2400
taatgtggga gatcttcact ttagggggct cgccctaccc agggattccc gtggaggaac 2460
tttttaagct gctgaaggaa ggacacagaa tggataagcc agccaactgc accaacgaac 2520
tgtacatgat gatgagggac tgttggcatg cagtgccctc ccagagacca acgttcaagc 2580
agttggtaga agacttggat cgaattctca ctctcacaac caatgaggaa tacttggacc 2640
tcagccaacc tctcgaacag tattcaccta gttaccctga cacaagaagt tcttgttctt 2700
caggagatga ttctgttttt tctccagacc ccatgcctta cgaaccatgc cttcctcagt 2760
atccacacat aaacggcagt gttaaaacat gaatgactgt gtctgcctgt ccccaaacag 2820
gacagcactg ggaacctagc tacactgagc agggagacca tgcctcccag agcttgttgt 2880
ctccacttgt atatatggat cagaggagta aataattgga aaagtaatca gcatatgtgt 2940
aaagatttat acagttgaaa acttgtaatc ttccccagga ggagaagaag gtttctggag 3000
cagtggactg ccacaagcca ccatgtaacc cctctcacct gccgtgcgtt ctggctgtgg 3060
accagtagga ctcaaggtgg acgtgcgttc tgccttcctt gttaattttg taataattgg 3120
agaagattta tgtcagcaca cacttacaga gcacaaatgc agtatatagg tgctggatgt 3180
atgtaaatat attcaaatta tgtataaata tatattatat atttacaagg agttattttt 3240
tgtattgatt ttaaatggat gtcccaatgc acctagaaaa ttggtctctc tttttttaat 3300
agctatttgc taaatgctgt tcttacacat aatttcttaa ttttcaccga gcagaggtgg 3360
aaaaatactt ttgctttcag ggaaaatggt ataacgttaa tttattaata aattggtaat 3420
atacaaaaca attaatcatt tatagttttt tttgtaattt aagtggcatt tctatgcagg 3480
cagcacagca gactagttaa tctattgctt ggacttaact agttatcaga tcctttgaaa 3540

agagaatatt tacaatatat gactaatttg gggaaaatga agttttgatt tatttgtgtt 3600

taaatgctgc tgtcagacga ttgttcttag acctcctaaa tgccccatat taaaagaact 3660

cattcatagg aaggtgtttc attttggtgt gcaaccctgt cattacgtca acgcaacgtc 3720

taactggact tcccaagata aatggtacca gcgtcctctt aaaagatgcc ttaatccatt 3780

ccttgaggac agaccttagt tgaaatgata gcagaatgtg cttctctctg gcagctggcc 3840

ttctgcttct gagttgcaca ttaatcagat tagcctgatt ctcttcagtg aattttgata 3900

atggcttcca gactctttgc gttggagacg cctgttagga tcttcaagtc ccatcataga 3960

aaattgaaac acagagttgt tctgctgata gttttgggga tacgtccatc tttttaaggg 4020

attgctttca tctaattctg gcaggacctc accaaaagat ccagcctcat acctacatca 4080

gacaaaatat cgccgttgtt ccttctgtac taaagtattg tgttttgctt tggaaacacc 4140

cactcacttt gcaatagccg tgcaagatga atgcagatta cactgatctt atgtgttaca 4200

aaattggaga aagtatttaa taaaacctgt taatttttat actgacaata aaaatgtttc 4260

tacagatatt aatgttaaca agacaaaata aatgtcacgc aactt 4305

<210> 49

<211> 4222

<212> DNA

<213> Homo sapiens

<220>

<221> fibroblast growth factor receptor 2 transcript variant 12

<222> (1)..(4222)

<223> LocusID: 2263; NM_023030

<400> 49

gagcgggcga gggagcgcgc gcggccgcca caaagctcgg gcgccgcggg gctgcatgcg 60

gcgtacctgg cccggcgcgg cgactgctct ccgggctggc gggggccggc cgcgagcccc 120

gggggccccg aggccgcagc ttgcctgcgc gctctgagcc ttcgcaactc gcgagcaaag 180

tttggtggag gcaacgcaag cctgagtcct ttcttcctct cgttccccaa atccgagggc 240

agcccgcggg cgtcatggcg ctcctccgca gcctggggta cgcgtgaagc ccgggaggct 300

tggcgccggc gaagacccaa ggaccactct tctgcgtttg gagttgctcc ccgcaacccc 360

gggctcgtcg ctttctccat cccgacccac gcggggccgg ggacaacaca ggtcgcggag 420

gagcgttgcc attcaagtga ctgcagcagc agcgcagcgc ctcggttcct gagcccaccg 480
cagctgaagg cattgcgcgt agtccatgcc cgtagaggaa gtgtgcagat gggattaacg 540
tccacatgga gatatggaag aggaccgggg attggtaccg taaccatggt cagctggggt 600
cgtttcatct gcctggtcgt ggtcaccatg gcaaccttgt ccctggcccg gccctccttc 660
agtttagttg aggataccac attagagcca gaaggagcac catactggac caacacagaa 720
aagatggaaa agcggctcca tgctgtgcct gcggccaaca ctgtcaagtt tcgctgccca 780
gccgggggga acccaatgcc aaccatgcgg tggctgaaaa acgggaagga gtttaagcag 840
gagcatcgca ttggaggcta caaggtacga aaccagcact ggagcctcat tatggaaagt 900
gtggtcccat ctgacaaggg aaattatacc tgtgtagtgg agaatgaata cgggtccatc 960
aatcacacgt accacctgga tgttgtggag cgatcgcctc accggcccat cctccaagcc 1020
ggactgccgg caaatgcctc cacagtggtc ggaggagacg tagagtttgt ctgcaaggtt 1080
tacagtgatg cccagcccca catccagtgg atcaagcacg tggaaaagaa cggcagtaaa 1140
tacgggcccg acgggctgcc ctacctcaag gttctcaagc actcggggat aaatagttcc 1200
aatgcagaag tgctggctct gttcaatgtg accgaggcgg atgctgggga atatatatgt 1260
aaggtctcca attatatagg gcaggccaac cagtctgcct ggctcactgt cctgccaaaa 1320
cagcaagcgc ctggaagaga aaaggagatt acagcttccc cagactacct ggagatagcc 1380
atttactgca taggggtctt cttaatcgcc tgtatggtgg taacagtcat cctgtgccga 1440
atgaagaaca cgaccaagaa gccagacttc agcagccagc cggctgtgca caagctgacc 1500
aaacgtatcc ccctgcggag acaggtaaca gtttcggctg agtccagctc ctccatgaac 1560
tccaacaccc cgctggtgag gataacaaca cgcctctctt caacggcaga cacccccatg 1620
ctggcagggg tctccgagta tgaacttcca gaggacccaa aatgggagtt tccaagagat 1680
aagctgacac tgggcaagcc cctgggagaa ggttgctttg ggcaagtggt catggcggaa 1740
gcagtgggaa ttgacaaaga caagcccaag gaggcggtca ccgtggccgt gaagatgttg 1800
aaagatgatg ccacagagaa agacctttct gatctggtgt cagagatgga gatgatgaag 1860
atgattggga aacacaagaa tatcataaat cttcttggag cctgcacaca ggatgggcct 1920
ctctatgtca tagttgagta tgcctctaaa ggcaacctcc gagaatacct ccgagcccgg 1980
aggccacccg ggatggagta ctcctatgac attaaccgtg ttcctgagga gcagatgacc 2040
ttcaaggact tggtgtcatg cacctaccag ctggccagac ggatggagta cttggcttcc 2100
caaaaatgta ttcatcgaga tttagcagcc agaaatgttt tggtaacaga aaacaatgtg 2160
atgaaaatag cagactttgg actcgccaga gatatcaaca atatagacta ttacaaaaag 2220
accaccaatg ggcggcttcc agtcaagtgg atggctccag aagccctgtt tgatagagta 2280
tacactcatc agagtgatgt ctggtccttc ggggtgttaa tgtgggagat cttcacttta 2340

gggggctcgc cctacccagg gattcccgtg gaggaacttt ttaagctgct gaaggaagga 2400

cacagaatgg ataagccagc caactgcacc aacgaactgt acatgatgat gagggactgt 2460

tggcatgcag tgccctccca gagaccaacg ttcaagcagt tggtagaaga cttggatcga 2520

attctcactc tcacaaccaa tgaggaatac ttggacctca gccaacctct cgaacagtat 2580

tcacctagtt accctgacac aagaagttct tgttcttcag gagatgattc tgttttttct 2640

ccagacccca tgccttacga accatgcctt cctcagtatc cacacataaa cggcagtgtt 2700

aaaacatgaa tgactgtgtc tgcctgtccc caaacaggac agcactggga acctagctac 2760

actgagcagg gagaccatgc ctcccagagc ttgttgtctc cacttgtata tatggatcag 2820

aggagtaaat aattggaaaa gtaatcagca tatgtgtaaa gatttataca gttgaaaact 2880

tgtaatcttc cccaggagga gaagaaggtt tctggagcag tggactgcca caagccacca 2940

tgtaacccct ctcacctgcc gtgcgttctg gctgtggacc agtaggactc aaggtggacg 3000

tgcgttctgc cttccttgtt aattttgtaa taattggaga agatttatgt cagcacacac 3060

ttacagagca caaatgcagt atataggtgc tggatgtatg taaatatatt caaattatgt 3120

ataaatatat attatatatt tacaaggagt tattttttgt attgatttta aatggatgtc 3180

ccaatgcacc tagaaaattg gtctctcttt ttttaatagc tatttgctaa atgctgttct 3240

tacacataat ttcttaattt tcaccgagca gaggtggaaa aatacttttg ctttcaggga 3300

aaatggtata acgttaattt attaataaat tggtaatata caaaacaatt aatcatttat 3360

agtttttttt gtaatttaag tggcatttct atgcaggcag cacagcagac tagttaatct 3420

attgcttgga cttaactagt tatcagatcc tttgaaaaga gaatatttac aatatatgac 3480

taatttgggg aaaatgaagt tttgatttat ttgtgtttaa atgctgctgt cagacgattg 3540

ttcttagacc tcctaaatgc cccatattaa aagaactcat tcataggaag gtgtttcatt 3600

ttggtgtgca accctgtcat tacgtcaacg caacgtctaa ctggacttcc caagataaat 3660

ggtaccagcg tcctcttaaa agatgcctta atccattcct tgaggacaga ccttagttga 3720

aatgatagca gaatgtgctt ctctctggca gctggccttc tgcttctgag ttgcacatta 3780

atcagattag cctgattctc ttcagtgaat tttgataatg gcttccagac tctttgcgtt 3840

ggagacgcct gttaggatct tcaagtccca tcatagaaaa ttgaaacaca gagttgttct 3900

gctgatagtt ttggggatac gtccatcttt ttaagggatt gctttcatct aattctggca 3960

ggacctcacc aaaagatcca gcctcatacc tacatcagac aaaatatcgc cgttgttcct 4020

tctgtactaa agtattgtgt tttgctttgg aaacacccac tcactttgca atagccgtgc 4080

aagatgaatg cagattacac tgatcttatg tgttacaaaa ttggagaaag tatttaataa 4140

aacctgttaa tttttatact gacaataaaa atgtttctac agatattaat gttaacaaga 4200

caaaataaat gtcacgcaac tt 4222

<210>  50

<211>  4216

<212>  DNA

<213>  Homo sapiens

<220>

<221>  fibroblast growth factor receptor 2t ranscript variant 13

<222>  (1)..(4216)

<223>  LocusID: 2263; NM_023031

<400>  50

gagcgggcga gggagcgcgc gcggccgcca caaagctcgg gcgccgcggg gctgcatgcg    60

gcgtacctgg cccggcgcgg cgactgctct ccgggctggc gggggccggc cgcgagcccc   120

gggggccccg aggccgcagc ttgcctgcgc gctctgagcc ttcgcaactc gcgagcaaag   180

tttggtggag gcaacgcaag cctgagtcct ttcttcctct cgttccccaa atccgagggc   240

agcccgcggg cgtcatggcg ctcctccgca gcctggggta cgcgtgaagc ccgggaggct   300

tggcgccggc gaagacccaa ggaccactct tctgcgtttg gagttgctcc ccgcaacccc   360

gggctcgtcg ctttctccat cccgacccac gcggggccgg ggacaacaca ggtcgcggag   420

gagcgttgcc attcaagtga ctgcagcagc agcgcagcgc ctcggttcct gagcccaccg   480

cagctgaagg cattgcgcgt agtccatgcc cgtagaggaa gtgtgcagat gggattaacg   540

tccacatgga gatatggaag aggaccgggg attggtaccg taaccatggt cagctggggt   600

cgtttcatct gcctggtcgt ggtcaccatg gcaaccttgt ccctggcccg gccctccttc   660

agtttagttg aggataccac attagagcca gaaggagcac catactggac caacacagaa   720

aagatggaaa agcggctcca tgctgtgcct gcggccaaca ctgtcaagtt tcgctgccca   780

gccgggggga acccaatgcc aaccatgcgg tggctgaaaa acgggaagga gtttaagcag   840

gagcatcgca ttggaggcta caaggtacga aaccagcact ggagcctcat tatggaaagt   900

gtggtcccat ctgacaaggg aaattatacc tgtgtagtgg agaatgaata cgggtccatc   960

aatcacacgt accacctgga tgttgtggag cgatcgcctc accggcccat cctccaagcc  1020

ggactgccgg caaatgcctc cacagtggtc ggaggagacg tagagtttgt ctgcaaggtt  1080

tacagtgatg cccagcccca catccagtgg atcaagcacg tggaaaagaa cggcagtaaa  1140

tacgggcccg acgggctgcc ctacctcaag gttctcaagc actcggggat aaatagttcc  1200

aatgcagaag tgctggctct gttcaatgtg accgaggcgg atgctgggga atatatatgt  1260

aaggtctcca attatatagg gcaggccaac cagtctgcct ggctcactgt cctgccaaaa  1320

cagcaagcgc ctggaagaga aaaggagatt acagcttccc cagactacct ggagatagcc  1380

atttactgca taggggtctt cttaatcgcc tgtatggtgg taacagtcat cctgtgccga 1440

atgaagaaca cgaccaagaa gccagacttc agcagccagc cggctgtgca caagctgacc 1500

aaacgtatcc ccctgcggag acaggtttcg gctgagtcca gctcctccat gaactccaac 1560

accccgctgg tgaggataac aacacgcctc tcttcaacgg cagacacccc catgctggca 1620

ggggtctccg agtatgaact tccagaggac ccaaaatggg agtttccaag agataagctg 1680

acactgggca agcccctggg agaaggttgc tttgggcaag tggtcatggc ggaagcagtg 1740

ggaattgaca aagacaagcc caaggaggcg gtcaccgtgg ccgtgaagat gttgaaagat 1800

gatgccacag agaaagacct ttctgatctg gtgtcagaga tggagatgat gaagatgatt 1860

gggaaacaca agaatatcat aaatcttctt ggagcctgca cacaggatgg gcctctctat 1920

gtcatagttg agtatgcctc taaaggcaac ctccgagaat acctccgagc ccggaggcca 1980

cccgggatgg agtactccta tgacattaac cgtgttcctg aggagcagat gaccttcaag 2040

gacttggtgt catgcaccta ccagctggcc agacggatgg agtacttggc ttcccaaaaa 2100

tgtattcatc gagatttagc agccagaaat gttttggtaa cagaaaacaa tgtgatgaaa 2160

atagcagact ttggactcgc cagagatatc aacaatatag actattacaa aaagaccacc 2220

aatgggcggc ttccagtcaa gtggatggct ccagaagccc tgtttgatag agtatacact 2280

catcagagtg atgtctggtc cttcggggtg ttaatgtggg agatcttcac tttagggggc 2340

tcgccctacc cagggattcc cgtggaggaa ctttttaagc tgctgaagga aggacacaga 2400

atggataagc cagccaactg caccaacgaa ctgtacatga tgatgaggga ctgttggcat 2460

gcagtgccct cccagagacc aacgttcaag cagttggtag aagacttgga tcgaattctc 2520

actctcacaa ccaatgagga atacttggac ctcagccaac ctctcgaaca gtattcacct 2580

agttaccctg acacaagaag ttcttgttct tcaggagatg attctgtttt ttctccagac 2640

cccatgcctt acgaaccatg ccttcctcag tatccacaca taaacggcag tgttaaaaca 2700

tgaatgactg tgtctgcctg tccccaaaca ggacagcact gggaacctag ctacactgag 2760

cagggagacc atgcctccca gagcttgttg tctccacttg tatatatgga tcagaggagt 2820

aaataattgg aaaagtaatc agcatatgtg taaagattta tacagttgaa aacttgtaat 2880

cttccccagg aggagaagaa ggtttctgga gcagtggact gccacaagcc accatgtaac 2940

ccctctcacc tgccgtgcgt tctggctgtg gaccagtagg actcaaggtg gacgtgcgtt 3000

ctgccttcct tgttaatttt gtaataattg gagaagattt atgtcagcac acacttacag 3060

agcacaaatg cagtatatag gtgctggatg tatgtaaata tattcaaatt atgtataaat 3120

atatattata tatttacaag gagttatttt ttgtattgat tttaaatgga tgtcccaatg 3180

cacctagaaa attggtctct ctttttttaa tagctatttg ctaaatgctg ttcttacaca 3240

taatttctta attttcaccg agcagaggtg gaaaaatact tttgctttca gggaaaatgg 3300

tataacgtta atttattaat aaattggtaa tatacaaaac aattaatcat ttatagtttt 3360

ttttgtaatt taagtggcat ttctatgcag gcagcacagc agactagtta atctattgct 3420

tggacttaac tagttatcag atcctttgaa aagagaatat ttacaatata tgactaattt 3480

ggggaaaatg aagttttgat ttatttgtgt ttaaatgctg ctgtcagacg attgttctta 3540

gacctcctaa atgccccata ttaaaagaac tcattcatag gaaggtgttt cattttggtg 3600

tgcaaccctg tcattacgtc aacgcaacgt ctaactggac ttcccaagat aaatggtacc 3660

agcgtcctct taaaagatgc cttaatccat tccttgagga cagaccttag ttgaaatgat 3720

agcagaatgt gcttctctct ggcagctggc cttctgcttc tgagttgcac attaatcaga 3780

ttagcctgat tctcttcagt gaattttgat aatggcttcc agactctttg cgttggagac 3840

gcctgttagg atcttcaagt cccatcatag aaaattgaaa cacagagttg ttctgctgat 3900

agttttgggg atacgtccat ctttttaagg gattgctttc atctaattct ggcaggacct 3960

caccaaaaga tccagcctca tacctacatc agacaaaata tcgccgttgt tccttctgta 4020

ctaaagtatt gtgttttgct ttggaaacac ccactcactt tgcaatagcc gtgcaagatg 4080

aatgcagatt acactgatct tatgtgttac aaaattggag aaagtattta ataaaacctg 4140

ttaattttta tactgacaat aaaaatgttt ctacagatat taatgttaac aagacaaaat 4200

aaatgtcacg caactt 4216

<210> 51

<211> 3682

<212> DNA

<213> Homo sapiens

<220>

<221> tumor necrosis factor receptor superfamily, member 1B

<222> (1)..(3682)

<223> LocusID: 7133; NM_001066

<400> 51

gcgagcgcag cggagcctgg agagaaggcg ctgggctgcg agggcgcgag ggcgcgaggg 60

cagggggcaa ccggaccccg cccgcaccca tggcgcccgt cgccgtctgg gccgcgctgg 120

ccgtcggact ggagctctgg gctgcggcgc acgccttgcc cgcccaggtg gcatttacac 180

cctacgcccc ggagcccggg agcacatgcc ggctcagaga atactatgac cagacagctc 240

agatgtgctg cagcaaatgc tcgccgggcc aacatgcaaa agtcttctgt accaagacct 300

cggacaccgt gtgtgactcc tgtgaggaca gcacatacac ccagctctgg aactgggttc 360
ccgagtgctt gagctgtggc tcccgctgta gctctgacca ggtggaaact caagcctgca 420
ctcgggaaca gaaccgcatc tgcacctgca ggcccggctg gtactgcgcg ctgagcaagc 480
aggaggggtg ccggctgtgc gcgccgctgc gcaagtgccg cccgggcttc ggcgtggcca 540
gaccaggaac tgaaacatca gacgtggtgt gcaagccctg tgccccgggg acgttctcca 600
acacgacttc atccacggat atttgcaggc cccaccagat ctgtaacgtg gtggccatcc 660
ctgggaatgc aagcatggat gcagtctgca cgtccacgtc ccccacccgg agtatggccc 720
caggggcagt acacttaccc cagccagtgt ccacacgatc ccaacacacg cagccaactc 780
cagaacccag cactgctcca agcacctcct tcctgctccc aatgggcccc agccccccag 840
ctgaagggag cactggcgac ttcgctcttc cagttggact gattgtgggt gtgacagcct 900
tgggtctact aataatagga gtggtgaact gtgtcatcat gacccaggtg aaaaagaagc 960
ccttgtgcct gcagagagaa gccaaggtgc ctcacttgcc tgccgataag gcccggggta 1020
cacagggccc cgagcagcag cacctgctga tcacagcgcc gagctccagc agcagctccc 1080
tggagagctc ggccagtgcg ttggacagaa gggcgcccac tcggaaccag ccacaggcac 1140
caggcgtgga ggccagtggg gccggggagg cccgggccag caccgggagc tcagattctt 1200
cccctggtgg ccatgggacc caggtcaatg tcacctgcat cgtgaacgtc tgtagcagct 1260
ctgaccacag ctcacagtgc tcctcccaag ccagctccac aatgggagac acagattcca 1320
gcccctcgga gtccccgaag gacgagcagg tccccttctc caaggaggaa tgtgcctttc 1380
ggtcacagct ggagacgcca gagaccctgc tggggagcac cgaagagaag cccctgcccc 1440
ttggagtgcc tgatgctggg atgaagccca gttaaccagg ccggtgtggg ctgtgtcgta 1500
gccaaggtgg gctgagccct ggcaggatga ccctgcgaag gggccctggt ccttccaggc 1560
ccccaccact aggactctga ggctctttct gggccaagtt cctctagtgc cctccacagc 1620
cgcagcctcc ctctgacctg caggccaaga gcagaggcag cgagttgtgg aaagcctctg 1680
ctgccatggc gtgtccctct cggaaggctg gctgggcatg gacgttcggg gcatgctggg 1740
gcaagtccct gactctctgt gacctgcccc gcccagctgc acctgccagc ctggcttctg 1800
gagcccttgg gttttttgtt tgtttgtttg tttgtttgtt tgtttctccc cctgggctct 1860
gccccagctc tggcttccag aaaaccccag catccttttc tgcagagggg ctttctggag 1920
aggagggatg ctgcctgagt cacccatgaa gacaggacag tgcttcagcc tgaggctgag 1980
actgcgggat ggtcctgggg ctctgtgcag ggaggaggtg gcagccctgt agggaacggg 2040
gtccttcaag ttagctcagg aggcttggaa agcatcacct caggccaggt gcagtggctc 2100
acgcctatga tcccagcact ttgggaggct gaggcgggtg gatcacctga ggttaggagt 2160
tcgagaccag cctggccaac atggtaaaac cccatctcta ctaaaaatac agaaattagc 2220

cgggcgtggt ggcgggcacc tatagtccca gctactcaga agcctgaggc tgggaaatcg 2280
tttgaacccg ggaagcggag gttgcaggga gccgagatca cgccactgca ctccagcctg 2340
ggcgacagag cgagagtctg tctcaaaaga aaaaaaaaag caccgcctcc aaatgccaac 2400
ttgtcctttt gtaccatggt gtgaaagtca gatgcccaga gggcccaggc aggccaccat 2460
attcagtgct gtggcctggg caagataacg cacttctaac tagaaatctg ccaatttttt 2520
aaaaaagtaa gtaccactca ggccaacaag ccaacgacaa agccaaactc tgccagccac 2580
atccaacccc ccacctgcca tttgcaccct ccgccttcac tccggtgtgc ctgcagcccc 2640
gcgcctcctt ccttgctgtc ctaggccaca ccatctcctt tcagggaatt tcaggaacta 2700
gagatgactg agtcctcgta gccatctctc tactcctacc tcagcctaga ccctcctcct 2760
cccccagagg ggtgggttcc tcttccccac tccccacctt caattcctgg gccccaaacg 2820
ggctgccctg ccactttggt acatggccag tgtgatccca agtgccagtc ttgtgtctgc 2880
gtctgtgttg cgtgtcgtgg gtgtgtgtag ccaaggtcgg taagttgaat ggcctgcctt 2940
gaagccactg aagctgggat tcctccccat tagagtcagc cttccccctc ccagggccag 3000
ggccctgcag aggggaaacc agtgtagcct tgcccggatt ctgggaggaa gcaggttgag 3060
gggctcctgg aaaggctcag tctcaggagc atggggataa aggagaaggc atgaaattgt 3120
ctagcagagc aggggcaggg tgataaattg ttgataaatt ccactggact tgagcttggc 3180
agctgaacta ttggagggtg ggagagccca gccattacca tggagacaag aagggttttc 3240
caccctggaa tcaagatgtc agactggctg gctgcagtga cgtgcacctg tactcaggag 3300
gctgagggga ggatcactgg agcccaggag tttgaggctg cagcgagcta tgatcgcgcc 3360
actacactcc agcctgagca acagagtgag accctgtctc ttaaagaaaa aaaaagtcag 3420
actgctggga ctggccaggt ttctgcccac attggaccca catgaggaca tgatggagcg 3480
cacctgcccc ctggtggaca gtcctgggag aacctcaggc ttccttggca tcacagggca 3540
gagccgggaa gcgatgaatt tggagactct gtggggcctt ggttcccttg tgtgtgtgtg 3600
ttgatcccaa gacaatgaaa gtttgcactg tatgctggac ggcattcctg cttatcaata 3660
aacctgtttg ttttaaaaaa aa 3682

<210> 52
<211> 1770
<212> DNA
<213> Homo sapiens
<220>

<221>  tumor necrosis factor (ligand) superfamily, member 10

<222>  (1)..(1770)

<223>  LocusID: 8743; NM_003810

<400>  52

tttcatttcc tcactgacta taaaagaata gagaaggaag ggcttcagtg accggctgcc    60

tggctgactt acagcagtca gactctgaca ggatcatggc tatgatggag gtccaggggg   120

gacccagcct gggacagacc tgcgtgctga tcgtgatctt cacagtgctc ctgcagtctc   180

tctgtgtggc tgtaacttac gtgtacttta ccaacgagct gaagcagatg caggacaagt   240

actccaaaag tggcattgct tgtttcttaa aagaagatga cagttattgg gaccccaatg   300

acgaagagag tatgaacagc ccctgctggc aagtcaagtg gcaactccgt cagctcgtta   360

gaaagatgat tttgagaacc tctgaggaaa ccatttctac agttcaagaa aagcaacaaa   420

atatttctcc cctagtgaga gaaagaggtc ctcagagagt agcagctcac ataactggga   480

ccagaggaag aagcaacaca ttgtcttctc caaactccaa gaatgaaaag gctctgggcc   540

gcaaaataaa ctcctgggaa tcatcaagga gtgggcattc attcctgagc aacttgcact   600

tgaggaatgg tgaactggtc atccatgaaa aagggtttta ctacatctat tcccaaacat   660

actttcgatt tcaggaggaa ataaaagaaa acacaaagaa cgacaaacaa atggtccaat   720

atatttacaa atacacaagt tatcctgacc ctatattgtt gatgaaaagt gctagaaata   780

gttgttggtc taaagatgca gaatatggac tctattccat ctatcaaggg ggaatatttg   840

agcttaagga aaatgacaga atttttgttt ctgtaacaaa tgagcacttg atagacatgg   900

accatgaagc cagttttttc ggggcctttt tagttggcta actgacctgg aaagaaaaag   960

caataacctc aaagtgacta ttcagttttc aggatgatac actatgaaga tgtttcaaaa  1020

aatctgacca aaacaaacaa acagaaaaca gaaaacaaaa aaacctctat gcaatctgag  1080

tagagcagcc acaaccaaaa aattctacaa cacacactgt tctgaaagtg actcacttat  1140

cccaagagaa tgaaattgct gaaagatctt tcaggactct acctcatatc agtttgctag  1200

cagaaatcta gaagactgtc agcttccaaa cattaatgca atggttaaca tcttctgtct  1260

ttataatcta ctccttgtaa agactgtaga agaaagagca acaatccatc tctcaagtag  1320

tgtatcacag tagtagcctc caggtttcct taagggacaa catccttaag tcaaaagaga  1380

gaagaggcac cactaaaaga tcgcagtttg cctggtgcag tggctcacac ctgtaatccc  1440

aacattttgg gaacccaagg tgggtagatc acgagatcaa gagatcaaga ccatagtgac  1500

caacatagtg aaaccccatc tctactgaaa gtacaaaaat tagctgggtg tgttggcaca  1560

tgcctgtagt cccagctact tgagaggctg aggcaagaga attgtttgaa cccgggaggc  1620

agaggttgca gtgtggtgag atcatgccac tacactccag cctggcgaca gagcgagact  1680

tggtttcaaa aaaaaaaaaa aaaaaaactt cagtaagtac gtgttatttt tttcaataaa 1740

attctattac agtatgtcaa aaaaaaaaaa 1770

<210> 53

<211> 4338

<212> DNA

<213> Homo sapiens

<220>

<221> angiopoietin 1 (ANGPT1), transcript variant 1

<222> (1)..(4338)

<223> LocusID: 284; NM_001146

<400> 53

ggggcacact catgcattcc tgtcaagtca tcttgtgaaa ggctgcctgc ttccagcttg 60

gcttggatgt gcaaccttaa taaaactcac tgaggtctgg gagaaaatag cagatctgca 120

gcagataggg tagaggaaag ggtctagaat atgtacacgc agctgactca ggcaggctcc 180

atgctgaacg gtcacacaga gaggaaacaa taaatctcag ctactatgca ataaatatct 240

caagttttaa cgaagaaaaa catcattgca gtgaaataaa aaattttaaa attttagaac 300

aaagctaaca aatggctagt tttctatgat tcttcttcaa acgctttctt tgagggggaa 360

agagtcaaac aaacaagcag ttttacctga aataaagaac tagttttaga ggtcagaaga 420

aaggagcaag ttttgcgaga ggcacggaag gagtgtgctg gcagtacaat gacagttttc 480

ctttcctttg ctttcctcgc tgccattctg actcacatag ggtgcagcaa tcagcgccga 540

agtccagaaa acagtgggag aagatataac cggattcaac atgggcaatg tgcctacact 600

ttcattcttc cagaacacga tggcaactgt cgtgagagta cgacagacca gtacaacaca 660

aacgctctgc agagagatgc tccacacgtg gaaccggatt tctcttccca gaaacttcaa 720

catctggaac atgtgatgga aaattatact cagtggctgc aaaaacttga gaattacatt 780

gtggaaaaca tgaagtcgga gatggcccag atacagcaga atgcagttca gaaccacacg 840

gctaccatgc tggagatagg aaccagcctc ctctctcaga ctgcagagca gaccagaaag 900

ctgacagatg ttgagaccca ggtactaaat caaacttctc gacttgagat acagctgctg 960

gagaattcat tatccaccta caagctagag aagcaacttc ttcaacagac aaatgaaatc 1020

ttgaagatcc atgaaaaaaa cagtttatta gaacataaaa tcttagaaat ggaaggaaaa 1080

cacaaggaag agttggacac cttaaaggaa gagaaagaga accttcaagg cttggttact 1140

cgtcaaacat atataatcca ggagctggaa aagcaattaa acagagctac caccaacaac 1200

agtgtccttc agaagcagca actggagctg atggacacag tccacaacct tgtcaatctt 1260

tgcactaaag aaggtgtttt actaaaggga ggaaaaagag aggaagagaa accatttaga 1320

gactgtgcag atgtatatca agctggtttt aataaaagtg gaatctacac tatttatatt 1380

aataatatgc cagaacccaa aaaggtgttt tgcaatatgg atgtcaatgg gggaggttgg 1440

actgtaatac aacatcgtga agatggaagt ctagatttcc aaagaggctg gaaggaatat 1500

aaaatgggtt ttggaaatcc ctccggtgaa tattggctgg ggaatgagtt tatttttgcc 1560

attaccagtc agaggcagta catgctaaga attgagttaa tggactggga agggaaccga 1620

gcctattcac agtatgacag attccacata ggaaatgaaa agcaaaacta taggttgtat 1680

ttaaaaggtc acactgggac agcaggaaaa cagagcagcc tgatcttaca cggtgctgat 1740

ttcagcacta aagatgctga taatgacaac tgtatgtgca aatgtgccct catgttaaca 1800

ggaggatggt ggtttgatgc ttgtggcccc tccaatctaa atggaatgtt ctatactgcg 1860

ggacaaaacc atggaaaact gaatgggata aagtggcact acttcaaagg gcccagttac 1920

tccttacgtt ccacaactat gatgattcga cctttagatt tttgaaagcg caatgtcaga 1980

agcgattatg aaagcaacaa agaaatccgg agaagctgcc aggtgagaaa ctgtttgaaa 2040

acttcagaag caaacaatat tgtctccctt ccagcaataa gtggtagtta tgtgaagtca 2100

ccaaggttct tgaccgtgaa tctggagccg tttgagttca caagagtctc tacttggggt 2160

gacagtgctc acgtggctcg actatagaaa actccactga ctgtcgggct ttaaaaaggg 2220

aagaaactgc tgagcttgct gtgcttcaaa ctactactgg accttatttt ggaactatgg 2280

tagccagatg ataaatatgg ttaatttcat gtaaaacaga aaaaaagagt gaaaaagaga 2340

atatacatga agaatagaaa caagcctgcc ataatccttt ggaaaagatg tattatacca 2400

gtgaaaaggt gttatatcta tgcaaaccta ctaacaaatt atactgttgc acaattttga 2460

taaaaattta gaacagcatt gtcctctgag ttggttaaat gttaatggat ttcagaagcc 2520

taattccagt atcatactta ctagttgatt tctgcttacc catcttcaaa tgaaaattcc 2580

atttttgtaa gccataatga actgtagtac atggacaata agtgtgtggt agaaacaaac 2640

tccattactc tgatttttga tacagttttc agaaaaagaa atgaacataa tcaagtaagg 2700

atgtatgtgg tgaaaactta ccacccccat actatggttt tcatttactc taaaaactga 2760

ttgaatgata tataaatata tttatagcct gagtaaagtt aaaagaatgt aaaatatatc 2820

atcaagttct taaaataata tacatgcatt taatatttcc tttgatatta tacaggaaag 2880

caatattttg gagtatgtta agttgaagta aaagcaagta ctctggagca gttcatttta 2940

cagtatctac ttgcatgtgt atacatacat gtaacttcat tattttaaaa atatttttag 3000

aactccaata ctcaccctgt tatgtcttgc taatttaaat tttgctaatt aactgaaaca 3060

tgcttaccag attcacactg ttccagtgtc tataaaagaa acactttgaa gtctataaaa 3120

aataaaataa ttataaatat cattgtacat agcatgttta tatctgcaaa aaacctaata 3180

gctaattaat ctggaatatg caacattgtc cttaattgat gcaaataaca caaatgctca 3240

aagaaatcta ctatatccct taatgaaata catcattctt catatatttc tccttcagtc 3300

cattccctta ggcaattttt aatttttaaa aattattatc aggggagaaa aattggcaaa 3360

actattatat gtaagggaaa tatatacaaa aagaaaatta atcatagtca cctgactaag 3420

aaattctgac tgctagttgc cataaataac tcaatggaaa tattcctatg ggataatgta 3480

ttttaagtga atttttgggg tgcttgaagt tactgcatta ttttatcaag aagtcttctc 3540

tgcctgtaag tgtccaaggt tatgacagta aacagttttt attaaaacat gagtcactat 3600

gggatgagaa aattgaaata aagctactgg gcctcctctc ataaaagaga cagttgttgg 3660

caaggtagca ataccagttt caaacttggt gacttgatcc actatgcctt aatggtttcc 3720

tccatttgag aaaataaagc tattcacatt gttaagaaaa atacttttta aagtttacca 3780

tcaagtcttt tttatattta tgtgtctgta ttctacccct ttttgcctta caagtgatat 3840

ttgcaggtat tataccattt ttctattctt ggtggcttct tcatagcagg taagcctctc 3900

cttctaaaaa cttctcaact gttttcattt aagggaaaga aaatgagtat tttgtccttt 3960

tgtgttccta cagacacttt cttaaaccag tttttggata aagaatacta tttccaaact 4020

catattacaa aaacaaaata aaataataaa aaaagaaagc atgatattta ctgttttgtt 4080

gtctgggttt gagaaatgaa atattgtttc caattattta taataaatca gtataaaatg 4140

ttttatgatt gttatgtgta ttatgtaata cgtacatgtt tatggcaatt taacatgtgt 4200

attcttttaa ttgtttcaga ataggataat taggtattcg aattttgtct ttaaaattca 4260

tgtggtttct atgcaaagtt cttcatatca tcacaacatt atttgattta aataaaattg 4320

aaagtaatat ttgtgcaa 4338

<210> 54

<211> 2360

<212> DNA

<213> Homo sapiens

<220>

<221> angiopoietin 1 (ANGPT1), transcript variant 2

<222> (1)..(2360)

<223> LocusID: 284; NM_139290

<400>  54

ggggcacact catgcattcc tgtcaagtca tcttgtgaaa ggctgcctgc ttccagcttg      60

gcttggatgt gcaaccttaa taaaactcac tgaggtctgg gagaaaatag cagatctgca     120

gcagataggg tagaggaaag ggtctagaat atgtacacgc agctgactca ggcaggctcc     180

atgctgaacg gtcacacaga gaggaaacaa taaatctcag ctactatgca ataaatatct     240

caagttttaa cgaagaaaaa catcattgca gtgaaataaa aaattttaaa attttagaac     300

aaagctaaca aatggctagt tttctatgat tcttcttcaa acgctttctt tgagggggaa     360

agagtcaaac aaacaagcag ttttacctga aataaagaac tagttttaga ggtcagaaga     420

aaggagcaag ttttgcgaga ggcacggaag gagtgtgctg gcagtacaat gacagttttc     480

ctttcctttg ctttcctcgc tgccattctg actcacatag ggtgcagcaa tcagcgccga     540

agtccagaaa acagtgggag aagatataac cggattcaac atgggcaatg tgcctacact     600

ttcattcttc cagaacacga tggcaactgt cgtgagagta cgacagacca gtacaacaca     660

aacgctctgc agagagatgc tccacacgtg gaaccggatt tctcttccca gaaacttcaa     720

catctggaac atgtgatgga aaattatact cagtggctgc aaaaacttga gaattacatt     780

gtggaaaaca tgaagtcgga gatggcccag atacagcaga atgcagttca gaaccacacg     840

gctaccatgc tggagatagg aaccagcctc ctctctcaga ctgcagagca gaccagaaag     900

ctgacagatg ttgagaccca ggtactaaat caaacttctc gacttgagat acagctgctg     960

gagaattcat tatccaccta caagctagag aagcaacttc ttcaacagac aaatgaaatc    1020

ttgaagatcc atgaaaaaaa cagtttatta gaacataaaa tcttagaaat ggaaggaaaa    1080

cacaaggaag agttggacac cttaaaggaa gagaaagaga accttcaagg cttggttact    1140

cgtcaaacat atataatcca ggagctggaa aagcaattaa acagagctac caccaacaac    1200

agtgtccttc agaagcagca actggagctg atggacacag tccacaacct tgtcaatctt    1260

tgcactaaag aaggtgtttt actaaaggga ggaaaaagag aggaagagaa accatttaga    1320

gactgtgcag atgtatatca agctggtttt aataaaagtg gaatctacac tatttatatt    1380

aataatatgc cagaacccaa aaaggtgttt tgcaatatgg atgtcaatgg gggaggttgg    1440

actgtaatac aacatcgtga agatggaagt ctagatttcc aaagaggctg gaaggaatat    1500

aaaatgggtt ttggaaatcc ctccggtgaa tattggctgg ggaatgagtt tatttttgcc    1560

attaccagtc agaggcagta catgctaaga attgagttaa tggactggga agggaaccga    1620

gcctattcac agtatgacag attccacata ggaaatgaaa agcaaaacta taggtaagtc    1680

atgctacagt gtagtgttcg actacctttt acctagccac ttaataattg tagtgggaaa    1740

atatgaaaaa aagaaatgaa aatcatcctt caaaatgaaa attctttttt ttttgtctag    1800

acctattaca aagaggtaca aaaaagcctg taaagacttt ttcaaagatt taaatttcag    1860

ttttcgtagg ttgactatct ttgatattgg cttaaatttt agggaatggt gaatgaagtc 1920

aagcatactg ttgttctgtg gttatcatgc agttgcagtg aattcatata tataaaactg 1980

ataagtttca tactcaaact cattggctta aataatctct ttattctaaa aaaagatttt 2040

ctggttctac tttttttgat gacctttctt gaattagcat ttttcaaatg atcttgaaga 2100

tgacaaaagt aaaatttttt tatgtttgaa tgatttacat aaaagagaag atgaggcatg 2160

atttagaggg ttttcttggt aaatctaaaa agcaaaaata aattgtaata atggcatcaa 2220

tatacataat acatgattgc agatgtaaaa atagataaac tatacactta atgatacatg 2280

tttccaatta tcctttttca atgagaagct cgtctactag ggaaaaataa acacacaaaa 2340

atatggctaa aaaaaaaaaa 2360

<210> 55

<211> 5430

<212> DNA

<213> Homo sapiens

<220>

<221> retinoid X receptor, alpha

<222> (1)..(5430)

<223> LocusID: 6256; NM_002957

<400> 55

gcgccggggg ccgccgcgcc cgccgcccgc tgcctgcgcc gccggccggg catgagttag 60

tcgcagacat ggacaccaaa catttcctgc cgctcgattt ctccacccag gtgaactcct 120

ccctcacctc cccgacgggg cgaggctcca tggctgcccc ctcgctgcac ccgtccctgg 180

ggcctggcat cggctccccg ggacagctgc attctcccat cagcaccctg agctccccca 240

tcaacggcat gggcccgcct ttctcggtca tcagctcccc catgggcccc cactccatgt 300

cggtgcccac cacacccacc ctgggcttca gcactggcag cccccagctc agctcaccta 360

tgaaccccgt cagcagcagc gaggacatca agccccccct gggcctcaat ggcgtcctca 420

aggtccccgc ccacccctca ggaaacatgg cttccttcac caagcacatc tgcgccatct 480

gcggggaccg ctcctcaggc aagcactatg gagtgtacag ctgcgagggg tgcaagggct 540

tcttcaagcg gacggtgcgc aaggacctga cctacacctg ccgcgacaac aaggactgcc 600

tgattgacaa gcggcagcgg aaccggtgcc agtactgccg ctaccagaag tgcctggcca 660

tgggcatgaa gcgggaagcc gtgcaggagg agcggcagcg tggcaaggac cggaacgaga 720

atgaggtgga gtcgaccagc agcgccaacg aggacatgcc ggtggagagg atcctggagg 780
ctgagctggc cgtggagccc aagaccgaga cctacgtgga ggcaaacatg gggctgaacc 840
ccagctcgcc gaacgaccct gtcaccaaca tttgccaagc agccgacaaa cagcttttca 900
ccctggtgga gtgggccaag cggatcccac acttctcaga gctgcccctg gacgaccagg 960
tcatcctgct gcgggcaggc tggaatgagc tgctcatcgc ctccttctcc caccgctcca 1020
tcgccgtgaa ggacgggatc ctcctggcca ccgggctgca cgtccaccgg aacagcgccc 1080
acagcgcagg ggtgggcgcc atctttgaca gggtgctgac ggagcttgtg tccaagatgc 1140
gggacatgca gatggacaag acggagctgg gctgcctgcg cgccatcgtc ctctttaacc 1200
ctgactccaa ggggctctcg aacccggccg aggtggaggc gctgagggag aaggtctatg 1260
cgtccttgga ggcctactgc aagcacaagt acccagagca gccgggaagg ttcgctaagc 1320
tcttgctccg cctgccggct ctgcgctcca tcgggctcaa atgcctggaa catctcttct 1380
tcttcaagct catcggggac acacccattg acaccttcct tatggagatg ctggaggcgc 1440
cgcaccaaat gacttaggcc tgcgggccca tcctttgtgc ccacccgttc tggccaccct 1500
gcctggacgc cagctgttct tctcagcctg agccctgtcc ctgcccttct ctgcctggcc 1560
tgtttggact ttggggcaca gcctgtcact gctctgccta agagatgtgt tgtcaccctc 1620
cttatttctg ttactacttg tctgtggccc agggcagtgg ctttcctgag gcagcagcct 1680
tcgtggcaag aactagcgtg agcccagcca ggcgcctccc caccgggctc tcaggacacc 1740
ctgccacacc ccacggggct tgggcgacta cagggtcttc gggccccagc cctggagctg 1800
caggagttgg gaacggggct tttgtttccg ttgctgttta tcgatgctgg ttttcagaat 1860
tcctgtgtgg ccctcctgtc tggagtgaca tcttcatctg ctctgaatac tggtgcccag 1920
ccagcccgtg acagcttccc cctaatcagg aggggacagc tgggggcgca agctggtgtg 1980
tcatcagcaa agacctcagc cgcctcgggg atgagagggg actcgtgggg caagcaagct 2040
gccctgtgct ctgagtgagg gggaaggtag cccctttttc caaagataac tcacagtttt 2100
gccctcgagc caatgagaac atgagctgcc ctctgtgcaa ggtttcgggg ccacctccag 2160
gctgcagggg cgggtcactc acccccctgt tttctctctg ccttggtgtt ctggtttcag 2220
actcccgact ccccgttcag accagagtgc cccggcccct ccccagcctg agtcttctcc 2280
ttgctctgcg gggtgggctg aggcttgtcc ttgtttcctg cagggctggc cctggctcgg 2340
gcagggtggg gcatcaccac ctcactggcc ttgctggagg cacagggctc tgcggacctg 2400
cagccatctg tgaggcccgc ggggatggga ggggaggagg gtggcctgtt ggtttccctc 2460
agagggggca ggtggcctgg agagagaggg gctcaggaac tgggagcctc gtgggtgggg 2520
cagatgctcc gcggcctgga gtggctctgc cggggcattg gtgggacccc tgctcaggcc 2580
ttctctctgg ctgccagttg tgtctaaaag actcttggaa tctgagaacc cggagtcgca 2640

gcgccctcgg gcctgggcca cacgcaggcc ctggtgggac cacccagcct ggtattgtcc 2700
acggacagcg ttgttcaccc agagccttac ttgggagcct cactgaacgc ctgctctggt 2760
tgaaggtggg gtgggggcgg ggcttggggc ctccctggct cagcccagtg cggcctggcg 2820
ctcctcccgc aggctctgcc cccgggctcc ggtggtgcgg ggccctctca ggttgaactc 2880
gcctcttttg cactggaagg ccctcccttt ggcctgagta cttttcccgt tcacgcctca 2940
gtcccgtgga cccagccttt gtcagtggca ggtgcctgaa cagagggtgg atggggggga 3000
taccggaggg ggtcttgtct tcccagccgc agtctaggaa tgatgcgggg gggtggacgc 3060
cttctccata gtctttcccc acctggagca ggggcttcct cagtggtgag gggagctgcc 3120
tacaggttgg accgggaggc agtggcttgg agaggcagct ttccagcctt ggtggggaag 3180
aaagtgtcca ttctttgcct tcctggagct cccagccaga gctgagctta ggcacccgag 3240
tggagcctgc agctgagtct gtgcccgaga caggctgtca gagattccag aagcctctcc 3300
tccccgccgc cctccacccc tgcctttcag cgttgtggat ccctagaggt ggccccctgc 3360
ccgatccacc gtcctgaggc agagtgttga gcctcatacc tgtaccaggt ccccggccag 3420
ctgggcccct cccaggcact gccaggaagc cccagctgcc cctggcgggt gtggtggaaa 3480
tggcaggagg gtgcaggtac tcttggggcc ccagcggtgg gagtgcaaaa gacccaacgc 3540
caacacctgg tgccttttgc agccagcgcc cacccatccg tgcccggacc cttgggaatg 3600
cccgcggctc cagaggaaaa agcccaggga cggggcctcc gttgcggggg gtcggctgct 3660
tcttgggaac tttgtcgttt ccggcgctgg ctggctggct ggctgtaaag cactgaagcc 3720
ccccggccgc caacccctga aagcagaacc tggcctccct ggccacagca gccttaccca 3780
ccgctctacg tgtcccgggc acttcccgca gccttcccgt ccctttctca tcggccttgt 3840
agttgtacag tgctgttggt ttgaaaaggt gatgtgtggg gagtgcggct catcactgag 3900
tagagaggta gaatttctat ttaaccagac ctgtagtagt attaccaatc cagttcaatt 3960
aaggtgattt tttgtaatta ttattatttt ggtgggacaa tctttaattt tctaaagata 4020
gcactaacat cagctcatta gccacctgtg cctgtccccg ccttggcccg gctggatgaa 4080
gcggcttccc cgcagggccc ccacttccca gtggctgctt cctggggacc cagggcaccc 4140
cggcaccttc aggcacgctc ctcagctggt cacctcccgg ctttgccgtt cagatggggc 4200
tcctgaggct caggagtgaa gatgccacag agccgggctc ccctaggctg cgtcgggcat 4260
gcttggaagc tggcctgcca ggaccttcca ccctggggcc tgtgtcagcc gccggccctc 4320
cgcaccctgg aagcacacgg cctctgggaa ggacagccct gaccttcggt tttccgagca 4380
cggtgtttcc caagaattct gggctggcgg cctggtggca gtgctggaga tgaccccgag 4440
cccctccccg tggggcaccc aggagggccc tgccggaatg tgcagcctgt gggtagtcgg 4500
ctggtgtccc tgtcgtggag ctggggtgcg tgatctggtg ctcgtccacg caggtgtgtg 4560

gtgtaaacat gtatgtgctg tacagagaga cgcgtgtgga gagagccgca caccagcgcc 4620

acccaggaaa ggcggagcgg ttaccagtgt tttgtgttta tttttaatca agacgtttcc 4680

cctgttttcc tataaatttg cttcgtgtaa gcaagtacat aaggaccctc ctttggtgaa 4740

atccgggttc gaatgaatat ctcaaggcag gagatgcatc tattttaaga tgctttggag 4800

cagacagctt tagccgttcc caatccttag caatgcctta gctgggacgc atagctaata 4860

ctttagagag gatgacagat ccataaagag agtaaagata agagaaaatg tctaaagcat 4920

ctggaaaggt aaaaaaaaaa aatctatttt tgtacaaatg taattttatc cctcatgtat 4980

acttggatat ggcgggggga gggctgggac tgtttcgttt ctgcttctag agattgaggt 5040

gaaagcttcg tccgagaaac gccaggacag acgatggcag aggagagggc tcctgtgacg 5100

gcggcgaggc ttgggaggaa accgccgcaa tgggggtgtc ttccctcggg gcaggagggt 5160

gggcctgagg ctttcaaggg ttttcttccc tttcgagtaa tttttaaagc cttgctctgt 5220

tgtgtcctgt tgccggctct ggccttcctg tgactgactg tgaagtggct tctccgtacg 5280

attgtctctg aaacatcgtg gcctcaggtg ccagggtttg atggacagta gcattagaat 5340

tgtggaaaag gaacacgcaa agggagaagt gtgagaggag aaacaaaata tgagcgttta 5400

aaatacatcg ccattcagtt cgttaaaaaa 5430

<210> 56

<211> 2050

<212> DNA

<213> Homo sapiens

<220>

<221> degenerative spermatocyte homolog, lipid desaturase transcript
variant 1

<222> (1)..(2050)

<223> LocusID: 8560; NM_003676

<400> 56

cacagccggc cgacaccaca ccagccgggg agccgccgcc gccgccgcca cctctgagca 60

gccggctggg agcgagagcc gacagctagt ctgcaagcca ccgctgtcgc catggggagc 120

cgcgtctcgc gggaagactt cgagtgggtc tacaccgacc agccgcacgc cgaccggcgc 180

cgggagatcc tggcaaagta tccagagata aagtccttga tgaaacctga tcccaatttg 240

atatggatta taattatgat ggttctcacc cagttgggtg cattttacat agtaaaagac 300

ttggactgga aatgggtcat atttggggcc tatgcgtttg gcagttgcat taaccactca 360

atgactctgg ctattcatga gattgcccac aatgctgcct ttggcaactg caaagcaatg 420

tggaatcgct ggtttggaat gtttgctaat cttcctattg ggattccata ttcaatttcc 480

tttaagaggt atcacatgga tcatcatcgg taccttggag ctgatggcgt cgatgtagat 540

attcctaccg attttgaggg ctggttcttc tgtaccgctt tcagaaagtt tatatgggtt 600

attcttcagc ctctctttta tgcctttcga cctctgttca tcaaccccaa accaattacg 660

tatctggaag ttatcaatac cgtggcacag gtcacttttg acattttaat ttattacttt 720

ttgggaatta aatccttagt ctacatgttg gcagcatctt tacttggcct gggtttgcac 780

ccaatttctg gacattttat agctgagcat tacatgttct taaagggtca tgaaacttac 840

tcatattatg ggcctctgaa tttacttacc ttcaatgtgg gttatcataa tgaacatcat 900

gatttcccca acattcctgg aaaaagtctt ccactggtga ggaaaatagc agctgaatac 960

tatgacaacc tccctcacta caattcctgg ataaaagtac tgtatgattt tgtgatggat 1020

gatacaataa gtccctactc aagaatgaag aggcaccaaa aaggagagat ggtgctggag 1080

taaatatcat tagtgccaaa gggattcttc tccaaaactt tagatgataa aatggaattt 1140

ttgcattatt aaacttgaga ccagtgatgc tcagaagctc ccctggcaca atttcagagt 1200

aagagctcgg tgataccaag aagtgaatct ggcttttaaa cagtcagcct gactctgtac 1260

tgctcagttt cactcacagg aaacttgtga cttgtgtatt atcgtcattg aggatgtttc 1320

actcatgtct gtcattttat aagcatatca tttaaaaagc ttctaaaaag ctatttcgcc 1380

aggcacggtg gctcatgcct ataatcccag cactttggga ggccaaggtg ggtggatcac 1440

ctgaggtcag gagttcgaga ccagcctggc caacacggtg aaaccccatc tctactaaaa 1500

atgcaaaaat tagccgggcg tggcggcaca tgcctgtaat cccagctaca tgggaggctg 1560

aggtgggaga attgcttgaa cccaggaggc ggaggcagag gctgcagtga cccaagattg 1620

tgccactgca ctccaccctg ggcaacagag caagacccca tctcaaaaat aaataaatat 1680

atataaaaaa taaaaagcta tttctagttt atttcactat aaagttttgc tttattaaaa 1740

agctaataaa cagctattaa tcacagtgta ttagtatttg ttacattttt gtatttcact 1800

atctttatac tatataatat ggtaacttgg gtaccggggg aactttaaaa tttcatctca 1860

aaaataattt ttaaaaagcc tgaggtatga tatagcataa aagattgaga tgaaaatata 1920

tttccctgta agctgaatta ctcatttaaa aattttaact tctatatggg acccgaatta 1980

gacactgctg aatcctgtac agccttactc ataaataaag tacttactga atttccacca 2040

ttcaaaaaaa 2050

<210> 57

<211> 1670

<212> DNA

<213> Homo sapiens

<220>

<221> degenerative spermatocyte homolog, lipid desaturase transcript
      variant 2

<222> (1)..(1670)

<223> LocusID: 8560; NM_144780

<400> 57

cacagccggc cgacaccaca ccagccgggg agccgccgcc gccgccgcca cctctgagca    60

gccggctggg agcgagagcc gacagctagt ctgcaagcca ccgctgtcgc catggggagc   120

cgcgtctcgc gggaagactt cgagtgggtc tacaccgacc agccgcacgc cgaccggcgc   180

cgggagatcc tggcaaagta tccagagata aagtccttga tgaaacctga tcccaatttg   240

atatggatta taattatgat ggttctcacc cagttgggtg cattttacat agtaaaagac   300

ttggactgga aatgggtcat atttggggcc tatgcgtttg gcagttgcat taaccactca   360

atgactctgg ctattcatga gattgcccac aatgctgcct ttggcaactg caaagcaatg   420

tggaatcgct ggtttggaat gtttgctaat cttcctattg ggattccata ttcaatttcc   480

tttaagaggt atcacatgga tcatcatcgg taccttggag ctgatggcgt cgatgtagat   540

attcctaccg attttgaggg ctggttcttc tgtaccgctt tcagaaagtt tatatgggtt   600

attcttcagc ctctctttta tgcctttcga cctctgttca tcaaccccaa accaattacg   660

tatctggaag ttatcaatac cgtggcacag gtcacttttg acattttaat ttattacttt   720

ttgggaatta aatccttagt ctacatgttg gcagcatctt tacttggcct gggtttgcac   780

ccaatttctg gacattttat agctgagcat tacatgttct taaagggtca tgaaacttac   840

tcatattatg ggcctctgaa tttacttacc ttcaatgtgg gttatcataa tgaacatcat   900

gatttcccca acattcctgg aaaaagtctt ccactggtga ggaaaatagc agctgaatac   960

tatgacaacc tccctcacta caattcctgg ataaaagtac tgtatgattt tgtgatggat  1020

gatacaataa gtccctactc aagaatgaag aggcaccaaa aaggagagat ggtgctggag  1080

taaatatcat tagtgccaaa gggattcttc tccaaaactt tagatgataa aattagccgg  1140

gcgtggcggc acatgcctgt aatcccagct acatggaggg ctgaggtggg agaattgctt  1200

gaacccagga ggcggaggca gaggctgcag tgacccaaga ttgtgccact gcactccacc  1260

ctgggcaaca gagcaagacc ccatctcaaa aataaataaa tatatataaa aaataaaaag  1320

ctatttctag tttatttcac tataaagttt tgctttatta aaaagctaat aaacagctat 1380

taatcacagt gtattagtat ttgttacatt tttgtatttc actatcttta tactatataa 1440

tatggtaact tgggtaccgg gggaacttta aaatttcatc tcaaaaataa tttttaaaaa 1500

gcctgaggta tgatatagca taaaagattg agatgaaaat atatttccct gtaagctgaa 1560

ttactcattt aaaaatttta acttctatat gggacccgaa ttagacactg ctgaatcctg 1620

tacagcctta ctcataaata aagtacttac tgaatttcca ccattcaaaa 1670

<210> 58

<211> 2447

<212> DNA

<213> Homo sapiens

<220>

<221> solute carrier family 7 member 7

<222> (1)..(2447)

<223> LocusID: 9056; NM_003982

<400> 58

gcatggagga cacagagaga gagagtgctg tgtattcctt ccccgctact gtcctgtcct 60

cagctaactt gctctgggac agcttcccca gggctacaga tactgcactc agctgactgt 120

cctttcttct gggcccctgg tcccagagca gagctgacaa aggagattcc tgagagagca 180

ccttcttatc acagaaagtg ctgagccaag agctcctagc tgcccctttt gcagatgtga 240

agggccagtg aaccttggac ccagatggtt gcttaatact cctttccccc tccctcactc 300

cttcctttgc gggctgcctc acctcctcca cccttcttgc ttaaatccat aggcatttgt 360

ctggccttcc cttttactgc tggctgggaa ggaggagcat cagaccacag atcctggaag 420

gcacttctct ccctgactgc cgctcacact gccgtgagaa cctgcttata tccaggacca 480

aggaggcaat gccaggaagc tggtgaaggg tttcctctcc tccaccatgg ttgacagcac 540

tgagtatgaa gtggcctccc agcctgaggt ggaaacctcc cctttgggtg atggggccag 600

cccagggccg gagcaggtga agctgaagaa ggagatctca ctgcttaacg gcgtgtgcct 660

gattgtgggg aacatgatcg gctcaggcat ctttgtttcc cccaagggtg tgctcatata 720

cagtgcctcc tttggtctct ctctggtcat ctgggctgtc gggggcctct tctccgtctt 780

tggggccctt tgttatgtgg aactgggcac caccattaag aaatctgggg ccagctatgc 840

ctatatcctg gaggcctttg gaggattcct tgctttcatc agactctgga cctccctgct 900

catcattgag cccaccagcc aggccatcat tgccatcacc tttgccaact acatggtaca 960

gcctctcttc ccgagctgct tcgcccctta tgctgccagc cgcctgctgg ctgctgcctg 1020

catttgtctc ttaaccttca ttaactgtgc ctatgtcaaa tggggaaccc tggtacaaga 1080

tattttcacc tatgctaaag tattggcact gatcgcggtc atcgttgcag gcattgttag 1140

acttggccag ggagcctcta ctcattttga gaattccttt gagggttcat catttgcagt 1200

gggtgacatt gccctggcac tgtactcagc tctgttctcc tactcaggct gggacaccct 1260

caactatgtc actgaagaga tcaagaatcc tgagaggaac ctgcccctct ccattggcat 1320

ctccatgccc attgtcacca tcatctatat cttgaccaat gtggcctatt atactgtgct 1380

agacatgaga gacatcttgg ccagtgatgc tgttgctgtg acttttgcag atcagatatt 1440

tggaatattt aactggataa ttccactgtc agttgcatta tcctgttttg gtggcctcaa 1500

tgcctccatt gtggctgctt ctaggctttt ctttgtgggc tcaagagaag gccatctccc 1560

tgatgccatc tgcatgatcc atgttgagcg gttcacacca gtgccttctc tgctcttcaa 1620

tggtatcatg gcattgatct acttgtgcgt ggaagacatc ttccagctca ttaactacta 1680

cagcttcagc tactggttct ttgtggggct ttctattgtg ggtcagcttt atctgcgctg 1740

gaaggagcct gatcgacctc gtcccctcaa gctcagcgtt ttcttcccga ttgtcttctg 1800

cctctgcacc atcttcctgg tggctgttcc actttacagt gatactatca actccctcat 1860

cggcattgcc attgccctct caggcctgcc cttttacttc ctcatcatca gagtgccaga 1920

acataagcga ccgctttacc tccgaaggat cgtggggtct gccacaaggt acctccaggt 1980

cctgtgtatg tcagttgctg cagaaatgga tttggaagat ggaggagaga tgcccaagca 2040

acgggatccc aagtctaact aaacaccatc tggaatcctg atgtggaaag caggggtttc 2100

tggtctactg gctagagcta aggaagttga aaaggaaagc tcacttcttt ggaggcacct 2160

gtccagaagc ctggcctagg cagcttcaac ctttgaactt actttttgaa atgaaaagta 2220

atttatttgt tttgctacat actgttccag acttttaaag gggacaatga aggtgactgt 2280

ggggaggagc atgtcaggtt tgggcttggt tgttttagaa gcacctgggt gtgcctacct 2340

actcctcttt tcttttaaaa gggcccacaa tgctccaatt tcctgtctcc tttagagaga 2400

catgaaacta tcacaggtgc tggatgacaa taaaagttta tgttcct 2447

<210> 59

<211> 3490

<212> DNA

<213> Homo sapiens

<220>

<221>  procollagen-lysine, 2-oxoglutarate 5-dioxygenase 2 transcript
       variant 2

<222>  (1)..(3490)

<223>  LocusID: 5352; NM_000935

<400>  59

atggggggat gcacggtgaa gcctcagctg ctgctcctgg cgctcgtcct ccacccctgg    60

aatccctgtc tgggtgcgga ctcggagaag ccctcgagca tccccacaga taaattatta   120

gtcataactg tagcaacaaa agaaagtgat ggattccatc gatttatgca gtcagccaaa   180

tatttcaatt atactgtgaa ggtccttggt caaggagaag aatggagagg tggtgatgga   240

attaatagta ttggaggggg ccagaaagtg agattaatga aagaagtcat ggaacactat   300

gctgatcaag atgatctggt tgtcatgttt actgaatgct ttgatgtcat atttgctggt   360

ggtccagaag aagttctaaa aaaattccaa aaggcaaacc acaaagtggt ctttgcagca   420

gatggaattt tgtggccaga taaaagacta gcagacaagt atcctgttgt gcacattggg   480

aaacgctatc tgaattcagg aggatttatt ggctatgctc catatgtcaa ccgtatagtt   540

caacaatgga atctccagga taatgatgat gatcagctct tttacactaa agtttacatt   600

gatccactga aaagggaagc tattaacatc acattggatc acaaatgcaa aattttccag   660

accttaaatg gagctgtaga tgaagttgtt ttaaaatttg aaaatggcaa agccagagct   720

aagaatacat tttatgaaac attaccagtg gcaattaatg gaaatggacc caccaagatt   780

ctcctgaatt attttggaaa ctatgtaccc aattcatgga cacaggataa tggctgcact   840

ctttgtgaat tcgatacagt cgacttgtct gcagtagatg tccatccaaa cgtatcaata   900

ggtgttttta ttgagcaacc aacccctttt ctacctcggt ttctggacat attgttgaca   960

ctggattacc caaaagaagc acttaaactt tttattcata acaaagaagt ttatcatgaa  1020

aaggacatca aggtattttt tgataaagct aagcatgaaa tcaaaactat aaaaatagta  1080

ggaccagaag aaaatctaag tcaagcggaa gccagaaaca tgggaatgga cttttgccgt  1140

caggatgaaa agtgtgatta ttactttagt gtggatgcag atgttgtttt gacaaatcca  1200

aggactttaa aaattttgat tgaacaaaac agaaagatca ttgctcctct tgtaactcgt  1260

catggaaagc tgtggtccaa tttctgggga gcattgagtc ctgatggata ctatgcacga  1320

tctgaagatt atgtggatat tgttcaaggg aatagagtag gagtatggaa tgtcccatat  1380

atggctaatg tgtacttaat taaaggaaag acactccgat cagagatgaa tgaaaggaac  1440

tattttgttc gtgataaact ggatcctgat atggctcttt gccgaaatgc tagagaaatg  1500

ggtgtattta tgtacatttc taatagacat gaatttggaa ggctattatc cactgctaat  1560

tacaatactt cccattataa caatgacctc tggcagattt ttgaaaatcc tgtggactgg 1620

aaggaaaagt atataaaccg tgattattca aagattttca ctgaaaatat agttgaacag 1680

ccctgtccag atgtcttttg gttccccata ttttctgaaa aagcctgtga tgaattggta 1740

gaagaaatgg aacattacgg caaatggtct gggggaaaac atcatgatag ccgtatatct 1800

ggtggttatg aaaatgtccc aactgatgat atccacatga agcaagttga tctggagaat 1860

gtatggcttg attttatccg ggagttcatt gcaccagtta cactgaaggt ctttgcaggc 1920

tattatacga agggatttgc actactgaat tttgtagtaa aatactcccc tgaacgacag 1980

cgttctcttc gtcctcatca tgatgcttct acatttacca taaacattgc acttaataac 2040

gtgggagaag actttcaggg aggtggttgc aaatttctaa ggtacaattg ctctattgag 2100

tcaccacgaa aaggctggag cttcatgcat cctgggagac tcacacattt gcatgaagga 2160

cttcctgtta aaaatggaac aagatacatt gcagtgtcat ttatagatcc ctaagttatt 2220

tacttttcat tgaattgaaa tttattttgg gtgaatgact ggcatgaaca cgtctttgaa 2280

gttgtggctg agaagatgag aggaatattt aaataacatc aacagaacaa cttcactttg 2340

ggccaaacat ttgaaaaact ttttataaaa aattgtttga tatttcttaa tgtctgctct 2400

gagccttaaa acacagattg aagaagaaaa gaaagaaaaa acttaaatat ttatttctat 2460

gctttgttgc ctctgagaat aatgacaatt tatgaatttg tgtttcaaat tgataaaata 2520

tttaggtaca aataacaaga ctaataatat tttcttattt aaaaaaagca tgggaagatt 2580

tttatttatc aaaatataga ggaaatgtag acaaaatgga tataaatgaa aattaccatg 2640

ttgtaaaacc ttgaaaatca gattctaact gattgtatgc aactaagtat ttctgaacac 2700

ctatgcaggt cttatttaca gtgttactaa gggaacacac aaagaattac acaacgtttt 2760

cctcaagaaa atggtacaaa acacaaccga ggagcgtata cagttgaaaa catttttgtt 2820

ttgattggaa ggcagattat tttatattag tattaaaaat caaaccctat gtttctttca 2880

gatgaatctt ccaaagtgga ttatattaag caggtattag atttagaaaa cctttccatt 2940

tcttaaagta ttatcaagtg tcaagatcag caagtgtcct taagtcaaat aggttttttt 3000

ttgttggtgg ttgtgcttgc tttccttttt tagaaagttc tagaaaatag gaaaacgaaa 3060

aatttcattg agatgagtag tgcatttaat tattttttaa aaaacttttt aagtacttga 3120

attttatatc aggaaaacaa agttgttgag ccttgcttct tccgttttgc cctttgtctc 3180

gctccttatt ctttttgggg gggagggtta tttgcttttt tatcttcctg gcataatttc 3240

cattttattc ttctgagtgt ctatgttaac ttccctctat cccgcttata aaaaaattct 3300

ccaacaaaaa tacttgttga cttgatgttt tatcacttct ctaagtaagg ttgaaatatc 3360

cttattgtag ctactgtttt taatgtaaag gttaaacttg aaaagaaatt cttaatcacg 3420

gtgccaaaat tcattttcta acaccatgtg ttagaaaatt ataaaaaata aaataatttt 3480

aaaaaaaaaa                                                          349

<210>  60

<211>  3730

<212>  DNA

<213>  Homo sapiens

<220>

<221>  procollagen-lysine, 2-oxoglutarate 5-dioxygenase 2 transcript
       variant 1

<222>  (1)..(3730)

<223>  LocusID: 5352; NM_182943

<400>  60
ccacgcgtcc gctcagctgc gttacgcgcc gctccggctg ccggccgcgc gccttgcccg      60

ccggctcccg cccgcaatcg gcggctcagg gcggacccgg gtctctgcgt tctcgcgaga     120

agcgcggcgc tgcggggccg tgggcgcctg agcccgcgcg gccctcgagg gccgaatatg     180

gggggatgca cggtgaagcc tcagctgctg ctcctggcgc tcgtcctcca cccctggaat     240

ccctgtctgg gtgcggactc ggagaagccc tcgagcatcc ccacagataa attattagtc     300

ataactgtag caacaaaaga aagtgatgga ttccatcgat ttatgcagtc agccaaatat     360

ttcaattata ctgtgaaggt ccttggtcaa ggagaagaat ggagaggtgg tgatggaatt     420

aatagtattg gagggggcca gaaagtgaga ttaatgaaag aagtcatgga acactatgct     480

gatcaagatg atctggttgt catgtttact gaatgctttg atgtcatatt tgctggtggt     540

ccagaagaag ttctaaaaaa attccaaaag gcaaaccaca aagtggtctt tgcagcagat     600

ggaattttgt ggccagataa aagactagca gacaagtatc ctgttgtgca cattgggaaa     660

cgctatctga attcaggagg atttattggc tatgctccat atgtcaaccg tatagttcaa     720

caatggaatc tccaggataa tgatgatgat cagctctttt acactaaagt ttacattgat     780

ccactgaaaa gggaagctat taacatcaca ttggatcaca aatgcaaaat tttccagacc     840

ttaaatggag ctgtagatga agttgtttta aaatttgaaa atggcaaagc cagagctaag     900

aatacatttt atgaaacatt accagtggca attaatggaa atggacccac caagattctc     960

ctgaattatt ttggaaacta tgtacccaat tcatggacac aggataatgg ctgcactctt    1020

tgtgaattcg atacagtcga cttgtctgca gtagatgtcc atccaaacgt atcaataggt    1080

gtttttattg agcaaccaac cccttttcta cctcggtttc tggacatatt gttgacactg    1140

gattacccaa aagaagcact taaacttttt attcataaca aagaagttta tcatgaaaag 1200
gacatcaagg tattttttga taaagctaag catgaaatca aaactataaa aatagtagga 1260
ccagaagaaa atctaagtca agcggaagcc agaaacatgg gaatggactt ttgccgtcag 1320
gatgaaaagt gtgattatta ctttagtgtg gatgcagatg ttgttttgac aaatccaagg 1380
actttaaaaa ttttgattga acaaaacaga aagatcattg ctcctcttgt aactcgtcat 1440
ggaaagctgt ggtccaattt ctggggagca ttgagtcctg atggatacta tgcacgatct 1500
gaagattatg tggatattgt tcaagggaat agagtaggag tatggaatgt cccatatatg 1560
gctaatgtgt acttaattaa aggaaagaca ctccgatcag agatgaatga aaggaactat 1620
tttgttcgtg ataaactgga tcctgatatg gctctttgcc gaaatgctag agaaatgact 1680
ttacaaaggg aaaaagactc ccctactccg gaaacattcc aaatgctcag ccccccaaag 1740
ggtgtattta tgtacatttc taatagacat gaatttggaa ggctattatc cactgctaat 1800
tacaatactt cccattataa caatgacctc tggcagattt ttgaaaatcc tgtggactgg 1860
aaggaaaagt atataaaccg tgattattca aagattttca ctgaaaatat agttgaacag 1920
ccctgtccag atgtcttttg gttccccata ttttctgaaa aagcctgtga tgaattggta 1980
gaagaaatgg aacattacgg caaatggtct gggggaaaac atcatgatag ccgtatatct 2040
ggtggttatg aaaatgtccc aactgatgat atccacatga agcaagttga tctggagaat 2100
gtatggcttc attttatccg ggagttcatt gcaccagtta cactgaaggt ctttgcaggc 2160
tattatacga agggatttgc actactgaat tttgtagtaa aatactcccc tgaacgacag 2220
cgttctcttc gtcctcatca tgatgcttct acatttacca taaacattgc acttaataac 2280
gtgggagaag actttcaggg aggtggttgc aaatttctaa ggtacaattg ctctattgag 2340
tcaccacgaa aaggctggag cttcatgcat cctgggagac tcacacattt gcatgaagga 2400
cttcctgtta aaaatggaac aagatacatt gcagtgtcat ttatagatcc ctaagttatt 2460
tacttttcat tgaattgaaa tttattttgg gtgaatgact ggcatgaaca cgtctttgaa 2520
gttgtggctg agaagatgag aggaatattt aaataacatc aacagaacaa cttcactttg 2580
ggccaaacat ttgaaaaact ttttataaaa aattgtttga tatttcttaa tgtctgctct 2640
gagccttaaa acacagattg aagaagaaaa gaaagaaaaa acttaaatat ttatttctat 2700
gctttgttgc ctctgagaat aatgacaatt tatgaatttg tgtttcaaat tgataaaata 2760
tttaggtaca aataacaaga ctaataatat tttcttattt aaaaaaagca tgggaagatt 2820
tttatttatc aaaatataga ggaaatgtag acaaaatgga tataaatgaa aattaccatg 2880
ttgtaaaacc ttgaaaatca gattctaact ggatttgtat gcaactaagt atttttctga 2940
acacctatgc aggtcttatt tacagtagtt actaagggaa cacacaaaga attacacaac 3000

gttttcctca agaaaatggt acaaaacaca accgaggagc gtatacagtt gaaaacattt 3060

ttgttttgat tggaaggcag attattttat attagtatta aaaatcaaac cctatgtttc 3120

tttcagatga atcttccaaa gtggattata ttaagcaggt attagattta ggaaaacctt 3180

tccatttctt aaagtattat caagtgtcaa gatcagcaag tgtccttaag tcaaacaggt 3240

tttttttgtt gttgtttttg ctttgtttcc ttttttagaa agttctagaa aataggaaaa 3300

cgaaaaattt cattgagatg agtagtgcat ttaattattt tttaaaaaac tttttaagta 3360

cttgaatttt atatcaggaa aacaaagttg ttgagccttg cttcttccgt tttgcccttt 3420

gtctcgctcc ttattctttt tttgggggga gggttatttg cttttttatc ttcctggcat 3480

aatttccatt ttattcttct gagtgtctat gttaacttcc ctctatcccg cttataaaaa 3540

aattctccaa caaaaatact tgttgacttg atgttttatc acttctctaa gtaaggttga 3600

aatatcctta ttgtagctac tgtttttaat gtaaaggtta aacttgaaaa gaaattctta 3660

atcacggtgc caaaattcat tttctaacac catgtgttag aaaattataa aaaataaaat 3720

aattttagaa 3730

<210> 61

<211> 2950

<212> DNA

<213> Homo sapiens

<220>

<221> UDP-glucose dehydrogenase

<222> (1)..(2950)

<223> LocusID: 7358; NM_003359

<400> 61

gcggcccgta ggaaggtgct gtccgatcga tcgggatagg agcggtccct gcgcttgctg 60

ctgggaagtg gtacaatcat gtttgaaatt aagaagatct gttgcatcgg tgcaggctat 120

gttggaggac ccacatgtag tgtcattgct catatgtgtc ctgaaatcag ggtaacggtt 180

gttgatgtca atgaatcaag aatcaatgcg tggaattctc ctacacttcc tatttatgag 240

ccaggactaa aagaagtggt agaatcctgt cgaggaaaaa atcttttttt ttctaccaat 300

attgatgatg ccatcaaaga agctgatctt gtatttattt ctgtgaatac tccaacaaaa 360

acctatggaa tggggaaagg ccgggcagca gatctgaagt atattgaagc ttgtgctaga 420

cgcattgtgc aaaactcaaa tgggtacaaa attgtgactg agaaaagcac agttccagtg 480

cgggcagcag aaagtatccg tcgcatattt gatgcaaaca caaaacccaa cttgaattta 540
caggtgctgt ccaaccctga gtttctggca gagggaacag ccatcaagga cctaaagaac 600
ccagacagag tactgattgg aggggatgaa actccagagg gccagagagc tgtgcaggcc 660
ctgtgtgctg tatatgagca ctgggttccc agagaaaaga tcctcaccac taatacttgg 720
tcttcagagc tttccaaact ggcagcaaat gcttttcttg cccagagaat aagcagcatt 780
aactccataa gtgctctgtg tgaagcaaca ggagctgatg tagaagaggt agcaacagcg 840
attggaatgg accagagaat tggaaacaag tttctaaaag ccagtgttgg gtttggtggg 900
agctgtttcc aaaaggatgt tctgaatttg gtttatctct gtgaggctct gaatttgcca 960
gaagtagctc gttattggca gcaggtcata gacatgaatg actaccagag gaggaggttt 1020
gcttcccgga tcatagatag tctgtttaat acagtaactg ataagaagat agctattttg 1080
ggatttgcat tcaaaaagga cactggtgat acaagagaat cttctagtat atatattagc 1140
aaatatttga tggatgaagg tgcacatcta catatatatg atccaaaagt acctagggaa 1200
caaatagttg tggatctttc tcatccaggt gtttcagagg atgaccaagt gtcccggctc 1260
gtgaccattt ccaaggatcc atatgaagca tgtgatggtg cccatgctgt tgttatttgc 1320
actgagtggg acatgtttaa ggaattggat tatgaacgca ttcataaaaa aatgctaaag 1380
ccagccttta tcttcgatgg acggcgtgtc ctggatgggc tccacaatga actacaaacc 1440
attggcttcc agattgaaac aattggcaaa aaggtgtctt caaagagaat tccatatgct 1500
ccttctggtg aaattccgaa gtttagtctt caagatccac ctaacaagaa acctaaagtg 1560
tagagattgc catttttatt tgtgattttt tttttttttt ggtacttcag gatagcaaat 1620
atctatctgc tattaaatgg taaatgaacc aagtgttttt ttttgttttt tttttgagac 1680
agagtctcac tgttgcccag gctggagtgc agtggtgcaa tctcggctca ctgcaagctc 1740
tgcttcccag gttcacgcca ttctcctggc tcagcctccc aagtagctgg gactacaggc 1800
acccgccaca gtgcctgggc taattttttg tatttttagt agagacaggg tttcaccatg 1860
tgagccagga tggtctcaat ctcctgacct tgtgaaccac ccgtctcggc ctcccaaagt 1920
gctgggatta caggtgtgag ccaccacgcc tgggcccatg aaccaagtgt ttttaaggaa 1980
acaaaactat ttttttaatc atcagattta tactagctat atggatatta gcatatctgg 2040
taattatgaa tctagaattt ttttacatat ttttataata ctgttagctc agttattgga 2100
tgagtgaaag ataatcatgt tggttttaat agtgtcaatt tttgtaaaat aaaaattaaa 2160
cttcaaactc tttactttat aaattgtcca taggccacac tttaatatca cattataaag 2220
ggaaggacag tcttcattcc tcctggttat tggtttgttt gtcattaaag atatattttg 2280
aatccatgaa attgctatgc taaacagcct ttacatgtat ggtctggtta aagttccttt 2340
gttccttttg ttttaataaa atgtgtcact gattttttag ctcaaaatca tcactgttaa 2400

tttccagtca ccccaaatat ggttaaaaga tttttttttt taatcatgaa gagaaaatta 2460

gtagcatttc tttctctccc cattatttat tggttttcct cactaatctt ttttttttta 2520

gtccaaaagc caaaaatatt tatcttggtt ttacatttta atttccattc ttaattgtaa 2580

tttttttctt taaataagga aaccaatata atctcatgta taaaaactta aatattttac 2640

aagttacata tagcatcatt ctaaaataag aatttttttt gttttctgtc tgcttttttc 2700

ttatgtctct tgttgagttt tatattttca gtggttattt ttgcttgtgt tagatcatta 2760

ttaaaatata tccaatgtcc ctttgatact tgtgctctgc tgagaatgta cagtttgcat 2820

taaacatccc aggtctcatc cttcaggaat tttgcagttc aatgagaaga gggagacaaa 2880

tataaagatg aggacagaag catctctaca gatgaaaatt acataaataa aacattctcc 2940

atcaacaaaa 2950

<210> 62

<211> 3878

<212> DNA

<213> Homo sapiens

<220>

<221> phosphatidylinositol-4-phosphate 5-kinase, type II beta
      transcript variant 1

<222> (1)..(3878)

<223> LocusID: 8396; NM_003559

<400> 62

ttgcgggaaa gagccaaacc ctggcgttgg ggggcccggg cggggagccc ctcccgcggt 60

ccacagcgac gcctgcccag ccctcctccc cttccggctc cggcacgggg ccccgaggcg 120

ttcggaggcc aggcgggttt ctgtcaggcc cggggaggag gggcgggcgg ggcggccgct 180

gcctccccgg gacgggccgt accacgcgga cggggaggac ggggccaggg gactgcaggg 240

cggctgcacc gcccgggggc ggggtgcgga gcgggccggc gggctccccg gggcggggcg 300

ggagggcggg gcgtggggcg gacggaacca ccggggcggg gtgggaggta acgggacggg 360

cgcgaccatg gcgcggtgag ggagcggggg tggggatcgg tccgggggag gcctgaggcc 420

gctggcttgt gcgctgtctc cgccgccccc ctctttcgcc gccgccgccg ccgccccggg 480

catgtcgtcc aactgcacca gcaccacggc ggtggcggtg gcgccgctca gcgccagcaa 540

gaccaagacc aagaagaagc atttcgtgtg ccagaaagtg aagctattcc gggccagcga 600

gccgatcctc agcgtcctga tgtggggggt gaaccacacg atcaatgagc tgagcaatgt 660
tcctgttcct gtcatgctaa tgccagatga cttcaaagcc tacagcaaga tcaaggtgga 720
caatcatctc ttcaataagg agaacctgcc cagccgcttt aagtttaagg agtattgccc 780
catggtgttc cgaaaccttc gggagaggtt tggaattgat gatcaggatt accagaattc 840
agtgacgcgc agcgccccca tcaacagtga cagccagggt cggtgtggca cgcgtttcct 900
caccacctac gaccggcgct ttgtcatcaa gactgtgtcc agcgaggacg tggcggagat 960
gcacaacatc ttaaagaaat accaccagtt tatagtggag tgtcatggca acacgctttt 1020
gccacagttc ctgggcatgt accgcctgac cgtggatggt gtggaaacct acatggtggt 1080
taccaggaac gtgttcagcc atcggctcac tgtgcatcgc aagtatgacc tcaagggttc 1140
tacggttgcc agagaagcga gcgacaagga gaaggccaag gacttgccaa cattcaaaga 1200
caatgacttc ctcaatgaag ggcagaagct gcatgtggga gaggagagta aaaagaactt 1260
cctggagaaa ctgaagcggg acgttgagtt cttggcacag ctgaagatca tggactacag 1320
cctgctggtg ggcatccacg acgtggaccg ggcagagcag gaggagatgg aggtggagga 1380
gcgggcagag gacgaggagt gtgagaatga tggggtgggt ggcaacctac tctgctccta 1440
tggcacacct ccggacagcc ctggcaacct cctcagcttt cctcggttct ttggtcctgg 1500
ggaattcgac ccctctgttg acgtctatgc catgaaaagc catgaaagtt cccccaagaa 1560
ggaggtgtat ttcatggcca tcattgatat cctcacgcca tacgatacaa agaagaaagc 1620
tgcacatgct gccaaaacgg tgaaacacgg ggcaggggcc gagatctcga ctgtgaaccc 1680
tgagcagtac tccaaacgct tcaacgagtt tatgtccaac atcctgacgt agttctcttc 1740
taccttcagc cagagccaga gagctggata tggggtcggg gatcgggagt tagggagaag 1800
ggtgtatttg ggctagatgg gagggtggga gcagagtcgg gtttgggagg gctttagcaa 1860
tgagactgca gcctgtgaca ccgaaagaga ctttagctga agaggagggg gatgtgctgt 1920
gtgtgcacct gctcacagga tgtaacccca ccttctgctt acccttgatt ttttctcccc 1980
atttgacacc caggttaaaa aggggttccc tttttggtac cttgtaacct tttaagatac 2040
cttggggcta gagatgactt cgtgggttta tttgggtttt gtttctgaaa tttcattgct 2100
ccaggtttgc tatttataat catatttcat cagcctaccc accctcccca tctttgctga 2160
gctctcagtt cccttcaatt aaagagatac ccggtagacc cagcacaagg gtccttccag 2220
aaccaagtgc tatggatgcc agattggaga ggtcagacac ctcgccctgc tgcatttgct 2280
cttgtctgga ttaactttgt aatttatgga gtattgtgca caacttcctc cacctttccc 2340
ttggattcaa gtgaaaactg ttgcattatt cctccatcct gtctggaata caccaggtca 2400
acaccagaga tctcagatca gaatcagaga tctcagaggg gaataagttc atcctcatgg 2460
gatggtgagg ggcaggaaag cggctgggct cttggacacc tggttctcag agaaccctgt 2520

gatgatcacc caagccccag gctgtcttag cccctggagt tcagaagtcc tctctgtaaa 2580

gcctgcctcc cactaggtca agaggaacta gagtaccttt ggatttatca ggaccctcat 2640

gtttaaatgg ttatttccct ttgggaaaac ttcagaaact gatgtatcaa atgaggccct 2700

gtgccctcga tctatttcct tcttccttct gacctcctcc caggcactct tacttctagc 2760

cgaactctta gctctgggca gatctccaag cgcctggagt gctttttagc agagacacct 2820

cgttaagctc cgggatgacc ttgtaggaga tctgtctccc tgtgcctgga gagttacagc 2880

cagcaaggtg cccccatctt agagtgtggt gtccaaacgt gaggtggctt cctagttaca 2940

tgaggatgtg atccaggaaa tccagtttgg aggcttgatg tgggttttga cctggcctca 3000

gccttggggc tgtttttcct tgttgccccg ctctagactt ttagcagatc tgcagcccac 3060

aggctttttt ggaaggagtg gcttcctgca ggtgttccac ctgccttcgg agcctgccac 3120

ccaggccctc agaactgagc cacaggctgc tctggccagg agagaaacag ctctgttgtt 3180

ctgcattggg ggaggtacat tcctgcatct tctcaccccc tcaaccagga actggggatt 3240

tgggatgaga tatggtcaga cttgtagata accccaaaga tgtgaagatc gcttgtgaaa 3300

ccattttgaa tgaatagatt ggtttcctgt ggctccctcc aaacctggcc aagcccagct 3360

tccgaagcag gaaccagcac tgtctctgtg cctgactcac agcatatagg tcaggaaaga 3420

atggagacgg cattcttgga cttcactggg gctgctggat tggatgggaa accttctgga 3480

agaggcagat gggggtcaaa ccactgcctt ggccccagga aggggccata ggtaggtctg 3540

aacaactgcc gcaagaccac tacatgactt agggaacttg aaaccaactg gctcatggag 3600

aaaacaaatt tgacttggga aagggattat gtaggaataa tgtttggact tgatttcccc 3660

acgtcataat gaagaatgga agtttggatc tgctcctcgt caggcgcagc atctctgaag 3720

cttggaaagc tgtcttccag cagcctccgt ggcctcgggt tcctaccggc ttctctgcat 3780

ttggtctgct gatcatgttg ccataatgtg tatggaaagt gtaacacatt cttactggtt 3840

aaagacgact accaggtatc taacttgttt aacattga 3878

<210> 63

<211> 1430

<212> DNA

<213> Homo sapiens

<220>

<221> phosphatidylinositol-4-phosphate 5-kinase, type II,beta
      transcript variant 2

<222>  (1)..(1430)

<223>  LocusID: 8396; NM_138687

<400>  63

ttgcgggaaa gagccaaacc ctggcgttgg ggggcccggg cggggagccc ctcccgcggt    60

ccacagcgac gcctgcccag ccctcctccc cttccggctc cggcacgggg ccccgaggcg   120

ttcggaggcc aggcgggttt ctgtcaggcc cggggaggag gggcgggcgg ggcggccgct   180

gcctccccgg gacgggccgt accacgcgga cggggaggac ggggccaggg gactgcaggg   240

cggctgcacc gcccgggggc ggggtgcgga gcgggccggc gggctccccg gggcggggcg   300

ggagggcggg gcgtggggcg gacggaacca ccggggcggg gtgggaggta acgggacggg   360

cgcgaccatg gcgcggtgag ggagcggggg tggggatcgg tccgggggag gcctgaggcc   420

gctggcttgt gcgctgtctc cgccgccccc ctctttcgcc gccgccgccg ccgccccggg   480

catgtcgtcc aactgcacca gcaccacggc ggtggcggtg gcgccgctca gcgccagcaa   540

gaccaagacc aagaagaagc atttcgtgtg ccagaaagtg aagctattcc gggccagcga   600

gccgatcctc agcgtcctga tgtggggggt gaaccacacg atcaatgagc tgagcaatgt   660

tcctgttcct gtcatgctaa tgccagatga cttcaaagcc tacagcaaga tcaaggtgga   720

caatcatctc ttcaataagg agaacctgcc cagccgcttt aagtttaagg agtattgccc   780

catggtgttc cgaaaccttc gggagaggtt tggaattgat gatcaggatt accagaattc   840

agtgacgcgc agcgccccca tcaacagtga cagccagggt cggtgtggca cgcgtttcct   900

caccacctac gaccggcgct ttgtcatcaa gactgtgtcc agcgaggacg tggcggagat   960

gcacaacatc ttaaagaaat accaccagtt tatagtggag tgtcatggca acacgctttt  1020

gccacagttc ctgggcatgt accgcctgac cgtggatggt gtggaaacct acatggtggt  1080

taccaggaac gtgttcagcc atcggctcac tgtgcatcgc aagtatgacc tcaagggttc  1140

tacggttgcc agagaagcga gcgacaagga gaaggccaag gacttgccaa cattcaaaga  1200

caatgacttc ctcaatgaag ggcagaagct gcatgtggga gaggagagta aaaagaactt  1260

cctggagaaa ctgaagcggg acgttgagga gattctcgtt ctgtccccag gctggagaat  1320

cgcttgaacc caggaggcgg aggttgcggt gaccctccag cttgggcaac atgttatgaa  1380

tgaaactcca tctcaaaaaa taaaaaaaaa aaaaggaagc aaaaaaaaaa              1430

<210>  64

<211>  1750

<212>  DNA

<213>  Homo sapiens

<220>

<221>  MAP kinase interacting serine/threonine kinase 2 sequence1

<222>  (1)..(1750)

<223>  LocusID: 2872; NM_017572

<400>  64

cagcggcggt ggcccaggta gaggggtccg cgctggcggc ggcggcggcg ctgttccccg    60

cgcggtccgc ggagcggggt ccgggctgcg cgacgtgggg cggcggcggc actgcggccc   120

cggcccaagc ccgaccccgg gtcccctcct cggccgcccc ccgcccggcc gcccgccctc   180

gggcctcccc ccgggccctc ggtcccctcc cccgctggcg gggcccggac agaagatggt   240

gcagaagaaa ccagccgaac ttcagggttt ccaccgttcg ttcaaggggc agaacccctt   300

cgagctggcc ttctccctag accagcccga ccacggagac tctgactttg gcctgcagtg   360

ctcagcccgc cctgacatgc ccgccagcca gcccattgac atcccggacg ccaagaagag   420

gggcaagaag aagaagcgcg gccgggccac cgacagcttc tcgggcaggt ttgaagacgt   480

ctaccagctg caggaagatg tgctggggga gggcgctcat gcccgagtgc agacctgcat   540

caacctgatc accagccagg agtacgccgt caagatcatt gagaagcagc caggccacat   600

tcggagcagg gttttcaggg aggtggagat gctgtaccag tgccagggac acaggaacgt   660

cctagagctg attgagttct tcgaggagga ggaccgcttc tacctggtgt ttgagaagat   720

gcggggaggc tccatcctga gccacatcca caagcgccgg cacttcaacg agctggaggc   780

cagcgtggtg gtgcaggacg tggccagcgc cttggacttt ctgcataaca aaggcatcgc   840

ccacagggac ctaaagccgg aaaacatcct ctgtgagcac cccaaccagg tctcccccgt   900

gaagatctgt gacttcgacc tgggcagcgg catcaaactc aacggggact gctcccctat   960

ctccaccccg gagctgctca ctccgtgcgg ctcggcggag tacatggccc cggaggtagt  1020

ggaggccttc agcgaggagg ctagcatcta cgacaagcgc tgcgacctgt ggagcctggg  1080

cgtcatcttg tatatcctac tcagcggcta cccgcccttc gtgggccgct gtggcagcga  1140

ctgcggctgg gaccgcggcg aggcctgccc tgcctgccag aacatgctgt ttgagagcat  1200

ccaggagggc aagtacgagt tccccgacaa ggactgggcc cacatctcct gcgctgccaa  1260

agacctcatc tccaagctgc tggtccgtga cgccaagcag aggctgagtg ccgcccaagt  1320

cctgcagcac ccctgggttc aggggtgcgc cccggagaac accttgccca ctcccatggt  1380

cctgcagagg tgggacagtc acttcctcct ccctccccac ccctgtcgca tccacgtgcg  1440

acctggagga ctggtcagaa ccgttactgt gaatgagtga agatcctgga ggaccctggg  1500

ccccaggcca gctcccatcg ctgggggacg gtgaacggcc atgtgttaat gttacgatgt  1560

ttttaaaaga caaaaaaaaa aaaaaaacct caaaagtttt tttaaagtgg gggaaaaaca 1620

tccaagcact ttaattccaa tgtaccaggt gaactgacgg agctcagaag ttttccttta 1680

caccaactgt caatgccgga attttgtatt ctgttttgta aagatttaat aaaagtcaaa 1740

aaacttgcaa 1750

<210> 65

<211> 3790

<212> DNA

<213> Homo sapiens

<220>

<221> MAP kinase interacting serine/threonine kinase 2 sequence 2

<222> (1)..(3790)

<223> LocusID: 2872; NM_199054

<400> 65

cagcggcggt ggcccaggta gaggggtccg cgctggcggc ggcggcggcg ctgttccccg 60

cgcggtccgc ggagcggggt ccgggctgcg cgacgtgggg cggcggcggc actgcggccc 120

cggcccaagc ccgaccccgg gtcccctcct cggccgcccc ccgcccggcc gcccgccctc 180

gggcctcccc ccgggccctc ggtcccctcc cccgctggcg gggcccggac agaagatggt 240

gcagaagaaa ccagccgaac ttcagggttt ccaccgttcg ttcaaggggc agaacccctt 300

cgagctggcc ttctccctag accagcccga ccacggagac tctgactttg gcctgcagtg 360

ctcagcccgc cctgacatgc ccgccagcca gcccattgac atcccggacg ccaagaagag 420

gggcaagaag aagaagcgcg gccgggccac cgacagcttc tcgggcaggt ttgaagacgt 480

ctaccagctg caggaagatg tgctggggga gggcgctcat gcccgagtgc agacctgcat 540

caacctgatc accagccagg agtacgccgt caagatcatt gagaagcagc caggccacat 600

tcggagcagg gttttcaggg aggtggagat gctgtaccag tgccagggac acaggaacgt 660

cctagagctg attgagttct tcgaggagga ggaccgcttc tacctggtgt ttgagaagat 720

gcggggaggc tccatcctga gccacatcca caagcgccgg cacttcaacg agctggaggc 780

cagcgtggtg gtgcaggacg tggccagcgc cttggacttt ctgcataaca aaggcatcgc 840

ccacagggac ctaaagccgg aaaacatcct ctgtgagcac cccaaccagg tctcccccgt 900

gaagatctgt gacttcgacc tgggcagcgg catcaaactc aacggggact gctcccctat 960

ctccaccccg gagctgctca ctccgtgcgg ctcggcggag tacatggccc cggaggtagt 1020
ggaggccttc agcgaggagg ctagcatcta cgacaagcgc tgcgacctgt ggagcctggg 1080
cgtcatcttg tatatcctac tcagcggcta cccgcccttc gtgggccgct gtggcagcga 1140
ctgcggctgg gaccgcggcg aggcctgccc tgcctgccag aacatgctgt ttgagagcat 1200
ccaggagggc aagtacgagt tccccgacaa ggactgggcc cacatctcct gcgctgccaa 1260
agacctcatc tccaagctgc tggtccgtga cgccaagcag aggctgagtg ccgcccaagt 1320
cctgcagcac ccctgggttc aggggtgcgc cccggagaac accttgccca ctcccatggt 1380
cctgcagagg aacagctgtg ccaaagacct cacgtccttc gcggctgagg ccattgccat 1440
gaaccggcag ctggcccagc acgacgagga cctggctgag gaggaggccg cggggcaggg 1500
ccagcccgtc ctggtccgag ctacctcacg ctgcctgcag ctgtctccac cctcccagtc 1560
caagctggcg cagcggcggc aaagggccag tctgtcctcg gccccagtgg tcctggtggg 1620
agaccacgcc tgaccctccc atctcccctc tgtacatagg tcacccgtcc cccaatcaaa 1680
tctaaaggtt ttttaagcta tcgccagccg gtgtccagcg ggctgcccct cctctgcctg 1740
gattcccagg cactaagctc agctgagggg ggtgttttat agaaggtttt tgcttttggg 1800
tttttttttt cctgtttcca cccctccccg ttatttttcc ctttggatgg ttaaaagcat 1860
tgcaggcacc cgggaaggtg agcagagggt aggtgggtgg gcttgtcccc tccccggtcc 1920
cccgccctgc tcacctctac tatgaaggtg cccccaggtc acctgtgctg cccgccatct 1980
gcccacgtgg cttgcagtga ctcaggagag caggcccaca gcgtttgcca tcttgcagag 2040
ctggggaggg gcacaggacc ctgccctcgt gttccctccc agcccgcagt atttcaggga 2100
caggctcttc ccctctatcc ctcaccctga gagcacccct ggtggcttgg ttggggaagg 2160
gaggggctgc ctgtctctgg aggtgtcagg caggcaggtg gcaggcagct cacccaccca 2220
ccccatggga tcccccagcc cttcacccgc gcctgccttg tccccatgat agttgacaat 2280
cggggcttcc tgcaaggccc gtctgtctgt ccaggactcc tggtggccag attcggcctc 2340
cgaccttgac cttaaactgc agctgacccc aggggctcgc cgctgcccct cccctccaca 2400
ccaaggcctg agacagcagg agccccgcct ggcccgaagc cgtttccacc gcagcaggca 2460
gaggggctgg acaggcactg tcagccaatg tggggggtcc tgaagacacc cccttggggc 2520
acccgagtgc cccttctcag ggctcagtct gaccgtagcc acgtcctgcc tcgcgccgcc 2580
cctcgggcct gacctggaag ctccgtcagc tccgtccttg tccttagagc tgagcccaga 2640
ccccggggtc tggccgaatc ctcaccccca gggcagtgtt tttggtctgc caccttcagg 2700
aaaacggctg cggcctcggc ctcccttcgg gcacccagga atgcgggggt ctgctcagtc 2760
cccccaccct ccatgctcca acccccgggg gctgcggagc ctgctgcccc ctccccgcgg 2820

gtggggacgt tctatgcaat acagggttcc actttagaag tgcgcgcggc tagggtcacc 2880

gcccgccctt cccggcgcag cccccgagct ccacagctgg ggcagcccct ctggcttcta 2940

aatccgcggt cgggattctt cctcctgttt agttttttag tttttcctta aaaaaaaaca 3000

acacatcgat ggactttgct tccctgttct tgaagaatac ttgaatgtcg gggggcctgg 3060

gggtgggggc ctcggagacc gtctgcctgg ccctgctgcc cctcctgaat ctcgtatgat 3120

ggtcacagtc cggtggccgt gggggtgctc tgccttccct ggtccccact gcccatatct 3180

gtggactgcc ccttccaaag acccctgggg ggggtggggc attccgccca cccctttccc 3240

ccatcacttc tcgcctgtca gtgattccat gtttcgtaac gggggattct ctgccttttt 3300

gtatcaaaga acaagcaaat ggacccccgc ccgctgcagg cgcccatagc catcgggtct 3360

ctaaagctga gtggctagca gcgtttgttt gtttgttttt tttttttttt ctgaaggtgg 3420

gacagtcact tcctcctccc tccccacccc tgtcgcatcc acgtgcgacc tggaggactg 3480

gtcagaaccg ttactgtgaa tgagtgaaga tcctggagga ccctgggccc caggccagct 3540

cccatcgctg ggggacggtg aacggccatg tgttaatgtt acgatgtttt taaaagacaa 3600

aaaaaaaaaa aaaacctcaa aagttttttt aaagtggggg aaaaacatcc aagcacttta 3660

attccaatgt accaggtgaa ctgacggagc tcagaagttt tcctttacac caactgtcaa 3720

tgccggaatt ttgtattctg ttttgtaaag atttaataaa agtcaaaaaa cttgcaaaaa 3780

aaaaaaaaaa 3790

<210> 66

<211> 2443

<212> DNA

<213> Homo sapiens

<220>

<221> carboxypeptidase E

<222> (1)..(2443)

<223> LocusID: 1363; NM_001873

<400> 66

aaatggcgtg cccgtctctc cgccggcccc ctgcctcgca gtggtttctc ctgcagctcc 60

cctgggctcc gcggccagta gtgcagcccg tggagccgcg gctttgcccg tctcctctgg 120

gtggccccag tgcgcgggct gacactcatt cagccgggga aggtgaggcg agtagaggct 180

ggtgcggaac ttgccgcccc cagcagcgcc ggcgggctaa gcccagggcc gggcagacaa 240

aagaggccgc ccgcgtagga aggcacggcc ggcggcggcg gagcgcagcg atggccgggc 300
gagggggcag cgcgctgctg gctctgtgcg gggcactggc tgcctgcggg tggctcctgg 360
gcgccgaagc ccaggagccc ggggcgcccg cggcgggcat gaggcggcgc cggcggctgc 420
agcaagagga cggcatctcc ttcgagtacc accgctaccc cgagctgcgc gaggcgctcg 480
tgtccgtgtg gctgcagtgc accgccatca gcaggattta cacggtgggg cgcagcttcg 540
agggccggga gctcctggtc atcgagctgt ccgacaaccc tggcgtccat gagcctggtg 600
agcctgaatt taaatacatt gggaatatgc atgggaatga ggctgttgga cgagaactgc 660
tcattttctt ggcccagtac ctatgcaacg aataccagaa ggggaacgag acaattgtca 720
acctgatcca cagtacccgc attcacatca tgccttccct gaacccagat ggctttgaga 780
aggcagcgtc tcagcctggt gaactcaagg actggtttgt gggtcgaagc aatgcccagg 840
gaatagatct gaaccggaac tttccagacc tggataggat agtgtacgtg aatgagaaag 900
aaggtggtcc aaataatcat ctgttgaaaa atatgaagaa aattgtggat caaaacacaa 960
agcttgctcc tgagaccaag gctgtcattc attggattat ggatattcct tttgtgcttt 1020
ctgccaatct ccatggagga gaccttgtgg ccaattatcc atatgatgag acgcggagtg 1080
gtagtgctca cgaatacagc tcctccccag atgacgccat tttccaaagc ttggcccggg 1140
catactcttc tttcaacccg gccatgtctg accccaatcg gccaccatgt cgcaagaatg 1200
atgatgacag cagctttgta gatggaacca ccaacggtgg tgcttggtac agcgtacctg 1260
gagggatgca agacttcaat taccttagca gcaactgttt tgagatcacc gtggagctta 1320
gctgtgagaa gttcccacct gaagagactc tgaagaccta ctgggaggat aacaaaaact 1380
ccctcattag ctaccttgag cagatacacc gaggagttaa aggatttgtc cgagaccttc 1440
aaggtaaccc aattgcgaat gccaccatct ccgtggaagg aatagaccac gatgttacat 1500
ccgcaaagga tggtgattac tggagattgc ttatacctgg aaactataaa cttacagcct 1560
cagctccagg ctatctggca ataacaaaga aagtggcagt tccttacagc cctgctgctg 1620
ggttgatttt tgaactggag tcattttctg aaaggaaaga agaggagaag gaagaattga 1680
tggaatggtg gaaaatgatg tcagaaactt taaattttta aaaaggcttc tagttagctg 1740
ctttaaatct atctatataa tgtagtatga tgtaatgtgg tctttttttt agattttgtg 1800
cagttaatac ttaacattga tttatttttt aatcatttaa atattaatca actttcctta 1860
aaataaatag cctcttaggt aaaaatataa gaacttgata tatttcattc tcttatatag 1920
tattcatttt cctacctata ttacacaaaa aagtatagaa aagatttaag taattttgcc 1980
atcctaggct taaatgcaat attcctggta ttatttacaa tgcagaattt tttgagtaat 2040
tctagctttc aaaaattagt gaagttcttt tactgtaatt ggtgacaatg tcacataatg 2100
aatgctattg aaaaggttaa cagatacagc tcggagttgt gagcactcta ctgcaagact 2160

taaatagttc agtataaatt gtcgtttttt tcttgtgctg actaactata agcatgatct 2220

tgttaatgca tttttgatgg gaagaaaagg tacatgttta caaagaggtt ttatgaaaag 2280

aataaaaatt gacttcttgc ttgtacatat aggagcaata ctattatatt atgtagtccg 2340

ttaacactac ttaaaagttt agggttttct cttggttgta gagtggccca gaattgcatt 2400

ctgaatgaat aaaggttaaa aaaaaatccc cagtgaaaaa aaa 2443

<210> 67

<211> 1633

<212> DNA

<213> Homo sapiens

<220>

<221> carboxypeptidase A3 (mast cell)

<222> (1)..(1633)

<223> LocusID: 1359; NM_001870

<400> 67

aaagaagaac catgaggctc atcctgcctg tgggtttgat tgctaccact cttgcaattg 60

ctcctgtccg ctttgacagg gagaaggtgt tccgcgtgaa gccccaggat gaaaaacaag 120

cagacatcat aaaggacttg gccaaaacca atgagcttga cttctggtat ccaggtgcca 180

cccaccacgt agctgctaat atgatggtgg atttccgagt tagtgagaag gaatcccaag 240

ccatccagtc tgccttggat caaaataaaa tgcactatga aatcttgatt catgatctac 300

aagaagagat tgagaaacag tttgatgtta aagaagatat cccaggcagg cacagctacg 360

caaaatacaa taattgggaa aagattgtgg cttggactga aaagatgatg gataagtatc 420

ctgaaatggt ctctcgtatt aaaattggat ctactgttga agataatcca ctatatgttc 480

tgaagattgg ggaaaagaat gaaagaagaa aggctatttt tatggattgt ggcattcacg 540

cacgagaatg ggtctcccca gcattctgcc agtggtttgt ctatcaggca accaaaactt 600

atgggagaaa caaaattatg accaaactct tggaccgaat gaatttttac attcttcctg 660

tgttcaatgt tgatggatat atttggtcat ggacaaagaa ccgcatgtgg agaaaaaatc 720

gttccaagaa ccaaaactcc aaatgcatcg gcactgacct caacaggaat tttaatgctt 780

catggaactc cattcctaac accaatgacc catgtgcaga taactatcgg ggctctgcac 840

cagagtccga gaaagagacg aaagctgtca ctaatttcat tagaagccac ctgaatgaaa 900

tcaaggttta catcaccttc cattcctact cccagatgct attgtttccc tatggatata 960

catcaaaact gccacctaac catgaggact tggccaaagt tgcaaagatt ggcactgatg 1020
ttctatcaac tcgatatgaa acccgctaca tctatggccc aatagaatca acaatttacc 1080
cgatatcagg ttcttcttta gactgggctt atgacctggg catcaaacac acatttgcct 1140
ttgagctccg agataaaggc aaatttggtt ttctccttcc agaatcccgg ataaagccaa 1200
cgtgcagaga gaccatgcta gctgtcaaat ttattgccaa gtatatcctc aagcatactt 1260
cctaaagaac tgccctctgt ttggaataag ccaattaatc cttttttgtg cctttcatca 1320
gaaagtcaat cttcagttat ccccaaatgc agcttctatt tcacctgaat ccttctcttg 1380
ctcatttaag tcccatgtta ctgctgtttg cttttactta ctttcagtag caccataacg 1440
aagtagcttt aagtgaaacc ttttaactac ctttctttgc tccaagtgaa gtttggaccc 1500
agcagaaagc attattttga aaggtgatat acagtggggc acagaaaaca aatgaaaacc 1560
ctcagtttct cacagatttt caccatgtgg cttcatcaat ttatgtgcta atacaataaa 1620
ataaaatgca ctt 1633

<210> 68
<211> 1516
<212> DNA
<213> Homo sapiens
<220>
<221> solute carrier family 38, member 6
<222> (1)..(1516)
<223> LocusID: 145389; NM_153811
<400> 68
gtacggaatg ccggaagggc cgggctcaaa gctccgcctc tggcgcgacc gacgactgga 60
gcgcagggca ggggtagagg ctcgtagatg gaactggtag tcagctggag agcagcatgg 120
aggcgtcctg ggggagcttc aacgctgagc ggggctggta tgtctctgtc cagcagcctg 180
aagaagcgga ggccgaagag ttgagtccgt tgctaagcaa cgaacttcac agacagcgat 240
ccccaggtgt ttcatttggt ttatcagtgt ttaatttgat gaatgccatc atgggaagtg 300
gcatccttgg cttagcttat gttatggcta ataccggtgt ctttggattt agcttcttgc 360
tgctgacagt tgctctcctg gcttcttact cagtccatct tctgcttagt atgtgtattc 420
agacagctgt aacatcttat gaagatcttg gactctttgc atttggatta cctggaaagt 480
tggtggtggc aggcaccata ataattcaga atattggagc tatgtcatct tatcttttaa 540

ttattaaaac agagcttcct gctgctattg cagaattttt gactggagac tatagtagat 600

attggtatct tgatggacaa acactactaa taatcatatg tgttggcatt gtgttccctc 660

ttgcacttct tcccaaaata ggctttcttg gctacacaag tagtttatca tttttcttta 720

tgatgttctt tgctcttgtg gtaataatta aaaaatggtc catcccttgt cctctgacat 780

taaattatgt agagaaaggc ttccagattt caaatgttac agatgattgt aagccaaagc 840

tctttcattt ctccaaagag agtgcttatg ccttaccaac catggctttt tcatttctct 900

gccatacctc aatattgccc atatactgtg aacttcaaag tccttcaaag aaaagaatgc 960

agaatgttac caatacagca attgctttaa gttttctcat ttattttata tctgcactct 1020

ttgggtacct cactttttat gacaaagtgg agtcagaatt actaaaaggt tatagtaaat 1080

acttatcaca tgatgttgtt gtcatgactg tgaagttatg catactattt gctgtgcttt 1140

tgacagtccc tctaatccac ttccctgcca gaaaagctgt aacaatgatg tttttctcca 1200

attttccatt ctcatggatt cgccattttt tgatcactct agcactcaat attatcatcg 1260

ttttacttgc aatatatgtt cctgacatta gaaatgtatt tggtgtagtt ggtgccagta 1320

catcaacatg tttgattttt atattcccag gactatttta tcttaaactt agcagagagg 1380

attttctgtc atggaaaaag cttggggcat tcgttttgct catctttgga attttggttg 1440

ggaattttag tttagcactc atcatttttg attggattaa taaataaaag aaatattttc 1500

ctacttctta caagaa 1516

<210> 69

<211> 3630

<212> DNA

<213> Homo sapiens

<220>

<221> very low density lipoprotein receptor

<222> (1)..(3630)

<223> LocusID: 7436; NM_003383

<400> 69

ctctgcgggc cgcgggtgcg ggtcgtcgct accggctctc tccgttctgt gctctcttct 60

gctctcggct ccccaccccc tctcccttcc ctcctctccc cttgcctccc ctcctctgca 120

gcgcctgcat tattttctgc ccgcagctcg gcttgcactg ctgctgcagc ccggggaggt 180

ggctgggtgg gtggggagga gactgtgcaa gttgtagggg agggggtgcc ctcttcttcc 240
ccgctccctt ccccagccaa gtggttcccc tccttctccc cctttcccct cccagccccc 300
accttcttcc tctttcggaa gggctggtaa cttgtcgtgc ggagcgaacg gcggcggcgg 360
cggcggcggc ggcaccatcc aggcgggcac catgggcacg tccgcgctct gggccgtctg 420
gctgctgctc gcgctgtgct gggcgccccg ggagagcggc gccaccggaa ccgggagaaa 480
agccaaatgt gaaccctccc aattccagtg cacaaatggt cgctgtatta cgctgttgtg 540
gaaatgtgat ggggatgaag actgtgttga cggcagtgat gaaaagaact gtgtaaagaa 600
gacgtgtgct gaatctgact tcgtgtgcaa caatggccag tgtgttccca gccgatggaa 660
gtgtgatgga gatcctgact gcgaagatgg ttcagatgaa agcccagaac agtgccatat 720
gagaacatgc cgcatacatg aaatcagctg tggcgcccat tctactcagt gtatcccagt 780
gtcctggaga tgtgatggtg aaaatgattg tgacagtgga gaagatgaag aaaactgtgg 840
caatataaca tgtagtcccg acgagttcac ctgctccagt ggccgctgca tctccaggaa 900
ctttgtatgc aatggccagg atgactgcag cgatggcagt gatgagctgg actgtgcccc 960
gccaacctgt ggcgcccatg agttccagtg cagcacctcc tcctgcatcc ccatcagctg 1020
ggtatgcgac gatgatgcag actgctccga ccaatctgat gagtccctgg agcagtgtgg 1080
ccgtcagcca gtcatacaca ccaagtgtcc agccagcgaa atccagtgcg gctctggcga 1140
gtgcatccat aagaagtggc gatgtgatgg ggaccctgac tgcaaggatg gcagtgatga 1200
ggtcaactgt ccctctcgaa cttgccgacc tgaccaattt gaatgtgagg atggcagctg 1260
catccatggc agcaggcagt gtaatggtat ccgagactgt gtcgatggtt ccgatgaagt 1320
caactgcaaa aatgtcaatc agtgcttggg ccctggaaaa ttcaagtgca gaagtggaga 1380
atgcatagat atcagcaaag tatgtaacca ggagcaggac tgcagggact ggagtgatga 1440
gcccctgaaa gagtgtcata taaacgaatg cttggtaaat aatggtggat gttctcatat 1500
ctgcaaagac ctagttatag gctacgagtg tgactgtgca gctgggtttg aactgataga 1560
taggaaaacc tgtggagata ttgatgaatg ccaaaatcca ggaatctgca gtcaaatttg 1620
tatcaactta aaaggcggtt acaagtgtga atgtagtcgt gcctatcaaa tggatcttgc 1680
tactggcgtg tgcaaggcag taggcaaaga gccaagtctg atcttcacta atcgaagaga 1740
catcaggaag attggcttag agaggaaaga atatatccaa ctagttgaac agctaagaaa 1800
cactgtggct ctcgatgctg acattgctgc ccagaaacta ttctgggccg atctaagcca 1860
aaaggctatc ttcagtgcct caattgatga caaggttggt agacatgtta aaatgatcga 1920
caatgtctat aatcctgcag ccattgctgt tgattgggtg tacaagacca tctactggac 1980
tgatgcggct tctaagacta tttcagtagc taccctagat ggaaccaaga ggaagttcct 2040
gtttaactct gacttgcgag agcctgcctc catagctgtg gacccactgt ctggctttgt 2100

ttactggtca gactggggtg aaccagctaa aatagaaaaa gcaggaatga atggattcga 2160

tagacgtcca ctggtgacag cggatatcca gtggcctaac ggaattacac ttgaccttat 2220

aaaaagtcgc ctctattggc ttgattctaa gttgcacatg ttatccagcg tggacttgaa 2280

tggccaagat cgtaggatag tactaaagtc tctggagttc ctagctcatc ctcttgcact 2340

aacaatattt gaggatcgtg tctactggat agatggggaa aatgaagcag tctatggtgc 2400

caataaattc actggatcag agcatgccac tctagtcaac aacctgaatg atgcccaaga 2460

catcattgtc tatcatgaac ttgtacagcc atcaggtaaa aattggtgtg aagaagacat 2520

ggagaatgga ggatgtgaat acctatgcct gccagcacca cagattaatg atcactctcc 2580

aaaatatacc tgttcctgtc ccagtgggta caatgtagag gaaaatggcc gagactgtca 2640

aagtactgca actactgtga cttacagtga gacaaaagat acgaacacaa cagaaatttc 2700

agcaactagt ggactagttc ctggagggat caatgtgacc acagcagtat cagaggtcag 2760

tgttccccca aaagggactt ctgccgcatg ggccattctt cctctcttgc tcttagtgat 2820

ggcagcagta ggtggctact tgatgtggcg gaattggcaa cacaagaaca tgaaaagcat 2880

gaactttgac aatcctgtgt acttgaaaac cactgaagag gacctctcca tagacattgg 2940

tagacacagt gcttctgttg gacacacgta cccagcaata tcagttgtaa gcacagatga 3000

tgatctagct tgacttctgt gacaaatgtt gacctttgag gtctaaacaa ataatacccc 3060

cgtcggaatg gtaaccgagc cagcagctga agtctctttt tcttcctctc ggctggaaga 3120

acatcaagat acctttgcgt ggatcaagct tgctgtactt gaccgttttt atattacttt 3180

tgtaaatatt cttgtccaca ttctacttca gctttggatg tggttaccga gtatctgtaa 3240

cccttgaatt tctagacagt attgccacct ctggccaaat atgcactttc cctagaaagc 3300

catattccag cagtgaaact tgtgctatag tgtataccac ctgtacatac attgtatagg 3360

ccatctgtaa atatcccaga gaacaatcac tattcttaag cactttgaaa atatttctat 3420

gtaaattatt gtaaactttt tcaatggttg ggacaatggc aataggacaa aacgggttac 3480

taagatgaaa ttgccaaaaa aatttataaa ctaattttgg tacgtatgaa tgatatcttt 3540

gacctcaatg gaggtttgca aagactgagt gttcaaacta ctgtacattt tttttcaagt 3600

gctaaaaaat taaaccaagc agcttaaaaa 3630

<210> 70

<211> 924

<212> DNA

<213> Homo sapiens

<220>

<221>  cathepsin G

<222>  (1)..(924)

<223>  LocusID: 1511; NM_001911

<400>  70

gcacagcagc aactgactgg gcagcctttc aggaaagatg cagccactcc tgcttctgct    60

ggcctttctc ctacccactg gggctgaggc aggggagatc atcggaggcc gggagagcag   120

gccccactcc cgcccctaca tggcgtatct tcagatccag agtccagcag gtcagagcag   180

atgtggaggg ttcctggtgc gagaagactt tgtgctgaca gcagctcatt gctggggaag   240

caatataaat gtcaccctgg gcgcccacaa tatccagaga cgggaaaaca cccagcaaca   300

catcactgcg cgcagagcca tccgccaccc tcaatataat cagcggacca tccagaatga   360

catcatgtta ttgcagctga gcagaagagt cagacggaat cgaaacgtga acccagtggc   420

tctgcctaga gcccaggagg gactgagacc cgggacgctg tgcactgtgg ccggctgggg   480

cagggtcagc atgaggaggg gaacagatac actccgagag gtgcagctga gagtgcagag   540

ggataggcag tgcctccgca tcttcggttc ctacgacccc cgaaggcaga tttgtgtggg   600

ggaccggcgg gaacggaagg ctgccttcaa gggggattcc ggaggccccc tgctgtgtaa   660

caatgtggcc cacggcatcg tctcctatgg aaagtcgtca ggggttcctc cagaagtctt   720

caccagggtc tcaagtttcc tgccctggat aaggacaaca atgagaagct tcaaactgct   780

ggatcagatg gagacccccc tgtgactgac tcttcttctc ggggacacag gccagctcca   840

cagtgttgcc agagccttaa taaacgtcca cagagtataa ataaccaatt cctcatttgt   900

tcattaaacg tcattcagta ctta                                          924

<210>  71

<211>  3208

<212>  DNA

<213>  Homo sapiens

<220>

<221>  galactokinase 2 (GALK2), transcript variant 2

<222>  (1)..(3208)

<223>  LocusID: 2585; NM_001001556

<400> 71

ggttggtgcc ttagcaacta aagctggcaa ctgggacatc gtccggtgct gcaggtctca 60

gccgaagggc gtcggaaact gcgctcgcat cgagcagttt ccagcctcct gggtaaagga 120

gcagtctcct cccttgcttg ggactctgga cgcatctcat tccggtgaaa gtaagggaca 180

gcttaggacc agaagccttt cgcggagaaa aggctgacat gcccgtccta tatgacaggt 240

tactgaagct aaaggagatg tttaactcca agtttggatc tattcccaag ttttatgttc 300

gagcaccagg aagagtcaac ataataggag agcatataga ttattgtgga tattctgttc 360

ttcctatggc tgtagaacaa gatgtgctaa tagctgtaga acctgtgaaa acgtacgctc 420

tccaactggc caatacaaat cccttgtatc cggacttcag tactagtgct aataacatcc 480

agattgataa aaccaagcct ttgtggcaca actatttctt atgtggactt aaaggaattc 540

aggaacactt tggtcttagt aacctgactg gaatgaactg cctggtagat ggaaatatcc 600

caccaagttc tggcctctcc agctccagtg ctttggtctg ttgtgctggc ttggtgacgc 660

tcacagtgct gggaaggaat ctatccaagg tggaacttgc agaaatctgt gccaagagtg 720

agcgttacat tggcactgaa ggaggaggca tggaccagtc tatatcattt cttgcagaag 780

aaggaactgc caagttgata gaatttagtc ctctgagggc aaccgatgta aaactcccaa 840

gtggagcagt gtttgtgatt gccaacagtt gtgtggagat gaataaggca gcaacttccc 900

atttcaatat cagggtgatg gagtgtcggc tggctgcgaa gctcctggct aaatacaaaa 960

gcttgcaatg ggacaaagta ctgaggctgg aggaggtgca ggctaaacta gggattagtc 1020

tagaagaaat gctgttggtc acagaagatg cccttcatcc tgaaccctat aaccctgagg 1080

agatctgcag gtgtctggga attagcctgg aggaactccg aacccaaatc ctgagtccaa 1140

acactcaaga tgtgctcatc ttcaaactct atcagcgggc aaagcatgtg tacagcgagg 1200

ctgcgcgagt gctccagttt aagaagatat gtgaagaagc acctgaaaac atggtccagc 1260

tgctgggaga gttgatgaac cagagccaca tgagctgccg ggacatgtat gagtgcagct 1320

gccccgagct ggatcagctg gtggacatct gtcggaagtt tggggctcaa gggtcacgac 1380

ttactggagc aggatgggga ggctgcacag tatcaatggt acctgcggac aagctgccca 1440

gctttctagc aaatgtgcac aaagcttatt accagaggag tgatggaagc ttagcaccgg 1500

agaagcaaag tttgtttgct accaaacctg gaggtggggc tttggttttg cttgaggcct 1560

gaaaaaatgt aaaaagtctg agagaaacta cttagggcac ttaggaattg gcaggacttt 1620

ctgtgccaca gtaaattaat cttccttctg ttttgtatta tgatgaacgg ttgctattat 1680

atcaagatat attttcaaag aaatggttga aagctctcta tgcttcataa tgattctttt 1740

tccatcttaa aatatggttt tactattaag agccaagatc atgcttggac agatctttta 1800

agaataactt actgagattt attgatttga agattttaaa gatgaatggt aaaacacact 1860

cttaatactg attacatgga ttggacttga attaaatata ttgttacaat taaactgata 1920

ccactgaatt gtatgcatta ttcttgaata gagttcattt ctggtttctc ttagtattct 1980

tcttcctcaa agttgtagtt gtctgttgat gatggtgatg atgatgatga tgacgatagt 2040

gatgccacac attctctctc aatttcagct tcggaacgct atgaaaataa tacatgatta 2100

aagtttcaca gatcttcttg gacattgtat aattgaattt gaatgtgaga tttctccagt 2160

tatcaagaga ctaaggattt tttttttttt ttgacaaaag gaggatacag gaagaaaatt 2220

cagactcatt tgaatatttg taaaccatgt aatatataaa taaccacttt caattctttt 2280

ggccctgagc tatctccatt acttaataga aaaacttaga taaaaaacac tttaagactc 2340

ttctattcac attgaaataa agaattatct agatgataat tcagataatt cagtttgttc 2400

atagaattac tttcttatca ctctttttct actacttttt ctcaaatacc tctctaatcc 2460

aagaggcact tccaagaaat tccacacatt ccttatatcc cttttttgct tgtctagcaa 2520

agcttgtttg tatatatgca ccccattgta aagcaggtat gcaggtatgt gggtgaaagt 2580

gactatgaaa agacttaatg aattctggga tttgtaatgg tattgatggg tatctctgta 2640

tgtatatcaa gagtgggcag aaatgtttgg ctggtgatgg caaatgactg gctttctctt 2700

gtggatggac tataggaagc actttctgaa ttatgtaatg atttggacca aaaagtattt 2760

tgctgaaata ctcaggtaat tgagatagca atggttttat ggcatgaatt atccaaaaaa 2820

atatcagaat tacttattat tctgtgattt cattagctca ttaatgttta actcacagat 2880

tgggcatcac catctagaat ttcagtttaa gggaggcctg cgcaaagcta gttttatttc 2940

ttaaaggata acagtcatac atagaatact aggaaagcca atattctatt taaaaataaa 3000

cttctgatgc attttcattc ttagcagaaa ggtaaatgct tgagaaagct tgagtcaaat 3060

ttgttaaaag aattttgagt tctataaatc caaaaatctg aaacctgaat ttatttaaat 3120

ttataatcct ttatttttta ataccgtgcc attttccaca aaggatttgt ggttggtata 3180

taattcttta aagatacctg gatcagtg 3208

<210> 72

<211> 3121

<212> DNA

<213> Homo sapiens

<220>

<221> galactokinase 2 (GALK2), transcript variant 1

<222> (1)..(3121)

<223>  LocusID: 2586; NM_002044

<400>  72

ccggaagaaa acggctcctg tcacagaagt ctcgtgattg ctctgggagc tttgcttaga   60

cacttgaaac tacaggagaa agaaggatct agcgaaatat ggctacagag agccctgcta   120

cgcgtcgggt ccaggtggca gaacatccta ggttactgaa gctaaaggag atgtttaact   180

ccaagtttgg atctattccc aagttttatg ttcgagcacc aggaagagtc aacataatag   240

gagagcatat agattattgt ggatattctg ttcttcctat ggctgtagaa caagatgtgc   300

taatagctgt agaacctgtg aaaacgtacg ctctccaact ggccaataca aatcccttgt   360

atccggactt cagtactagt gctaataaca tccagattga taaaaccaag cctttgtggc   420

acaactattt cttatgtgga cttaaaggaa ttcaggaaca ctttggtctt agtaacctga   480

ctggaatgaa ctgcctggta gatggaaata tcccaccaag ttctggcctc tccagctcca   540

gtgctttggt ctgttgtgct ggcttggtga cgctcacagt gctgggaagg aatctatcca   600

aggtggaact tgcagaaatc tgtgccaaga gtgagcgtta cattggcact gaaggaggag   660

gcatggacca gtctatatca tttcttgcag aagaaggaac tgccaagttg atagaattta   720

gtcctctgag ggcaaccgat gtaaaactcc caagtggagc agtgtttgtg attgccaaca   780

gttgtgtgga gatgaataag gcagcaactt cccatttcaa tatcagggtg atggagtgtc   840

ggctggctgc gaagctcctg gctaaataca aaagcttgca atgggacaaa gtactgaggc   900

tggaggaggt gcaggctaaa ctagggatta gtctagaaga aatgctgttg gtcacagaag   960

atgcccttca tcctgaaccc tataaccctg aggagatctg caggtgtctg ggaattagcc   1020

tggaggaact ccgaacccaa atcctgagtc caaacactca agatgtgctc atcttcaaac   1080

tctatcagcg ggcaaagcat gtgtacagcg aggctgcgcg agtgctccag tttaagaaga   1140

tatgtgaaga agcacctgaa aacatggtcc agctgctggg agagttgatg aaccagagcc   1200

acatgagctg ccgggacatg tatgagtgca gctgccccga gctggatcag ctggtggaca   1260

tctgtcggaa gtttggggct caagggtcac gacttactgg agcaggatgg ggaggctgca   1320

cagtatcaat ggtacctgcg gacaagctgc ccagctttct agcaaatgtg cacaaagctt   1380

attaccagag gagtgatgga agcttagcac cggagaagca aagtttgttt gctaccaaac   1440

ctggaggtgg ggctttggtt ttgcttgagg cctgaaaaaa tgtaaaaagt ctgagagaaa   1500

ctacttaggg cacttaggaa ttggcaggac tttctgtgcc acagtaaatt aatcttcctt   1560

ctgttttgta ttatgatgaa cggttgctat tatatcaaga tatattttca aagaaatggt   1620

tgaaagctct ctatgcttca taatgattct ttttccatct taaaatatgg ttttactatt   1680

aagagccaag atcatgcttg gacagatctt ttaagaataa cttactgaga tttattgatt   1740

tgaagatttt aaagatgaat ggtaaaacac actcttaata ctgattacat ggattggact   1800

tgaattaaat atattgttac aattaaactg ataccactga attgtatgca ttattcttga 1860

atagagttca tttctggttt ctcttagtat tcttcttcct caaagttgta gttgtctgtt 1920

gatgatggtg atgatgatga tgatgacgat agtgatgcca cacattctct ctcaatttca 1980

gcttcggaac gctatgaaaa taatacatga ttaaagtttc acagatcttc ttggacattg 2040

tataattgaa tttgaatgtg agatttctcc agttatcaag agactaagga tttttttttt 2100

tttttgacaa aaggaggata caggaagaaa attcagactc atttgaatat ttgtaaacca 2160

tgtaatatat aaataaccac tttcaattct tttggccctg agctatctcc attacttaat 2220

agaaaaactt agataaaaaa cactttaaga ctcttctatt cacattgaaa taaagaatta 2280

tctagatgat aattcagata attcagtttg ttcatagaat tactttctta tcactctttt 2340

tctactactt tttctcaaat acctctctaa tccaagaggc acttccaaga aattccacac 2400

attccttata tccctttttt gcttgtctag caaagcttgt ttgtatatat gcaccccatt 2460

gtaaagcagg tatgcaggta tgtgggtgaa agtgactatg aaaagactta atgaattctg 2520

ggatttgtaa tggtattgat gggtatctct gtatgtatat caagagtggg cagaaatgtt 2580

tggctggtga tggcaaatga ctggctttct cttgtggatg gactatagga agcactttct 2640

gaattatgta atgatttgga ccaaaaagta ttttgctgaa atactcaggt aattgagata 2700

gcaatggttt tatggcatga attatccaaa aaaatatcag aattacttat tattctgtga 2760

tttcattagc tcattaatgt ttaactcaca gattgggcat caccatctag aatttcagtt 2820

taagggaggc ctgcgcaaag ctagttttat ttcttaaagg ataacagtca tacatagaat 2880

actaggaaag ccaatattct atttaaaaat aaacttctga tgcattttca ttcttagcag 2940

aaaggtaaat gcttgagaaa gcttgagtca aatttgttaa aagaattttg agttctataa 3000

atccaaaaat ctgaaacctg aatttattta aatttataat cctttatttt ttaataccgt 3060

gccattttcc acaaaggatt tgtggttggt atataattct ttaaagatac ctggatcagt 3120

g 3121

<210> 73

<211> 820

<212> DNA

<213> Homo sapiens

<220>

<221> prostaglandin D2 synthase

<222> (1)..(820)

<223>  LocusID: 5730; NM_000954

<400>  73

gctcctcctg cacacctccc tcgctctccc acaccactgg caccaggccc cggacacccg    60

ctctgctgca ggagaatggc tactcatcac acgctgtgga tgggactggc cctgctgggg   120

gtgctgggcg acctgcaggc agcaccggag gcccaggtct ccgtgcagcc caacttccag   180

caggacaagt tcctggggcg ctggttcagc gcgggcctcg cctccaactc gagctggctc   240

cgggagaaga aggcggcgtt gtccatgtgc aagtctgtgg tggcccctgc cacggatggt   300

ggcctcaacc tgacctccac cttcctcagg aaaaaccagt gtgagacccg aaccatgctg   360

ctgcagcccg cggggtccct cggctcctac agctaccgga gtccccactg gggcagcacc   420

tactccgtgt cagtggtgga gaccgactac gaccagtacg cgctgctgta cagccagggc   480

agcaagggcc ctggcgagga cttccgcatg gccaccctct acagccgaac ccagaccccc   540

agggctgagt taaaggagaa attcaccgcc ttctgcaagg cccagggctt cacagaggat   600

accattgtct tcctgcccca aaccgataag tgcatgacgg aacaatagga ctccccaggg   660

ctgaagctgg gatcccggcc agccaggtga cccccacgct ctggatgtct ctgctctgtt   720

ccttccccga gcccctgccc cggctccccg ccaaagcaac cctgcccact caggcttcat   780

cctgcacaat aaactccgga agcaagtcag taaaaaaaaa                         820

<210>  74

<211>  1930

<212>  DNA

<213>  Homo sapiens

<220>

<221>  mitogen-activated protein kinase 9 transcript variant 1

<222>  (1)..(1930)

<223>  LocusID: 5601; NM_002752

<400>  74

agggatctga aacttgccca cccttcggga tattgcagga cgctgcatca tgagcgacag    60

taaatgtgac agtcagtttt atagtgtgca agtggcagac tcaaccttca ctgtcctaaa   120

acgttaccag cagctgaaac caattggctc tggggcccaa gggattgttt gtgctgcatt   180

tgatacagtt cttgggataa atgttgcagt caagaaacta agccgtcctt ttcagaacca   240

aactcatgca aagagagctt atcgtgaact tgtcctctta aaatgtgtca atcataaaaa   300

tataattagt ttgttaaatg tgtttacacc acaaaaaact ctagaagaat ttcaagatgt 360

gtatttggtt atggaattaa tggatgctaa cttatgtcag gttattcaca tggagctgga 420

tcatgaaaga atgtcctacc ttctttacca gatgctttgt ggtattaaac atctgcattc 480

agctggtata attcatagag atttgaagcc tagcaacatt gttgtgaaat cagactgcac 540

cctgaagatc cttgactttg gcctggcccg gacagcgtgc actaacttca tgatgacccc 600

ttacgtggtg acacggtact accgggcgcc cgaagtcatc ctgggtatgg gctacaaaga 660

gaacgttgat atctggtcag tgggttgcat catgggagag ctggtgaaag gttgtgtgat 720

attccaaggc actgaccata ttgatcagtg gaataaagtt attgagcagc tgggaacacc 780

atcagcagag ttcatgaaga aacttcagcc aactgtgagg aattatgtcg aaaacagacc 840

aaagtatcct ggaatcaaat ttgaagaact ctttccagat tggatattcc catcagaatc 900

tgagcgagac aaaataaaaa caagtcaagc cagagatctg ttatcaaaaa tgttagtgat 960

tgatcctgac aagcggatct ctgtagacga agctctgcgt cacccataca tcactgtttg 1020

gtatgacccc gccgaagcag aagccccacc acctcaaatt tatgatgccc agttggaaga 1080

aagagaacat gcaattgaag aatggaaaga gctaatttac aaagaagtca tggattggga 1140

agaaagaagc aagaatggtg ttgtaaaaga tcagccttca gatgcagcag taagtagcaa 1200

cgccactcct tctcagtctt catcgatcaa tgacatttca tccatgtcca ctgagcagac 1260

gctggcctca gacacagaca gcagtcttga tgcctcgacg ggaccccttg aaggctgtcg 1320

atgataggtt agaaatagca aacctgtcag cattgaagga actctcacct ccgtgggcct 1380

gaaatgcttg ggagttgatg gaaccaaata gaaaaactcc atgttctgca tgtaagaaac 1440

acaatgcctt gccctattca gacctgatag gattgcctgc ttagatgata aaatgaggca 1500

gaatatgtct gaagaaaaaa attgcaagcc acacttctag agattttgtt caagatcatt 1560

tcaggtgagc agttagagta ggtgaatttg tttcaaattg tactagtgac agtttctcat 1620

catctgtaac tgttgagatg tatgtgcatg tgaccacaaa tgcttgcttg gacttgccca 1680

tctagcactt tggaaatcag tatttaaatg ccaaataatc ttccaggtag tgctgcttct 1740

gaagttatct cttaatcctc ttaagtaatt tggtgtctgt ccagaaaaag tcgatttatg 1800

tgtattaatt ggccatcatg atgttatcat atcttattcc cttttatgct atgatttatt 1860

ctatcttttg tatttcagaa gacatataat taaatctatt taataaataa aaatatatag 1920

ctttaaaaaa 1930

<210> 75

<211> 1940

<212>  DNA

<213>  Homo sapiens

<220>

<221>  mitogen-activated protein kinase 9 transcript variant 2

<222>  (1)..(1940)

<223>  LocusID: 5601; NM_139068

<400>  75

agggatctga aacttgccca cccttcggga tattgcagga cgctgcatca tgagcgacag    60

taaatgtgac agtcagtttt atagtgtgca agtggcagac tcaaccttca ctgtcctaaa   120

acgttaccag cagctgaaac caattggctc tggggcccaa gggattgttt gtgctgcatt   180

tgatacagtt cttgggataa atgttgcagt caagaaacta agccgtcctt ttcagaacca   240

aactcatgca aagagagctt atcgtgaact tgtcctctta aaatgtgtca atcataaaaa   300

tataattagt ttgttaaatg tgtttacacc acaaaaaact ctagaagaat ttcaagatgt   360

gtatttggtt atggaattaa tggatgctaa cttatgtcag gttattcaca tggagctgga   420

tcatgaaaga atgtcctacc ttctttacca gatgctttgt ggtattaaac atctgcattc   480

agctggtata attcatagag atttgaagcc tagcaacatt gttgtgaaat cagactgcac   540

cctgaagatc cttgactttg gcctggcccg gacagcgtgc actaacttca tgatgacccc   600

ttacgtggtg acacggtact accgggcgcc cgaagtcatc ctgggtatgg gctacaaaga   660

gaacgttgat atctggtcag tgggttgcat catgggagag ctggtgaaag gttgtgtgat   720

attccaaggc actgaccata ttgatcagtg gaataaagtt attgagcagc tgggaacacc   780

atcagcagag ttcatgaaga aacttcagcc aactgtgagg aattatgtcg aaaacagacc   840

aaagtatcct ggaatcaaat ttgaagaact ctttccagat tggatattcc catcagaatc   900

tgagcgagac aaaataaaaa caagtcaagc cagagatctg ttatcaaaaa tgttagtgat   960

tgatcctgac aagcggatct ctgtagacga agctctgcgt cacccataca tcactgtttg  1020

gtatgacccc gccgaagcag aagccccacc acctcaaatt tatgatgccc agttggaaga  1080

aagagaacat gcaattgaag aatggaaaga gctaatttac aaagaagtca tggattggga  1140

agaaagaagc aagaatggtg ttgtaaaaga tcagccttca gcacagatgc agcagtaagt  1200

agcaacgcca ctccttctca gtcttcatcg atcaatgaca tttcatccat gtccactgag  1260

cagacgctgg cctcagacac agacagcagt cttgatgcct cgacgggacc ccttgaaggc  1320

tgtcgatgat aggttagaaa tagcaaacct gtcagcattg aaggaactct cacctccgtg  1380

ggcctgaaat gcttgggagt tgatggaacc aaatagaaaa actccatgtt ctgcatgtaa  1440

gaaacacaat gccttgccct attcagacct gataggattg cctgcttaga tgataaaatg  1500

aggcagaata tgtctgaaga aaaaaattgc aagccacact tctagagatt ttgttcaaga 1560

tcatttcagg tgagcagtta gagtaggtga atttgtttca aattgtacta gtgacagttt 1620

ctcatcatct gtaactgttg agatgtatgt gcatgtgacc acaaatgctt gcttggactt 1680

gcccatctag cactttggaa atcagtattt aaatgccaaa taatcttcca ggtagtgctg 1740

cttctgaagt tatctcttaa tcctcttaag taatttggtg tctgtccaga aaaagtcgat 1800

ttatgtgtat taattggcca tcatgatgtt atcatatctt attccctttt atgctatgat 1860

ttattctatc ttttgtattt cagaagacat ataattaaat ctatttaata aataaaaata 1920

tatagcttta aaaaaaaaaa 1940

<210> 76

<211> 1940

<212> DNA

<213> Homo sapiens

<220>

<221> mitogen-activated protein kinase 9 transcript variant 3

<222> (1)..(1940)

<223> LocusID: 5601; NM_139069

<400> 76

agggatctga aacttgccca cccttcggga tattgcagga cgctgcatca tgagcgacag 60

taaatgtgac agtcagtttt atagtgtgca agtggcagac tcaaccttca ctgtcctaaa 120

acgttaccag cagctgaaac caattggctc tggggcccaa gggattgttt gtgctgcatt 180

tgatacagtt cttgggataa atgttgcagt caagaaacta agccgtcctt ttcagaacca 240

aactcatgca aagagagctt atcgtgaact tgtcctctta aaatgtgtca atcataaaaa 300

tataattagt ttgttaaatg tgtttacacc acaaaaaact ctagaagaat ttcaagatgt 360

gtatttggtt atggaattaa tggatgctaa cttatgtcag gttattcaca tggagctgga 420

tcatgaaaga atgtcctacc ttctttacca gatgctttgt ggtattaaac atctgcattc 480

agctggtata attcatagag atttgaagcc tagcaacatt gttgtgaaat cagactgcac 540

cctgaagatc cttgactttg gcctggcccg gacagcgtgc actaacttca tgatgacccc 600

ttacgtggtg acacggtact accgggcgcc cgaagtcatc ctgggtatgg gctacaaaga 660

gaacgttgat atctggtcag tcgggtgcat catggcagaa atggtcctcc ataaagtcct 720

gttcccggga agagactata ttgatcagtg gaataaagtt attgagcagc tgggaacacc 780

atcagcagag ttcatgaaga aacttcagcc aactgtgagg aattatgtcg aaaacagacc 840

aaagtatcct ggaatcaaat ttgaagaact ctttccagat tggatattcc catcagaatc 900

tgagcgagac aaaataaaaa caagtcaagc cagagatctg ttatcaaaaa tgttagtgat 960

tgatcctgac aagcggatct ctgtagacga agctctgcgt cacccataca tcactgtttg 1020

gtatgacccc gccgaagcag aagccccacc acctcaaatt tatgatgccc agttggaaga 1080

aagagaacat gcaattgaag aatggaaaga gctaatttac aaagaagtca tggattggga 1140

agaaagaagc aagaatggtg ttgtaaaaga tcagccttca gcacagatgc agcagtaagt 1200

agcaacgcca ctccttctca gtcttcatcg atcaatgaca tttcatccat gtccactgag 1260

cagacgctgg cctcagacac agacagcagt cttgatgcct cgacgggacc ccttgaaggc 1320

tgtcgatgat aggttagaaa tagcaaacct gtcagcattg aaggaactct cacctccgtg 1380

ggcctgaaat gcttgggagt tgatggaacc aaatagaaaa actccatgtt ctgcatgtaa 1440

gaaacacaat gccttgccct attcagacct gataggattg cctgcttaga tgataaaatg 1500

aggcagaata tgtctgaaga aaaaaattgc aagccacact tctagagatt ttgttcaaga 1560

tcatttcagg tgagcagtta gagtaggtga atttgtttca aattgtacta gtgacagttt 1620

ctcatcatct gtaactgttg agatgtatgt gcatgtgacc acaaatgctt gcttggactt 1680

gcccatctag cactttggaa atcagtattt aaatgccaaa taatcttcca ggtagtgctg 1740

cttctgaagt tatctcttaa tcctcttaag taatttggtg tctgtccaga aaaagtcgat 1800

ttatgtgtat taattggcca tcatgatgtt atcatatctt attccctttt atgctatgat 1860

ttattctatc ttttgtattt cagaagacat ataattaaat ctatttaata aataaaaata 1920

tatagcttta aaaaaaaaaa 1940

<210> 77

<211> 1930

<212> DNA

<213> Homo sapiens

<220>

<221> mitogen-activated protein kinase 9 transcript variant 4

<222> (1)..(1930)

<223> LocusID: 5601; NM_139070

<400> 77

agggatctga aacttgccca cccttcggga tattgcagga cgctgcatca tgagcgacag 60

taaatgtgac agtcagtttt atagtgtgca agtggcagac tcaaccttca ctgtcctaaa 120

acgttaccag cagctgaaac caattggctc tggggcccaa gggattgttt gtgctgcatt 180

tgatacagtt cttgggataa atgttgcagt caagaaacta agccgtcctt ttcagaacca 240

aactcatgca aagagagctt atcgtgaact tgtcctctta aaatgtgtca atcataaaaa 300

tataattagt ttgttaaatg tgtttacacc acaaaaaact ctagaagaat ttcaagatgt 360

gtatttggtt atggaattaa tggatgctaa cttatgtcag gttattcaca tggagctgga 420

tcatgaaaga atgtcctacc ttctttacca gatgctttgt ggtattaaac atctgcattc 480

agctggtata attcatagag atttgaagcc tagcaacatt gttgtgaaat cagactgcac 540

cctgaagatc cttgactttg gcctggcccg gacagcgtgc actaacttca tgatgacccc 600

ttacgtggtg acacggtact accgggcgcc cgaagtcatc ctgggtatgg gctacaaaga 660

gaacgttgat atctggtcag tcgggtgcat catggcagaa atggtcctcc ataaagtcct 720

gttcccggga agagactata ttgatcagtg gaataaagtt attgagcagc tgggaacacc 780

atcagcagag ttcatgaaga aacttcagcc aactgtgagg aattatgtcg aaaacagacc 840

aaagtatcct ggaatcaaat ttgaagaact ctttccagat tggatattcc catcagaatc 900

tgagcgagac aaaataaaaa caagtcaagc cagagatctg ttatcaaaaa tgttagtgat 960

tgatcctgac aagcggatct ctgtagacga agctctgcgt cacccataca tcactgtttg 1020

gtatgacccc gccgaagcag aagccccacc acctcaaatt tatgatgccc agttggaaga 1080

aagagaacat gcaattgaag aatggaaaga gctaatttac aaagaagtca tggattggga 1140

agaaagaagc aagaatggtg ttgtaaaaga tcagccttca gatgcagcag taagtagcaa 1200

cgccactcct tctcagtctt catcgatcaa tgacatttca tccatgtcca ctgagcagac 1260

gctggcctca gacacagaca gcagtcttga tgcctcgacg ggaccccttg aaggctgtcg 1320

atgataggtt agaaatagca aacctgtcag cattgaagga actctcacct ccgtgggcct 1380

gaaatgcttg ggagttgatg gaaccaaata gaaaaactcc atgttctgca tgtaagaaac 1440

acaatgcctt gccctattca gacctgatag gattgcctgc ttagatgata aaatgaggca 1500

gaatatgtct gaagaaaaaa attgcaagcc acacttctag agattttgtt caagatcatt 1560

tcaggtgagc agttagagta ggtgaatttg tttcaaattg tactagtgac agtttctcat 1620

catctgtaac tgttgagatg tatgtgcatg tgaccacaaa tgcttgcttg gacttgccca 1680

tctagcactt tggaaatcag tatttaaatg ccaaataatc ttccaggtag tgctgcttct 1740

gaagttatct cttaatcctc ttaagtaatt tggtgtctgt ccagaaaaag tcgatttatg 800

tgtattaatt ggccatcatg atgttatcat atcttattcc cttttatgct atgatttatt 1860

ctatcttttg tatttcagaa gacatataat taaatctatt taataaataa aaatatatag 1920

ctttaaaaaa 1930

<210> 78

<211> 1480

<212> DNA

<213> Homo sapiens

<220>

<221> interleukin 15 transcript variant 3

<222> (1)..(1480)

<223> LocusID: 3600; NM_000585

<400> 78

gactccgggt ggcaggcgcc cgggggaatc ccagctgact cgctcactgc cttcgaagtc 60

cggcgccccc cgggagggaa ctgggtggcc gcaccctccc ggctgcggtg gctgtcgccc 120

cccaccctgc agccaggact cgatggagaa tccattccaa tatatggcca tgtggctctt 180

tggagcaatg ttccatcatg ttccatgctg ctgctgacgt cacatggagc acagaaatca 240

atgttagcag atagccagcc catacaagat cgtattgtat tgtaggaggc atcgtggatg 300

gatggctgct ggaaacccct tgccatagcc agctcttctt caatacttaa ggatttaccg 360

tggctttgag taatgagaat ttcgaaacca catttgagaa gtatttccat ccagtgctac 420

ttgtgtttac ttctaaacag tcattttcta actgaagctg gcattcatgt cttcattttg 480

ggctgtttca gtgcagggct tcctaaaaca gaagccaact gggtgaatgt aataagtgat 540

ttgaaaaaaa ttgaagatct tattcaatct atgcatattg atgctacttt atatacggaa 600

agtgatgttc accccagttg caaagtaaca gcaatgaagt gctttctctt ggagttacaa 660

gttatttcac ttgagtccgg agatgcaagt attcatgata cagtagaaaa tctgatcatc 720

ctagcaaaca acagtttgtc ttctaatggg aatgtaacag aatctggatg caaagaatgt 780

gaggaactgg aggaaaaaaa tattaaagaa tttttgcaga gttttgtaca tattgtccaa 840

atgttcatca acacttcttg attgcaattg attcttttta aagtgtttct gttattaaca 900

aacatcactc tgctgcttag acataacaaa acactcggca tttcaaatgt gctgtcaaaa 960

caagtttttc tgtcaagaag atgatcagac cttggatcag atgaactctt agaaatgaag 1020

gcagaaaaat gtcattgagt aatatagtga ctatgaactt ctctcagact tactttactc 1080

atttttttaa tttattattg aaattgtaca tatttgtgga ataatgtaaa atgttgaata 1140

aaaatatgta caagtgttgt tttttaagtt gcactgatat tttacctctt attgcaaaat 1200

agcatttgtt taagggtgat agtcaaatta tgtattggtg gggctgggta ccaatgctgc 1260

aggtcaacag ctatgctggt aggctcctgc cagtgtggaa ccactgacta ctggctctca 1320

ttgacttcct tactaagcat agcaaacaga ggaagaattt gttatcagta agaaaaagaa 1380

gaactatatg tgaatcctct tctttatact gtaatttagt tattgatgta taaagcaact 1440
gttatgaaat aaagaaattg caataactgg caaaaaaaaa 1480

<210> 79
<211> 1960
<212> DNA
<213> Homo sapiens
<220>
<221> interleukin 15 transcript variant 1
<222> (1)..(1960)
<223> LocusID: 3600; NM_172174
<400> 79
gactccgggt ggcaggcgcc cgggggaatc ccagctgact cgctcactgc cttcgaagtc 60
cggcgccccc cgggagggaa ctgggtggcc gcaccctccc ggctgcggtg gctgtcgccc 120
cccaccctgc agccaggact cgatggaggt acagagctcg gcttctttgc cttgggaggg 180
gagtggtggt ggttgaaagg gcgatggaat tttccccgaa agcctacgcc cagggcccct 240
cccagctcca gcgttaccct ccggtctatc ctactggccg agctgccccg ccttctcatg 300
gggaaaactt agccgcaact tcaatttttg gtttttcctt taatgacact tctgaggctc 360
tcctagccat cctcccgctt ccggaggagc gcagatcgca ggtccctttg cccctggcgt 420
gcgactccct actgcgctgc gctcttacgg cgttccaggc tgctggctag cgcaaggcgg 480
gccgggcacc ccgcgctccg ctgggagggt gagggacgcg cgtctggcgg ccccagccaa 540
gctgcgggtt tctgagaaga cgctgtcccg cagccctgag ggctgagttc tgcacccagt 600
caagctcagg aaggccaaga aaagaatcca ttccaatata tggccatgtg gctctttgga 660
gcaatgttcc atcatgttcc atgctgctga cgtcacatgg agcacagaaa tcaatgttag 720
cagatagcca gcccatacaa gatcgtattg tattgtagga ggcatcgtgg atggatggct 780
gctggaaacc ccttgccata gccagctctt cttcaatact taaggattta ccgtggcttt 840
gagtaatgag aatttcgaaa ccacatttga gaagtatttc catccagtgc tacttgtgtt 900
tacttctaaa cagtcatttt ctaactgaag ctggcattca tgtcttcatt ttgggctgtt 960
tcagtgcagg gcttcctaaa acagaagcca actgggtgaa tgtaataagt gatttgaaaa 1020
aaattgaaga tcttattcaa tctatgcata ttgatgctac tttatatacg gaaagtgatg 1080
ttcaccccag ttgcaaagta acagcaatga agtgctttct cttggagtta caagttattt 1140

cacttgagtc cggagatgca agtattcatg atacagtaga aaatctgatc atcctagcaa 1200

acaacagttt gtcttctaat gggaatgtaa cagaatctgg atgcaaagaa tgtgaggaac 1260

tggaggaaaa aaatattaaa gaatttttgc agagttttgt acatattgtc caaatgttca 1320

tcaacacttc ttgattgcaa ttgattcttt ttaaagtgtt tctgttatta acaaacatca 1380

ctctgctgct tagacataac aaaacactcg gcatttcaaa tgtgctgtca aaacaagttt 1440

ttctgtcaag aagatgatca gaccttggat cagatgaact cttagaaatg aaggcagaaa 1500

aatgtcattg agtaatatag tgactatgaa cttctctcag acttacttta ctcatttttt 1560

taatttatta ttgaaattgt acatatttgt ggaataatgt aaaatgttga ataaaaatat 1620

gtacaagtgt tgttttttaa gttgcactga tattttacct cttattgcaa aatagcattt 1680

gtttaagggt gatagtcaaa ttatgtattg gtggggctgg gtaccaatgc tgcaggtcaa 1740

cagctatgct ggtaggctcc tgccagtgtg gaaccactga ctactggctc tcattgactt 1800

ccttactaag catagcaaac agaggaagaa tttgttatca gtaagaaaaa gaagaactat 1860

atgtgaatcc tcttctttat actgtaattt agttattgat gtataaagca actgttatga 1920

aataaagaaa ttgcaataac tggcaaaaaa aaaaaaaaaa 1960

<210> 80

<211> 1550

<212> DNA

<213> Homo sapiens

<220>

<221> interleukin 15 transcript variant 2

<222> (1)..(1550)

<223> LocusID: 3600; NM_172175

<400> 80

aaatcctggc tgtcatgtac ttgctatggg cctagagtag cttacctaaa tgctactaac 60

cttcctccat accattattg taaagattaa aggtgatgca tctgttaagt aactaataga 120

gtgcttatta aaaggtaggt gttcaataag tattaattcc ctccctttct ttttcttact 180

agtgcacttg tgtttttaat ggatcatact ttaccctaga ttgtattgta ggaggcatcg 240

tggatggatg gctgctggaa accccttgcc atagccagct cttcttcaat acttaaggat 300

ttaccgtggc tttgagtaat gagaatttcg aaaccacatt tgagaagtat ttccatccag 360

tgctacttgt gtttacttct aaacagtcat tttctaactg aagctggcat tcatgtcttc 420

attttgggat gcagctaata tacccagttg gcccaaagca cctaacctat agttatataa 480

tctgactctc agttcagttt tactctacta atgccttcat ggtattggga accatagatt 540

tgtgcagctg tttcagtgca gggcttccta aaacagaagc caactgggtg aatgtaataa 600

gtgatttgaa aaaaattgaa gatcttattc aatctatgca tattgatgct actttatata 660

cggaaagtga tgttcacccc agttgcaaag taacagcaat gaagtgcttt ctcttggagt 720

tacaagttat ttcacttgag tccggagatg caagtattca tgatacagta gaaaatctga 780

tcatcctagc aaacaacagt ttgtcttcta atgggaatgt aacagaatct ggatgcaaag 840

aatgtgagga actggaggaa aaaaatatta aagaattttt gcagagtttt gtacatattg 900

tccaaatgtt catcaacact tcttgattgc aattgattct ttttaaagtg tttctgttat 960

taacaaacat cactctgctg cttagacata acaaaacact cggcatttca aatgtgctgt 1020

caaaacaagt ttttctgtca agaagatgat cagaccttgg atcagatgaa ctcttagaaa 1080

tgaaggcaga aaaatgtcat tgagtaatat agtgactatg aacttctctc agacttactt 1140

tactcatttt tttaatttat tattgaaatt gtacatattt gtggaataat gtaaaatgtt 1200

gaataaaaat atgtacaagt gttgtttttt aagttgcact gatattttac ctcttattgc 1260

aaaatagcat ttgtttaagg gtgatagtca aattatgtat tggtggggct gggtaccaat 1320

gctgcaggtc aacagctatg ctggtaggct cctgccagtg tggaaccact gactactggc 1380

tctcattgac ttccttacta agcatagcaa acagaggaag aatttgttat cagtaagaaa 1440

aagaagaact atatgtgaat cctcttcttt atactgtaat ttagttattg atgtataaag 1500

caactgttat gaaataaaga aattgcaata actggcaaaa aaaaaaaaaa 1550

<210> 81

<211> 5029

<212> DNA

<213> Homo sapiens

<220>

<221> mucosa associated lymphoid tissue lymphoma translocation gene 1
transcript variant 1

<222> (1)..(5029)

<223> Locus ID: 10892; NM_006785

<400> 81

cgaggctccg tgccccgccc cccgggtgcc ccgccccttt gcgcggctgg cgcggccagc 60

cggccaggct cccctcggca aacctgtcta attggggcgg ggagcggagc ttcctcctct 120
gagggccgtg ccgcgctgcc agatttgttc ttccgcccct gcctccgcgg ctcggaggcg 180
agcggaaggt gccccggggc cgaggcccgt gacggggcgg gcgggagccc cggcagtccg 240
gggtcgccgg cgagggccat gtcgctgttg ggggacccgc tacaggccct gccgccctcg 300
gccgccccca cggggccgct gctcgcccct ccggccggcg cgaccctcaa ccgcctgcgg 360
gagccgctgc tgcggaggct cagcgagctc ctggatcagg cgcccgaggg ccggggctgg 420
aggagactgg cggagctggc ggggagtcgc gggcgcctcc gcctcagttg cctagacctg 480
gagcagtgtt ctcttaaggt actggagcct gaaggaagcc ccagcctgtg tctgctgaag 540
ttaatgggtg aaaaaggttg cacagtcaca gaattgagtg atttcctgca ggctatggaa 600
cacactgaag ttcttcagct tctcagcccc ccaggaataa agattactgt aaacccagag 660
tcaaaggcag tcttggctgg acagtttgtg aaactgtgtt gccgggcaac tggacatcct 720
tttgttcaat atcagtggtt caaaatgaat aaagagattc caaatggaaa tacatcagag 780
cttattttta atgcagtgca tgtaaaagat gcaggctttt atgtctgtcg agttaataac 840
aatttcacct ttgaattcag ccagtggtca cagctggatg tttgcgacat cccagagagc 900
ttccagagaa gtgttgatgg cgtctctgaa tccaagttgc aaatctgtgt tgaaccaact 960
tcccaaaagc tgatgccagg cagcacattg gttttacagt gtgttgctgt tggaagccct 1020
attcctcact accagtggtt caaaaatgaa ttaccattaa cacatgagac caaaaagcta 1080
tacatggtgc cttatgtgga tttggaacac caaggaacct actggtgtca tgtatataat 1140
gatcgagaca gtcaagatag caagaaggta gaaatcatca taggaagaac agatgaggca 1200
gtggagtgca ctgaagatga attaaataat cttggtcatc ctgataataa agagcaaaca 1260
actgaccagc ctttggcgaa ggacaaggtt gcccttttga taggaaatat gaattaccgg 1320
gagcacccca agctcaaagc tcctttggtg gatgtgtacg aattgactaa cttactgaga 1380
cagctggact tcaaagtggt ttcactgttg gatcttactg aatatgagat gcgtaatgct 1440
gtggatgagt ttttactcct tttagacaag ggagtatatg ggttattata ttatgcagga 1500
catggttatg aaaattttgg gaacagcttc atggtccccg ttgatgctcc aaatccatat 1560
aggtctgaaa attgtctgtg tgtacaaaat atactgaaat tgatgcaaga aaaagaaact 1620
ggacttaatg tgttcttatt ggatatgtgt aggaaaagaa atgactacga tgataccatt 1680
ccaatcttgg atgcactaaa agtcaccgcc aatattgtgt ttggatatgc cacgtgtcaa 1740
ggagcagaag cttttgaaat ccagcattct ggattggcaa atggaatctt tatgaaattt 1800
ttaaaagaca gattattaga agataagaaa atcactgtgt tactggatga agttgcagaa 1860
gatatgggta agtgtcacct taccaaaggc aaacaggctc tagagattcg aagtagttta 1920
tctgagaaga gagcacttac tgatccaata cagggaacag aatattctgc tgaatctctt 1980

gtgcggaatc tacagtgggc caaggctcat gaacttccag aaagtatgtg tcttaagttt 2040
gactgtggtg ttcagattca attaggattt gcagctgagt tttccaatgt catgatcatc 2100
tatacaagta tagtttacaa accaccggag ataataatgt gtgatgccta cgttactgat 2160
tttccacttg atctagatat tgatccaaaa gatgcaaata aaggcacacc tgaagaaact 2220
ggcagctact tggtatcaaa ggatcttccc aagcattgcc tctataccag actcagttca 2280
ctgcaaaaat taaaggaaca tctagtcttc acagtatgtt tatcatatca gtactcagga 2340
ttggaagata ctgtagagga caagcaggaa gtgaatgttg ggaaacctct cattgctaaa 2400
ttagacatgc atcgaggttt gggaaggaag acttgctttc aaacttgtct tatgtctaat 2460
ggtccttacc agagttctgc agccacctca ggaggagcag ggcattatca ctcattgcaa 2520
gacccattcc atggtgttta ccattcacat cctggtaatc caagtaatgt tacaccagca 2580
gatagctgtc attgcagccg gactccagat gcatttattt caagtttcgc tcaccatgct 2640
tcatgtcatt ttagtagaag taatgtgcca gtagagacaa ctgatgaaat accatttagt 2700
ttctctgaca ggctcagaat ttctgaaaaa tgacctcctt gtttttgaaa gttagcataa 2760
ttttagatgc ctgtgaaata gtactgcact tacataaagt gagacattgt gaaaaggcaa 2820
atttgtatat gtagagaaag aatagtagta actgtttcat agcaaacttc aggactttga 2880
gatgttgaaa ttacattatt taattacaga cttcctcttt ctaagatttt gtgaattggt 2940
tgaatagttc tatacaaatg aagtatggag gtgtgtatgt ttatatgtat ataacaaaat 3000
attttcattg tgaccactct gaagtaagag caatgggaat ggcattattg tagaataagt 3060
cattgtattt ttaacaccag aaagaacctt gccgatcacc aggcataacc taattttatc 3120
catggaagaa acacagaaag gcatctaagt tagagctggc accagaactg agacctccag 3180
aaatctattc cagtattttt tccactacac aactgccttc ctgacaggtt ctgagataag 3240
tgttatgttt gtagatagag tgaaatatat ttatatatat ataaatatat acagatacat 3300
atctgtgtat tatctcaagg aatgtacaaa ctttagtttt tgattataag gacttcactg 3360
caagttttag ttaagaggtt tgtatataaa tctgttatag aacaggctga aatttcttgt 3420
tcataagatt atgaaaccac atgagaagtg ataaaatgtt tgttaaagct agatagaggt 3480
taagaatcaa gatataatgg ataattttca tagctgccta tcagaatttc ccaaatattt 3540
agcatcttcc ttgataatat gtattttctt cttgaatttc actggcctaa tgagataata 3600
ctcttatctt tggctctacc taaaagttgg ttaaaaatgc aattggcatt aacaaggaaa 3660
aatactgaat tagtaatttt aaaagtctca caaagaaaat cccaggccta gatggctgca 3720
ttgttgaatt ctgccaaaca ttaaaattag cactaatttt ttgcacactg tttctaaaag 3780
taggagagga aagaacactt cccaacttac tctaggtcag tattaccctg atactagact 3840
agacatcaca agaaaactat aagccaatat tccttattaa tacaaacaca aaaatcatta 3900

acaaaaatat tagcaaactg aatccagcaa cctataaaaa ggattctata tcatgaccaa 3960

gtggaattta tcccaggaat tcaaggttgg ttcaacatct aaaaatcaaa taagctaata 4020

tacagtcagt tctcattatt cacagtaatt atgttctaca gaatattctc ccataaacac 4080

tgaattaaat atggaacaac tgcttttagg agaaagtgta tttgtgtata tgtgtgtata 4140

catatgttta tctcacacac attatgagct tgaattctta attcatccta gcaaattcta 4200

ccttatttta cagaagtgaa agtgaggtat cagaagtgtt aagtgacatg cctgaggcac 4260

ccccctaaca ggtgtcagag ctgaaattca aaccccatcc agctggcccc ggagccagag 4320

cttcttgtac tacacagaat tgcccctgcc atttccaccc tccagtcatt tctctatgag 4380

actgaagcag gaaggcagag catcatcttg ttcagcctca gctgggaaca tgtgtactgg 4440

gtgactcaaa tttttcaccc atttacacat atccacaaat gactgcaaaa gtgccacgga 4500

tatcaatttg agggttataa attttagcaa gttggtaaat tcacaaatac ataaccttga 4560

ataatgagga tcaactgtac catatttaat aaagcacaaa acccacacag attgtcttat 4620

tacagcattt gataaaatcc aaaactcttt cataaaaaca ctcaacaaac ttaggaataa 4680

aaggaatctt cctagatatg ataaatataa catctatgaa aagcccacac ctaacattat 4740

acttcatggt gatagactga aggctgaatg ttttcccctt aagattggga agaaggacaa 4800

ggatgttcac tcggcactac ttctattcag cattgtactt gaagttctag ccacagcagt 4860

taggttagga attcaaggtt tgttcaacat ctaaaaatca aataagctaa taaagaaaag 4920

aggtttatac tgcaaaagaa gtgaaactat atgtattcac agttgataca tagttgtata 4980

tagaaaatgc taaagaatcc ataaaaagta ataaatgagt tcaacaggt 5029

<210> 82

<211> 4996

<212> DNA

<213> Homo sapiens

<220>

<221> mucosa associated lymphoid tissue lymphoma translocation gene 1
transcript variant 2

<222> (1)..(4996)

<223> LocusID: 10892; NM_173844

<400> 82

cgaggctccg tgccccgccc cccgggtgcc ccgccccttt gcgcggctgg cgcggccagc 60

cggccaggct cccctcggca aacctgtcta attggggcgg ggagcggagc ttcctcctct 120

gagggccgtg ccgcgctgcc agatttgttc ttccgcccct gcctccgcgg ctcggaggcg 180

agcggaaggt gccccggggc cgaggcccgt gacggggcgg gcgggagccc cggcagtccg 240

gggtcgccgg cgagggccat gtcgctgttg ggggacccgc tacaggccct gccgccctcg 300

gccgccccca cggggccgct gctcgcccct ccggccggcg cgaccctcaa ccgcctgcgg 360

gagccgctgc tgcggaggct cagcgagctc ctggatcagg cgcccgaggg ccggggctgg 420

aggagactgg cggagctggc ggggagtcgc gggcgcctcc gcctcagttg cctagacctg 480

gagcagtgtt ctcttaaggt actggagcct gaaggaagcc ccagcctgtg tctgctgaag 540

ttaatgggtg aaaaaggttg cacagtcaca gaattgagtg atttcctgca ggctatggaa 600

cacactgaag ttcttcagct tctcagcccc ccaggaataa agattactgt aaacccagag 660

tcaaaggcag tcttggctgg acagtttgtg aaactgtgtt gccgggcaac tggacatcct 720

tttgttcaat atcagtggtt caaaatgaat aaagagattc caaatggaaa tacatcagag 780

cttattttta atgcagtgca tgtaaaagat gcaggctttt atgtctgtcg agttaataac 840

aatttcacct ttgaattcag ccagtggtca cagctggatg tttgcgacat cccagagagc 900

ttccagagaa gtgttgatgg cgtctctgaa tccaagttgc aaatctgtgt tgaaccaact 960

tcccaaaagc tgatgccagg cagcacattg gttttacagt gtgttgctgt tggaagccct 1020

attcctcact accagtggtt caaaaatgaa ttaccattaa cacatgagac caaaaagcta 1080

tacatggtgc cttatgtgga tttggaacac caaggaacct actggtgtca tgtatataat 1140

gatcgagaca gtcaagatag caagaaggta gaaatcatca tagatgaatt aaataatctt 1200

ggtcatcctg ataataaaga gcaaacaact gaccagcctt tggcgaagga caaggttgcc 260

cttttgatag gaaatatgaa ttaccgggag caccccaagc tcaaagctcc tttggtggat 1320

gtgtacgaat tgactaactt actgagacag ctggacttca aagtggtttc actgttggat 1380

cttactgaat atgagatgcg taatgctgtg gatgagtttt tactcctttt agacaaggga 1440

gtatatgggt tattatatta tgcaggacat ggttatgaaa attttgggaa cagcttcatg 1500

gtccccgttg atgctccaaa tccatatagg tctgaaaatt gtctgtgtgt acaaaatata 1560

ctgaaattga tgcaagaaaa agaaactgga cttaatgtgt tcttattgga tatgtgtagg 1620

aaaagaaatg actacgatga taccattcca atcttggatg cactaaaagt caccgccaat 1680

attgtgtttg gatatgccac gtgtcaagga gcagaagctt ttgaaatcca gcattctgga 1740

ttggcaaatg gaatctttat gaaattttta aaagacagat tattagaaga taagaaaatc 1800

actgtgttac tggatgaagt tgcagaagat atgggtaagt gtcaccttac caaaggcaaa 1860

caggctctag agattcgaag tagtttatct gagaagagag cacttactga tccaatacag 1920

ggaacagaat attctgctga atctcttgtg cggaatctac agtgggccaa ggctcatgaa 1980

cttccagaaa gtatgtgtct taagtttgac tgtggtgttc agattcaatt aggatttgca 2040
gctgagtttt ccaatgtcat gatcatctat acaagtatag tttacaaacc accggagata 2100
ataatgtgtg atgcctacgt tactgatttt ccacttgatc tagatattga tccaaaagat 2160
gcaaataaag gcacacctga agaaactggc agctacttgg tatcaaagga tcttcccaag 2220
cattgcctct ataccagact cagttcactg caaaaattaa aggaacatct agtcttcaca 2280
gtatgtttat catatcagta ctcaggattg gaagatactg tagaggacaa gcaggaagtg 2340
aatgttggga aacctctcat tgctaaatta gacatgcatc gaggtttggg aaggaagact 2400
tgctttcaaa cttgtcttat gtctaatggt ccttaccaga gttctgcagc cacctcagga 2460
ggagcagggc attatcactc attgcaagac ccattccatg gtgtttacca ttcacatcct 2520
ggtaatccaa gtaatgttac accagcagat agctgtcatt gcagccggac tccagatgca 2580
tttatttcaa gtttcgctca ccatgcttca tgtcatttta gtagaagtaa tgtgccagta 2640
gagacaactg atgaaatacc atttagtttc tctgacaggc tcagaatttc tgaaaaatga 2700
cctccttgtt tttgaaagtt agcataattt tagatgcctg tgaaatagta ctgcacttac 2760
ataaagtgag acattgtgaa aaggcaaatt tgtatatgta gagaaagaat agtagtaact 2820
gtttcatagc aaacttcagg actttgagat gttgaaatta cattatttaa ttacagactt 2880
cctctttcta agattttgtg aattggttga atagttctat acaaatgaag tatggaggtg 2940
tgtatgttta tatgtatata acaaaatatt ttcattgtga ccactctgaa gtaagagcaa 3000
tgggaatggc attattgtag aataagtcat tgtattttta acaccagaaa gaaccttgcc 3060
gatcaccagg cataacctaa ttttatccat ggaagaaaca cagaaaggca tctaagttag 3120
agctggcacc agaactgaga cctccagaaa tctattccag tattttttcc actacacaac 3180
tgccttcctg acaggttctg agataagtgt tatgtttgta gatagagtga aatatattta 3240
tatatatata aatatataca gatacatatc tgtgtattat ctcaaggaat gtacaaactt 3300
tagtttttga ttataaggac ttcactgcaa gttttagtta agaggtttgt atataaatct 3360
gttatagaac aggctgaaat ttcttgttca taagattatg aaaccacatg agaagtgata 3420
aaatgtttgt taaagctaga tagaggttaa gaatcaagat ataatggata attttcatag 3480
ctgcctatca gaatttccca aatatttagc atcttccttg ataatatgta ttttcttctt 3540
gaatttcact ggcctaatga gataatactc ttatctttgg ctctacctaa aagttggtta 3600
aaaatgcaat tggcattaac aaggaaaaat actgaattag taattttaaa agtctcacaa 3660
agaaaatccc aggcctagat ggctgcattg ttgaattctg ccaaacatta aaattagcac 3720
taattttttg cacactgttt ctaaaagtag gagaggaaag aacacttccc aacttactct 3780
aggtcagtat taccctgata ctagactaga catcacaaga aaactataag ccaatattcc 3840

ttattaatac aaacacaaaa atcattaaca aaaatattag caaactgaat ccagcaacct 3900

ataaaaagga ttctatatca tgaccaagtg gaatttatcc caggaattca aggttggttc 3960

aacatctaaa aatcaaataa gctaatatac agtcagttct cattattcac agtaattatg 4020

ttctacagaa tattctccca taaacactga attaaatatg gaacaactgc ttttaggaga 4080

aagtgtattt gtgtatatgt gtgtatacat atgtttatct cacacacatt atgagcttga 4140

attcttaatt catcctagca aattctacct tattttacag aagtgaaagt gaggtatcag 4200

aagtgttaag tgacatgcct gaggcacccc cctaacaggt gtcagagctg aaattcaaac 4260

cccatccagc tggccccgga gccagagctt cttgtactac acagaattgc ccctgccatt 4320

tccaccctcc agtcatttct ctatgagact gaagcaggaa ggcagagcat catcttgttc 4380

agcctcagct gggaacatgt gtactgggtg actcaaattt ttcacccatt tacacatatc 4440

cacaaatgac tgcaaaagtg ccacggatat caatttgagg gttataaatt ttagcaagtt 4500

ggtaaattca caaatacata accttgaata atgaggatca actgtaccat atttaataaa 4560

gcacaaaacc cacacagatt gtcttattac agcatttgat aaaatccaaa actctttcat 4620

aaaaacactc aacaaactta ggaataaaag gaatcttcct agatatgata aatataacat 4680

ctatgaaaag cccacaccta acattatact tcatggtgat agactgaagg ctgaatgttt 4740

tccccttaag attgggaaga aggacaagga tgttcactcg gcactacttc tattcagcat 4800

tgtacttgaa gttctagcca cagcagttag gttaggaatt caaggtttgt tcaacatcta 4860

aaaatcaaat aagctaataa agaaaagagg tttatactgc aaaagaagtg aaactatatg 4920

tattcacagt tgatacatag ttgtatatag aaaatgctaa agaatccata aaaagtaata 4980

aatgagttca acaggt 4996

<210> 83

<211> 1946

<212> DNA

<213> Homo sapiens

<220>

<221> lysyl oxidase

<222> (1)..(1946)

<223> LocusID: 4015; NM_002317

<400> 83

agacactgcc cgctctccgg gactccgcgc cgctccccgt tgccttccag gactgagaaa 60

ggggaaaggg aagggtgcca cgtccgagca gccgccttga ctggggaagg gtctgaatcc 120
cacccttggc attgcttggt ggagactgag atacccgtgc tccgctcgcc tccttggttg 180
aagatttctc cttccctcac gtgatttgag ccccgttttt attttctgtg agccacgtcc 240
tcctcgagcg gggtcaatct ggcaaaagga gtgatgcgct tcgcctggac cgtgctcctg 300
ctcgggcctt tgcagctctg cgcgctagtg cactgcgccc ctcccgccgc cggccaacag 360
cagcccccgc gcgagccgcc ggcggctccg ggcgcctggc gccagcagat ccaatgggag 420
aacaacgggc aggtgttcag cttgctgagc ctgggctcac agtaccagcc tcagcgccgc 480
cgggacccgg gcgccgccgt ccctggtgca gccaacgcct ccgcccagca gccccgcact 540
ccgatcctgc tgatccgcga caaccgcacc gccgcggcgc gaacgcggac ggccggctca 600
tctggagtca ccgctggccg ccccaggccc accgcccgtc actggttcca agctggctac 660
tcgacatcta gagcccgcga agctggcgcc tcgcgcgcgg agaaccagac agcgccggga 720
gaagttcctg cgctcagtaa cctgcggccg cccagccgcg tggacggcat ggtgggcgac 780
gacccttaca acccctacaa gtactctgac gacaaccctt attacaacta ctacgatact 840
tatgaaaggc ccagacctgg gggcaggtac cggcccggat acggcactgg ctacttccag 900
tacggtctcc cagacctggt ggccgacccc tactacatcc aggcgtccac gtacgtgcag 960
aagatgtcca tgtacaacct gagatgcgcg gcggaggaaa actgtctggc cagtacagca 1020
tacagggcag atgtcagaga ttatgatcac agggtgctgc tcagatttcc ccaaagagtg 1080
aaaaaccaag ggacatcaga tttcttaccc agccgaccaa gatattcctg ggaatggcac 1140
agttgtcatc aacattacca cagtatggat gagtttagcc actatgacct gcttgatgcc 1200
aacacccaga ggagagtggc tgaaggccac aaagcaagtt tctgtcttga agacacatcc 1260
tgtgactatg gctaccacag gcgatttgca tgtactgcac acacacaggg attgagtcct 1320
ggctgttatg atacctatgg tgcagacata gactgccagt ggattgatat tacagatgta 1380
aaacctggaa actatatcct aaaggtcagt gtaaacccca gctacctggt tcctgaatct 1440
gactatacca acaatgttgt gcgctgtgac attcgctaca caggacatca tgcgtatgcc 1500
tcaggctgca caatttcacc gtattagaag gcaaagcaaa actcccaatg gataaatcag 1560
tgcctggtgt tctgaagtgg gaaaaaatag actaacttca gtaggattta tgtattttga 1620
aaaagagaac agaaaacaac aaaagaattt ttgtttggac tgttttcaat aacaaagcac 1680
ataactggat tttgaacgct taagtcatca ttacttggga aatttttaat gtttattatt 1740
tacatcactt tgtgaattaa cacagtgttt caattctgta attacatatt tgactctttc 1800
aaagaaatcc aaatttctca tgttcctttt gaaattgtag tgcaaaatgg tcagtattat 1860
ctaaatgaat gagccaaaat gactttgaac tgaaactttt ctaaagtgct ggaactttag 1920
tgaaacataa taataatggg tttata 1946

<210> 84

<211> 3380

<212> DNA

<213> Homo sapiens

<220>

<221> integrin, beta 5

<222> (1)..(3380)

<223> Locus ID: 3693; NM_002213

<400> 84

gcggagccag cccctcccct acccggagca gcccgctggg gccgtcccga gcggcgacac 60

actaggagtc ccggccggcc agccagggca gccgcggtcc cgggactcgg ccgtgagtgc 120

tgcgggacgg atggtggcgg cggggcgcgg gccagcgcgg gcgccgtgag ccggagctgc 180

gcgcggggca tgcggctgcg gcccccggcc ctcggccccc gcgctccggc cccagccccg 240

gccgccggcc cccgcggagt gcagcgaccg cgccgccgct gagggaggcg ccccaccatg 300

ccgcgggccc cggcgccgct gtacgcctgc ctcctggggc tctgcgcgct cctgccccgg 360

ctcgcaggtc tcaacatatg cactagtgga agtgccacct catgtgaaga atgtctgcta 420

atccacccaa aatgtgcctg gtgctccaaa gaggacttcg gaagcccacg gtccatcacc 480

tctcggtgtg atctgagggc aaaccttgtc aaaaatggct gtggaggtga gatagagagc 540

ccagccagca gcttccatgt cctgaggagc ctgcccctca gcagcaaggg ttcgggctct 600

gcaggctggg acgtcattca gatgacacca caggagattg ccgtgaacct ccggcccggt 660

gacaagacca ccttccagct acaggttcgc caggtggagg actatcctgt ggacctgtac 720

tacctgatgg acctctccct gtccatgaag gatgacttgg acaatatccg gagcctgggc 780

accaaactcg cggaggagat gaggaagctc accagcaact tccggttggg atttgggtct 840

tttgttgata aggacatctc tcctttctcc tacacggcac cgaggtacca gaccaatccg 900

tgcattggtt acaagttgtt tccaaattgc gtcccctcct ttgggttccg ccatctgctg 960

cctctcacag acagagtgga cagcttcaat gaggaagttc ggaaacagag ggtgtcccgg 1020

aaccgagatg cccctgaggg gggctttgat gcagtactcc aggcagccgt ctgcaaggag 1080

aagattggct ggcgaaagga tgcactgcat ttgctggtgt tcacaacaga tgatgtgccc 1140

cacatcgcat tggatggaaa attgggaggc ctggtgcagc cacacgatgg ccagtgccac 1200

ctgaacgagg ccaacgagta cactgcatcc aaccagatgg actatccatc ccttgccttg 1260

cttggagaga aattggcaga gaacaacatc aacctcatct ttgcagtgac aaaaaaccat 1320

tatatgctgt acaagaattt tacagccctg atacctggaa caacggtgga gattttagat 1380
ggagactcca aaaatattat tcaactgatt attaatgcat acaatagtat ccggtctaaa 1440
gtggagttgt cagtctggga tcagcctgag gatcttaatc tcttctttac tgctacctgc 1500
caagatgggg tatcctatcc tggtcagagg aagtgtgagg gtctgaagat tggggacacg 1560
gcatcttttg aagtatcatt ggaggcccga agctgtccca gcagacacac ggagcatgtg 1620
tttgccctgc ggccggtggg attccgggac agcctggagg tgggggtcac ctacaactgc 1680
acgtgcggct gcagcgtggg gctggaaccc aacagtgcca ggtgcaacgg gagcgggacc 1740
tatgtctgcg gcctgtgtga gtgcagcccc ggctacctgg gcaccaggtg cgagtgccag 1800
gatggggaga accagagcgt gtaccagaac ctgtgccggg aggcagaggg caagccactg 1860
tgcagcgggc gtggggactg cagctgcaac cagtgctcct gcttcgagag cgagttcggc 1920
aagatctatg ggcctttctg tgagtgcgac aacttctcct gtgccaggaa caagggagtc 1980
ctctgctcag gccatggcga gtgtcactgc ggggaatgca agtgccatgc aggttacatc 2040
ggggacaact gtaactgctc gacagacatc agcacatgcc ggggcagaga tggccagatc 2100
tgcagcgagc gtgggcactg tctctgtggg cagtgccaat gcacggagcc gggggccttt 2160
ggggagatgt gtgagaagtg ccccacctgc ccggatgcat gcagcaccaa gagagattgc 2220
gtcgagtgcc tgctgctcca ctctgggaaa cctgacaacc agacctgcca cagcctatgc 2280
agggatgagg tgatcacatg ggtggacacc atcgtgaaag atgaccagga ggctgtgcta 2340
tgtttctaca aaaccgccaa ggactgcgtc atgatgttca cctatgtgga gctccccagt 2400
gggaagtcca acctgaccgt cctcagggag ccagagtgtg gaaacacccc caacgccatg 2460
accatcctcc tggctgtggt cggtagcatc ctccttgttg ggcttgcact cctggctatc 2520
tggaagctgc ttgtcaccat ccacgaccgg agggagtttg caaagtttca gagcgagcga 2580
tccagggccc gctatgaaat ggcttcaaat ccattataca gaaagcctat ctccacgcac 2640
actgtggact tcaccttcaa caagttcaac aaatcctaca atggcactgt ggactgatgt 2700
ttccttctcc gaggggctgg agcggggatc tgatgaaaag gtcagactga aacgccttgc 2760
acggctgctc ggcttgatca cagctcccta ggtaggcacc acagagaaga ccttctagtg 2820
agcctgggcc aggagcccac agtgcctgta caggaaggtg cctggccatg tcacctggct 2880
gctaggccag agccatgcca ggctgcgtcc ctccgagctt gggataaagc aaggggacct 2940
tggcactctc agctttccct gccacatcca gcttgttgtc ccaatgaaat actgagatgc 3000
tgggctgtct ctcccttcca ggaatgctgg gcccccagcc tggccagaca agacgactgt 3060
caggaagggt cggagtctgt aaaaccagca tacagtttgg cttttttcac attgatcatt 3120
tttatatgaa ataaaaagat cctgcattta tggtgtagtt ctgagtcctg agacttttcc 3180
gcgtgatggc tatgccttgc acacaggtgt tggtgatggg gctgttgaga tgcctgttga 3240

aggtacatcg tttgcaaatg tcagtttcct ctcctgtccg tgtttgttta gtacttttat 3300

aatgaaaaga aacaagattg tttgggattg gaagtaaaga ttaaaaccaa aagaatttgt 3360

gtttgtctga taaaaaaaaa 3380

<210> 85

<211> 3670

<212> DNA

<213> Homo sapiens

<220>

<221> aldehyde dehydrogenase 3 family, member A2

<222> (1)..(3670)

<223> LocusID: 224; NM_000382

<400> 85

agtgggactc agcgggcgtg gaggtcgcgg cggacggagc gagccctggg cgagtgaatt 60

gtggctgtgg gttgacggtg gagacacccc ccggagaggc ggagggaagg gaggcgaggc 120

tgcacctgca tgcttcccgc ctcccactcc ccagcgcccc cggaccgtgc agttctctgc 180

aggaccaggc catggagctc gaagtccggc gggtccgaca ggcgttcctg tccggccggt 240

cgcgacctct gcggtttcgg ctgcagcagc tggaggccct gcggaggatg gtgcaggagc 300

gcgagaagga tatcctgacg gccatcgccg ccgacctgtg caagagtgaa ttcaatgtgt 360

acagtcagga agtcattact gtccttgggg aaattgattt tatgcttgag aatcttcctg 420

aatgggttac tgctaaacca gttaagaaga acgtgctcac catgctggat gaggcctata 480

ttcagccaca gcctctggga gtggtgctga taatcggagc ttggaattac cccttcgttc 540

tcaccattca gccactgata ggagccatcg ctgcaggaaa tgctgtgatt ataaagcctt 600

ctgaactgag tgaaaataca gccaagatct tggcaaagct tctccctcag tatttagacc 660

aggatctcta tattgttatt aatggtggtg ttgaggaaac cacggagctc ctgaagcagc 720

gatttgacca cattttctat acgggaaaca ctgcggttgg caaaattgtc atggaagctg 780

ctgccaagca tctgacccct gtgactcttg aactgggagg gaaaagtcca tgttatattg 840

ataaagattg tgacctggac attgtttgca gacgcataac ctggggaaaa tacatgaatt 900

gtggccaaac ctgcattgca cccgactata ttctctgtga agcatccctc caaaatcaaa 960

ttgtatggaa gattaaggaa acagtgaagg aattttatgg agaaaatata aaagagtctc 1020

ctgattatga aaggatcatc aatcttcgtc attttaagag gatactaagt ttgcttgaag 1080

gacaaaagat agcttttggt ggggagactg atgaggccac acgctacata gccccaacag 1140

tacttaccga tgttgatcct aaaaccaagg tgatgcaaga agaaattttt ggaccaattc 1200

ttccaatagt gcctgtgaaa aatgtagatg aggccataaa tttcataaat gaacgtgaaa 1260

agcctctggc tctttatgta ttttcgcata accataagct catcaaacgg atgattgatg 1320

agacatccag tggaggtgtc acaggcaatg acgtcattat gcacttcacg ctcaactctt 1380

tcccatttgg aggagtgggt tccagtggga tgggagctta tcacggaaaa catagttttg 1440

atactttttc tcatcagcgt ccctgtttat taaaaagttt aaagagagaa ggtgctaaca 1500

aactcagata tcctcccaac agccagtcaa aggtggattg gggaaatttt tttctcttga 1560

aacggttcaa caaagaaaaa ctcggtctcc tgttgctcac tttcctgggt attgtagccg 1620

ctgtgcttgt caaggcagaa tattactgaa gaatgatcct gttcaacctc ctagtgcctc 1680

tactgaatta ttcctctttt aaatggttaa tgaaccaata atttttaaat cataccaaaa 1740

atagtaagaa aatatgcaaa cactctgtga tcaaacttaa aagtcattgc cattcatcat 1800

taataaaagt tgccatttca actacgtccc aacattccct aatagggtat tcagggaacc 1860

tgtcttaaat tgtgcttatc taaatcttgg aactttgagc taggggagga gaatgtatta 1920

gactaaatac aaactgcggg gttgtaaggg agtctcagaa cctcactgaa tccttcactc 1980

cagttaatgg cactgctcac ttcctgcctc tgctgccacc atcactgtgt gaagctttca 2040

agagcttggt acttcccagg gctaccggca gtcctctgta gtccagagag gtgagattag 2100

atcttcttgg ttccctgtga ggtttcaggc actaaaactc tatgtgggga agggaggggt 2160

tactcctcct ccaatgggac tcaaggactt gacctccagg agtaggcccc tggtcagaag 2220

tgccatctca ccagtggtct tcattcttcc tcattcattc tttatcatcc tgtgttctgt 2280

ttagttgcaa caatctcttg tgactaatgt cactcaaagc atcttgtaac ctagggcttc 2340

ctggaagtta gttgccaaag tcatgcaagc atcacctgtc attcttgtgt tggagttata 2400

gaattctaca tcttataaaa cctaactggc atttaaaaaa tactgtggcc gggcgtggtg 2460

gctcatgcct gtaatcccag cactttggga ggccgaggtg ggaggattgc ttgagtccag 2520

gaatttgaga ccagcctgga caacacagtg agacctcatc tctatcaaaa aataaaaatt 2580

agctagatgt ggtggcatga gcctgtgttc ccagctgctt aggaggctga agcaggagga 2640

ttgattgagc ctgcgaggcc aaggctgcag caggctgtga ttgcaccact gcacttcagc 2700

ttgggcaaca gagcaagacc ctgtctccga aacaaataaa aaatactgta ataaaagtac 2760

ttataaacat actaatcctc tttcaggacc ctaaagttgc aggttagtag gtcttcaagg 2820

acaaatctgt aagtttcttt tctgtagtgc aagtaaaatt tcactttttg aaactataga 2880

gagatccctt tctgattagc ctacagaact taaagtgagg gaaccatttc ctctcacaga 2940

caaagaggcc tgggatatta ggactttggg gtttgagagc atcatggggc agacagatgg 3000

tggatggtct ggacaagaag cgagtaagcc actgcggttg gtcatactga agggaattga 3060

tggcaagagg atcccctgag caagtcagaa gttactctca tcagtcgttc atggtcacaa 3120

cctgaggtac tctgctgagt gggcaaggct gaagtaagag gcctgtggaa tgcagcatta 3180

cctgctggac agagcagggc aggcagttct atgccttgga gctcctgact gcagggactc 3240

tgtccccaca ctcagaaaga ctcagctcac tcaatgagag aatgtgattt actttataga 3300

acgtataatc aactttgttg aataatttgt tctattaagg ctgtctaaaa tgtgatgtct 3360

tcatcatagt atgaagtgtt gaaaattaat aacgagccta gtttaggaaa aagctgctta 3420

aaactgtggc tctaagagag taatcataaa ataccttaga taaaattgca ctatggaatt 3480

ttcattgagt atgtttaaat tattggcttg tctactaata catctgcttc aaaatgaaca 3540

tatttcataa aattggcatc aattttaatg acgctcctgg tatggaacct cagatatacc 3600

ctattggaga caatcctttg atcataaatt ctccccaact ataaatcatt ttatgtcttt 3660

aaaaaaaaaa 3670

<210> 86

<211> 463

<212> PRT

<213> Homo sapiens

<220>

<221> retinoid X receptor, gamma

<222> (1)..(463)

<223> LocusID: 6258; NM_006917

<400> 86

Met Tyr Gly Asn Tyr Ser His Phe Met Lys Phe Pro Ala Gly Tyr Gly

1               5                   10                  15

Gly Ser Pro Gly His Thr Gly Ser Thr Ser Met Ser Pro Ser Ala Ala

            20                  25                  30

Leu Ser Thr Gly Lys Pro Met Asp Ser His Pro Ser Tyr Thr Asp Thr

        35                  40                  45

Pro Val Ser Ala Pro Arg Thr Leu Ser Ala Val Gly Thr Pro Leu Asn

50                  55                  60

Ala Leu Gly Ser Pro Tyr Arg Val Ile Thr Ser Ala Met Gly Pro Pro

65                  70                  75                  80

Ser Gly Ala Leu Ala Ala Pro Pro Gly Ile Asn Leu Val Ala Pro Pro

                85                  90                  95

Ser Ser Gln Leu Asn Val Val Asn Ser Val Ser Ser Ser Glu Asp Ile

            100                 105                 110

Lys Pro Leu Pro Gly Leu Pro Gly Ile Gly Asn Met Asn Tyr Pro Ser

        115                 120                 125

Thr Ser Pro Gly Ser Leu Val Lys His Ile Cys Ala Ile Cys Gly Asp

    130                 135                 140

Arg Ser Ser Gly Lys His Tyr Gly Val Tyr Ser Cys Glu Gly Cys Lys

145                 150                 155                 160

Gly Phe Phe Lys Arg Thr Ile Arg Lys Asp Leu Ile Tyr Thr Cys Arg

                165                 170                 175

Asp Asn Lys Asp Cys Leu Ile Asp Lys Arg Gln Arg Asn Arg Cys Gln

            180                 185                 190

Tyr Cys Arg Tyr Gln Lys Cys Leu Val Met Gly Met Lys Arg Glu Ala

        195                 200                 205

Val Gln Glu Glu Arg Gln Arg Ser Arg Glu Arg Ala Glu Ser Glu Ala

    210                 215                 220

Glu Cys Ala Thr Ser Gly His Glu Asp Met Pro Val Glu Arg Ile Leu

225                 230                 235                 240

Glu Ala Glu Leu Ala Val Glu Pro Lys Thr Glu Ser Tyr Gly Asp Met

                245                 250                 255

Asn Met Glu Asn Ser Thr Asn Asp Pro Val Thr Asn Ile Cys His Ala

            260                 265                 270

Ala Asp Lys Gln Leu Phe Thr Leu Val Glu Trp Ala Lys Arg Ile Pro

        275                 280                 285

His Phe Ser Asp Leu Thr Leu Glu Asp Gln Val Ile Leu Leu Arg Ala

    290                 295                 300

Gly Trp Asn Glu Leu Leu Ile Ala Ser Phe Ser His Arg Ser Val Ser

305                 310                 315                 320

Val Gln Asp Gly Ile Leu Leu Ala Thr Gly Leu His Val His Arg Ser

                325                 330                 335

Ser Ala His Ser Ala Gly Val Gly Ser Ile Phe Asp Arg Val Leu Thr

            340                 345                 350

Glu Leu Val Ser Lys Met Lys Asp Met Gln Met Asp Lys Ser Glu Leu

        355                 360                 365

Gly Cys Leu Arg Ala Ile Val Leu Phe Asn Pro Asp Ala Lys Gly Leu

    370                 375                 380

Ser Asn Pro Ser Glu Val Glu Thr Leu Arg Glu Lys Val Tyr Ala Thr

385                 390                 395                 400

Leu Glu Ala Tyr Thr Lys Gln Lys Tyr Pro Glu Gln Pro Gly Arg Phe

                405                 410                 415

Ala Lys Leu Leu Leu Arg Leu Pro Ala Leu Arg Ser Ile Gly Leu Lys

            420                 425                 430

Cys Leu Glu His Leu Phe Phe Phe Lys Leu Ile Gly Asp Thr Pro Ile

        435                 440                 445

Asp Thr Phe Leu Met Glu Met Leu Glu Thr Pro Leu Gln Ile Thr

    450                 455                 460

<210> 87

<211> 215

<212> PRT

<213> Homo sapiens

<220>

<221> vascular endothelial growth factor

<222> (1)..(215)

<223> LocusID; 7422; NM_003376

<400> 87

Met Asn Phe Leu Leu Ser Trp Val His Trp Ser Leu Ala Leu Leu Leu

1               5                   10                  15

Tyr Leu His His Ala Lys Trp Ser Gln Ala Ala Pro Met Ala Glu Gly

                20                  25                  30

Gly Gly Gln Asn His His Glu Val Val Lys Phe Met Asp Val Tyr Gln

        35                  40                  45

Arg Ser Tyr Cys His Pro Ile Glu Thr Leu Val Asp Ile Phe Gln Glu

    50                  55                  60

Tyr Pro Asp Glu Ile Glu Tyr Ile Phe Lys Pro Ser Cys Val Pro Leu

65                  70                  75                  80

Met Arg Cys Gly Gly Cys Cys Asn Asp Glu Gly Leu Glu Cys Val Pro

                85                  90                  95

Thr Glu Glu Ser Asn Ile Thr Met Gln Ile Met Arg Ile Lys Pro His

            100                 105                 110

Gln Gly Gln His Ile Gly Glu Met Ser Phe Leu Gln His Asn Lys Cys

        115                 120                 125

Glu Cys Arg Pro Lys Lys Asp Arg Ala Arg Gln Glu Lys Lys Ser Val

    130                 135                 140

Arg Gly Lys Gly Lys Gly Gln Lys Arg Lys Arg Lys Lys Ser Arg Tyr

145                 150                 155                 160

Lys Ser Trp Ser Val Pro Cys Gly Pro Cys Ser Glu Arg Arg Lys His

                165                 170                 175

Leu Phe Val Gln Asp Pro Gln Thr Cys Lys Cys Ser Cys Lys Asn Thr

            180                 185                 190

Asp Ser Arg Cys Lys Ala Arg Gln Leu Glu Leu Asn Glu Arg Thr Cys

        195                 200                 205

Arg Cys Asp Lys Pro Arg Arg

    210                 215

<210>  88

<211>  472

<212>  PRT

<213>  Homo sapiens

<220>

<221>  cannabinoid receptor 1 (brain) variant 1

<222>  (1)..(472)

<223>  LocusID: 1268; NM_016083

<400>  88

Met Lys Ser Ile Leu Asp Gly Leu Ala Asp Thr Thr Phe Arg Thr Ile
1               5                   10                  15

Thr Thr Asp Leu Leu Tyr Val Gly Ser Asn Asp Ile Gln Tyr Glu Asp
            20                  25                  30

Ile Lys Gly Asp Met Ala Ser Lys Leu Gly Tyr Phe Pro Gln Lys Phe
        35                  40                  45

Pro Leu Thr Ser Phe Arg Gly Ser Pro Phe Gln Glu Lys Met Thr Ala
    50                  55                  60

Gly Asp Asn Pro Gln Leu Val Pro Ala Asp Gln Val Asn Ile Thr Glu
65                  70                  75                  80

Phe Tyr Asn Lys Ser Leu Ser Ser Phe Lys Glu Asn Glu Glu Asn Ile
                85                  90                  95

Gln Cys Gly Glu Asn Phe Met Asp Ile Glu Cys Phe Met Val Leu Asn
            100                 105                 110

Pro Ser Gln Gln Leu Ala Ile Ala Val Leu Ser Leu Thr Leu Gly Thr
        115                 120                 125

Phe Thr Val Leu Glu Asn Leu Leu Val Leu Cys Val Ile Leu His Ser
    130                 135                 140

Arg Ser Leu Arg Cys Arg Pro Ser Tyr His Phe Ile Gly Ser Leu Ala
145                 150                 155                 160

Val Ala Asp Leu Leu Gly Ser Val Ile Phe Val Tyr Ser Phe Ile Asp
                165                 170                 175

Phe His Val Phe His Arg Lys Asp Ser Arg Asn Val Phe Leu Phe Lys
            180                 185                 190

Leu Gly Gly Val Thr Ala Ser Phe Thr Ala Ser Val Gly Ser Leu Phe

        195                 200                 205
Leu Thr Ala Ile Asp Arg Tyr Ile Ser Ile His Arg Pro Leu Ala Tyr
    210                 215                 220
Lys Arg Ile Val Thr Arg Pro Lys Ala Val Val Ala Phe Cys Leu Met
225                 230                 235                 240
Trp Thr Ile Ala Ile Val Ile Ala Val Leu Pro Leu Leu Gly Trp Asn
                245                 250                 255
Cys Glu Lys Leu Gln Ser Val Cys Ser Asp Ile Phe Pro His Ile Asp
            260                 265                 270
Glu Thr Tyr Leu Met Phe Trp Ile Gly Val Thr Ser Val Leu Leu Leu
        275                 280                 285
Phe Ile Val Tyr Ala Tyr Met Tyr Ile Leu Trp Lys Ala His Ser His
    290                 295                 300
Ala Val Arg Met Ile Gln Arg Gly Thr Gln Lys Ser Ile Ile Ile His
305                 310                 315                 320
Thr Ser Glu Asp Gly Lys Val Gln Val Thr Arg Pro Asp Gln Ala Arg
                325                 330                 335
Met Asp Ile Arg Leu Ala Lys Thr Leu Val Leu Ile Leu Val Val Leu
            340                 345                 350
Ile Ile Cys Trp Gly Pro Leu Leu Ala Ile Met Val Tyr Asp Val Phe
        355                 360                 365
Gly Lys Met Asn Lys Leu Ile Lys Thr Val Phe Ala Phe Cys Ser Met
    370                 375                 380
Leu Cys Leu Leu Asn Ser Thr Val Asn Pro Ile Ile Tyr Ala Leu Arg
385                 390                 395                 400
Ser Lys Asp Leu Arg His Ala Phe Arg Ser Met Phe Pro Ser Cys Glu
                405                 410                 415
Gly Thr Ala Gln Pro Leu Asp Asn Ser Met Gly Asp Ser Asp Cys Leu
            420                 425                 430
His Lys His Ala Asn Asn Ala Ala Ser Val His Arg Ala Ala Glu Ser
        435                 440                 445
Cys Ile Lys Ser Thr Val Lys Ile Ala Lys Val Thr Met Ser Val Ser

450                 455                 460

Thr Asp Thr Ser Ala Glu Ala Leu

465                 470

<210>  89

<211>  411

<212>  PRT

<213>  Homo sapiens

<220>

<221>  cannabinoid receptor 1 (brain) variant 2

<222>  (1)..(411)

<223>  LocusID: 1268; NM_033181

<400>  89

Met Ala Leu Gln Ile Pro Pro Ser Ala Pro Ser Pro Leu Thr Ser Cys

1               5                   10                  15

Thr Trp Ala Gln Met Thr Phe Ser Thr Lys Thr Ser Lys Glu Asn Glu

            20                  25                  30

Glu Asn Ile Gln Cys Gly Glu Asn Phe Met Asp Ile Glu Cys Phe Met

        35                  40                  45

Val Leu Asn Pro Ser Gln Gln Leu Ala Ile Ala Val Leu Ser Leu Thr

    50                  55                  60

Leu Gly Thr Phe Thr Val Leu Glu Asn Leu Leu Val Leu Cys Val Ile

65                  70                  75                  80

Leu His Ser Arg Ser Leu Arg Cys Arg Pro Ser Tyr His Phe Ile Gly

                85                  90                  95

Ser Leu Ala Val Ala Asp Leu Leu Gly Ser Val Ile Phe Val Tyr Ser

            100                 105                 110

Phe Ile Asp Phe His Val Phe His Arg Lys Asp Ser Arg Asn Val Phe

        115                 120                 125

Leu Phe Lys Leu Gly Gly Val Thr Ala Ser Phe Thr Ala Ser Val Gly

    130                 135                 140

Ser Leu Phe Leu Thr Ala Ile Asp Arg Tyr Ile Ser Ile His Arg Pro

145                 150                 155                 160

Leu Ala Tyr Lys Arg Ile Val Thr Arg Pro Lys Ala Val Val Ala Phe

                165                 170                 175

Cys Leu Met Trp Thr Ile Ala Ile Val Ile Ala Val Leu Pro Leu Leu

            180                 185                 190

Gly Trp Asn Cys Glu Lys Leu Gln Ser Val Cys Ser Asp Ile Phe Pro

        195                 200                 205

His Ile Asp Glu Thr Tyr Leu Met Phe Trp Ile Gly Val Thr Ser Val

    210                 215                 220

Leu Leu Leu Phe Ile Val Tyr Ala Tyr Met Tyr Ile Leu Trp Lys Ala

225                 230                 235                 240

His Ser His Ala Val Arg Met Ile Gln Arg Gly Thr Gln Lys Ser Ile

                245                 250                 255

Ile Ile His Thr Ser Glu Asp Gly Lys Val Gln Val Thr Arg Pro Asp

            260                 265                 270

Gln Ala Arg Met Asp Ile Arg Leu Ala Lys Thr Leu Val Leu Ile Leu

        275                 280                 285

Val Val Leu Ile Ile Cys Trp Gly Pro Leu Leu Ala Ile Met Val Tyr

    290                 295                 300

Asp Val Phe Gly Lys Met Asn Lys Leu Ile Lys Thr Val Phe Ala Phe

305                 310                 315                 320

Cys Ser Met Leu Cys Leu Leu Asn Ser Thr Val Asn Pro Ile Ile Tyr

                325                 330                 335

Ala Leu Arg Ser Lys Asp Leu Arg His Ala Phe Arg Ser Met Phe Pro

            340                 345                 350

Ser Cys Glu Gly Thr Ala Gln Pro Leu Asp Asn Ser Met Gly Asp Ser

        355                 360                 365

Asp Cys Leu His Lys His Ala Asn Asn Ala Ala Ser Val His Arg Ala

    370                 375                 380

Ala Glu Ser Cys Ile Lys Ser Thr Val Lys Ile Ala Lys Val Thr Met

385                 390                 395                 400

Ser Val Ser Thr Asp Thr Ser Ala Glu Ala Leu

                405                 410

<210>  90

<211>  321

<212>  PRT

<213>  Homo sapiens

<220>

<221>  cathepsin O

<222>  (1)..(321)

<223>  LocusID: 1519; NM_001334

<400>  90

Met Asp Val Arg Ala Leu Pro Trp Leu Pro Trp Leu Leu Trp Leu Leu

1               5                   10                  15

Cys Arg Gly Gly Gly Asp Ala Asp Ser Arg Ala Pro Phe Thr Pro Thr

            20                  25                  30

Trp Pro Arg Ser Arg Glu Arg Glu Ala Ala Ala Phe Arg Glu Ser Leu

        35                  40                  45

Asn Arg His Arg Tyr Leu Asn Ser Leu Phe Pro Ser Glu Asn Ser Thr

    50                  55                  60

Ala Phe Tyr Gly Ile Asn Gln Phe Ser Tyr Leu Phe Pro Glu Glu Phe

65                  70                  75                  80

Lys Ala Ile Tyr Leu Arg Ser Lys Pro Ser Lys Phe Pro Arg Tyr Ser

                85                  90                  95

Ala Glu Val His Met Ser Ile Pro Asn Val Ser Leu Pro Leu Arg Phe

            100                 105                 110

Asp Trp Arg Asp Lys Gln Val Val Thr Gln Val Arg Asn Gln Gln Met

        115                 120                 125

Cys Gly Gly Cys Trp Ala Phe Ser Val Val Gly Ala Val Glu Ser Ala

130                 135                 140

Tyr Ala Ile Lys Gly Lys Pro Leu Glu Asp Leu Ser Val Gln Gln Val

145                 150                 155                 160

Ile Asp Cys Ser Tyr Asn Asn Tyr Gly Cys Asn Gly Gly Ser Thr Leu

                165                 170                 175

Asn Ala Leu Asn Trp Leu Asn Lys Met Gln Val Lys Leu Val Lys Asp

            180                 185                 190

Ser Glu Tyr Pro Phe Lys Ala Gln Asn Gly Leu Cys His Tyr Phe Ser

        195                 200                 205

Gly Ser His Ser Gly Phe Ser Ile Lys Gly Tyr Ser Ala Tyr Asp Phe

    210                 215                 220

Ser Asp Gln Glu Asp Glu Met Ala Lys Ala Leu Leu Thr Phe Gly Pro

225                 230                 235                 240

Leu Val Val Ile Val Asp Ala Val Ser Trp Gln Asp Tyr Leu Gly Gly

                245                 250                 255

Ile Ile Gln His His Cys Ser Ser Gly Glu Ala Asn His Ala Val Leu

            260                 265                 270

Ile Thr Gly Phe Asp Lys Thr Gly Ser Thr Pro Tyr Trp Ile Val Arg

        275                 280                 285

Asn Ser Trp Gly Ser Ser Trp Gly Val Asp Gly Tyr Ala His Val Lys

    290                 295                 300

Met Gly Ser Asn Val Cys Gly Ile Ala Asp Ser Val Ser Ser Ile Phe

305                 310                 315                 320

Val

<210>  91

<211>  290

<212>  PRT

<213>  Homo sapiens

<220>

<221>  enoyl Coenzyme A hydratase, short chain, 1

<222>  (1)..(290)

<223>  LocusID: 1892; NM_004092

<400>  91

Met Ala Ala Leu Arg Val Leu Leu Ser Cys Ala Arg Gly Pro Leu Arg
1               5                   10                  15

Pro Pro Val Arg Cys Pro Ala Trp Arg Pro Phe Ala Ser Gly Ala Asn
            20                  25                  30

Phe Glu Tyr Ile Ile Ala Glu Lys Arg Gly Lys Asn Asn Thr Val Gly
        35                  40                  45

Leu Ile Gln Leu Asn Arg Pro Lys Ala Leu Asn Ala Leu Cys Asp Gly
    50                  55                  60

Leu Ile Asp Glu Leu Asn Gln Ala Leu Lys Ile Phe Glu Glu Asp Pro
65                  70                  75                  80

Ala Val Gly Ala Ile Val Leu Thr Gly Gly Asp Lys Ala Phe Ala Ala
                85                  90                  95

Gly Ala Asp Ile Lys Glu Met Gln Asn Leu Ser Phe Gln Asp Cys Tyr
            100                 105                 110

Ser Ser Lys Phe Leu Lys His Trp Asp His Leu Thr Gln Val Lys Lys
        115                 120                 125

Pro Val Ile Ala Ala Val Asn Gly Tyr Ala Phe Gly Gly Gly Cys Glu
    130                 135                 140

Leu Ala Met Met Cys Asp Ile Ile Tyr Ala Gly Glu Lys Ala Gln Phe
145                 150                 155                 160

Ala Gln Pro Glu Ile Leu Ile Gly Thr Ile Pro Gly Ala Gly Gly Thr
                165                 170                 175

Gln Arg Leu Thr Arg Ala Val Gly Lys Ser Leu Ala Met Glu Met Val
            180                 185                 190

Leu Thr Gly Asp Arg Ile Ser Ala Gln Asp Ala Lys Gln Ala Gly Leu
        195                 200                 205

Val Ser Lys Ile Cys Pro Val Glu Thr Leu Val Glu Glu Ala Ile Gln

210                 215                 220

Cys Ala Glu Lys Ile Ala Ser Asn Ser Lys Ile Val Val Ala Met Ala

225                 230                 235                 240

Lys Glu Ser Val Asn Ala Ala Phe Glu Met Thr Leu Thr Glu Gly Ser

                245                 250                 255

Lys Leu Glu Lys Lys Leu Phe Tyr Ser Thr Phe Ala Thr Asp Asp Arg

            260                 265                 270

Lys Glu Gly Met Thr Ala Phe Val Glu Lys Arg Lys Ala Asn Phe Lys

        275                 280                 285

Asp Gln

    290

<210>  92

<211>  207

<212>  PRT

<213>  Homo sapiens

<220>

<221>  vascular endothelial growth factor B

<222>  (1)..(207)

<223>  LocusID: 7423; NM_003377

<400>  92

Met Ser Pro Leu Leu Arg Arg Leu Leu Leu Ala Ala Leu Leu Gln Leu

1               5                   10                  15

Ala Pro Ala Gln Ala Pro Val Ser Gln Pro Asp Ala Pro Gly His Gln

            20                  25                  30

Arg Lys Val Val Ser Trp Ile Asp Val Tyr Thr Arg Ala Thr Cys Gln

        35                  40                  45

Pro Arg Glu Val Val Val Pro Leu Thr Val Glu Leu Met Gly Thr Val

    50                  55                  60

Ala Lys Gln Leu Val Pro Ser Cys Val Thr Val Gln Arg Cys Gly Gly

65                  70                  75                  80

Cys Cys Pro Asp Asp Gly Leu Glu Cys Val Pro Thr Gly Gln His Gln

                85                  90                  95

Val Arg Met Gln Ile Leu Met Ile Arg Tyr Pro Ser Ser Gln Leu Gly

            100                 105                 110

Glu Met Ser Leu Glu Glu His Ser Gln Cys Glu Cys Arg Pro Lys Lys

        115                 120                 125

Lys Asp Ser Ala Val Lys Pro Asp Arg Ala Ala Thr Pro His His Arg

    130                 135                 140

Pro Gln Pro Arg Ser Val Pro Gly Trp Asp Ser Ala Pro Gly Ala Pro

145                 150                 155                 160

Ser Pro Ala Asp Ile Thr His Pro Thr Pro Ala Pro Gly Pro Ser Ala

                165                 170                 175

His Ala Ala Pro Ser Thr Thr Ser Ala Leu Thr Pro Gly Pro Ala Ala

            180                 185                 190

Ala Ala Ala Asp Ala Ala Ala Ser Ser Val Ala Lys Gly Gly Ala

        195                 200                 205

<210>  93

<211>  1178

<212>  PRT

<213>  Homo sapiens

<220>

<221>  pyruvate carboxylase variant A

<222>  (1)..(1178)

<223>  LocusID: 5091; NM_000920

<400>  93

Met Leu Lys Phe Arg Thr Val His Gly Gly Leu Arg Leu Leu Gly Ile

1               5                   10                  15

Arg Arg Thr Ser Thr Ala Pro Ala Ala Ser Pro Asn Val Arg Arg Leu

20                  25                  30
Glu Tyr Lys Pro Ile Lys Lys Val Met Val Ala Asn Arg Gly Glu Ile
        35                  40                  45
Ala Ile Arg Val Phe Arg Ala Cys Thr Glu Leu Gly Ile Arg Thr Val
    50                  55                  60
Ala Ile Tyr Ser Glu Gln Asp Thr Gly Gln Met His Arg Gln Lys Ala
65                  70                  75                  80
Asp Glu Ala Tyr Leu Ile Gly Arg Gly Leu Ala Pro Val Gln Ala Tyr
                85                  90                  95
Leu His Ile Pro Asp Ile Ile Lys Val Ala Lys Glu Asn Asn Val Asp
            100                 105                 110
Ala Val His Pro Gly Tyr Gly Phe Leu Ser Glu Arg Ala Asp Phe Ala
        115                 120                 125
Gln Ala Cys Gln Asp Ala Gly Val Arg Phe Ile Gly Pro Ser Pro Glu
    130                 135                 140
Val Val Arg Lys Met Gly Asp Lys Val Glu Ala Arg Ala Ile Ala Ile
145                 150                 155                 160
Ala Ala Gly Val Pro Val Val Pro Gly Thr Asp Ala Pro Ile Thr Ser
                165                 170                 175
Leu His Glu Ala His Glu Phe Ser Asn Thr Tyr Gly Phe Pro Ile Ile
            180                 185                 190
Phe Lys Ala Ala Tyr Gly Gly Gly Gly Arg Gly Met Arg Val Val His
        195                 200                 205
Ser Tyr Glu Glu Leu Glu Glu Asn Tyr Thr Arg Ala Tyr Ser Glu Ala
    210                 215                 220
Leu Ala Ala Phe Gly Asn Gly Ala Leu Phe Val Glu Lys Phe Ile Glu
225                 230                 235                 240
Lys Pro Arg His Ile Glu Val Gln Ile Leu Gly Asp Gln Tyr Gly Asn
                245                 250                 255
Ile Leu His Leu Tyr Glu Arg Asp Cys Ser Ile Gln Arg Arg His Gln
            260                 265                 270
Lys Val Val Glu Ile Ala Pro Ala Ala His Leu Asp Pro Gln Leu Arg

        275                 280                 285
Thr Arg Leu Thr Ser Asp Ser Val Lys Leu Ala Lys Gln Val Gly Tyr
    290                 295                 300
Glu Asn Ala Gly Thr Val Glu Phe Leu Val Asp Arg His Gly Lys His
305                 310                 315                 320
Tyr Phe Ile Glu Val Asn Ser Arg Leu Gln Val Glu His Thr Val Thr
                325                 330                 335
Glu Glu Ile Thr Asp Val Asp Leu Val His Ala Gln Ile His Val Ser
            340                 345                 350
Glu Gly Arg Ser Leu Pro Asp Leu Gly Leu Arg Gln Glu Asn Ile Arg
        355                 360                 365
Ile Asn Gly Cys Ala Ile Gln Cys Arg Val Thr Thr Glu Asp Pro Ala
    370                 375                 380
Arg Ser Phe Gln Pro Asp Thr Gly Arg Ile Glu Val Phe Arg Ser Gly
385                 390                 395                 400
Glu Gly Met Gly Ile Arg Leu Asp Asn Ala Ser Ala Phe Gln Gly Ala
                405                 410                 415
Val Ile Ser Pro His Tyr Asp Ser Leu Leu Val Lys Val Ile Ala His
            420                 425                 430
Gly Lys Asp His Pro Thr Ala Ala Thr Lys Met Ser Arg Ala Leu Ala
        435                 440                 445
Glu Phe Arg Val Arg Gly Val Lys Thr Asn Ile Ala Phe Leu Gln Asn
    450                 455                 460
Val Leu Asn Asn Gln Gln Phe Leu Ala Gly Thr Val Asp Thr Gln Phe
465                 470                 475                 480
Ile Asp Glu Asn Pro Glu Leu Phe Gln Leu Arg Pro Ala Gln Asn Arg
                485                 490                 495
Ala Gln Lys Leu Leu His Tyr Leu Gly His Val Met Val Asn Gly Pro
            500                 505                 510
Thr Thr Pro Ile Pro Val Lys Ala Ser Pro Ser Pro Thr Asp Pro Val
        515                 520                 525
Val Pro Ala Val Pro Ile Gly Pro Pro Pro Ala Gly Phe Arg Asp Ile

530                 535                 540

Leu Leu Arg Glu Gly Pro Glu Gly Phe Ala Arg Ala Val Arg Asn His
545                 550                 555                 560

Pro Gly Leu Leu Leu Met Asp Thr Thr Phe Arg Asp Ala His Gln Ser
                565                 570                 575

Leu Leu Ala Thr Arg Val Arg Thr His Asp Leu Lys Lys Ile Ala Pro
            580                 585                 590

Tyr Val Ala His Asn Phe Ser Lys Leu Phe Ser Met Glu Asn Trp Gly
        595                 600                 605

Gly Ala Thr Phe Asp Val Ala Met Arg Phe Leu Tyr Glu Cys Pro Trp
    610                 615                 620

Arg Arg Leu Gln Glu Leu Arg Glu Leu Ile Pro Asn Ile Pro Phe Gln
625                 630                 635                 640

Met Leu Leu Arg Gly Ala Asn Ala Val Gly Tyr Thr Asn Tyr Pro Asp
                645                 650                 655

Asn Val Val Phe Lys Phe Cys Glu Val Ala Lys Glu Asn Gly Met Asp
            660                 665                 670

Val Phe Arg Val Phe Asp Ser Leu Asn Tyr Leu Pro Asn Met Leu Leu
        675                 680                 685

Gly Met Glu Ala Ala Gly Ser Ala Gly Gly Val Val Glu Ala Ala Ile
    690                 695                 700

Ser Tyr Thr Gly Asp Val Ala Asp Pro Ser Arg Thr Lys Tyr Ser Leu
705                 710                 715                 720

Gln Tyr Tyr Met Gly Leu Ala Glu Glu Leu Val Arg Ala Gly Thr His
                725                 730                 735

Ile Leu Cys Ile Lys Asp Met Ala Gly Leu Leu Lys Pro Thr Ala Cys
            740                 745                 750

Thr Met Leu Val Ser Ser Leu Arg Asp Arg Phe Pro Asp Leu Pro Leu
        755                 760                 765

His Ile His Thr His Asp Thr Ser Gly Ala Gly Val Ala Ala Met Leu
    770                 775                 780

Ala Cys Ala Gln Ala Gly Ala Asp Val Val Asp Val Ala Ala Asp Ser

785                 790                 795                 800

Met Ser Gly Met Thr Ser Gln Pro Ser Met Gly Ala Leu Val Ala Cys

                805                 810                 815

Thr Arg Gly Thr Pro Leu Asp Thr Glu Val Pro Met Glu Arg Val Phe

            820                 825                 830

Asp Tyr Ser Glu Tyr Trp Glu Gly Ala Arg Gly Leu Tyr Ala Ala Phe

        835                 840                 845

Asp Cys Thr Ala Thr Met Lys Ser Gly Asn Ser Asp Val Tyr Glu Asn

    850                 855                 860

Glu Ile Pro Gly Gly Gln Tyr Thr Asn Leu His Phe Gln Ala His Ser

865                 870                 875                 880

Met Gly Leu Gly Ser Lys Phe Lys Glu Val Lys Lys Ala Tyr Val Glu

                885                 890                 895

Ala Asn Gln Met Leu Gly Asp Leu Ile Lys Val Thr Pro Ser Ser Lys

            900                 905                 910

Ile Val Gly Asp Leu Ala Gln Phe Met Val Gln Asn Gly Leu Ser Arg

        915                 920                 925

Ala Glu Ala Glu Ala Gln Ala Glu Glu Leu Ser Phe Pro Arg Ser Val

    930                 935                 940

Val Glu Phe Leu Gln Gly Tyr Ile Gly Val Pro His Gly Gly Phe Pro

945                 950                 955                 960

Glu Pro Phe Arg Ser Lys Val Leu Lys Asp Leu Pro Arg Val Glu Gly

                965                 970                 975

Arg Pro Gly Ala Ser Leu Pro Pro Leu Asp Leu Gln Ala Leu Glu Lys

            980                 985                 990

Glu Leu Val Asp Arg His Gly Glu  Glu Val Thr Pro Glu  Asp Val Leu

        995                 1000                 1005

Ser Ala  Ala Met Tyr Pro Asp  Val Phe Ala His Phe  Lys Asp Phe

    1010                 1015                 1020

Thr Ala  Thr Phe Gly Pro Leu  Asp Ser Leu Asn Thr  Arg Leu Phe

    1025                 1030                 1035

Leu Gln  Gly Pro Lys Ile Ala  Glu Glu Phe Glu Val  Glu Leu Glu

1040                    1045                    1050

Arg Gly  Lys Thr Leu His Ile  Lys Ala Leu Ala Val  Ser Asp Leu

    1055                    1060                    1065

Asn Arg  Ala Gly Gln Arg Gln  Val Phe Phe Glu Leu  Asn Gly Gln

    1070                    1075                    1080

Leu Arg  Ser Ile Leu Val Lys  Asp Thr Gln Ala Met  Lys Glu Met

    1085                    1090                    1095

His Phe  His Pro Lys Ala Leu  Lys Asp Val Lys Gly  Gln Ile Gly

    1100                    1105                    1110

Ala Pro  Met Pro Gly Lys Val  Ile Asp Ile Lys Val  Val Ala Gly

    1115                    1120                    1125

Ala Lys  Val Ala Lys Gly Gln  Pro Leu Cys Val Leu  Ser Ala Met

    1130                    1135                    1140

Lys Met  Glu Thr Val Val Thr  Ser Pro Met Glu Gly  Thr Val Arg

    1145                    1150                    1155

Lys Val  His Val Thr Lys Asp  Met Thr Leu Glu Gly  Asp Asp Leu

    1160                    1165                    1170

Ile Leu  Glu Ile Glu

    1175

<210>  94

<211>  1178

<212>  PRT

<213>  Homo sapiens

<220>

<221>  pyruvate carboxylase variant 2

<222>  (1)..(1178)

<223>  LocusID: 5091; NM_022172

<400>  94

Met Leu Lys Phe Arg Thr Val His Gly Gly Leu Arg Leu Leu Gly Ile
1               5                   10                  15
Arg Arg Thr Ser Thr Ala Pro Ala Ala Ser Pro Asn Val Arg Arg Leu
            20                  25                  30
Glu Tyr Lys Pro Ile Lys Lys Val Met Val Ala Asn Arg Gly Glu Ile
        35                  40                  45
Ala Ile Arg Val Phe Arg Ala Cys Thr Glu Leu Gly Ile Arg Thr Val
    50                  55                  60
Ala Ile Tyr Ser Glu Gln Asp Thr Gly Gln Met His Arg Gln Lys Ala
65                  70                  75                  80
Asp Glu Ala Tyr Leu Ile Gly Arg Gly Leu Ala Pro Val Gln Ala Tyr
                85                  90                  95
Leu His Ile Pro Asp Ile Ile Lys Val Ala Lys Glu Asn Asn Val Asp
            100                 105                 110
Ala Val His Pro Gly Tyr Gly Phe Leu Ser Glu Arg Ala Asp Phe Ala
        115                 120                 125
Gln Ala Cys Gln Asp Ala Gly Val Arg Phe Ile Gly Pro Ser Pro Glu
    130                 135                 140
Val Val Arg Lys Met Gly Asp Lys Val Glu Ala Arg Ala Ile Ala Ile
145                 150                 155                 160
Ala Ala Gly Val Pro Val Val Pro Gly Thr Asp Ala Pro Ile Thr Ser
                165                 170                 175
Leu His Glu Ala His Glu Phe Ser Asn Thr Tyr Gly Phe Pro Ile Ile
            180                 185                 190
Phe Lys Ala Ala Tyr Gly Gly Gly Gly Arg Gly Met Arg Val Val His
        195                 200                 205
Ser Tyr Glu Glu Leu Glu Glu Asn Tyr Thr Arg Ala Tyr Ser Glu Ala
    210                 215                 220
Leu Ala Ala Phe Gly Asn Gly Ala Leu Phe Val Glu Lys Phe Ile Glu
225                 230                 235                 240
Lys Pro Arg His Ile Glu Val Gln Ile Leu Gly Asp Gln Tyr Gly Asn
                245                 250                 255

Ile Leu His Leu Tyr Glu Arg Asp Cys Ser Ile Gln Arg Arg His Gln
            260                 265                 270
Lys Val Val Glu Ile Ala Pro Ala Ala His Leu Asp Pro Gln Leu Arg
        275                 280                 285
Thr Arg Leu Thr Ser Asp Ser Val Lys Leu Ala Lys Gln Val Gly Tyr
    290                 295                 300
Glu Asn Ala Gly Thr Val Glu Phe Leu Val Asp Arg His Gly Lys His
305                 310                 315                 320
Tyr Phe Ile Glu Val Asn Ser Arg Leu Gln Val Glu His Thr Val Thr
                325                 330                 335
Glu Glu Ile Thr Asp Val Asp Leu Val His Ala Gln Ile His Val Ser
            340                 345                 350
Glu Gly Arg Ser Leu Pro Asp Leu Gly Leu Arg Gln Glu Asn Ile Arg
        355                 360                 365
Ile Asn Gly Cys Ala Ile Gln Cys Arg Val Thr Thr Glu Asp Pro Ala
    370                 375                 380
Arg Ser Phe Gln Pro Asp Thr Gly Arg Ile Glu Val Phe Arg Ser Gly
385                 390                 395                 400
Glu Gly Met Gly Ile Arg Leu Asp Asn Ala Ser Ala Phe Gln Gly Ala
                405                 410                 415
Val Ile Ser Pro His Tyr Asp Ser Leu Leu Val Lys Val Ile Ala His
            420                 425                 430
Gly Lys Asp His Pro Thr Ala Ala Thr Lys Met Ser Arg Ala Leu Ala
        435                 440                 445
Glu Phe Arg Val Arg Gly Val Lys Thr Asn Ile Ala Phe Leu Gln Asn
    450                 455                 460
Val Leu Asn Asn Gln Gln Phe Leu Ala Gly Thr Val Asp Thr Gln Phe
465                 470                 475                 480
Ile Asp Glu Asn Pro Glu Leu Phe Gln Leu Arg Pro Ala Gln Asn Arg
                485                 490                 495
Ala Gln Lys Leu Leu His Tyr Leu Gly His Val Met Val Asn Gly Pro
            500                 505                 510

Thr Thr Pro Ile Pro Val Lys Ala Ser Pro Ser Pro Thr Asp Pro Val
        515                 520                 525
Val Pro Ala Val Pro Ile Gly Pro Pro Pro Ala Gly Phe Arg Asp Ile
    530                 535                 540
Leu Leu Arg Glu Gly Pro Glu Gly Phe Ala Arg Ala Val Arg Asn His
545                 550                 555                 560
Pro Gly Leu Leu Leu Met Asp Thr Thr Phe Arg Asp Ala His Gln Ser
                565                 570                 575
Leu Leu Ala Thr Arg Val Arg Thr His Asp Leu Lys Lys Ile Ala Pro
            580                 585                 590
Tyr Val Ala His Asn Phe Ser Lys Leu Phe Ser Met Glu Asn Trp Gly
        595                 600                 605
Gly Ala Thr Phe Asp Val Ala Met Arg Phe Leu Tyr Glu Cys Pro Trp
    610                 615                 620
Arg Arg Leu Gln Glu Leu Arg Glu Leu Ile Pro Asn Ile Pro Phe Gln
625                 630                 635                 640
Met Leu Leu Arg Gly Ala Asn Ala Val Gly Tyr Thr Asn Tyr Pro Asp
                645                 650                 655
Asn Val Val Phe Lys Phe Cys Glu Val Ala Lys Glu Asn Gly Met Asp
            660                 665                 670
Val Phe Arg Val Phe Asp Ser Leu Asn Tyr Leu Pro Asn Met Leu Leu
        675                 680                 685
Gly Met Glu Ala Ala Gly Ser Ala Gly Gly Val Val Glu Ala Ala Ile
    690                 695                 700
Ser Tyr Thr Gly Asp Val Ala Asp Pro Ser Arg Thr Lys Tyr Ser Leu
705                 710                 715                 720
Gln Tyr Tyr Met Gly Leu Ala Glu Glu Leu Val Arg Ala Gly Thr His
                725                 730                 735
Ile Leu Cys Ile Lys Asp Met Ala Gly Leu Leu Lys Pro Thr Ala Cys
            740                 745                 750
Thr Met Leu Val Ser Ser Leu Arg Asp Arg Phe Pro Asp Leu Pro Leu
        755                 760                 765

His Ile His Thr His Asp Thr Ser Gly Ala Gly Val Ala Ala Met Leu
    770                 775                 780
Ala Cys Ala Gln Ala Gly Ala Asp Val Val Asp Val Ala Ala Asp Ser
785                 790                 795                 800
Met Ser Gly Met Thr Ser Gln Pro Ser Met Gly Ala Leu Val Ala Cys
                805                 810                 815
Thr Arg Gly Thr Pro Leu Asp Thr Glu Val Pro Met Glu Arg Val Phe
            820                 825                 830
Asp Tyr Ser Glu Tyr Trp Glu Gly Ala Arg Gly Leu Tyr Ala Ala Phe
        835                 840                 845
Asp Cys Thr Ala Thr Met Lys Ser Gly Asn Ser Asp Val Tyr Glu Asn
    850                 855                 860
Glu Ile Pro Gly Gly Gln Tyr Thr Asn Leu His Phe Gln Ala His Ser
865                 870                 875                 880
Met Gly Leu Gly Ser Lys Phe Lys Glu Val Lys Lys Ala Tyr Val Glu
                885                 890                 895
Ala Asn Gln Met Leu Gly Asp Leu Ile Lys Val Thr Pro Ser Ser Lys
            900                 905                 910
Ile Val Gly Asp Leu Ala Gln Phe Met Val Gln Asn Gly Leu Ser Arg
        915                 920                 925
Ala Glu Ala Glu Ala Gln Ala Glu Glu Leu Ser Phe Pro Arg Ser Val
    930                 935                 940
Val Glu Phe Leu Gln Gly Tyr Ile Gly Val Pro His Gly Gly Phe Pro
945                 950                 955                 960
Glu Pro Phe Arg Ser Lys Val Leu Lys Asp Leu Pro Arg Val Glu Gly
                965                 970                 975
Arg Pro Gly Ala Ser Leu Pro Pro Leu Asp Leu Gln Ala Leu Glu Lys
            980                 985                 990
Glu Leu Val Asp Arg His Gly Glu Glu Val Thr Pro Glu Asp Val Leu
        995                 1000                1005
Ser Ala Ala Met Tyr Pro Asp Val Phe Ala His Phe Lys Asp Phe
    1010                1015                1020

Thr Ala Thr Phe Gly Pro Leu Asp Ser Leu Asn Thr Arg Leu Phe
    1025                1030                1035

Leu Gln Gly Pro Lys Ile Ala Glu Glu Phe Glu Val Glu Leu Glu
    1040                1045                1050

Arg Gly Lys Thr Leu His Ile Lys Ala Leu Ala Val Ser Asp Leu
    1055                1060                1065

Asn Arg Ala Gly Gln Arg Gln Val Phe Phe Glu Leu Asn Gly Gln
    1070                1075                1080

Leu Arg Ser Ile Leu Val Lys Asp Thr Gln Ala Met Lys Glu Met
    1085                1090                1095

His Phe His Pro Lys Ala Leu Lys Asp Val Lys Gly Gln Ile Gly
    1100                1105                1110

Ala Pro Met Pro Gly Lys Val Ile Asp Ile Lys Val Val Ala Gly
    1115                1120                1125

Ala Lys Val Ala Lys Gly Gln Pro Leu Cys Val Leu Ser Ala Met
    1130                1135                1140

Lys Met Glu Thr Val Val Thr Ser Pro Met Glu Gly Thr Val Arg
    1145                1150                1155

Lys Val His Val Thr Lys Asp Met Thr Leu Glu Gly Asp Asp Leu
    1160                1165                1170

Ile Leu Glu Ile Glu
    1175

<210> 95
<211> 314
<212> PRT
<213> Homo sapiens
<220>
<221> L-3-hydroxyacyl-Coenzyme A dehydrogenase, short chain
<222> (1)..(314)
<223> LocusID: 3033; NM_005327

<400> 95

Met Ala Phe Val Thr Arg Gln Phe Met Arg Ser Val Ser Ser Ser Ser
1               5                   10                  15

Thr Ala Ser Ala Ser Ala Lys Lys Ile Ile Val Lys His Val Thr Val
            20                  25                  30

Ile Gly Gly Gly Leu Met Gly Ala Gly Ile Ala Gln Val Ala Ala Ala
        35                  40                  45

Thr Gly His Thr Val Val Leu Val Asp Gln Thr Glu Asp Ile Leu Ala
    50                  55                  60

Lys Ser Lys Lys Gly Ile Glu Glu Ser Leu Arg Lys Val Ala Lys Lys
65                  70                  75                  80

Lys Phe Ala Glu Asn Pro Lys Ala Gly Asp Glu Phe Val Glu Lys Thr
                85                  90                  95

Leu Ser Thr Ile Ala Thr Ser Thr Asp Ala Ala Ser Val Val His Ser
            100                 105                 110

Thr Asp Leu Val Val Glu Ala Ile Val Glu Asn Leu Lys Val Lys Asn
        115                 120                 125

Glu Leu Phe Lys Arg Leu Asp Lys Phe Ala Ala Glu His Thr Ile Phe
    130                 135                 140

Ala Ser Asn Thr Ser Ser Leu His Ile Thr Ser Ile Ala Asn Ala Thr
145                 150                 155                 160

Thr Arg Gln Asp Arg Phe Ala Gly Leu His Phe Phe Asn Pro Val Pro
                165                 170                 175

Val Met Lys Leu Val Glu Val Ile Lys Thr Pro Met Thr Ser Gln Lys
            180                 185                 190

Thr Phe Glu Ser Leu Val Asp Phe Ser Lys Ala Leu Gly Lys His Pro
        195                 200                 205

Val Ser Cys Lys Asp Thr Pro Gly Phe Ile Val Asn Arg Leu Leu Val
    210                 215                 220

Pro Tyr Leu Met Glu Ala Ile Arg Leu Tyr Glu Arg Gly Asp Ala Ser
225                 230                 235                 240

Lys Glu Asp Ile Asp Thr Ala Met Lys Leu Gly Ala Gly Tyr Pro Met
                245                 250                 255
Gly Pro Phe Glu Leu Leu Asp Tyr Val Gly Leu Asp Thr Thr Lys Phe
            260                 265                 270
Ile Val Asp Gly Trp His Glu Met Asp Ala Glu Asn Pro Leu His Gln
        275                 280                 285
Pro Ser Pro Ser Leu Asn Lys Leu Val Ala Glu Asn Lys Phe Gly Lys
    290                 295                 300
Lys Thr Gly Glu Gly Phe Tyr Lys Tyr Lys
305                 310

<210> 96
<211> 407
<212> PRT
<213> Homo sapiens
<220>
<221> pyruvate dehydrogenase kinase, isoenzyme 2
<222> (1)..(407)
<223> LocusID: 5164; NM_002611
<400> 96

Met Arg Trp Val Trp Ala Leu Leu Lys Asn Ala Ser Leu Ala Gly Ala
1               5                   10                  15
Pro Lys Tyr Ile Glu His Phe Ser Lys Phe Ser Pro Ser Pro Leu Ser
            20                  25                  30
Met Lys Gln Phe Leu Asp Phe Gly Ser Ser Asn Ala Cys Glu Lys Thr
        35                  40                  45
Ser Phe Thr Phe Leu Arg Gln Glu Leu Pro Val Arg Leu Ala Asn Ile
    50                  55                  60
Met Lys Glu Ile Asn Leu Leu Pro Asp Arg Val Leu Ser Thr Pro Ser
65                  70                  75                  80

Val Gln Leu Val Gln Ser Trp Tyr Val Gln Ser Leu Leu Asp Ile Met
                85                  90                  95
Glu Phe Leu Asp Lys Asp Pro Glu Asp His Arg Thr Leu Ser Gln Phe
            100                 105                 110
Thr Asp Ala Leu Val Thr Ile Arg Asn Arg His Asn Asp Val Val Pro
        115                 120                 125
Thr Met Ala Gln Gly Val Leu Glu Tyr Lys Asp Thr Tyr Gly Asp Asp
    130                 135                 140
Pro Val Ser Asn Gln Asn Ile Gln Tyr Phe Leu Asp Arg Phe Tyr Leu
145                 150                 155                 160
Ser Arg Ile Ser Ile Arg Met Leu Ile Asn Gln His Thr Leu Ile Phe
                165                 170                 175
Asp Gly Ser Thr Asn Pro Ala His Pro Lys His Ile Gly Ser Ile Asp
            180                 185                 190
Pro Asn Cys Asn Val Ser Glu Val Val Lys Asp Ala Tyr Asp Met Ala
        195                 200                 205
Lys Leu Leu Cys Asp Lys Tyr Tyr Met Ala Ser Pro Asp Leu Glu Ile
    210                 215                 220
Gln Glu Ile Asn Ala Ala Asn Ser Lys Gln Pro Ile His Met Val Tyr
225                 230                 235                 240
Val Pro Ser His Leu Tyr His Met Leu Phe Glu Leu Phe Lys Asn Ala
                245                 250                 255
Met Arg Ala Thr Val Glu Ser His Glu Ser Ser Leu Ile Leu Pro Pro
            260                 265                 270
Ile Lys Val Met Val Ala Leu Gly Glu Glu Asp Leu Ser Ile Lys Met
        275                 280                 285
Ser Asp Arg Gly Gly Gly Val Pro Leu Arg Lys Ile Glu Arg Leu Phe
    290                 295                 300
Ser Tyr Met Tyr Ser Thr Ala Pro Thr Pro Gln Pro Gly Thr Gly Gly
305                 310                 315                 320
Thr Pro Leu Ala Gly Phe Gly Tyr Gly Leu Pro Ile Ser Arg Leu Tyr
                325                 330                 335

Ala Lys Tyr Phe Gln Gly Asp Leu Gln Leu Phe Ser Met Glu Gly Phe
            340                 345                 350
Gly Thr Asp Ala Val Ile Tyr Leu Lys Ala Leu Ser Thr Asp Ser Val
        355                 360                 365
Glu Arg Leu Pro Val Tyr Asn Lys Ser Ala Trp Arg His Tyr Gln Thr
    370                 375                 380
Ile Gln Glu Ala Gly Asp Trp Cys Val Pro Ser Thr Glu Pro Lys Asn
385                 390                 395                 400
Thr Ser Thr Tyr Arg Val Ser
                405

<210> 97
<211> 466
<212> PRT
<213> Homo sapiens
<220>
<221> citrate synthase variant 1
<222> (1)..(466)
<223> LocusID: 1431; NM_004077
<400> 97

<210>  98
<211>  400
<212>  PRT
<213>  Homo sapiens
<220>
<221>  citrate synthase variant 2
<222>  (1)..(400)
<223>  LocusID: 1431; NM_198324
<400>  98

Met Met Tyr Gly Gly Met Arg Gly Met Lys Gly Leu Val Tyr Glu Thr
1               5                   10                  15

Ser Val Leu Asp Pro Asp Glu Gly Ile Arg Phe Arg Gly Phe Ser Ile
            20                  25                  30
Pro Glu Cys Gln Lys Leu Leu Pro Lys Ala Lys Gly Gly Glu Glu Pro
        35                  40                  45
Leu Pro Glu Gly Leu Phe Trp Leu Leu Val Thr Gly His Ile Pro Thr
    50                  55                  60
Glu Glu Gln Val Ser Trp Leu Ser Lys Glu Trp Ala Lys Arg Ala Ala
65                  70                  75                  80
Leu Pro Ser His Val Val Thr Met Leu Asp Asn Phe Pro Thr Asn Leu
                85                  90                  95
His Pro Met Ser Gln Leu Ser Ala Ala Val Thr Ala Leu Asn Ser Glu
            100                 105                 110
Ser Asn Phe Ala Arg Ala Tyr Ala Gln Gly Ile Ser Arg Thr Lys Tyr
        115                 120                 125
Trp Glu Leu Ile Tyr Glu Asp Ser Met Asp Leu Ile Ala Lys Leu Pro
    130                 135                 140
Cys Val Ala Ala Lys Ile Tyr Arg Asn Leu Tyr Arg Glu Gly Ser Gly
145                 150                 155                 160
Ile Gly Ala Ile Asp Ser Asn Leu Asp Trp Ser His Asn Phe Thr Asn
                165                 170                 175
Met Leu Gly Tyr Thr Asp His Gln Phe Thr Glu Leu Thr Arg Leu Tyr
            180                 185                 190
Leu Thr Ile His Ser Asp His Glu Gly Gly Asn Val Ser Ala His Thr
        195                 200                 205
Ser His Leu Val Gly Ser Ala Leu Ser Asp Pro Tyr Leu Ser Phe Ala
    210                 215                 220
Ala Ala Met Asn Gly Leu Ala Gly Pro Leu His Gly Leu Ala Asn Gln
225                 230                 235                 240
Glu Val Leu Val Trp Leu Thr Gln Leu Gln Lys Glu Val Gly Lys Asp
                245                 250                 255
Val Ser Asp Glu Lys Leu Arg Asp Tyr Ile Trp Asn Thr Leu Asn Ser
            260                 265                 270

Gly Arg Val Val Pro Gly Tyr Gly His Ala Val Leu Arg Lys Thr Asp
        275                 280                 285
Pro Arg Tyr Thr Cys Gln Arg Glu Phe Ala Leu Lys His Leu Pro Asn
    290                 295                 300
Asp Pro Met Phe Lys Leu Val Ala Gln Leu Tyr Lys Ile Val Pro Asn
305                 310                 315                 320
Val Leu Leu Glu Gln Gly Lys Ala Lys Asn Pro Trp Pro Asn Val Asp
                325                 330                 335
Ala His Ser Gly Val Leu Leu Gln Tyr Tyr Gly Met Thr Glu Met Asn
            340                 345                 350
Tyr Tyr Thr Val Leu Phe Gly Val Ser Arg Ala Leu Gly Val Leu Ala
        355                 360                 365
Gln Leu Ile Trp Ser Arg Ala Leu Gly Phe Pro Leu Glu Arg Pro Lys
    370                 375                 380
Ser Met Ser Thr Glu Gly Leu Met Lys Phe Val Asp Ser Lys Ser Gly
385                 390                 395                 400

<210>  99
<211>  821
<212>  PRT
<213>  Homo sapiens
<220>
<221>  fibroblast growth factor receptor 2 variant 1
<222>  (1)..(821)
<223>  LocusID: 2263; NM_000141
<400>  99

<210> 100
<211> 822
<212> PRT
<213> Homo sapiens
<220>
<221> fibroblast growth factor receptor 2 variant 2
<222> (1)..(822)
<223> LocusID: 2263; NM_022969
<400> 100

Met Val Ser Trp Gly Arg Phe Ile Cys Leu Val Val Val Thr Met Ala
1               5                   10                  15
Thr Leu Ser Leu Ala Arg Pro Ser Phe Ser Leu Val Glu Asp Thr Thr
            20                  25                  30
Leu Glu Pro Glu Glu Pro Pro Thr Lys Tyr Gln Ile Ser Gln Pro Glu
        35                  40                  45
Val Tyr Val Ala Ala Pro Gly Glu Ser Leu Glu Val Arg Cys Leu Leu
    50                  55                  60
Lys Asp Ala Ala Val Ile Ser Trp Thr Lys Asp Gly Val His Leu Gly
65                  70                  75                  80
Pro Asn Asn Arg Thr Val Leu Ile Gly Glu Tyr Leu Gln Ile Lys Gly
                85                  90                  95
Ala Thr Pro Arg Asp Ser Gly Leu Tyr Ala Cys Thr Ala Ser Arg Thr
            100                 105                 110
Val Asp Ser Glu Thr Trp Tyr Phe Met Val Asn Val Thr Asp Ala Ile
        115                 120                 125

Ser Ser Gly Asp Asp Glu Asp Asp Thr Asp Gly Ala Glu Asp Phe Val
    130                 135                 140
Ser Glu Asn Ser Asn Asn Lys Arg Ala Pro Tyr Trp Thr Asn Thr Glu
145                 150                 155                 160
Lys Met Glu Lys Arg Leu His Ala Val Pro Ala Ala Asn Thr Val Lys
                165                 170                 175
Phe Arg Cys Pro Ala Gly Gly Asn Pro Met Pro Thr Met Arg Trp Leu
            180                 185                 190
Lys Asn Gly Lys Glu Phe Lys Gln Glu His Arg Ile Gly Gly Tyr Lys
        195                 200                 205
Val Arg Asn Gln His Trp Ser Leu Ile Met Glu Ser Val Val Pro Ser
    210                 215                 220
Asp Lys Gly Asn Tyr Thr Cys Val Val Glu Asn Glu Tyr Gly Ser Ile
225                 230                 235                 240
Asn His Thr Tyr His Leu Asp Val Val Glu Arg Ser Pro His Arg Pro
                245                 250                 255
Ile Leu Gln Ala Gly Leu Pro Ala Asn Ala Ser Thr Val Val Gly Gly
            260                 265                 270
Asp Val Glu Phe Val Cys Lys Val Tyr Ser Asp Ala Gln Pro His Ile
        275                 280                 285
Gln Trp Ile Lys His Val Glu Lys Asn Gly Ser Lys Tyr Gly Pro Asp
    290                 295                 300
Gly Leu Pro Tyr Leu Lys Val Leu Lys His Ser Gly Ile Asn Ser Ser
305                 310                 315                 320
Asn Ala Glu Val Leu Ala Leu Phe Asn Val Thr Glu Ala Asp Ala Gly
                325                 330                 335
Glu Tyr Ile Cys Lys Val Ser Asn Tyr Ile Gly Gln Ala Asn Gln Ser
            340                 345                 350
Ala Trp Leu Thr Val Leu Pro Lys Gln Gln Ala Pro Gly Arg Glu Lys
        355                 360                 365
Glu Ile Thr Ala Ser Pro Asp Tyr Leu Glu Ile Ala Ile Tyr Cys Ile
    370                 375                 380

Gly Val Phe Leu Ile Ala Cys Met Val Val Thr Val Ile Leu Cys Arg
385                 390                 395                 400
Met Lys Asn Thr Thr Lys Lys Pro Asp Phe Ser Ser Gln Pro Ala Val
                405                 410                 415
His Lys Leu Thr Lys Arg Ile Pro Leu Arg Arg Gln Val Thr Val Ser
            420                 425                 430
Ala Glu Ser Ser Ser Ser Met Asn Ser Asn Thr Pro Leu Val Arg Ile
        435                 440                 445
Thr Thr Arg Leu Ser Ser Thr Ala Asp Thr Pro Met Leu Ala Gly Val
    450                 455                 460
Ser Glu Tyr Glu Leu Pro Glu Asp Pro Lys Trp Glu Phe Pro Arg Asp
465                 470                 475                 480
Lys Leu Thr Leu Gly Lys Pro Leu Gly Glu Gly Cys Phe Gly Gln Val
                485                 490                 495
Val Met Ala Glu Ala Val Gly Ile Asp Lys Asp Lys Pro Lys Glu Ala
            500                 505                 510
Val Thr Val Ala Val Lys Met Leu Lys Asp Asp Ala Thr Glu Lys Asp
        515                 520                 525
Leu Ser Asp Leu Val Ser Glu Met Glu Met Met Lys Met Ile Gly Lys
    530                 535                 540
His Lys Asn Ile Ile Asn Leu Leu Gly Ala Cys Thr Gln Asp Gly Pro
545                 550                 555                 560
Leu Tyr Val Ile Val Glu Tyr Ala Ser Lys Gly Asn Leu Arg Glu Tyr
                565                 570                 575
Leu Arg Ala Arg Arg Pro Pro Gly Met Glu Tyr Ser Tyr Asp Ile Asn
            580                 585                 590
Arg Val Pro Glu Glu Gln Met Thr Phe Lys Asp Leu Val Ser Cys Thr
        595                 600                 605
Tyr Gln Leu Ala Arg Gly Met Glu Tyr Leu Ala Ser Gln Lys Cys Ile
    610                 615                 620
His Arg Asp Leu Ala Ala Arg Asn Val Leu Val Thr Glu Asn Asn Val
625                 630                 635                 640

Met Lys Ile Ala Asp Phe Gly Leu Ala Arg Asp Ile Asn Asn Ile Asp
                645                 650                 655

Tyr Tyr Lys Lys Thr Thr Asn Gly Arg Leu Pro Val Lys Trp Met Ala
            660                 665                 670

Pro Glu Ala Leu Phe Asp Arg Val Tyr Thr His Gln Ser Asp Val Trp
        675                 680                 685

Ser Phe Gly Val Leu Met Trp Glu Ile Phe Thr Leu Gly Gly Ser Pro
    690                 695                 700

Tyr Pro Gly Ile Pro Val Glu Glu Leu Phe Lys Leu Leu Lys Glu Gly
705                 710                 715                 720

His Arg Met Asp Lys Pro Ala Asn Cys Thr Asn Glu Leu Tyr Met Met
                725                 730                 735

Met Arg Asp Cys Trp His Ala Val Pro Ser Gln Arg Pro Thr Phe Lys
            740                 745                 750

Gln Leu Val Glu Asp Leu Asp Arg Ile Leu Thr Leu Thr Thr Asn Glu
        755                 760                 765

Glu Tyr Leu Asp Leu Ser Gln Pro Leu Glu Gln Tyr Ser Pro Ser Tyr
    770                 775                 780

Pro Asp Thr Arg Ser Ser Cys Ser Ser Gly Asp Asp Ser Val Phe Ser
785                 790                 795                 800

Pro Asp Pro Met Pro Tyr Glu Pro Cys Leu Pro Gln Tyr Pro His Ile
                805                 810                 815

Asn Gly Ser Val Lys Thr
            820

<210>  101

<211>  705

<212>  PRT

<213>  Homo sapiens

<220>

<221>  fibroblast growth factor receptor 2 variant 3

<222>  (1)..(705)

<223>  LocusID: 2263; NM_022970

<400>  101

Met Val Ser Trp Gly Arg Phe Ile Cys Leu Val Val Val Thr Met Ala
1               5                   10                  15

Thr Leu Ser Leu Ala Arg Pro Ser Phe Ser Leu Val Glu Asp Thr Thr
            20                  25                  30

Leu Glu Pro Glu Glu Pro Pro Thr Lys Tyr Gln Ile Ser Gln Pro Glu
        35                  40                  45

Val Tyr Val Ala Ala Pro Gly Glu Ser Leu Glu Val Arg Cys Leu Leu
    50                  55                  60

Lys Asp Ala Ala Val Ile Ser Trp Thr Lys Asp Gly Val His Leu Gly
65                  70                  75                  80

Pro Asn Asn Arg Thr Val Leu Ile Gly Glu Tyr Leu Gln Ile Lys Gly
                85                  90                  95

Ala Thr Pro Arg Asp Ser Gly Leu Tyr Ala Cys Thr Ala Ser Arg Thr
            100                 105                 110

Val Asp Ser Glu Thr Trp Tyr Phe Met Val Asn Val Thr Asp Ala Ile
        115                 120                 125

Ser Ser Gly Asp Asp Glu Asp Asp Thr Asp Gly Ala Glu Asp Phe Val
    130                 135                 140

Ser Glu Asn Ser Asn Asn Lys Arg Ala Pro Tyr Trp Thr Asn Thr Glu
145                 150                 155                 160

Lys Met Glu Lys Arg Leu His Ala Val Pro Ala Ala Asn Thr Val Lys
                165                 170                 175

Phe Arg Cys Pro Ala Gly Gly Asn Pro Met Pro Thr Met Arg Trp Leu
            180                 185                 190

Lys Asn Gly Lys Glu Phe Lys Gln Glu His Arg Ile Gly Gly Tyr Lys
        195                 200                 205

Val Arg Asn Gln His Trp Ser Leu Ile Met Glu Ser Val Val Pro Ser
    210                 215                 220

Asp Lys Gly Asn Tyr Thr Cys Val Val Glu Asn Glu Tyr Gly Ser Ile
225                 230                 235                 240
Asn His Thr Tyr His Leu Asp Val Val Glu Arg Ser Pro His Arg Pro
                245                 250                 255
Ile Leu Gln Ala Gly Leu Pro Ala Asn Ala Ser Thr Val Val Gly Gly
            260                 265                 270
Asp Val Glu Phe Val Cys Lys Val Tyr Ser Asp Ala Gln Pro His Ile
        275                 280                 285
Gln Trp Ile Lys His Val Glu Lys Asn Gly Ser Lys Tyr Gly Pro Asp
    290                 295                 300
Gly Leu Pro Tyr Leu Lys Val Leu Lys Val Ser Ala Glu Ser Ser Ser
305                 310                 315                 320
Ser Met Asn Ser Asn Thr Pro Leu Val Arg Ile Thr Thr Arg Leu Ser
                325                 330                 335
Ser Thr Ala Asp Thr Pro Met Leu Ala Gly Val Ser Glu Tyr Glu Leu
            340                 345                 350
Pro Glu Asp Pro Lys Trp Glu Phe Pro Arg Asp Lys Leu Thr Leu Gly
        355                 360                 365
Lys Pro Leu Gly Glu Gly Cys Phe Gly Gln Val Val Met Ala Glu Ala
    370                 375                 380
Val Gly Ile Asp Lys Asp Lys Pro Lys Glu Ala Val Thr Val Ala Val
385                 390                 395                 400
Lys Met Leu Lys Asp Asp Ala Thr Glu Lys Asp Leu Ser Asp Leu Val
                405                 410                 415
Ser Glu Met Glu Met Met Lys Met Ile Gly Lys His Lys Asn Ile Ile
            420                 425                 430
Asn Leu Leu Gly Ala Cys Thr Gln Asp Gly Pro Leu Tyr Val Ile Val
        435                 440                 445
Glu Tyr Ala Ser Lys Gly Asn Leu Arg Glu Tyr Leu Arg Ala Arg Arg
    450                 455                 460
Pro Pro Gly Met Glu Tyr Ser Tyr Asp Ile Asn Arg Val Pro Glu Glu
465                 470                 475                 480

Gln Met Thr Phe Lys Asp Leu Val Ser Cys Thr Tyr Gln Leu Ala Arg
                485                 490                 495
Gly Met Glu Tyr Leu Ala Ser Gln Lys Cys Ile His Arg Asp Leu Ala
            500                 505                 510
Ala Arg Asn Val Leu Val Thr Glu Asn Asn Val Met Lys Ile Ala Asp
        515                 520                 525
Phe Gly Leu Ala Arg Asp Ile Asn Asn Ile Asp Tyr Tyr Lys Lys Thr
    530                 535                 540
Thr Asn Gly Arg Leu Pro Val Lys Trp Met Ala Pro Glu Ala Leu Phe
545                 550                 555                 560
Asp Arg Val Tyr Thr His Gln Ser Asp Val Trp Ser Phe Gly Val Leu
                565                 570                 575
Met Trp Glu Ile Phe Thr Leu Gly Gly Ser Pro Tyr Pro Gly Ile Pro
            580                 585                 590
Val Glu Glu Leu Phe Lys Leu Leu Lys Glu Gly His Arg Met Asp Lys
        595                 600                 605
Pro Ala Asn Cys Thr Asn Glu Leu Tyr Met Met Met Arg Asp Cys Trp
    610                 615                 620
His Ala Val Pro Ser Gln Arg Pro Thr Phe Lys Gln Leu Val Glu Asp
625                 630                 635                 640
Leu Asp Arg Ile Leu Thr Leu Thr Thr Asn Glu Glu Tyr Leu Asp Leu
                645                 650                 655
Ser Gln Pro Leu Glu Gln Tyr Ser Pro Ser Tyr Pro Asp Thr Arg Ser
            660                 665                 670
Ser Cys Ser Ser Gly Asp Asp Ser Val Phe Ser Pro Asp Pro Met Pro
        675                 680                 685
Tyr Glu Pro Cys Leu Pro Gln Tyr Pro His Ile Asn Gly Ser Val Lys
    690                 695                 700
Thr
705

<210> 102

<211> 254

<212> PRT

<213> Homo sapiens

<220>

<221> fibroblast growth factor receptor 2 variant 4

<222> (1)..(254)

<223> LocusID; 2263; NM_022971

<400> 102

Met Val Ser Trp Gly Arg Phe Ile Cys Leu Val Val Val Thr Met Ala
1               5                   10                  15

Thr Leu Ser Leu Ala Arg Pro Ser Phe Ser Leu Val Glu Asp Thr Thr
            20                  25                  30

Leu Glu Pro Glu Glu Pro Pro Thr Lys Tyr Gln Ile Ser Gln Pro Glu
        35                  40                  45

Val Tyr Val Ala Ala Pro Gly Glu Ser Leu Glu Val Arg Cys Leu Leu
    50                  55                  60

Lys Asp Ala Ala Val Ile Ser Trp Thr Lys Asp Gly Val His Leu Gly
65                  70                  75                  80

Pro Asn Asn Arg Thr Val Leu Ile Gly Glu Tyr Leu Gln Ile Lys Gly
                85                  90                  95

Ala Thr Pro Arg Asp Ser Gly Leu Tyr Ala Cys Thr Ala Ser Arg Thr
            100                 105                 110

Val Asp Ser Glu Thr Trp Tyr Phe Met Val Asn Val Thr Asp Ala Ile
        115                 120                 125

Ser Ser Gly Asp Asp Glu Asp Asp Thr Asp Gly Ala Glu Asp Phe Val
    130                 135                 140

Ser Glu Asn Ser Asn Asn Lys Arg Ala Pro Tyr Trp Thr Asn Thr Glu
145                 150                 155                 160

Lys Met Glu Lys Arg Leu His Ala Val Pro Ala Ala Asn Thr Val Lys
                165                 170                 175

Phe Arg Cys Pro Ala Gly Gly Asn Pro Met Pro Thr Met Arg Trp Leu
            180                 185                 190
Lys Asn Gly Lys Glu Phe Lys Gln Glu His Arg Ile Gly Gly Tyr Lys
        195                 200                 205
Val Arg Asn Gln His Trp Ser Leu Ile Met Glu Ser Val Val Pro Ser
    210                 215                 220
Asp Lys Gly Asn Tyr Thr Cys Val Val Glu Asn Glu Tyr Gly Ser Ile
225                 230                 235                 240
Asn His Thr Tyr His Leu Asp Val Val Gly Ser Gln Gly Leu
                245                 250

<210> 103
<211> 822
<212> PRT
<213> Homo sapiens
<220>
<221> fibroblast growth factor receptor 2 variant 5
<222> (1)..(822)
<223> LocusID: 2263; NM_022972
<400> 103

Met Val Ser Trp Gly Arg Phe Ile Cys Leu Val Val Val Thr Met Ala
1               5                   10                  15
Thr Leu Ser Leu Ala Arg Pro Ser Phe Ser Leu Val Glu Asp Thr Thr
            20                  25                  30
Leu Glu Pro Glu Glu Pro Pro Thr Lys Tyr Gln Ile Ser Gln Pro Glu
        35                  40                  45
Val Tyr Val Ala Ala Pro Gly Glu Ser Leu Glu Val Arg Cys Leu Leu
    50                  55                  60
Lys Asp Ala Ala Val Ile Ser Trp Thr Lys Asp Gly Val His Leu Gly
65                  70                  75                  80

Pro Asn Asn Arg Thr Val Leu Ile Gly Glu Tyr Leu Gln Ile Lys Gly
                85                  90                  95
Ala Thr Pro Arg Asp Ser Gly Leu Tyr Ala Cys Thr Ala Ser Arg Thr
            100                 105                 110
Val Asp Ser Glu Thr Trp Tyr Phe Met Val Asn Val Thr Asp Ala Ile
        115                 120                 125
Ser Ser Gly Asp Asp Glu Asp Asp Thr Asp Gly Ala Glu Asp Phe Val
    130                 135                 140
Ser Glu Asn Ser Asn Asn Lys Arg Ala Pro Tyr Trp Thr Asn Thr Glu
145                 150                 155                 160
Lys Met Glu Lys Arg Leu His Ala Val Pro Ala Ala Asn Thr Val Lys
                165                 170                 175
Phe Arg Cys Pro Ala Gly Gly Asn Pro Met Pro Thr Met Arg Trp Leu
            180                 185                 190
Lys Asn Gly Lys Glu Phe Lys Gln Glu His Arg Ile Gly Gly Tyr Lys
        195                 200                 205
Val Arg Asn Gln His Trp Ser Leu Ile Met Glu Ser Val Val Pro Ser
    210                 215                 220
Asp Lys Gly Asn Tyr Thr Cys Val Val Glu Asn Glu Tyr Gly Ser Ile
225                 230                 235                 240
Asn His Thr Tyr His Leu Asp Val Val Glu Arg Ser Pro His Arg Pro
                245                 250                 255
Ile Leu Gln Ala Gly Leu Pro Ala Asn Ala Ser Thr Val Val Gly Gly
            260                 265                 270
Asp Val Glu Phe Val Cys Lys Val Tyr Ser Asp Ala Gln Pro His Ile
        275                 280                 285
Gln Trp Ile Lys His Val Glu Lys Asn Gly Ser Lys Tyr Gly Pro Asp
    290                 295                 300
Gly Leu Pro Tyr Leu Lys Val Leu Lys Val Leu Lys Ala Ala Gly Val
305                 310                 315                 320
Asn Thr Thr Asp Lys Glu Ile Glu Val Leu Tyr Ile Arg Asn Val Thr
                325                 330                 335

Phe Glu Asp Ala Gly Glu Tyr Thr Cys Leu Ala Gly Asn Ser Ile Gly
            340                 345                 350
Ile Ser Phe His Ser Ala Trp Leu Thr Val Leu Pro Ala Pro Gly Arg
        355                 360                 365
Glu Lys Glu Ile Thr Ala Ser Pro Asp Tyr Leu Glu Ile Ala Ile Tyr
    370                 375                 380
Cys Ile Gly Val Phe Leu Ile Ala Cys Met Val Val Thr Val Ile Leu
385                 390                 395                 400
Cys Arg Met Lys Asn Thr Thr Lys Lys Pro Asp Phe Ser Ser Gln Pro
                405                 410                 415
Ala Val His Lys Leu Thr Lys Arg Ile Pro Leu Arg Arg Gln Val Ser
            420                 425                 430
Ala Glu Ser Ser Ser Ser Met Asn Ser Asn Thr Pro Leu Val Arg Ile
        435                 440                 445
Thr Thr Arg Leu Ser Ser Thr Ala Asp Thr Pro Met Leu Ala Gly Val
    450                 455                 460
Ser Glu Tyr Glu Leu Pro Glu Asp Pro Lys Trp Glu Phe Pro Arg Asp
465                 470                 475                 480
Lys Leu Thr Leu Gly Lys Pro Leu Gly Glu Gly Cys Phe Gly Gln Val
                485                 490                 495
Val Met Ala Glu Ala Val Gly Ile Asp Lys Asp Lys Pro Lys Glu Ala
            500                 505                 510
Val Thr Val Ala Val Lys Met Leu Lys Asp Asp Ala Thr Glu Lys Asp
        515                 520                 525
Leu Ser Asp Leu Val Ser Glu Met Glu Met Met Lys Met Ile Gly Lys
    530                 535                 540
His Lys Asn Ile Ile Asn Leu Leu Gly Ala Cys Thr Gln Asp Gly Pro
545                 550                 555                 560
Leu Tyr Val Ile Val Glu Tyr Ala Ser Lys Gly Asn Leu Arg Glu Tyr
                565                 570                 575
Leu Arg Ala Arg Arg Pro Pro Gly Met Glu Tyr Ser Tyr Asp Ile Asn
            580                 585                 590

Arg Val Pro Glu Glu Gln Met Thr Phe Lys Asp Leu Val Ser Cys Thr
        595                 600                 605
Tyr Gln Leu Ala Arg Gly Met Glu Tyr Leu Ala Ser Gln Lys Cys Ile
    610                 615                 620
His Arg Asp Leu Ala Ala Arg Asn Val Leu Val Thr Glu Asn Asn Val
625                 630                 635                 640
Met Lys Ile Ala Asp Phe Gly Leu Ala Arg Asp Ile Asn Asn Ile Asp
                645                 650                 655
Tyr Tyr Lys Lys Thr Thr Asn Gly Arg Leu Pro Val Lys Trp Met Ala
            660                 665                 670
Pro Glu Ala Leu Phe Asp Arg Val Tyr Thr His Gln Ser Asp Val Trp
        675                 680                 685
Ser Phe Gly Val Leu Met Trp Glu Ile Phe Thr Leu Gly Gly Ser Pro
    690                 695                 700
Tyr Pro Gly Ile Pro Val Glu Glu Leu Phe Lys Leu Leu Lys Glu Gly
705                 710                 715                 720
His Arg Met Asp Lys Pro Ala Asn Cys Thr Asn Glu Leu Tyr Met Met
                725                 730                 735
Met Arg Asp Cys Trp His Ala Val Pro Ser Gln Arg Pro Thr Phe Lys
            740                 745                 750
Gln Leu Val Glu Asp Leu Asp Arg Ile Leu Thr Leu Thr Thr Asn Glu
        755                 760                 765
Glu Tyr Leu Asp Leu Ser Gln Pro Leu Glu Gln Tyr Ser Pro Ser Tyr
    770                 775                 780
Pro Asp Thr Arg Ser Ser Cys Ser Ser Gly Asp Asp Ser Val Phe Ser
785                 790                 795                 800
Pro Asp Pro Met Pro Tyr Glu Pro Cys Leu Pro Gln Tyr Pro His Ile
                805                 810                 815
Asn Gly Ser Val Lys Thr
            820

<210>  104

<211>  768

<212>  PRT

<213>  Homo sapiens

<220>

<221>  fibroblast growth factor receptor 2 variant 6

<222>  (1)..(768)

<223>  LocusID: 2263; NM_022973

<400>  104

Met Val Ser Trp Gly Arg Phe Ile Cys Leu Val Val Val Thr Met Ala
1               5                   10                  15

Thr Leu Ser Leu Ala Arg Pro Ser Phe Ser Leu Val Glu Asp Thr Thr
            20                  25                  30

Leu Glu Pro Glu Glu Pro Pro Thr Lys Tyr Gln Ile Ser Gln Pro Glu
        35                  40                  45

Val Tyr Val Ala Ala Pro Gly Glu Ser Leu Glu Val Arg Cys Leu Leu
    50                  55                  60

Lys Asp Ala Ala Val Ile Ser Trp Thr Lys Asp Gly Val His Leu Gly
65                  70                  75                  80

Pro Asn Asn Arg Thr Val Leu Ile Gly Glu Tyr Leu Gln Ile Lys Gly
                85                  90                  95

Ala Thr Pro Arg Asp Ser Gly Leu Tyr Ala Cys Thr Ala Ser Arg Thr
            100                 105                 110

Val Asp Ser Glu Thr Trp Tyr Phe Met Val Asn Val Thr Asp Ala Ile
        115                 120                 125

Ser Ser Gly Asp Asp Glu Asp Asp Thr Asp Gly Ala Glu Asp Phe Val
    130                 135                 140

Ser Glu Asn Ser Asn Asn Lys Arg Ala Pro Tyr Trp Thr Asn Thr Glu
145                 150                 155                 160

Lys Met Glu Lys Arg Leu His Ala Val Pro Ala Ala Asn Thr Val Lys
                165                 170                 175

Phe Arg Cys Pro Ala Gly Gly Asn Pro Met Pro Thr Met Arg Trp Leu
            180                 185                 190
Lys Asn Gly Lys Glu Phe Lys Gln Glu His Arg Ile Gly Gly Tyr Lys
        195                 200                 205
Val Arg Asn Gln His Trp Ser Leu Ile Met Glu Ser Val Val Pro Ser
    210                 215                 220
Asp Lys Gly Asn Tyr Thr Cys Val Val Glu Asn Glu Tyr Gly Ser Ile
225                 230                 235                 240
Asn His Thr Tyr His Leu Asp Val Val Glu Arg Ser Pro His Arg Pro
                245                 250                 255
Ile Leu Gln Ala Gly Leu Pro Ala Asn Ala Ser Thr Val Val Gly Gly
            260                 265                 270
Asp Val Glu Phe Val Cys Lys Val Tyr Ser Asp Ala Gln Pro His Ile
        275                 280                 285
Gln Trp Ile Lys His Val Glu Lys Asn Gly Ser Lys Tyr Gly Pro Asp
    290                 295                 300
Gly Leu Pro Tyr Leu Lys Val Leu Lys Ala Ala Gly Val Asn Thr Thr
305                 310                 315                 320
Asp Lys Glu Ile Glu Val Leu Tyr Ile Arg Asn Val Thr Phe Glu Asp
                325                 330                 335
Ala Gly Glu Tyr Thr Cys Leu Ala Gly Asn Ser Ile Gly Ile Ser Phe
            340                 345                 350
His Ser Ala Trp Leu Thr Val Leu Pro Ala Pro Gly Arg Glu Lys Glu
        355                 360                 365
Ile Thr Ala Ser Pro Asp Tyr Leu Glu Ile Ala Ile Tyr Cys Ile Gly
    370                 375                 380
Val Phe Leu Ile Ala Cys Met Val Val Thr Val Ile Leu Cys Arg Met
385                 390                 395                 400
Lys Asn Thr Thr Lys Lys Pro Asp Phe Ser Ser Gln Pro Ala Val His
                405                 410                 415
Lys Leu Thr Lys Arg Ile Pro Leu Arg Arg Gln Val Thr Val Ser Ala
            420                 425                 430

Glu Ser Ser Ser Ser Met Asn Ser Asn Thr Pro Leu Val Arg Ile Thr
        435                 440                 445
Thr Arg Leu Ser Ser Thr Ala Asp Thr Pro Met Leu Ala Gly Val Ser
    450                 455                 460
Glu Tyr Glu Leu Pro Glu Asp Pro Lys Trp Glu Phe Pro Arg Asp Lys
465                 470                 475                 480
Leu Thr Leu Gly Lys Pro Leu Gly Glu Gly Cys Phe Gly Gln Val Val
                485                 490                 495
Met Ala Glu Ala Val Gly Ile Asp Lys Asp Lys Pro Lys Glu Ala Val
            500                 505                 510
Thr Val Ala Val Lys Met Leu Lys Asp Asp Ala Thr Glu Lys Asp Leu
        515                 520                 525
Ser Asp Leu Val Ser Glu Met Glu Met Met Lys Met Ile Gly Lys His
    530                 535                 540
Lys Asn Ile Ile Asn Leu Leu Gly Ala Cys Thr Gln Asp Gly Pro Leu
545                 550                 555                 560
Tyr Val Ile Val Glu Tyr Ala Ser Lys Gly Asn Leu Arg Glu Tyr Leu
                565                 570                 575
Arg Ala Arg Arg Pro Pro Gly Met Glu Tyr Ser Tyr Asp Ile Asn Arg
            580                 585                 590
Val Pro Glu Glu Gln Met Thr Phe Lys Asp Leu Val Ser Cys Thr Tyr
        595                 600                 605
Gln Leu Ala Arg Gly Met Glu Tyr Leu Ala Ser Gln Lys Cys Ile His
    610                 615                 620
Arg Asp Leu Ala Ala Arg Asn Val Leu Val Thr Glu Asn Asn Val Met
625                 630                 635                 640
Lys Ile Ala Asp Phe Gly Leu Ala Arg Asp Ile Asn Asn Ile Asp Tyr
                645                 650                 655
Tyr Lys Lys Thr Thr Asn Gly Arg Leu Pro Val Lys Trp Met Ala Pro
            660                 665                 670
Glu Ala Leu Phe Asp Arg Val Tyr Thr His Gln Ser Asp Val Trp Ser
        675                 680                 685

Phe Gly Val Leu Met Trp Glu Ile Phe Thr Leu Gly Gly Ser Pro Tyr
    690                 695                 700
Pro Gly Ile Pro Val Glu Glu Leu Phe Lys Leu Leu Lys Glu Gly His
705                 710                 715                 720
Arg Met Asp Lys Pro Ala Asn Cys Thr Asn Glu Leu Tyr Met Met Met
                725                 730                 735
Arg Asp Cys Trp His Ala Val Pro Ser Gln Arg Pro Thr Phe Lys Gln
            740                 745                 750
Leu Val Glu Asp Leu Asp Arg Ile Leu Thr Leu Thr Thr Asn Glu Ile
        755                 760                 765

<210> 105
<211> 769
<212> PRT
<213> Homo sapiens
<220>
<221> fibroblast growth factor receptor 2 variant 7
<222> (1)..(769)
<223> LocusID: 2263; NM_022974
<400> 105

Met Val Ser Trp Gly Arg Phe Ile Cys Leu Val Val Val Thr Met Ala
1               5                   10                  15
Thr Leu Ser Leu Ala Arg Pro Ser Phe Ser Leu Val Glu Asp Thr Thr
            20                  25                  30
Leu Glu Pro Glu Glu Pro Pro Thr Lys Tyr Gln Ile Ser Gln Pro Glu
        35                  40                  45
Val Tyr Val Ala Ala Pro Gly Glu Ser Leu Glu Val Arg Cys Leu Leu
    50                  55                  60
Lys Asp Ala Ala Val Ile Ser Trp Thr Lys Asp Gly Val His Leu Gly
65                  70                  75                  80

Pro Asn Asn Arg Thr Val Leu Ile Gly Glu Tyr Leu Gln Ile Lys Gly
                85                  90                  95
Ala Thr Pro Arg Asp Ser Gly Leu Tyr Ala Cys Thr Ala Ser Arg Thr
            100                 105                 110
Val Asp Ser Glu Thr Trp Tyr Phe Met Val Asn Val Thr Asp Ala Ile
        115                 120                 125
Ser Ser Gly Asp Asp Glu Asp Asp Thr Asp Gly Ala Glu Asp Phe Val
    130                 135                 140
Ser Glu Asn Ser Asn Asn Lys Arg Ala Pro Tyr Trp Thr Asn Thr Glu
145                 150                 155                 160
Lys Met Glu Lys Arg Leu His Ala Val Pro Ala Ala Asn Thr Val Lys
                165                 170                 175
Phe Arg Cys Pro Ala Gly Gly Asn Pro Met Pro Thr Met Arg Trp Leu
            180                 185                 190
Lys Asn Gly Lys Glu Phe Lys Gln Glu His Arg Ile Gly Gly Tyr Lys
        195                 200                 205
Val Arg Asn Gln His Trp Ser Leu Ile Met Glu Ser Val Val Pro Ser
    210                 215                 220
Asp Lys Gly Asn Tyr Thr Cys Val Val Glu Asn Glu Tyr Gly Ser Ile
225                 230                 235                 240
Asn His Thr Tyr His Leu Asp Val Val Glu Arg Ser Pro His Arg Pro
                245                 250                 255
Ile Leu Gln Ala Gly Leu Pro Ala Asn Ala Ser Thr Val Val Gly Gly
            260                 265                 270
Asp Val Glu Phe Val Cys Lys Val Tyr Ser Asp Ala Gln Pro His Ile
        275                 280                 285
Gln Trp Ile Lys His Val Glu Lys Asn Gly Ser Lys Tyr Gly Pro Asp
    290                 295                 300
Gly Leu Pro Tyr Leu Lys Val Leu Lys His Ser Gly Ile Asn Ser Ser
305                 310                 315                 320
Asn Ala Glu Val Leu Ala Leu Phe Asn Val Thr Glu Ala Asp Ala Gly
                325                 330                 335

Glu Tyr Ile Cys Lys Val Ser Asn Tyr Ile Gly Gln Ala Asn Gln Ser
            340                 345                 350
Ala Trp Leu Thr Val Leu Pro Lys Gln Gln Ala Pro Gly Arg Glu Lys
        355                 360                 365
Glu Ile Thr Ala Ser Pro Asp Tyr Leu Glu Ile Ala Ile Tyr Cys Ile
    370                 375                 380
Gly Val Phe Leu Ile Ala Cys Met Val Val Thr Val Ile Leu Cys Arg
385                 390                 395                 400
Met Lys Asn Thr Thr Lys Lys Pro Asp Phe Ser Ser Gln Pro Ala Val
                405                 410                 415
His Lys Leu Thr Lys Arg Ile Pro Leu Arg Arg Gln Val Thr Val Ser
            420                 425                 430
Ala Glu Ser Ser Ser Ser Met Asn Ser Asn Thr Pro Leu Val Arg Ile
        435                 440                 445
Thr Thr Arg Leu Ser Ser Thr Ala Asp Thr Pro Met Leu Ala Gly Val
    450                 455                 460
Ser Glu Tyr Glu Leu Pro Glu Asp Pro Lys Trp Glu Phe Pro Arg Asp
465                 470                 475                 480
Lys Leu Thr Leu Gly Lys Pro Leu Gly Glu Gly Cys Phe Gly Gln Val
                485                 490                 495
Val Met Ala Glu Ala Val Gly Ile Asp Lys Asp Lys Pro Lys Glu Ala
            500                 505                 510
Val Thr Val Ala Val Lys Met Leu Lys Asp Asp Ala Thr Glu Lys Asp
        515                 520                 525
Leu Ser Asp Leu Val Ser Glu Met Glu Met Met Lys Met Ile Gly Lys
    530                 535                 540
His Lys Asn Ile Ile Asn Leu Leu Gly Ala Cys Thr Gln Asp Gly Pro
545                 550                 555                 560
Leu Tyr Val Ile Val Glu Tyr Ala Ser Lys Gly Asn Leu Arg Glu Tyr
                565                 570                 575
Leu Arg Ala Arg Arg Pro Pro Gly Met Glu Tyr Ser Tyr Asp Ile Asn
            580                 585                 590

Arg Val Pro Glu Glu Gln Met Thr Phe Lys Asp Leu Val Ser Cys Thr
        595                 600                 605
Tyr Gln Leu Ala Arg Gly Met Glu Tyr Leu Ala Ser Gln Lys Cys Ile
    610                 615                 620
His Arg Asp Leu Ala Ala Arg Asn Val Leu Val Thr Glu Asn Asn Val
625                 630                 635                 640
Met Lys Ile Ala Asp Phe Gly Leu Ala Arg Asp Ile Asn Asn Ile Asp
                645                 650                 655
Tyr Tyr Lys Lys Thr Thr Asn Gly Arg Leu Pro Val Lys Trp Met Ala
            660                 665                 670
Pro Glu Ala Leu Phe Asp Arg Val Tyr Thr His Gln Ser Asp Val Trp
        675                 680                 685
Ser Phe Gly Val Leu Met Trp Glu Ile Phe Thr Leu Gly Gly Ser Pro
    690                 695                 700
Tyr Pro Gly Ile Pro Val Glu Glu Leu Phe Lys Leu Leu Lys Glu Gly
705                 710                 715                 720
His Arg Met Asp Lys Pro Ala Asn Cys Thr Asn Glu Leu Tyr Met Met
                725                 730                 735
Met Arg Asp Cys Trp His Ala Val Pro Ser Gln Arg Pro Thr Phe Lys
            740                 745                 750
Gln Leu Val Glu Asp Leu Asp Arg Ile Leu Thr Leu Thr Thr Asn Glu
        755                 760                 765
Ile

<210> 106
<211> 731
<212> PRT
<213> Homo sapiens
<220>
<221> fibroblast growth factor receptor 2 variant 8

<222>  (1)..(731)

<223>  LocusID: 2263; NM_022975

<400>  106

Met Val Ser Trp Gly Arg Phe Ile Cys Leu Val Val Val Thr Met Ala
1               5                   10                  15

Thr Leu Ser Leu Ala Arg Pro Ser Phe Ser Leu Val Glu Asp Thr Thr
            20                  25                  30

Leu Glu Pro Glu Asp Ala Ile Ser Ser Gly Asp Asp Glu Asp Asp Thr
        35                  40                  45

Asp Gly Ala Glu Asp Phe Val Ser Glu Asn Ser Asn Asn Lys Arg Ala
    50                  55                  60

Pro Tyr Trp Thr Asn Thr Glu Lys Met Glu Lys Arg Leu His Ala Val
65                  70                  75                  80

Pro Ala Ala Asn Thr Val Lys Phe Arg Cys Pro Ala Gly Gly Asn Pro
                85                  90                  95

Met Pro Thr Met Arg Trp Leu Lys Asn Gly Lys Glu Phe Lys Gln Glu
            100                 105                 110

His Arg Ile Gly Gly Tyr Lys Val Arg Asn Gln His Trp Ser Leu Ile
        115                 120                 125

Met Glu Ser Val Val Pro Ser Asp Lys Gly Asn Tyr Thr Cys Val Val
    130                 135                 140

Glu Asn Glu Tyr Gly Ser Ile Asn His Thr Tyr His Leu Asp Val Val
145                 150                 155                 160

Glu Arg Ser Pro His Arg Pro Ile Leu Gln Ala Gly Leu Pro Ala Asn
                165                 170                 175

Ala Ser Thr Val Val Gly Gly Asp Val Glu Phe Val Cys Lys Val Tyr
            180                 185                 190

Ser Asp Ala Gln Pro His Ile Gln Trp Ile Lys His Val Glu Lys Asn
        195                 200                 205

Gly Ser Lys Tyr Gly Pro Asp Gly Leu Pro Tyr Leu Lys Val Leu Lys
    210                 215                 220

His Ser Gly Ile Asn Ser Ser Asn Ala Glu Val Leu Ala Leu Phe Asn
225                 230                 235                 240
Val Thr Glu Ala Asp Ala Gly Glu Tyr Ile Cys Lys Val Ser Asn Tyr
                245                 250                 255
Ile Gly Gln Ala Asn Gln Ser Ala Trp Leu Thr Val Leu Pro Lys Gln
            260                 265                 270
Gln Ala Pro Gly Arg Glu Lys Glu Ile Thr Ala Ser Pro Asp Tyr Leu
        275                 280                 285
Glu Ile Ala Ile Tyr Cys Ile Gly Val Phe Leu Ile Ala Cys Met Val
    290                 295                 300
Val Thr Val Ile Leu Cys Arg Met Lys Asn Thr Thr Lys Lys Pro Asp
305                 310                 315                 320
Phe Ser Ser Gln Pro Ala Val His Lys Leu Thr Lys Arg Ile Pro Leu
                325                 330                 335
Arg Arg Gln Val Ser Ala Glu Ser Ser Ser Ser Met Asn Ser Asn Thr
            340                 345                 350
Pro Leu Val Arg Ile Thr Thr Arg Leu Ser Ser Thr Ala Asp Thr Pro
        355                 360                 365
Met Leu Ala Gly Val Ser Glu Tyr Glu Leu Pro Glu Asp Pro Lys Trp
    370                 375                 380
Glu Phe Pro Arg Asp Lys Leu Thr Leu Gly Lys Pro Leu Gly Glu Gly
385                 390                 395                 400
Cys Phe Gly Gln Val Val Met Ala Glu Ala Val Gly Ile Asp Lys Asp
                405                 410                 415
Lys Pro Lys Glu Ala Val Thr Val Ala Val Lys Met Leu Lys Asp Asp
            420                 425                 430
Ala Thr Glu Lys Asp Leu Ser Asp Leu Val Ser Glu Met Glu Met Met
        435                 440                 445
Lys Met Ile Gly Lys His Lys Asn Ile Ile Asn Leu Leu Gly Ala Cys
    450                 455                 460
Thr Gln Asp Gly Pro Leu Tyr Val Ile Val Glu Tyr Ala Ser Lys Gly
465                 470                 475                 480

Asn Leu Arg Glu Tyr Leu Arg Ala Arg Arg Pro Pro Gly Met Glu Tyr
                485                 490                 495
Ser Tyr Asp Ile Asn Arg Val Pro Glu Glu Gln Met Thr Phe Lys Asp
            500                 505                 510
Leu Val Ser Cys Thr Tyr Gln Leu Ala Arg Gly Met Glu Tyr Leu Ala
        515                 520                 525
Ser Gln Lys Cys Ile His Arg Asp Leu Ala Ala Arg Asn Val Leu Val
    530                 535                 540
Thr Glu Asn Asn Val Met Lys Ile Ala Asp Phe Gly Leu Ala Arg Asp
545                 550                 555                 560
Ile Asn Asn Ile Asp Tyr Tyr Lys Lys Thr Thr Asn Gly Arg Leu Pro
                565                 570                 575
Val Lys Trp Met Ala Pro Glu Ala Leu Phe Asp Arg Val Tyr Thr His
            580                 585                 590
Gln Ser Asp Val Trp Ser Phe Gly Val Leu Met Trp Glu Ile Phe Thr
        595                 600                 605
Leu Gly Gly Ser Pro Tyr Pro Gly Ile Pro Val Glu Glu Leu Phe Lys
    610                 615                 620
Leu Leu Lys Glu Gly His Arg Met Asp Lys Pro Ala Asn Cys Thr Asn
625                 630                 635                 640
Glu Leu Tyr Met Met Met Arg Asp Cys Trp His Ala Val Pro Ser Gln
                645                 650                 655
Arg Pro Thr Phe Lys Gln Leu Val Glu Asp Leu Asp Arg Ile Leu Thr
            660                 665                 670
Leu Thr Thr Asn Glu Glu Tyr Leu Asp Leu Ser Gln Pro Leu Glu Gln
        675                 680                 685
Tyr Ser Pro Ser Tyr Pro Asp Thr Arg Ser Ser Cys Ser Ser Gly Asp
    690                 695                 700
Asp Ser Val Phe Ser Pro Asp Pro Met Pro Tyr Glu Pro Cys Leu Pro
705                 710                 715                 720
Gln Tyr Pro His Ile Asn Gly Ser Val Lys Thr
                725                 730

<210>  107

<211>  366

<212>  PRT

<213>  Homo sapiens

<220>

<221>  fibroblast growth factor receptor 2 variant 9

<222>  (1)..(366)

<223>  LocusID: 2263; NM_022976

<400>  107

Met Val Ser Trp Gly Arg Phe Ile Cys Leu Val Val Val Thr Met Ala
1               5                   10                  15

Thr Leu Ser Leu Ala Arg Pro Ser Phe Ser Leu Val Glu Asp Thr Thr
            20                  25                  30

Leu Glu Pro Glu Glu Pro Pro Thr Lys Tyr Gln Ile Ser Gln Pro Glu
        35                  40                  45

Val Tyr Val Ala Ala Pro Gly Glu Ser Leu Glu Val Arg Cys Leu Leu
    50                  55                  60

Lys Asp Ala Ala Val Ile Ser Trp Thr Lys Asp Gly Val His Leu Gly
65                  70                  75                  80

Pro Asn Asn Arg Thr Val Leu Ile Gly Glu Tyr Leu Gln Ile Lys Gly
                85                  90                  95

Ala Thr Pro Arg Asp Ser Gly Leu Tyr Ala Cys Thr Ala Ser Arg Thr
            100                 105                 110

Val Asp Ser Glu Thr Trp Tyr Phe Met Val Asn Val Thr Asp Ala Ile
        115                 120                 125

Ser Ser Gly Asp Asp Glu Asp Asp Thr Asp Gly Ala Glu Asp Phe Val
    130                 135                 140

Ser Glu Asn Ser Asn Asn Lys Arg Ala Pro Tyr Trp Thr Asn Thr Glu
145                 150                 155                 160

Lys Met Glu Lys Arg Leu His Ala Val Pro Ala Ala Asn Thr Val Lys
                165                 170                 175
Phe Arg Cys Pro Ala Gly Gly Asn Pro Met Pro Thr Met Arg Trp Leu
            180                 185                 190
Lys Asn Gly Lys Glu Phe Lys Gln Glu His Arg Ile Gly Gly Tyr Lys
        195                 200                 205
Val Arg Asn Gln His Trp Ser Leu Ile Met Glu Ser Val Val Pro Ser
    210                 215                 220
Asp Lys Gly Asn Tyr Thr Cys Val Val Glu Asn Glu Tyr Gly Ser Ile
225                 230                 235                 240
Asn His Thr Tyr His Leu Asp Val Val Glu Arg Ser Pro His Arg Pro
                245                 250                 255
Ile Leu Gln Ala Gly Leu Pro Ala Asn Ala Ser Thr Val Val Gly Gly
            260                 265                 270
Asp Val Glu Phe Val Cys Lys Val Tyr Ser Asp Ala Gln Pro His Ile
        275                 280                 285
Gln Trp Ile Lys His Val Glu Lys Asn Gly Ser Lys Tyr Gly Pro Asp
    290                 295                 300
Gly Leu Pro Tyr Leu Lys Val Leu Lys His Ser Gly Ile Asn Ser Ser
305                 310                 315                 320
Asn Ala Glu Val Leu Ala Leu Phe Asn Val Thr Glu Ala Asp Ala Gly
                325                 330                 335
Glu Tyr Ile Cys Lys Val Ser Asn Tyr Ile Gly Gln Ala Asn Gln Ser
            340                 345                 350
Ala Trp Leu Thr Val Leu Pro Lys Gln Gln Gly Arg Arg Cys
        355                 360                 365

<210>  108
<211>  788
<212>  PRT
<213>  Homo sapiens

<220>

<221>  fibroblast growth factor receptor 2 variant 10

<222>  (1)..(788)

<223>  LocusID: 2263; NM_023028

<400>  108

Met Val Ser Trp Gly Arg Phe Ile Cys Leu Val Val Val Thr Met Ala
1               5                   10                  15

Thr Leu Ser Leu Ala Arg Pro Ser Phe Ser Leu Val Glu Asp Thr Thr
            20                  25                  30

Leu Glu Pro Glu Glu Pro Pro Thr Lys Tyr Gln Ile Ser Gln Pro Glu
        35                  40                  45

Val Tyr Val Ala Ala Pro Gly Glu Ser Leu Glu Val Arg Cys Leu Leu
    50                  55                  60

Lys Asp Ala Ala Val Ile Ser Trp Thr Lys Asp Gly Val His Leu Gly
65                  70                  75                  80

Pro Asn Asn Arg Thr Val Leu Ile Gly Glu Tyr Leu Gln Ile Lys Gly
                85                  90                  95

Ala Thr Pro Arg Asp Ser Gly Leu Tyr Ala Cys Thr Ala Ser Arg Thr
            100                 105                 110

Val Asp Ser Glu Thr Trp Tyr Phe Met Val Asn Val Thr Asp Ala Ile
        115                 120                 125

Ser Ser Gly Asp Asp Glu Asp Asp Thr Asp Gly Ala Glu Asp Phe Val
    130                 135                 140

Ser Glu Asn Ser Asn Asn Lys Arg Ala Pro Tyr Trp Thr Asn Thr Glu
145                 150                 155                 160

Lys Met Glu Lys Arg Leu His Ala Val Pro Ala Ala Asn Thr Val Lys
                165                 170                 175

Phe Arg Cys Pro Ala Gly Gly Asn Pro Met Pro Thr Met Arg Trp Leu
            180                 185                 190

Lys Asn Gly Lys Glu Phe Lys Gln Glu His Arg Ile Gly Gly Tyr Lys
        195                 200                 205

Val Arg Asn Gln His Trp Ser Leu Ile Met Glu Ser Val Val Pro Ser
    210                 215                 220
Asp Lys Gly Asn Tyr Thr Cys Val Val Glu Asn Glu Tyr Gly Ser Ile
225                 230                 235                 240
Asn His Thr Tyr His Leu Asp Val Val Glu Arg Ser Pro His Arg Pro
                245                 250                 255
Ile Leu Gln Ala Gly Leu Pro Ala Asn Ala Ser Thr Val Val Gly Gly
            260                 265                 270
Asp Val Glu Phe Val Cys Lys Val Tyr Ser Asp Ala Gln Pro His Ile
        275                 280                 285
Gln Trp Ile Lys His Val Glu Lys Asn Gly Ser Lys Tyr Gly Pro Asp
    290                 295                 300
Gly Leu Pro Tyr Leu Lys Val Leu Lys Val Leu Lys Ala Ala Gly Val
305                 310                 315                 320
Asn Thr Thr Asp Lys Glu Ile Glu Val Leu Tyr Ile Arg Asn Val Thr
                325                 330                 335
Phe Glu Asp Ala Gly Glu Tyr Thr Cys Leu Ala Gly Asn Ser Ile Gly
            340                 345                 350
Ile Ser Phe His Ser Ala Trp Leu Thr Val Leu Pro Ala Pro Gly Arg
        355                 360                 365
Glu Lys Glu Ile Thr Ala Ser Pro Asp Tyr Leu Glu Ile Ala Ile Tyr
    370                 375                 380
Cys Ile Gly Val Phe Leu Ile Ala Cys Met Val Val Thr Val Ile Leu
385                 390                 395                 400
Cys Arg Met Lys Asn Thr Thr Lys Lys Pro Asp Phe Ser Ser Gln Pro
                405                 410                 415
Ala Val His Lys Leu Thr Lys Arg Ile Pro Leu Arg Arg Gln Val Ser
            420                 425                 430
Ala Glu Ser Ser Ser Ser Met Asn Ser Asn Thr Pro Leu Val Arg Ile
        435                 440                 445
Thr Thr Arg Leu Ser Ser Thr Ala Asp Thr Pro Met Leu Ala Gly Val
    450                 455                 460

Ser Glu Tyr Glu Leu Pro Glu Asp Pro Lys Trp Glu Phe Pro Arg Asp
465                 470                 475                 480
Lys Leu Thr Leu Gly Lys Pro Leu Gly Glu Gly Cys Phe Gly Gln Val
                485                 490                 495
Val Met Ala Glu Ala Val Gly Ile Asp Lys Asp Lys Pro Lys Glu Ala
            500                 505                 510
Val Thr Val Ala Val Lys Met Leu Lys Asp Asp Ala Thr Glu Lys Asp
        515                 520                 525
Leu Ser Asp Leu Val Ser Glu Met Glu Met Met Lys Met Ile Gly Lys
    530                 535                 540
His Lys Asn Ile Ile Asn Leu Leu Gly Ala Cys Thr Gln Asp Gly Pro
545                 550                 555                 560
Leu Tyr Val Ile Val Glu Tyr Ala Ser Lys Gly Asn Leu Arg Glu Tyr
                565                 570                 575
Leu Arg Ala Arg Arg Pro Pro Gly Met Glu Tyr Ser Tyr Asp Ile Asn
            580                 585                 590
Arg Val Pro Glu Glu Gln Met Thr Phe Lys Asp Leu Val Ser Cys Thr
        595                 600                 605
Tyr Gln Leu Ala Arg Gly Met Glu Tyr Leu Ala Ser Gln Lys Cys Ile
    610                 615                 620
His Arg Asp Leu Ala Ala Arg Asn Val Leu Val Thr Glu Asn Asn Val
625                 630                 635                 640
Met Lys Ile Ala Asp Phe Gly Leu Ala Arg Asp Ile Asn Asn Ile Asp
                645                 650                 655
Tyr Tyr Lys Lys Thr Thr Asn Gly Arg Leu Pro Val Lys Trp Met Ala
            660                 665                 670
Pro Glu Ala Leu Phe Asp Arg Val Tyr Thr His Gln Ser Asp Val Trp
        675                 680                 685
Ser Phe Gly Val Leu Met Trp Glu Ile Phe Thr Leu Gly Gly Ser Pro
    690                 695                 700
Tyr Pro Gly Ile Pro Val Glu Glu Leu Phe Lys Leu Leu Lys Glu Gly
705                 710                 715                 720

His Arg Met Asp Lys Pro Ala Asn Cys Thr Asn Glu Leu Tyr Met Met
                725                 730                 735
Met Arg Asp Cys Trp His Ala Val Pro Ser Gln Arg Pro Thr Phe Lys
            740                 745                 750
Gln Leu Val Glu Asp Leu Asp Arg Ile Leu Thr Leu Thr Thr Asn Glu
        755                 760                 765
Glu Tyr Leu Asp Leu Ser Gln Pro Leu Glu Pro Tyr Ser Pro Cys Tyr
    770                 775                 780
Pro Asp Pro Arg
785

<210> 109
<211> 732
<212> PRT
<213> Homo sapiens
<220>
<221> fibroblast growth factor receptor 2 variant 11
<222> (1)..(732)
<223> LocusID: 2263; NM_023029
<400> 109

Met Val Ser Trp Gly Arg Phe Ile Cys Leu Val Val Val Thr Met Ala
1               5                   10                  15
Thr Leu Ser Leu Ala Arg Pro Ser Phe Ser Leu Val Glu Asp Thr Thr
            20                  25                  30
Leu Glu Pro Glu Asp Ala Ile Ser Ser Gly Asp Asp Glu Asp Asp Thr
        35                  40                  45
Asp Gly Ala Glu Asp Phe Val Ser Glu Asn Ser Asn Asn Lys Arg Ala
    50                  55                  60
Pro Tyr Trp Thr Asn Thr Glu Lys Met Glu Lys Arg Leu His Ala Val
65                  70                  75                  80

Pro Ala Ala Asn Thr Val Lys Phe Arg Cys Pro Ala Gly Gly Asn Pro
                85                  90                  95
Met Pro Thr Met Arg Trp Leu Lys Asn Gly Lys Glu Phe Lys Gln Glu
            100                 105                 110
His Arg Ile Gly Gly Tyr Lys Val Arg Asn Gln His Trp Ser Leu Ile
        115                 120                 125
Met Glu Ser Val Val Pro Ser Asp Lys Gly Asn Tyr Thr Cys Val Val
    130                 135                 140
Glu Asn Glu Tyr Gly Ser Ile Asn His Thr Tyr His Leu Asp Val Val
145                 150                 155                 160
Glu Arg Ser Pro His Arg Pro Ile Leu Gln Ala Gly Leu Pro Ala Asn
                165                 170                 175
Ala Ser Thr Val Val Gly Gly Asp Val Glu Phe Val Cys Lys Val Tyr
            180                 185                 190
Ser Asp Ala Gln Pro His Ile Gln Trp Ile Lys His Val Glu Lys Asn
        195                 200                 205
Gly Ser Lys Tyr Gly Pro Asp Gly Leu Pro Tyr Leu Lys Val Leu Lys
    210                 215                 220
Ala Ala Gly Val Asn Thr Thr Asp Lys Glu Ile Glu Val Leu Tyr Ile
225                 230                 235                 240
Arg Asn Val Thr Phe Glu Asp Ala Gly Glu Tyr Thr Cys Leu Ala Gly
                245                 250                 255
Asn Ser Ile Gly Ile Ser Phe His Ser Ala Trp Leu Thr Val Leu Pro
            260                 265                 270
Ala Pro Gly Arg Glu Lys Glu Ile Thr Ala Ser Pro Asp Tyr Leu Glu
        275                 280                 285
Ile Ala Ile Tyr Cys Ile Gly Val Phe Leu Ile Ala Cys Met Val Val
    290                 295                 300
Thr Val Ile Leu Cys Arg Met Lys Asn Thr Thr Lys Lys Pro Asp Phe
305                 310                 315                 320
Ser Ser Gln Pro Ala Val His Lys Leu Thr Lys Arg Ile Pro Leu Arg
                325                 330                 335

Arg Gln Val Thr Val Ser Ala Glu Ser Ser Ser Ser Met Asn Ser Asn
            340                 345                 350
Thr Pro Leu Val Arg Ile Thr Thr Arg Leu Ser Ser Thr Ala Asp Thr
        355                 360                 365
Pro Met Leu Ala Gly Val Ser Glu Tyr Glu Leu Pro Glu Asp Pro Lys
    370                 375                 380
Trp Glu Phe Pro Arg Asp Lys Leu Thr Leu Gly Lys Pro Leu Gly Glu
385                 390                 395                 400
Gly Cys Phe Gly Gln Val Val Met Ala Glu Ala Val Gly Ile Asp Lys
                405                 410                 415
Asp Lys Pro Lys Glu Ala Val Thr Val Ala Val Lys Met Leu Lys Asp
            420                 425                 430
Asp Ala Thr Glu Lys Asp Leu Ser Asp Leu Val Ser Glu Met Glu Met
        435                 440                 445
Met Lys Met Ile Gly Lys His Lys Asn Ile Ile Asn Leu Leu Gly Ala
    450                 455                 460
Cys Thr Gln Asp Gly Pro Leu Tyr Val Ile Val Glu Tyr Ala Ser Lys
465                 470                 475                 480
Gly Asn Leu Arg Glu Tyr Leu Arg Ala Arg Arg Pro Pro Gly Met Glu
                485                 490                 495
Tyr Ser Tyr Asp Ile Asn Arg Val Pro Glu Glu Gln Met Thr Phe Lys
            500                 505                 510
Asp Leu Val Ser Cys Thr Tyr Gln Leu Ala Arg Gly Met Glu Tyr Leu
        515                 520                 525
Ala Ser Gln Lys Cys Ile His Arg Asp Leu Ala Ala Arg Asn Val Leu
    530                 535                 540
Val Thr Glu Asn Asn Val Met Lys Ile Ala Asp Phe Gly Leu Ala Arg
545                 550                 555                 560
Asp Ile Asn Asn Ile Asp Tyr Tyr Lys Lys Thr Thr Asn Gly Arg Leu
                565                 570                 575
Pro Val Lys Trp Met Ala Pro Glu Ala Leu Phe Asp Arg Val Tyr Thr
            580                 585                 590

His Gln Ser Asp Val Trp Ser Phe Gly Val Leu Met Trp Glu Ile Phe
        595                 600                 605
Thr Leu Gly Gly Ser Pro Tyr Pro Gly Ile Pro Val Glu Glu Leu Phe
    610                 615                 620
Lys Leu Leu Lys Glu Gly His Arg Met Asp Lys Pro Ala Asn Cys Thr
625                 630                 635                 640
Asn Glu Leu Tyr Met Met Met Arg Asp Cys Trp His Ala Val Pro Ser
                645                 650                 655
Gln Arg Pro Thr Phe Lys Gln Leu Val Glu Asp Leu Asp Arg Ile Leu
            660                 665                 670
Thr Leu Thr Thr Asn Glu Glu Tyr Leu Asp Leu Ser Gln Pro Leu Glu
        675                 680                 685
Gln Tyr Ser Pro Ser Tyr Pro Asp Thr Arg Ser Ser Cys Ser Ser Gly
    690                 695                 700
Asp Asp Ser Val Phe Ser Pro Asp Pro Met Pro Tyr Glu Pro Cys Leu
705                 710                 715                 720
Pro Gln Tyr Pro His Ile Asn Gly Ser Val Lys Thr
                725                 730

<210> 110
<211> 707
<212> PRT
<213> Homo sapiens
<220>
<221> fibroblast growth factor receptor 2 variant 12
<222> (1)..(707)
<223> LocusID: 2263; NM_023030
<400> 110

Met Val Ser Trp Gly Arg Phe Ile Cys Leu Val Val Val Thr Met Ala
1               5                   10                  15

Thr Leu Ser Leu Ala Arg Pro Ser Phe Ser Leu Val Glu Asp Thr Thr
            20                  25                  30
Leu Glu Pro Glu Gly Ala Pro Tyr Trp Thr Asn Thr Glu Lys Met Glu
        35                  40                  45
Lys Arg Leu His Ala Val Pro Ala Ala Asn Thr Val Lys Phe Arg Cys
    50                  55                  60
Pro Ala Gly Gly Asn Pro Met Pro Thr Met Arg Trp Leu Lys Asn Gly
65                  70                  75                  80
Lys Glu Phe Lys Gln Glu His Arg Ile Gly Gly Tyr Lys Val Arg Asn
                85                  90                  95
Gln His Trp Ser Leu Ile Met Glu Ser Val Val Pro Ser Asp Lys Gly
            100                 105                 110
Asn Tyr Thr Cys Val Val Glu Asn Glu Tyr Gly Ser Ile Asn His Thr
        115                 120                 125
Tyr His Leu Asp Val Val Glu Arg Ser Pro His Arg Pro Ile Leu Gln
    130                 135                 140
Ala Gly Leu Pro Ala Asn Ala Ser Thr Val Val Gly Gly Asp Val Glu
145                 150                 155                 160
Phe Val Cys Lys Val Tyr Ser Asp Ala Gln Pro His Ile Gln Trp Ile
                165                 170                 175
Lys His Val Glu Lys Asn Gly Ser Lys Tyr Gly Pro Asp Gly Leu Pro
            180                 185                 190
Tyr Leu Lys Val Leu Lys His Ser Gly Ile Asn Ser Ser Asn Ala Glu
        195                 200                 205
Val Leu Ala Leu Phe Asn Val Thr Glu Ala Asp Ala Gly Glu Tyr Ile
    210                 215                 220
Cys Lys Val Ser Asn Tyr Ile Gly Gln Ala Asn Gln Ser Ala Trp Leu
225                 230                 235                 240
Thr Val Leu Pro Lys Gln Gln Ala Pro Gly Arg Glu Lys Glu Ile Thr
                245                 250                 255
Ala Ser Pro Asp Tyr Leu Glu Ile Ala Ile Tyr Cys Ile Gly Val Phe
            260                 265                 270

Leu Ile Ala Cys Met Val Val Thr Val Ile Leu Cys Arg Met Lys Asn
        275                 280                 285
Thr Thr Lys Lys Pro Asp Phe Ser Ser Gln Pro Ala Val His Lys Leu
    290                 295                 300
Thr Lys Arg Ile Pro Leu Arg Arg Gln Val Thr Val Ser Ala Glu Ser
305                 310                 315                 320
Ser Ser Ser Met Asn Ser Asn Thr Pro Leu Val Arg Ile Thr Thr Arg
                325                 330                 335
Leu Ser Ser Thr Ala Asp Thr Pro Met Leu Ala Gly Val Ser Glu Tyr
            340                 345                 350
Glu Leu Pro Glu Asp Pro Lys Trp Glu Phe Pro Arg Asp Lys Leu Thr
        355                 360                 365
Leu Gly Lys Pro Leu Gly Glu Gly Cys Phe Gly Gln Val Val Met Ala
    370                 375                 380
Glu Ala Val Gly Ile Asp Lys Asp Lys Pro Lys Glu Ala Val Thr Val
385                 390                 395                 400
Ala Val Lys Met Leu Lys Asp Asp Ala Thr Glu Lys Asp Leu Ser Asp
                405                 410                 415
Leu Val Ser Glu Met Glu Met Met Lys Met Ile Gly Lys His Lys Asn
            420                 425                 430
Ile Ile Asn Leu Leu Gly Ala Cys Thr Gln Asp Gly Pro Leu Tyr Val
        435                 440                 445
Ile Val Glu Tyr Ala Ser Lys Gly Asn Leu Arg Glu Tyr Leu Arg Ala
    450                 455                 460
Arg Arg Pro Pro Gly Met Glu Tyr Ser Tyr Asp Ile Asn Arg Val Pro
465                 470                 475                 480
Glu Glu Gln Met Thr Phe Lys Asp Leu Val Ser Cys Thr Tyr Gln Leu
                485                 490                 495
Ala Arg Arg Met Glu Tyr Leu Ala Ser Gln Lys Cys Ile His Arg Asp
            500                 505                 510
Leu Ala Ala Arg Asn Val Leu Val Thr Glu Asn Asn Val Met Lys Ile
        515                 520                 525

Ala Asp Phe Gly Leu Ala Arg Asp Ile Asn Asn Ile Asp Tyr Tyr Lys
    530                 535                 540
Lys Thr Thr Asn Gly Arg Leu Pro Val Lys Trp Met Ala Pro Glu Ala
545                 550                 555                 560
Leu Phe Asp Arg Val Tyr Thr His Gln Ser Asp Val Trp Ser Phe Gly
                565                 570                 575
Val Leu Met Trp Glu Ile Phe Thr Leu Gly Gly Ser Pro Tyr Pro Gly
            580                 585                 590
Ile Pro Val Glu Glu Leu Phe Lys Leu Leu Lys Glu Gly His Arg Met
        595                 600                 605
Asp Lys Pro Ala Asn Cys Thr Asn Glu Leu Tyr Met Met Met Arg Asp
    610                 615                 620
Cys Trp His Ala Val Pro Ser Gln Arg Pro Thr Phe Lys Gln Leu Val
625                 630                 635                 640
Glu Asp Leu Asp Arg Ile Leu Thr Leu Thr Thr Asn Glu Glu Tyr Leu
                645                 650                 655
Asp Leu Ser Gln Pro Leu Glu Gln Tyr Ser Pro Ser Tyr Pro Asp Thr
            660                 665                 670
Arg Ser Ser Cys Ser Ser Gly Asp Asp Ser Val Phe Ser Pro Asp Pro
        675                 680                 685
Met Pro Tyr Glu Pro Cys Leu Pro Gln Tyr Pro His Ile Asn Gly Ser
    690                 695                 700
Val Lys Thr
705

<210> 111
<211> 705
<212> PRT
<213> Homo sapiens
<220>
<221> fibroblast growth factor receptor 2 variant 13

<222>  (1)..(705)

<223>  LocusID: 2263; NM_023031

<400>  111

Met Val Ser Trp Gly Arg Phe Ile Cys Leu Val Val Val Thr Met Ala
1               5                   10                  15

Thr Leu Ser Leu Ala Arg Pro Ser Phe Ser Leu Val Glu Asp Thr Thr
            20                  25                  30

Leu Glu Pro Glu Gly Ala Pro Tyr Trp Thr Asn Thr Glu Lys Met Glu
        35                  40                  45

Lys Arg Leu His Ala Val Pro Ala Ala Asn Thr Val Lys Phe Arg Cys
    50                  55                  60

Pro Ala Gly Gly Asn Pro Met Pro Thr Met Arg Trp Leu Lys Asn Gly
65                  70                  75                  80

Lys Glu Phe Lys Gln Glu His Arg Ile Gly Gly Tyr Lys Val Arg Asn
                85                  90                  95

Gln His Trp Ser Leu Ile Met Glu Ser Val Val Pro Ser Asp Lys Gly
            100                 105                 110

Asn Tyr Thr Cys Val Val Glu Asn Glu Tyr Gly Ser Ile Asn His Thr
        115                 120                 125

Tyr His Leu Asp Val Val Glu Arg Ser Pro His Arg Pro Ile Leu Gln
    130                 135                 140

Ala Gly Leu Pro Ala Asn Ala Ser Thr Val Val Gly Gly Asp Val Glu
145                 150                 155                 160

Phe Val Cys Lys Val Tyr Ser Asp Ala Gln Pro His Ile Gln Trp Ile
                165                 170                 175

Lys His Val Glu Lys Asn Gly Ser Lys Tyr Gly Pro Asp Gly Leu Pro
            180                 185                 190

Tyr Leu Lys Val Leu Lys His Ser Gly Ile Asn Ser Ser Asn Ala Glu
        195                 200                 205

Val Leu Ala Leu Phe Asn Val Thr Glu Ala Asp Ala Gly Glu Tyr Ile
    210                 215                 220

Cys Lys Val Ser Asn Tyr Ile Gly Gln Ala Asn Gln Ser Ala Trp Leu
225                 230                 235                 240
Thr Val Leu Pro Lys Gln Gln Ala Pro Gly Arg Glu Lys Glu Ile Thr
                245                 250                 255
Ala Ser Pro Asp Tyr Leu Glu Ile Ala Ile Tyr Cys Ile Gly Val Phe
            260                 265                 270
Leu Ile Ala Cys Met Val Val Thr Val Ile Leu Cys Arg Met Lys Asn
        275                 280                 285
Thr Thr Lys Lys Pro Asp Phe Ser Ser Gln Pro Ala Val His Lys Leu
    290                 295                 300
Thr Lys Arg Ile Pro Leu Arg Arg Gln Val Ser Ala Glu Ser Ser Ser
305                 310                 315                 320
Ser Met Asn Ser Asn Thr Pro Leu Val Arg Ile Thr Thr Arg Leu Ser
                325                 330                 335
Ser Thr Ala Asp Thr Pro Met Leu Ala Gly Val Ser Glu Tyr Glu Leu
            340                 345                 350
Pro Glu Asp Pro Lys Trp Glu Phe Pro Arg Asp Lys Leu Thr Leu Gly
        355                 360                 365
Lys Pro Leu Gly Glu Gly Cys Phe Gly Gln Val Val Met Ala Glu Ala
    370                 375                 380
Val Gly Ile Asp Lys Asp Lys Pro Lys Glu Ala Val Thr Val Ala Val
385                 390                 395                 400
Lys Met Leu Lys Asp Asp Ala Thr Glu Lys Asp Leu Ser Asp Leu Val
                405                 410                 415
Ser Glu Met Glu Met Met Lys Met Ile Gly Lys His Lys Asn Ile Ile
            420                 425                 430
Asn Leu Leu Gly Ala Cys Thr Gln Asp Gly Pro Leu Tyr Val Ile Val
        435                 440                 445
Glu Tyr Ala Ser Lys Gly Asn Leu Arg Glu Tyr Leu Arg Ala Arg Arg
    450                 455                 460
Pro Pro Gly Met Glu Tyr Ser Tyr Asp Ile Asn Arg Val Pro Glu Glu
465                 470                 475                 480

Gln Met Thr Phe Lys Asp Leu Val Ser Cys Thr Tyr Gln Leu Ala Arg
                485                 490                 495
Arg Met Glu Tyr Leu Ala Ser Gln Lys Cys Ile His Arg Asp Leu Ala
            500                 505                 510
Ala Arg Asn Val Leu Val Thr Glu Asn Asn Val Met Lys Ile Ala Asp
        515                 520                 525
Phe Gly Leu Ala Arg Asp Ile Asn Asn Ile Asp Tyr Tyr Lys Lys Thr
    530                 535                 540
Thr Asn Gly Arg Leu Pro Val Lys Trp Met Ala Pro Glu Ala Leu Phe
545                 550                 555                 560
Asp Arg Val Tyr Thr His Gln Ser Asp Val Trp Ser Phe Gly Val Leu
                565                 570                 575
Met Trp Glu Ile Phe Thr Leu Gly Gly Ser Pro Tyr Pro Gly Ile Pro
            580                 585                 590
Val Glu Glu Leu Phe Lys Leu Leu Lys Glu Gly His Arg Met Asp Lys
        595                 600                 605
Pro Ala Asn Cys Thr Asn Glu Leu Tyr Met Met Met Arg Asp Cys Trp
    610                 615                 620
His Ala Val Pro Ser Gln Arg Pro Thr Phe Lys Gln Leu Val Glu Asp
625                 630                 635                 640
Leu Asp Arg Ile Leu Thr Leu Thr Thr Asn Glu Glu Tyr Leu Asp Leu
                645                 650                 655
Ser Gln Pro Leu Glu Gln Tyr Ser Pro Ser Tyr Pro Asp Thr Arg Ser
            660                 665                 670
Ser Cys Ser Ser Gly Asp Asp Ser Val Phe Ser Pro Asp Pro Met Pro
        675                 680                 685
Tyr Glu Pro Cys Leu Pro Gln Tyr Pro His Ile Asn Gly Ser Val Lys
    690                 695                 700
Thr
705

<210> 112

<211> 461

<212> PRT

<213> Homo sapiens

<220>

<221> tumor necrosis factor receptor superfamily, member 1B

<222> (1)..(461)

<223> LocusID: 7133; NM_001066

<400> 112

Met Ala Pro Val Ala Val Trp Ala Ala Leu Ala Val Gly Leu Glu Leu
1               5                   10                  15

Trp Ala Ala Ala His Ala Leu Pro Ala Gln Val Ala Phe Thr Pro Tyr
            20                  25                  30

Ala Pro Glu Pro Gly Ser Thr Cys Arg Leu Arg Glu Tyr Tyr Asp Gln
        35                  40                  45

Thr Ala Gln Met Cys Cys Ser Lys Cys Ser Pro Gly Gln His Ala Lys
    50                  55                  60

Val Phe Cys Thr Lys Thr Ser Asp Thr Val Cys Asp Ser Cys Glu Asp
65                  70                  75                  80

Ser Thr Tyr Thr Gln Leu Trp Asn Trp Val Pro Glu Cys Leu Ser Cys
                85                  90                  95

Gly Ser Arg Cys Ser Ser Asp Gln Val Glu Thr Gln Ala Cys Thr Arg
            100                 105                 110

Glu Gln Asn Arg Ile Cys Thr Cys Arg Pro Gly Trp Tyr Cys Ala Leu
        115                 120                 125

Ser Lys Gln Glu Gly Cys Arg Leu Cys Ala Pro Leu Arg Lys Cys Arg
    130                 135                 140

Pro Gly Phe Gly Val Ala Arg Pro Gly Thr Glu Thr Ser Asp Val Val
145                 150                 155                 160

Cys Lys Pro Cys Ala Pro Gly Thr Phe Ser Asn Thr Thr Ser Ser Thr
                165                 170                 175

Asp Ile Cys Arg Pro His Gln Ile Cys Asn Val Val Ala Ile Pro Gly
            180                 185                 190
Asn Ala Ser Met Asp Ala Val Cys Thr Ser Thr Ser Pro Thr Arg Ser
        195                 200                 205
Met Ala Pro Gly Ala Val His Leu Pro Gln Pro Val Ser Thr Arg Ser
    210                 215                 220
Gln His Thr Gln Pro Thr Pro Glu Pro Ser Thr Ala Pro Ser Thr Ser
225                 230                 235                 240
Phe Leu Leu Pro Met Gly Pro Ser Pro Pro Ala Glu Gly Ser Thr Gly
                245                 250                 255
Asp Phe Ala Leu Pro Val Gly Leu Ile Val Gly Val Thr Ala Leu Gly
            260                 265                 270
Leu Leu Ile Ile Gly Val Val Asn Cys Val Ile Met Thr Gln Val Lys
        275                 280                 285
Lys Lys Pro Leu Cys Leu Gln Arg Glu Ala Lys Val Pro His Leu Pro
    290                 295                 300
Ala Asp Lys Ala Arg Gly Thr Gln Gly Pro Glu Gln Gln His Leu Leu
305                 310                 315                 320
Ile Thr Ala Pro Ser Ser Ser Ser Ser Ser Leu Glu Ser Ser Ala Ser
                325                 330                 335
Ala Leu Asp Arg Arg Ala Pro Thr Arg Asn Gln Pro Gln Ala Pro Gly
            340                 345                 350
Val Glu Ala Ser Gly Ala Gly Glu Ala Arg Ala Ser Thr Gly Ser Ser
        355                 360                 365
Asp Ser Ser Pro Gly Gly His Gly Thr Gln Val Asn Val Thr Cys Ile
    370                 375                 380
Val Asn Val Cys Ser Ser Ser Asp His Ser Ser Gln Cys Ser Ser Gln
385                 390                 395                 400
Ala Ser Ser Thr Met Gly Asp Thr Asp Ser Ser Pro Ser Glu Ser Pro
                405                 410                 415
Lys Asp Glu Gln Val Pro Phe Ser Lys Glu Glu Cys Ala Phe Arg Ser
            420                 425                 430

Gln Leu Glu Thr Pro Glu Thr Leu Leu Gly Ser Thr Glu Glu Lys Pro
        435                 440                 445
Leu Pro Leu Gly Val Pro Asp Ala Gly Met Lys Pro Ser
    450                 455                 460

<210> 113
<211> 281
<212> PRT
<213> Homo sapiens
<220>
<221> tumor necrosis factor (ligand) superfamily, member 10
<222> (1)..(281)
<223> LocusID: 8743; NM_003810
<400> 113

<210>  114
<211>  498
<212>  PRT
<213>  Homo sapiens
<220>
<221>  angiopoietin 1 variant 1
<222>  (1)..(498)
<223>  LocusID: 284; NM_001146
<400>  114

Met Thr Val Phe Leu Ser Phe Ala Phe Leu Ala Ala Ile Leu Thr His
1               5                   10                  15
Ile Gly Cys Ser Asn Gln Arg Arg Ser Pro Glu Asn Ser Gly Arg Arg
            20                  25                  30
Tyr Asn Arg Ile Gln His Gly Gln Cys Ala Tyr Thr Phe Ile Leu Pro
        35                  40                  45
Glu His Asp Gly Asn Cys Arg Glu Ser Thr Thr Asp Gln Tyr Asn Thr
    50                  55                  60
Asn Ala Leu Gln Arg Asp Ala Pro His Val Glu Pro Asp Phe Ser Ser
65                  70                  75                  80
Gln Lys Leu Gln His Leu Glu His Val Met Glu Asn Tyr Thr Gln Trp
                85                  90                  95
Leu Gln Lys Leu Glu Asn Tyr Ile Val Glu Asn Met Lys Ser Glu Met
            100                 105                 110
Ala Gln Ile Gln Gln Asn Ala Val Gln Asn His Thr Ala Thr Met Leu
        115                 120                 125
Glu Ile Gly Thr Ser Leu Leu Ser Gln Thr Ala Glu Gln Thr Arg Lys
    130                 135                 140
Leu Thr Asp Val Glu Thr Gln Val Leu Asn Gln Thr Ser Arg Leu Glu
145                 150                 155                 160
Ile Gln Leu Leu Glu Asn Ser Leu Ser Thr Tyr Lys Leu Glu Lys Gln
                165                 170                 175
Leu Leu Gln Gln Thr Asn Glu Ile Leu Lys Ile His Glu Lys Asn Ser
            180                 185                 190
Leu Leu Glu His Lys Ile Leu Glu Met Glu Gly Lys His Lys Glu Glu
        195                 200                 205
Leu Asp Thr Leu Lys Glu Glu Lys Glu Asn Leu Gln Gly Leu Val Thr
    210                 215                 220
Arg Gln Thr Tyr Ile Ile Gln Glu Leu Glu Lys Gln Leu Asn Arg Ala
225                 230                 235                 240
Thr Thr Asn Asn Ser Val Leu Gln Lys Gln Gln Leu Glu Leu Met Asp
                245                 250                 255

Thr Val His Asn Leu Val Asn Leu Cys Thr Lys Glu Gly Val Leu Leu
            260                 265                 270
Lys Gly Gly Lys Arg Glu Glu Glu Lys Pro Phe Arg Asp Cys Ala Asp
        275                 280                 285
Val Tyr Gln Ala Gly Phe Asn Lys Ser Gly Ile Tyr Thr Ile Tyr Ile
    290                 295                 300
Asn Asn Met Pro Glu Pro Lys Lys Val Phe Cys Asn Met Asp Val Asn
305                 310                 315                 320
Gly Gly Gly Trp Thr Val Ile Gln His Arg Glu Asp Gly Ser Leu Asp
                325                 330                 335
Phe Gln Arg Gly Trp Lys Glu Tyr Lys Met Gly Phe Gly Asn Pro Ser
            340                 345                 350
Gly Glu Tyr Trp Leu Gly Asn Glu Phe Ile Phe Ala Ile Thr Ser Gln
        355                 360                 365
Arg Gln Tyr Met Leu Arg Ile Glu Leu Met Asp Trp Glu Gly Asn Arg
    370                 375                 380
Ala Tyr Ser Gln Tyr Asp Arg Phe His Ile Gly Asn Glu Lys Gln Asn
385                 390                 395                 400
Tyr Arg Leu Tyr Leu Lys Gly His Thr Gly Thr Ala Gly Lys Gln Ser
                405                 410                 415
Ser Leu Ile Leu His Gly Ala Asp Phe Ser Thr Lys Asp Ala Asp Asn
            420                 425                 430
Asp Asn Cys Met Cys Lys Cys Ala Leu Met Leu Thr Gly Gly Trp Trp
        435                 440                 445
Phe Asp Ala Cys Gly Pro Ser Asn Leu Asn Gly Met Phe Tyr Thr Ala
    450                 455                 460
Gly Gln Asn His Gly Lys Leu Asn Gly Ile Lys Trp His Tyr Phe Lys
465                 470                 475                 480
Gly Pro Ser Tyr Ser Leu Arg Ser Thr Thr Met Met Ile Arg Pro Leu
                485                 490                 495
Asp Phe

<210> 115

<211> 402

<212> PRT

<213> Homo sapiens

<220>

<221> angiopoietin 1 variant 2

<222> (1)..(402)

<223> LocusID: 284; NM_139290

<400> 115

Met Thr Val Phe Leu Ser Phe Ala Phe Leu Ala Ala Ile Leu Thr His
1               5                   10                  15

Ile Gly Cys Ser Asn Gln Arg Arg Ser Pro Glu Asn Ser Gly Arg Arg
            20                  25                  30

Tyr Asn Arg Ile Gln His Gly Gln Cys Ala Tyr Thr Phe Ile Leu Pro
        35                  40                  45

Glu His Asp Gly Asn Cys Arg Glu Ser Thr Thr Asp Gln Tyr Asn Thr
    50                  55                  60

Asn Ala Leu Gln Arg Asp Ala Pro His Val Glu Pro Asp Phe Ser Ser
65                  70                  75                  80

Gln Lys Leu Gln His Leu Glu His Val Met Glu Asn Tyr Thr Gln Trp
                85                  90                  95

Leu Gln Lys Leu Glu Asn Tyr Ile Val Glu Asn Met Lys Ser Glu Met
            100                 105                 110

Ala Gln Ile Gln Gln Asn Ala Val Gln Asn His Thr Ala Thr Met Leu
        115                 120                 125

Glu Ile Gly Thr Ser Leu Leu Ser Gln Thr Ala Glu Gln Thr Arg Lys
    130                 135                 140

Leu Thr Asp Val Glu Thr Gln Val Leu Asn Gln Thr Ser Arg Leu Glu
145                 150                 155                 160

Ile Gln Leu Leu Glu Asn Ser Leu Ser Thr Tyr Lys Leu Glu Lys Gln
                165                 170                 175
Leu Leu Gln Gln Thr Asn Glu Ile Leu Lys Ile His Glu Lys Asn Ser
            180                 185                 190
Leu Leu Glu His Lys Ile Leu Glu Met Glu Gly Lys His Lys Glu Glu
        195                 200                 205
Leu Asp Thr Leu Lys Glu Glu Lys Glu Asn Leu Gln Gly Leu Val Thr
    210                 215                 220
Arg Gln Thr Tyr Ile Ile Gln Glu Leu Glu Lys Gln Leu Asn Arg Ala
225                 230                 235                 240
Thr Thr Asn Asn Ser Val Leu Gln Lys Gln Gln Leu Glu Leu Met Asp
                245                 250                 255
Thr Val His Asn Leu Val Asn Leu Cys Thr Lys Glu Gly Val Leu Leu
            260                 265                 270
Lys Gly Gly Lys Arg Glu Glu Glu Lys Pro Phe Arg Asp Cys Ala Asp
        275                 280                 285
Val Tyr Gln Ala Gly Phe Asn Lys Ser Gly Ile Tyr Thr Ile Tyr Ile
    290                 295                 300
Asn Asn Met Pro Glu Pro Lys Lys Val Phe Cys Asn Met Asp Val Asn
305                 310                 315                 320
Gly Gly Gly Trp Thr Val Ile Gln His Arg Glu Asp Gly Ser Leu Asp
                325                 330                 335
Phe Gln Arg Gly Trp Lys Glu Tyr Lys Met Gly Phe Gly Asn Pro Ser
            340                 345                 350
Gly Glu Tyr Trp Leu Gly Asn Glu Phe Ile Phe Ala Ile Thr Ser Gln
        355                 360                 365
Arg Gln Tyr Met Leu Arg Ile Glu Leu Met Asp Trp Glu Gly Asn Arg
    370                 375                 380
Ala Tyr Ser Gln Tyr Asp Arg Phe His Ile Gly Asn Glu Lys Gln Asn
385                 390                 395                 400
Tyr Arg

<210>  116

<211>  462

<212>  PRT

<213>  Homo sapiens

<220>

<221>  retinoid X receptor alpha

<222>  (1)..(462)

<223>  LocusID: 6256; NM_002957

<400>  116

Met Asp Thr Lys His Phe Leu Pro Leu Asp Phe Ser Thr Gln Val Asn
1               5                   10                  15

Ser Ser Leu Thr Ser Pro Thr Gly Arg Gly Ser Met Ala Ala Pro Ser
            20                  25                  30

Leu His Pro Ser Leu Gly Pro Gly Ile Gly Ser Pro Gly Gln Leu His
        35                  40                  45

Ser Pro Ile Ser Thr Leu Ser Ser Pro Ile Asn Gly Met Gly Pro Pro
    50                  55                  60

Phe Ser Val Ile Ser Ser Pro Met Gly Pro His Ser Met Ser Val Pro
65                  70                  75                  80

Thr Thr Pro Thr Leu Gly Phe Ser Thr Gly Ser Pro Gln Leu Ser Ser
                85                  90                  95

Pro Met Asn Pro Val Ser Ser Ser Glu Asp Ile Lys Pro Pro Leu Gly
            100                 105                 110

Leu Asn Gly Val Leu Lys Val Pro Ala His Pro Ser Gly Asn Met Ala
        115                 120                 125

Ser Phe Thr Lys His Ile Cys Ala Ile Cys Gly Asp Arg Ser Ser Gly
    130                 135                 140

Lys His Tyr Gly Val Tyr Ser Cys Glu Gly Cys Lys Gly Phe Phe Lys
145                 150                 155                 160

Arg Thr Val Arg Lys Asp Leu Thr Tyr Thr Cys Arg Asp Asn Lys Asp
                165                 170                 175
Cys Leu Ile Asp Lys Arg Gln Arg Asn Arg Cys Gln Tyr Cys Arg Tyr
            180                 185                 190
Gln Lys Cys Leu Ala Met Gly Met Lys Arg Glu Ala Val Gln Glu Glu
        195                 200                 205
Arg Gln Arg Gly Lys Asp Arg Asn Glu Asn Glu Val Glu Ser Thr Ser
    210                 215                 220
Ser Ala Asn Glu Asp Met Pro Val Glu Arg Ile Leu Glu Ala Glu Leu
225                 230                 235                 240
Ala Val Glu Pro Lys Thr Glu Thr Tyr Val Glu Ala Asn Met Gly Leu
                245                 250                 255
Asn Pro Ser Ser Pro Asn Asp Pro Val Thr Asn Ile Cys Gln Ala Ala
            260                 265                 270
Asp Lys Gln Leu Phe Thr Leu Val Glu Trp Ala Lys Arg Ile Pro His
        275                 280                 285
Phe Ser Glu Leu Pro Leu Asp Asp Gln Val Ile Leu Leu Arg Ala Gly
    290                 295                 300
Trp Asn Glu Leu Leu Ile Ala Ser Phe Ser His Arg Ser Ile Ala Val
305                 310                 315                 320
Lys Asp Gly Ile Leu Leu Ala Thr Gly Leu His Val His Arg Asn Ser
                325                 330                 335
Ala His Ser Ala Gly Val Gly Ala Ile Phe Asp Arg Val Leu Thr Glu
            340                 345                 350
Leu Val Ser Lys Met Arg Asp Met Gln Met Asp Lys Thr Glu Leu Gly
        355                 360                 365
Cys Leu Arg Ala Ile Val Leu Phe Asn Pro Asp Ser Lys Gly Leu Ser
    370                 375                 380
Asn Pro Ala Glu Val Glu Ala Leu Arg Glu Lys Val Tyr Ala Ser Leu
385                 390                 395                 400
Glu Ala Tyr Cys Lys His Lys Tyr Pro Glu Gln Pro Gly Arg Phe Ala
                405                 410                 415

Lys Leu Leu Leu Arg Leu Pro Ala Leu Arg Ser Ile Gly Leu Lys Cys
            420                 425                 430
Leu Glu His Leu Phe Phe Phe Lys Leu Ile Gly Asp Thr Pro Ile Asp
        435                 440                 445
Thr Phe Leu Met Glu Met Leu Glu Ala Pro His Gln Met Thr
    450                 455                 460

<210> 117
<211> 323
<212> PRT
<213> Homo sapiens
<220>
<221> degenerative spermatocyte homolog, lipid desaturase variant 1
<222> (1)..(323)
<223> LocusID: 8560; NM_003676
<400> 117

<210> 118
<211> 323

<212>  PRT

<213>  Homo sapiens

<220>

<221>  degenerative spermatocyte homolog, lipid desaturase variant 2

<222>  (1)..(323)

<223>  LocusID: 8560; NM_144780

<400>  118

<210> 119
<211> 511
<212> PRT
<213> Homo sapiens
<220>
<221> solute carrier family 7 member 7
<222> (1)..(511)
<223> LocusID: 9056; NM_003982
<400> 119

Met Val Asp Ser Thr Glu Tyr Glu Val Ala Ser Gln Pro Glu Val Glu
1               5                   10                  15

Thr Ser Pro Leu Gly Asp Gly Ala Ser Pro Gly Pro Glu Gln Val Lys
            20                  25                  30
Leu Lys Lys Glu Ile Ser Leu Leu Asn Gly Val Cys Leu Ile Val Gly
        35                  40                  45
Asn Met Ile Gly Ser Gly Ile Phe Val Ser Pro Lys Gly Val Leu Ile
    50                  55                  60
Tyr Ser Ala Ser Phe Gly Leu Ser Leu Val Ile Trp Ala Val Gly Gly
65                  70                  75                  80
Leu Phe Ser Val Phe Gly Ala Leu Cys Tyr Val Glu Leu Gly Thr Thr
                85                  90                  95
Ile Lys Lys Ser Gly Ala Ser Tyr Ala Tyr Ile Leu Glu Ala Phe Gly
            100                 105                 110
Gly Phe Leu Ala Phe Ile Arg Leu Trp Thr Ser Leu Leu Ile Ile Glu
        115                 120                 125
Pro Thr Ser Gln Ala Ile Ile Ala Ile Thr Phe Ala Asn Tyr Met Val
    130                 135                 140
Gln Pro Leu Phe Pro Ser Cys Phe Ala Pro Tyr Ala Ala Ser Arg Leu
145                 150                 155                 160
Leu Ala Ala Ala Cys Ile Cys Leu Leu Thr Phe Ile Asn Cys Ala Tyr
                165                 170                 175
Val Lys Trp Gly Thr Leu Val Gln Asp Ile Phe Thr Tyr Ala Lys Val
            180                 185                 190
Leu Ala Leu Ile Ala Val Ile Val Ala Gly Ile Val Arg Leu Gly Gln
        195                 200                 205
Gly Ala Ser Thr His Phe Glu Asn Ser Phe Glu Gly Ser Ser Phe Ala
    210                 215                 220
Val Gly Asp Ile Ala Leu Ala Leu Tyr Ser Ala Leu Phe Ser Tyr Ser
225                 230                 235                 240
Gly Trp Asp Thr Leu Asn Tyr Val Thr Glu Glu Ile Lys Asn Pro Glu
                245                 250                 255
Arg Asn Leu Pro Leu Ser Ile Gly Ile Ser Met Pro Ile Val Thr Ile
            260                 265                 270

Ile Tyr Ile Leu Thr Asn Val Ala Tyr Tyr Thr Val Leu Asp Met Arg
        275                 280                 285
Asp Ile Leu Ala Ser Asp Ala Val Ala Val Thr Phe Ala Asp Gln Ile
    290                 295                 300
Phe Gly Ile Phe Asn Trp Ile Ile Pro Leu Ser Val Ala Leu Ser Cys
305                 310                 315                 320
Phe Gly Gly Leu Asn Ala Ser Ile Val Ala Ala Ser Arg Leu Phe Phe
                325                 330                 335
Val Gly Ser Arg Glu Gly His Leu Pro Asp Ala Ile Cys Met Ile His
            340                 345                 350
Val Glu Arg Phe Thr Pro Val Pro Ser Leu Leu Phe Asn Gly Ile Met
        355                 360                 365
Ala Leu Ile Tyr Leu Cys Val Glu Asp Ile Phe Gln Leu Ile Asn Tyr
    370                 375                 380
Tyr Ser Phe Ser Tyr Trp Phe Phe Val Gly Leu Ser Ile Val Gly Gln
385                 390                 395                 400
Leu Tyr Leu Arg Trp Lys Glu Pro Asp Arg Pro Arg Pro Leu Lys Leu
                405                 410                 415
Ser Val Phe Phe Pro Ile Val Phe Cys Leu Cys Thr Ile Phe Leu Val
            420                 425                 430
Ala Val Pro Leu Tyr Ser Asp Thr Ile Asn Ser Leu Ile Gly Ile Ala
        435                 440                 445
Ile Ala Leu Ser Gly Leu Pro Phe Tyr Phe Leu Ile Ile Arg Val Pro
    450                 455                 460
Glu His Lys Arg Pro Leu Tyr Leu Arg Arg Ile Val Gly Ser Ala Thr
465                 470                 475                 480
Arg Tyr Leu Gln Val Leu Cys Met Ser Val Ala Ala Glu Met Asp Leu
                485                 490                 495
Glu Asp Gly Gly Glu Met Pro Lys Gln Arg Asp Pro Lys Ser Asn
            500                 505                 510

<210>  120

<211>  737

<212>  PRT

<213>  Homo sapiens

<220>

<221>  procollagen-lysine, 2-oxoglutarate 5-dioxygenase variant 2

<222>  (1)..(737)

<223>  LocusID: 5352; NM_000935

<400>  120

Met Gly Gly Cys Thr Val Lys Pro Gln Leu Leu Leu Leu Ala Leu Val
1               5                   10                  15

Leu His Pro Trp Asn Pro Cys Leu Gly Ala Asp Ser Glu Lys Pro Ser
            20                  25                  30

Ser Ile Pro Thr Asp Lys Leu Leu Val Ile Thr Val Ala Thr Lys Glu
        35                  40                  45

Ser Asp Gly Phe His Arg Phe Met Gln Ser Ala Lys Tyr Phe Asn Tyr
    50                  55                  60

Thr Val Lys Val Leu Gly Gln Gly Glu Glu Trp Arg Gly Gly Asp Gly
65                  70                  75                  80

Ile Asn Ser Ile Gly Gly Gly Gln Lys Val Arg Leu Met Lys Glu Val
                85                  90                  95

Met Glu His Tyr Ala Asp Gln Asp Asp Leu Val Val Met Phe Thr Glu
            100                 105                 110

Cys Phe Asp Val Ile Phe Ala Gly Gly Pro Glu Glu Val Leu Lys Lys
        115                 120                 125

Phe Gln Lys Ala Asn His Lys Val Val Phe Ala Ala Asp Gly Ile Leu
    130                 135                 140

Trp Pro Asp Lys Arg Leu Ala Asp Lys Tyr Pro Val Val His Ile Gly
145                 150                 155                 160

Lys Arg Tyr Leu Asn Ser Gly Gly Phe Ile Gly Tyr Ala Pro Tyr Val
                165                 170                 175

Asn Arg Ile Val Gln Gln Trp Asn Leu Gln Asp Asn Asp Asp Asp Gln
            180                 185                 190
Leu Phe Tyr Thr Lys Val Tyr Ile Asp Pro Leu Lys Arg Glu Ala Ile
        195                 200                 205
Asn Ile Thr Leu Asp His Lys Cys Lys Ile Phe Gln Thr Leu Asn Gly
    210                 215                 220
Ala Val Asp Glu Val Val Leu Lys Phe Glu Asn Gly Lys Ala Arg Ala
225                 230                 235                 240
Lys Asn Thr Phe Tyr Glu Thr Leu Pro Val Ala Ile Asn Gly Asn Gly
                245                 250                 255
Pro Thr Lys Ile Leu Leu Asn Tyr Phe Gly Asn Tyr Val Pro Asn Ser
            260                 265                 270
Trp Thr Gln Asp Asn Gly Cys Thr Leu Cys Glu Phe Asp Thr Val Asp
        275                 280                 285
Leu Ser Ala Val Asp Val His Pro Asn Val Ser Ile Gly Val Phe Ile
    290                 295                 300
Glu Gln Pro Thr Pro Phe Leu Pro Arg Phe Leu Asp Ile Leu Leu Thr
305                 310                 315                 320
Leu Asp Tyr Pro Lys Glu Ala Leu Lys Leu Phe Ile His Asn Lys Glu
                325                 330                 335
Val Tyr His Glu Lys Asp Ile Lys Val Phe Phe Asp Lys Ala Lys His
            340                 345                 350
Glu Ile Lys Thr Ile Lys Ile Val Gly Pro Glu Glu Asn Leu Ser Gln
        355                 360                 365
Ala Glu Ala Arg Asn Met Gly Met Asp Phe Cys Arg Gln Asp Glu Lys
    370                 375                 380
Cys Asp Tyr Tyr Phe Ser Val Asp Ala Asp Val Val Leu Thr Asn Pro
385                 390                 395                 400
Arg Thr Leu Lys Ile Leu Ile Glu Gln Asn Arg Lys Ile Ile Ala Pro
                405                 410                 415
Leu Val Thr Arg His Gly Lys Leu Trp Ser Asn Phe Trp Gly Ala Leu
            420                 425                 430

Ser Pro Asp Gly Tyr Tyr Ala Arg Ser Glu Asp Tyr Val Asp Ile Val
        435                 440                 445
Gln Gly Asn Arg Val Gly Val Trp Asn Val Pro Tyr Met Ala Asn Val
    450                 455                 460
Tyr Leu Ile Lys Gly Lys Thr Leu Arg Ser Glu Met Asn Glu Arg Asn
465                 470                 475                 480
Tyr Phe Val Arg Asp Lys Leu Asp Pro Asp Met Ala Leu Cys Arg Asn
                485                 490                 495
Ala Arg Glu Met Gly Val Phe Met Tyr Ile Ser Asn Arg His Glu Phe
            500                 505                 510
Gly Arg Leu Leu Ser Thr Ala Asn Tyr Asn Thr Ser His Tyr Asn Asn
        515                 520                 525
Asp Leu Trp Gln Ile Phe Glu Asn Pro Val Asp Trp Lys Glu Lys Tyr
    530                 535                 540
Ile Asn Arg Asp Tyr Ser Lys Ile Phe Thr Glu Asn Ile Val Glu Gln
545                 550                 555                 560
Pro Cys Pro Asp Val Phe Trp Phe Pro Ile Phe Ser Glu Lys Ala Cys
                565                 570                 575
Asp Glu Leu Val Glu Glu Met Glu His Tyr Gly Lys Trp Ser Gly Gly
            580                 585                 590
Lys His His Asp Ser Arg Ile Ser Gly Gly Tyr Glu Asn Val Pro Thr
        595                 600                 605
Asp Asp Ile His Met Lys Gln Val Asp Leu Glu Asn Val Trp Leu Asp
    610                 615                 620
Phe Ile Arg Glu Phe Ile Ala Pro Val Thr Leu Lys Val Phe Ala Gly
625                 630                 635                 640
Tyr Tyr Thr Lys Gly Phe Ala Leu Leu Asn Phe Val Val Lys Tyr Ser
                645                 650                 655
Pro Glu Arg Gln Arg Ser Leu Arg Pro His His Asp Ala Ser Thr Phe
            660                 665                 670
Thr Ile Asn Ile Ala Leu Asn Asn Val Gly Glu Asp Phe Gln Gly Gly
        675                 680                 685

Gly Cys Lys Phe Leu Arg Tyr Asn Cys Ser Ile Glu Ser Pro Arg Lys
    690                 695                 700

Gly Trp Ser Phe Met His Pro Gly Arg Leu Thr His Leu His Glu Gly
705                 710                 715                 720

Leu Pro Val Lys Asn Gly Thr Arg Tyr Ile Ala Val Ser Phe Ile Asp
                725                 730                 735

Pro

<210>  121

<211>  758

<212>  PRT

<213>  Homo sapiens

<220>

<221>  procollagen-lysine, 2-oxoglutarate 5-dioxygenase variant 1

<222>  (1)..(758)

<223>  LocusID: 5352; NM_182943

<400>  121

<210> 122
<211> 494
<212> PRT
<213> Homo sapiens
<220>
<221> UDP-glucose dehydrogenase
<222> (1)..(494)
<223> LocusID: 7358; NM_003359
<400> 122

<210>  123

<211>  416

<212>  PRT

<213>  Homo sapiens

<220>

<221>  phosphatidylinositol-4-phosphate 5-kinase, type II beta variant
1

<222>  (1)..(416)

<223>  LocusID: 8396; NM_003559

<400>  123

Met Ser Ser Asn Cys Thr Ser Thr Thr Ala Val Ala Val Ala Pro Leu
1               5                   10                  15

Ser Ala Ser Lys Thr Lys Thr Lys Lys Lys His Phe Val Cys Gln Lys
            20                  25                  30

Val Lys Leu Phe Arg Ala Ser Glu Pro Ile Leu Ser Val Leu Met Trp
        35                  40                  45

Gly Val Asn His Thr Ile Asn Glu Leu Ser Asn Val Pro Val Pro Val
    50                  55                  60

Met Leu Met Pro Asp Asp Phe Lys Ala Tyr Ser Lys Ile Lys Val Asp
65                  70                  75                  80

Asn His Leu Phe Asn Lys Glu Asn Leu Pro Ser Arg Phe Lys Phe Lys
                85                  90                  95

Glu Tyr Cys Pro Met Val Phe Arg Asn Leu Arg Glu Arg Phe Gly Ile
            100                 105                 110

Asp Asp Gln Asp Tyr Gln Asn Ser Val Thr Arg Ser Ala Pro Ile Asn
        115                 120                 125

Ser Asp Ser Gln Gly Arg Cys Gly Thr Arg Phe Leu Thr Thr Tyr Asp
    130                 135                 140

Arg Arg Phe Val Ile Lys Thr Val Ser Ser Glu Asp Val Ala Glu Met
145                 150                 155                 160

His Asn Ile Leu Lys Lys Tyr His Gln Phe Ile Val Glu Cys His Gly

                165                 170                 175
Asn Thr Leu Leu Pro Gln Phe Leu Gly Met Tyr Arg Leu Thr Val Asp
            180                 185                 190
Gly Val Glu Thr Tyr Met Val Val Thr Arg Asn Val Phe Ser His Arg
        195                 200                 205
Leu Thr Val His Arg Lys Tyr Asp Leu Lys Gly Ser Thr Val Ala Arg
    210                 215                 220
Glu Ala Ser Asp Lys Glu Lys Ala Lys Asp Leu Pro Thr Phe Lys Asp
225                 230                 235                 240
Asn Asp Phe Leu Asn Glu Gly Gln Lys Leu His Val Gly Glu Glu Ser
                245                 250                 255
Lys Lys Asn Phe Leu Glu Lys Leu Lys Arg Asp Val Glu Phe Leu Ala
            260                 265                 270
Gln Leu Lys Ile Met Asp Tyr Ser Leu Leu Val Gly Ile His Asp Val
        275                 280                 285
Asp Arg Ala Glu Gln Glu Glu Met Glu Val Glu Glu Arg Ala Glu Asp
    290                 295                 300
Glu Glu Cys Glu Asn Asp Gly Val Gly Gly Asn Leu Leu Cys Ser Tyr
305                 310                 315                 320
Gly Thr Pro Pro Asp Ser Pro Gly Asn Leu Leu Ser Phe Pro Arg Phe
                325                 330                 335
Phe Gly Pro Gly Glu Phe Asp Pro Ser Val Asp Val Tyr Ala Met Lys
            340                 345                 350
Ser His Glu Ser Ser Pro Lys Lys Glu Val Tyr Phe Met Ala Ile Ile
        355                 360                 365
Asp Ile Leu Thr Pro Tyr Asp Thr Lys Lys Lys Ala Ala His Ala Ala
    370                 375                 380
Lys Thr Val Lys His Gly Ala Gly Ala Glu Ile Ser Thr Val Asn Pro
385                 390                 395                 400
Glu Gln Tyr Ser Lys Arg Phe Asn Glu Phe Met Ser Asn Ile Leu Thr
                405                 410                 415

<210> 124

<211> 237

<212> PRT

<213> Homo sapiens

<220>

<221> phosphatidylinositol-4-phosphate 5-kinase, type II, beta variant
      2

<222> (1)..(237)

<223> LocusID: 8396; NM_138687

<400> 124

Val Leu Met Trp Gly Val Asn His Thr Ile Asn Glu Leu Ser Asn Val
1               5                   10                  15

Pro Val Pro Val Met Leu Met Pro Asp Asp Phe Lys Ala Tyr Ser Lys
            20                  25                  30

Ile Lys Val Asp Asn His Leu Phe Asn Lys Glu Asn Leu Pro Ser Arg
        35                  40                  45

Phe Lys Phe Lys Glu Tyr Cys Pro Met Val Phe Arg Asn Leu Arg Glu
    50                  55                  60

Arg Phe Gly Ile Asp Asp Gln Asp Tyr Gln Asn Ser Val Thr Arg Ser
65                  70                  75                  80

Ala Pro Ile Asn Ser Asp Ser Gln Gly Arg Cys Gly Thr Arg Phe Leu
                85                  90                  95

Thr Thr Tyr Asp Arg Arg Phe Val Ile Lys Thr Val Ser Ser Glu Asp
            100                 105                 110

Val Ala Glu Met His Asn Ile Leu Lys Lys Tyr His Gln Phe Ile Val
        115                 120                 125

Glu Cys His Gly Asn Thr Leu Leu Pro Gln Phe Leu Gly Met Tyr Arg
    130                 135                 140

Leu Thr Val Asp Gly Val Glu Thr Tyr Met Val Val Thr Arg Asn Val
145                 150                 155                 160

Phe Ser His Arg Leu Thr Val His Arg Lys Tyr Asp Leu Lys Gly Ser
                165                 170                 175
Thr Val Ala Arg Glu Ala Ser Asp Lys Glu Lys Ala Lys Asp Leu Pro
            180                 185                 190
Thr Phe Lys Asp Asn Asp Phe Leu Asn Glu Gly Gln Lys Leu His Val
        195                 200                 205
Gly Glu Glu Ser Lys Lys Asn Phe Leu Glu Lys Leu Lys Arg Asp Val
    210                 215                 220
Glu Glu Ile Leu Val Leu Ser Pro Gly Trp Arg Ile Ala
225                 230                 235

<210> 125
<211> 414
<212> PRT
<213> Homo sapiens
<220>
<221> MAP kinase interacting serine/threonine kinase 2 sequence 1
<222> (1)..(414)
<223> LocusID:2872; NM_017572
<400> 125

Met Val Gln Lys Lys Pro Ala Glu Leu Gln Gly Phe His Arg Ser Phe
1               5                   10                  15
Lys Gly Gln Asn Pro Phe Glu Leu Ala Phe Ser Leu Asp Gln Pro Asp
            20                  25                  30
His Gly Asp Ser Asp Phe Gly Leu Gln Cys Ser Ala Arg Pro Asp Met
        35                  40                  45
Pro Ala Ser Gln Pro Ile Asp Ile Pro Asp Ala Lys Lys Arg Gly Lys
    50                  55                  60
Lys Lys Lys Arg Gly Arg Ala Thr Asp Ser Phe Ser Gly Arg Phe Glu
65                  70                  75                  80

Asp Val Tyr Gln Leu Gln Glu Asp Val Leu Gly Glu Gly Ala His Ala
                85                  90                  95
Arg Val Gln Thr Cys Ile Asn Leu Ile Thr Ser Gln Glu Tyr Ala Val
            100                 105                 110
Lys Ile Ile Glu Lys Gln Pro Gly His Ile Arg Ser Arg Val Phe Arg
        115                 120                 125
Glu Val Glu Met Leu Tyr Gln Cys Gln Gly His Arg Asn Val Leu Glu
    130                 135                 140
Leu Ile Glu Phe Phe Glu Glu Glu Asp Arg Phe Tyr Leu Val Phe Glu
145                 150                 155                 160
Lys Met Arg Gly Gly Ser Ile Leu Ser His Ile His Lys Arg Arg His
                165                 170                 175
Phe Asn Glu Leu Glu Ala Ser Val Val Val Gln Asp Val Ala Ser Ala
            180                 185                 190
Leu Asp Phe Leu His Asn Lys Gly Ile Ala His Arg Asp Leu Lys Pro
        195                 200                 205
Glu Asn Ile Leu Cys Glu His Pro Asn Gln Val Ser Pro Val Lys Ile
    210                 215                 220
Cys Asp Phe Asp Leu Gly Ser Gly Ile Lys Leu Asn Gly Asp Cys Ser
225                 230                 235                 240
Pro Ile Ser Thr Pro Glu Leu Leu Thr Pro Cys Gly Ser Ala Glu Tyr
                245                 250                 255
Met Ala Pro Glu Val Val Glu Ala Phe Ser Glu Glu Ala Ser Ile Tyr
            260                 265                 270
Asp Lys Arg Cys Asp Leu Trp Ser Leu Gly Val Ile Leu Tyr Ile Leu
        275                 280                 285
Leu Ser Gly Tyr Pro Pro Phe Val Gly Arg Cys Gly Ser Asp Cys Gly
    290                 295                 300
Trp Asp Arg Gly Glu Ala Cys Pro Ala Cys Gln Asn Met Leu Phe Glu
305                 310                 315                 320
Ser Ile Gln Glu Gly Lys Tyr Glu Phe Pro Asp Lys Asp Trp Ala His
                325                 330                 335

Ile Ser Cys Ala Ala Lys Asp Leu Ile Ser Lys Leu Leu Val Arg Asp
            340                 345                 350
Ala Lys Gln Arg Leu Ser Ala Ala Gln Val Leu Gln His Pro Trp Val
        355                 360                 365
Gln Gly Cys Ala Pro Glu Asn Thr Leu Pro Thr Pro Met Val Leu Gln
    370                 375                 380
Arg Trp Asp Ser His Phe Leu Leu Pro Pro His Pro Cys Arg Ile His
385                 390                 395                 400
Val Arg Pro Gly Gly Leu Val Arg Thr Val Thr Val Asn Glu
                405                 410

<210> 126
<211> 465
<212> PRT
<213> Homo sapiens
<220>
<221> MAP kinase interacting serine/threonine kinase 2 sequence 2
<222> (1)..(465)
<223> LocusID: 2872; NM_199054
<400> 126

Met Val Gln Lys Lys Pro Ala Glu Leu Gln Gly Phe His Arg Ser Phe
1               5                   10                  15
Lys Gly Gln Asn Pro Phe Glu Leu Ala Phe Ser Leu Asp Gln Pro Asp
            20                  25                  30
His Gly Asp Ser Asp Phe Gly Leu Gln Cys Ser Ala Arg Pro Asp Met
        35                  40                  45
Pro Ala Ser Gln Pro Ile Asp Ile Pro Asp Ala Lys Lys Arg Gly Lys
    50                  55                  60
Lys Lys Lys Arg Gly Arg Ala Thr Asp Ser Phe Ser Gly Arg Phe Glu
65                  70                  75                  80

Asp Val Tyr Gln Leu Gln Glu Asp Val Leu Gly Glu Gly Ala His Ala
                85                  90                  95
Arg Val Gln Thr Cys Ile Asn Leu Ile Thr Ser Gln Glu Tyr Ala Val
            100                 105                 110
Lys Ile Ile Glu Lys Gln Pro Gly His Ile Arg Ser Arg Val Phe Arg
        115                 120                 125
Glu Val Glu Met Leu Tyr Gln Cys Gln Gly His Arg Asn Val Leu Glu
    130                 135                 140
Leu Ile Glu Phe Phe Glu Glu Glu Asp Arg Phe Tyr Leu Val Phe Glu
145                 150                 155                 160
Lys Met Arg Gly Gly Ser Ile Leu Ser His Ile His Lys Arg Arg His
                165                 170                 175
Phe Asn Glu Leu Glu Ala Ser Val Val Val Gln Asp Val Ala Ser Ala
            180                 185                 190
Leu Asp Phe Leu His Asn Lys Gly Ile Ala His Arg Asp Leu Lys Pro
        195                 200                 205
Glu Asn Ile Leu Cys Glu His Pro Asn Gln Val Ser Pro Val Lys Ile
    210                 215                 220
Cys Asp Phe Asp Leu Gly Ser Gly Ile Lys Leu Asn Gly Asp Cys Ser
225                 230                 235                 240
Pro Ile Ser Thr Pro Glu Leu Leu Thr Pro Cys Gly Ser Ala Glu Tyr
                245                 250                 255
Met Ala Pro Glu Val Val Glu Ala Phe Ser Glu Glu Ala Ser Ile Tyr
            260                 265                 270
Asp Lys Arg Cys Asp Leu Trp Ser Leu Gly Val Ile Leu Tyr Ile Leu
        275                 280                 285
Leu Ser Gly Tyr Pro Pro Phe Val Gly Arg Cys Gly Ser Asp Cys Gly
    290                 295                 300
Trp Asp Arg Gly Glu Ala Cys Pro Ala Cys Gln Asn Met Leu Phe Glu
305                 310                 315                 320
Ser Ile Gln Glu Gly Lys Tyr Glu Phe Pro Asp Lys Asp Trp Ala His
                325                 330                 335

Ile Ser Cys Ala Ala Lys Asp Leu Ile Ser Lys Leu Leu Val Arg Asp
            340                 345                 350
Ala Lys Gln Arg Leu Ser Ala Ala Gln Val Leu Gln His Pro Trp Val
        355                 360                 365
Gln Gly Cys Ala Pro Glu Asn Thr Leu Pro Thr Pro Met Val Leu Gln
    370                 375                 380
Arg Asn Ser Cys Ala Lys Asp Leu Thr Ser Phe Ala Ala Glu Ala Ile
385                 390                 395                 400
Ala Met Asn Arg Gln Leu Ala Gln His Asp Glu Asp Leu Ala Glu Glu
                405                 410                 415
Glu Ala Ala Gly Gln Gly Gln Pro Val Leu Val Arg Ala Thr Ser Arg
            420                 425                 430
Cys Leu Gln Leu Ser Pro Pro Ser Gln Ser Lys Leu Ala Gln Arg Arg
        435                 440                 445
Gln Arg Ala Ser Leu Ser Ser Ala Pro Val Val Leu Val Gly Asp His
    450                 455                 460
Ala
465

<210> 127
<211> 476
<212> PRT
<213> Homo sapiens
<220>
<221> carboxypeptidase E
<222> (1)..(476)
<223> LocusID: 1363; NM_001873
<400> 127

Met Ala Gly Arg Gly Gly Ser Ala Leu Leu Ala Leu Cys Gly Ala Leu
1               5                   10                  15

Ala Ala Cys Gly Trp Leu Leu Gly Ala Glu Ala Gln Glu Pro Gly Ala
            20                  25                  30
Pro Ala Ala Gly Met Arg Arg Arg Arg Arg Leu Gln Gln Glu Asp Gly
        35                  40                  45
Ile Ser Phe Glu Tyr His Arg Tyr Pro Glu Leu Arg Glu Ala Leu Val
    50                  55                  60
Ser Val Trp Leu Gln Cys Thr Ala Ile Ser Arg Ile Tyr Thr Val Gly
65                  70                  75                  80
Arg Ser Phe Glu Gly Arg Glu Leu Leu Val Ile Glu Leu Ser Asp Asn
                85                  90                  95
Pro Gly Val His Glu Pro Gly Glu Pro Glu Phe Lys Tyr Ile Gly Asn
            100                 105                 110
Met His Gly Asn Glu Ala Val Gly Arg Glu Leu Leu Ile Phe Leu Ala
        115                 120                 125
Gln Tyr Leu Cys Asn Glu Tyr Gln Lys Gly Asn Glu Thr Ile Val Asn
    130                 135                 140
Leu Ile His Ser Thr Arg Ile His Ile Met Pro Ser Leu Asn Pro Asp
145                 150                 155                 160
Gly Phe Glu Lys Ala Ala Ser Gln Pro Gly Glu Leu Lys Asp Trp Phe
                165                 170                 175
Val Gly Arg Ser Asn Ala Gln Gly Ile Asp Leu Asn Arg Asn Phe Pro
            180                 185                 190
Asp Leu Asp Arg Ile Val Tyr Val Asn Glu Lys Glu Gly Gly Pro Asn
        195                 200                 205
Asn His Leu Leu Lys Asn Met Lys Lys Ile Val Asp Gln Asn Thr Lys
    210                 215                 220
Leu Ala Pro Glu Thr Lys Ala Val Ile His Trp Ile Met Asp Ile Pro
225                 230                 235                 240
Phe Val Leu Ser Ala Asn Leu His Gly Gly Asp Leu Val Ala Asn Tyr
                245                 250                 255
Pro Tyr Asp Glu Thr Arg Ser Gly Ser Ala His Glu Tyr Ser Ser Ser
            260                 265                 270

Pro Asp Asp Ala Ile Phe Gln Ser Leu Ala Arg Ala Tyr Ser Ser Phe
        275                 280                 285
Asn Pro Ala Met Ser Asp Pro Asn Arg Pro Pro Cys Arg Lys Asn Asp
    290                 295                 300
Asp Asp Ser Ser Phe Val Asp Gly Thr Thr Asn Gly Gly Ala Trp Tyr
305                 310                 315                 320
Ser Val Pro Gly Gly Met Gln Asp Phe Asn Tyr Leu Ser Ser Asn Cys
                325                 330                 335
Phe Glu Ile Thr Val Glu Leu Ser Cys Glu Lys Phe Pro Pro Glu Glu
            340                 345                 350
Thr Leu Lys Thr Tyr Trp Glu Asp Asn Lys Asn Ser Leu Ile Ser Tyr
        355                 360                 365
Leu Glu Gln Ile His Arg Gly Val Lys Gly Phe Val Arg Asp Leu Gln
    370                 375                 380
Gly Asn Pro Ile Ala Asn Ala Thr Ile Ser Val Glu Gly Ile Asp His
385                 390                 395                 400
Asp Val Thr Ser Ala Lys Asp Gly Asp Tyr Trp Arg Leu Leu Ile Pro
                405                 410                 415
Gly Asn Tyr Lys Leu Thr Ala Ser Ala Pro Gly Tyr Leu Ala Ile Thr
            420                 425                 430
Lys Lys Val Ala Val Pro Tyr Ser Pro Ala Ala Gly Val Asp Phe Glu
        435                 440                 445
Leu Glu Ser Phe Ser Glu Arg Lys Glu Glu Glu Lys Glu Glu Leu Met
    450                 455                 460
Glu Trp Trp Lys Met Met Ser Glu Thr Leu Asn Phe
465                 470                 475

<210> 128
<211> 417
<212> PRT
<213> Homo sapiens

<220>

<221>  carboxypeptidase A3

<222>  (1)..(417)

<223>  LocusID: 1359; NM_001870

<400>  128

Met Arg Leu Ile Leu Pro Val Gly Leu Ile Ala Thr Thr Leu Ala Ile
1               5                   10                  15

Ala Pro Val Arg Phe Asp Arg Glu Lys Val Phe Arg Val Lys Pro Gln
            20                  25                  30

Asp Glu Lys Gln Ala Asp Ile Ile Lys Asp Leu Ala Lys Thr Asn Glu
        35                  40                  45

Leu Asp Phe Trp Tyr Pro Gly Ala Thr His His Val Ala Ala Asn Met
    50                  55                  60

Met Val Asp Phe Arg Val Ser Glu Lys Glu Ser Gln Ala Ile Gln Ser
65                  70                  75                  80

Ala Leu Asp Gln Asn Lys Met His Tyr Glu Ile Leu Ile His Asp Leu
                85                  90                  95

Gln Glu Glu Ile Glu Lys Gln Phe Asp Val Lys Glu Asp Ile Pro Gly
            100                 105                 110

Arg His Ser Tyr Ala Lys Tyr Asn Asn Trp Glu Lys Ile Val Ala Trp
        115                 120                 125

Thr Glu Lys Met Met Asp Lys Tyr Pro Glu Met Val Ser Arg Ile Lys
    130                 135                 140

Ile Gly Ser Thr Val Glu Asp Asn Pro Leu Tyr Val Leu Lys Ile Gly
145                 150                 155                 160

Glu Lys Asn Glu Arg Arg Lys Ala Ile Phe Met Asp Cys Gly Ile His
                165                 170                 175

Ala Arg Glu Trp Val Ser Pro Ala Phe Cys Gln Trp Phe Val Tyr Gln
            180                 185                 190

Ala Thr Lys Thr Tyr Gly Arg Asn Lys Ile Met Thr Lys Leu Leu Asp
        195                 200                 205

Arg Met Asn Phe Tyr Ile Leu Pro Val Phe Asn Val Asp Gly Tyr Ile
    210                 215                 220
Trp Ser Trp Thr Lys Asn Arg Met Trp Arg Lys Asn Arg Ser Lys Asn
225                 230                 235                 240
Gln Asn Ser Lys Cys Ile Gly Thr Asp Leu Asn Arg Asn Phe Asn Ala
                245                 250                 255
Ser Trp Asn Ser Ile Pro Asn Thr Asn Asp Pro Cys Ala Asp Asn Tyr
            260                 265                 270
Arg Gly Ser Ala Pro Glu Ser Glu Lys Glu Thr Lys Ala Val Thr Asn
        275                 280                 285
Phe Ile Arg Ser His Leu Asn Glu Ile Lys Val Tyr Ile Thr Phe His
    290                 295                 300
Ser Tyr Ser Gln Met Leu Leu Phe Pro Tyr Gly Tyr Thr Ser Lys Leu
305                 310                 315                 320
Pro Pro Asn His Glu Asp Leu Ala Lys Val Ala Lys Ile Gly Thr Asp
                325                 330                 335
Val Leu Ser Thr Arg Tyr Glu Thr Arg Tyr Ile Tyr Gly Pro Ile Glu
            340                 345                 350
Ser Thr Ile Tyr Pro Ile Ser Gly Ser Ser Leu Asp Trp Ala Tyr Asp
        355                 360                 365
Leu Gly Ile Lys His Thr Phe Ala Phe Glu Leu Arg Asp Lys Gly Lys
    370                 375                 380
Phe Gly Phe Leu Leu Pro Glu Ser Arg Ile Lys Pro Thr Cys Arg Glu
385                 390                 395                 400
Thr Met Leu Ala Val Lys Phe Ile Ala Lys Tyr Ile Leu Lys His Thr
                405                 410                 415
Ser

<210> 129
<211> 456

<212>  PRT

<213>  Homo sapiens

<220>

<221>  solute carrier family 38, member 6

<222>  (1)..(456)

<223>  LocusID: 145389; NM_153811

<400>  129

Met Glu Ala Ser Trp Gly Ser Phe Asn Ala Glu Arg Gly Trp Tyr Val
1               5                   10                  15

Ser Val Gln Gln Pro Glu Glu Ala Glu Ala Glu Glu Leu Ser Pro Leu
            20                  25                  30

Leu Ser Asn Glu Leu His Arg Gln Arg Ser Pro Gly Val Ser Phe Gly
        35                  40                  45

Leu Ser Val Phe Asn Leu Met Asn Ala Ile Met Gly Ser Gly Ile Leu
    50                  55                  60

Gly Leu Ala Tyr Val Met Ala Asn Thr Gly Val Phe Gly Phe Ser Phe
65                  70                  75                  80

Leu Leu Leu Thr Val Ala Leu Leu Ala Ser Tyr Ser Val His Leu Leu
                85                  90                  95

Leu Ser Met Cys Ile Gln Thr Ala Val Thr Ser Tyr Glu Asp Leu Gly
            100                 105                 110

Leu Phe Ala Phe Gly Leu Pro Gly Lys Leu Val Val Ala Gly Thr Ile
        115                 120                 125

Ile Ile Gln Asn Ile Gly Ala Met Ser Ser Tyr Leu Leu Ile Ile Lys
    130                 135                 140

Thr Glu Leu Pro Ala Ala Ile Ala Glu Phe Leu Thr Gly Asp Tyr Ser
145                 150                 155                 160

Arg Tyr Trp Tyr Leu Asp Gly Gln Thr Leu Leu Ile Ile Ile Cys Val
                165                 170                 175

Gly Ile Val Phe Pro Leu Ala Leu Leu Pro Lys Ile Gly Phe Leu Gly
            180                 185                 190

Tyr Thr Ser Ser Leu Ser Phe Phe Phe Met Met Phe Phe Ala Leu Val
        195                 200                 205
Val Ile Ile Lys Lys Trp Ser Ile Pro Cys Pro Leu Thr Leu Asn Tyr
    210                 215                 220
Val Glu Lys Gly Phe Gln Ile Ser Asn Val Thr Asp Asp Cys Lys Pro
225                 230                 235                 240
Lys Leu Phe His Phe Ser Lys Glu Ser Ala Tyr Ala Leu Pro Thr Met
                245                 250                 255
Ala Phe Ser Phe Leu Cys His Thr Ser Ile Leu Pro Ile Tyr Cys Glu
            260                 265                 270
Leu Gln Ser Pro Ser Lys Lys Arg Met Gln Asn Val Thr Asn Thr Ala
        275                 280                 285
Ile Ala Leu Ser Phe Leu Ile Tyr Phe Ile Ser Ala Leu Phe Gly Tyr
    290                 295                 300
Leu Thr Phe Tyr Asp Lys Val Glu Ser Glu Leu Leu Lys Gly Tyr Ser
305                 310                 315                 320
Lys Tyr Leu Ser His Asp Val Val Val Met Thr Val Lys Leu Cys Ile
                325                 330                 335
Leu Phe Ala Val Leu Leu Thr Val Pro Leu Ile His Phe Pro Ala Arg
            340                 345                 350
Lys Ala Val Thr Met Met Phe Phe Ser Asn Phe Pro Phe Ser Trp Ile
        355                 360                 365
Arg His Phe Leu Ile Thr Leu Ala Leu Asn Ile Ile Ile Val Leu Leu
    370                 375                 380
Ala Ile Tyr Val Pro Asp Ile Arg Asn Val Phe Gly Val Val Gly Ala
385                 390                 395                 400
Ser Thr Ser Thr Cys Leu Ile Phe Ile Phe Pro Gly Leu Phe Tyr Leu
                405                 410                 415
Lys Leu Ser Arg Glu Asp Phe Leu Ser Trp Lys Lys Leu Gly Ala Phe
            420                 425                 430
Val Leu Leu Ile Phe Gly Ile Leu Val Gly Asn Phe Ser Leu Ala Leu
        435                 440                 445

Ile Ile Phe Asp Trp Ile Asn Lys
    450                 455

<210> 130
<211> 873
<212> PRT
<213> Homo sapiens
<220>
<221> very low density lipoprotein receptor
<222> (1)..(873)
<223> LocusID: 7436; NM_003383
<400> 130

Met Gly Thr Ser Ala Leu Trp Ala Val Trp Leu Leu Leu Ala Leu Cys
1               5                   10                  15
Trp Ala Pro Arg Glu Ser Gly Ala Thr Gly Thr Gly Arg Lys Ala Lys
            20                  25                  30
Cys Glu Pro Ser Gln Phe Gln Cys Thr Asn Gly Arg Cys Ile Thr Leu
        35                  40                  45
Leu Trp Lys Cys Asp Gly Asp Glu Asp Cys Val Asp Gly Ser Asp Glu
    50                  55                  60
Lys Asn Cys Val Lys Lys Thr Cys Ala Glu Ser Asp Phe Val Cys Asn
65                  70                  75                  80
Asn Gly Gln Cys Val Pro Ser Arg Trp Lys Cys Asp Gly Asp Pro Asp
                85                  90                  95
Cys Glu Asp Gly Ser Asp Glu Ser Pro Glu Gln Cys His Met Arg Thr
            100                 105                 110
Cys Arg Ile His Glu Ile Ser Cys Gly Ala His Ser Thr Gln Cys Ile
        115                 120                 125
Pro Val Ser Trp Arg Cys Asp Gly Glu Asn Asp Cys Asp Ser Gly Glu
    130                 135                 140

Asp Glu Glu Asn Cys Gly Asn Ile Thr Cys Ser Pro Asp Glu Phe Thr
145                 150                 155                 160
Cys Ser Ser Gly Arg Cys Ile Ser Arg Asn Phe Val Cys Asn Gly Gln
                165                 170                 175
Asp Asp Cys Ser Asp Gly Ser Asp Glu Leu Asp Cys Ala Pro Pro Thr
            180                 185                 190
Cys Gly Ala His Glu Phe Gln Cys Ser Thr Ser Ser Cys Ile Pro Ile
        195                 200                 205
Ser Trp Val Cys Asp Asp Asp Ala Asp Cys Ser Asp Gln Ser Asp Glu
    210                 215                 220
Ser Leu Glu Gln Cys Gly Arg Gln Pro Val Ile His Thr Lys Cys Pro
225                 230                 235                 240
Ala Ser Glu Ile Gln Cys Gly Ser Gly Glu Cys Ile His Lys Lys Trp
                245                 250                 255
Arg Cys Asp Gly Asp Pro Asp Cys Lys Asp Gly Ser Asp Glu Val Asn
            260                 265                 270
Cys Pro Ser Arg Thr Cys Arg Pro Asp Gln Phe Glu Cys Glu Asp Gly
        275                 280                 285
Ser Cys Ile His Gly Ser Arg Gln Cys Asn Gly Ile Arg Asp Cys Val
    290                 295                 300
Asp Gly Ser Asp Glu Val Asn Cys Lys Asn Val Asn Gln Cys Leu Gly
305                 310                 315                 320
Pro Gly Lys Phe Lys Cys Arg Ser Gly Glu Cys Ile Asp Ile Ser Lys
                325                 330                 335
Val Cys Asn Gln Glu Gln Asp Cys Arg Asp Trp Ser Asp Glu Pro Leu
            340                 345                 350
Lys Glu Cys His Ile Asn Glu Cys Leu Val Asn Asn Gly Gly Cys Ser
        355                 360                 365
His Ile Cys Lys Asp Leu Val Ile Gly Tyr Glu Cys Asp Cys Ala Ala
    370                 375                 380
Gly Phe Glu Leu Ile Asp Arg Lys Thr Cys Gly Asp Ile Asp Glu Cys
385                 390                 395                 400

Gln Asn Pro Gly Ile Cys Ser Gln Ile Cys Ile Asn Leu Lys Gly Gly
                405                 410                 415
Tyr Lys Cys Glu Cys Ser Arg Ala Tyr Gln Met Asp Leu Ala Thr Gly
            420                 425                 430
Val Cys Lys Ala Val Gly Lys Glu Pro Ser Leu Ile Phe Thr Asn Arg
        435                 440                 445
Arg Asp Ile Arg Lys Ile Gly Leu Glu Arg Lys Glu Tyr Ile Gln Leu
    450                 455                 460
Val Glu Gln Leu Arg Asn Thr Val Ala Leu Asp Ala Asp Ile Ala Ala
465                 470                 475                 480
Gln Lys Leu Phe Trp Ala Asp Leu Ser Gln Lys Ala Ile Phe Ser Ala
                485                 490                 495
Ser Ile Asp Asp Lys Val Gly Arg His Val Lys Met Ile Asp Asn Val
            500                 505                 510
Tyr Asn Pro Ala Ala Ile Ala Val Asp Trp Val Tyr Lys Thr Ile Tyr
        515                 520                 525
Trp Thr Asp Ala Ala Ser Lys Thr Ile Ser Val Ala Thr Leu Asp Gly
    530                 535                 540
Thr Lys Arg Lys Phe Leu Phe Asn Ser Asp Leu Arg Glu Pro Ala Ser
545                 550                 555                 560
Ile Ala Val Asp Pro Leu Ser Gly Phe Val Tyr Trp Ser Asp Trp Gly
                565                 570                 575
Glu Pro Ala Lys Ile Glu Lys Ala Gly Met Asn Gly Phe Asp Arg Arg
            580                 585                 590
Pro Leu Val Thr Ala Asp Ile Gln Trp Pro Asn Gly Ile Thr Leu Asp
        595                 600                 605
Leu Ile Lys Ser Arg Leu Tyr Trp Leu Asp Ser Lys Leu His Met Leu
    610                 615                 620
Ser Ser Val Asp Leu Asn Gly Gln Asp Arg Arg Ile Val Leu Lys Ser
625                 630                 635                 640
Leu Glu Phe Leu Ala His Pro Leu Ala Leu Thr Ile Phe Glu Asp Arg
                645                 650                 655

Val Tyr Trp Ile Asp Gly Glu Asn Glu Ala Val Tyr Gly Ala Asn Lys
            660                 665                 670
Phe Thr Gly Ser Glu His Ala Thr Leu Val Asn Asn Leu Asn Asp Ala
        675                 680                 685
Gln Asp Ile Ile Val Tyr His Glu Leu Val Gln Pro Ser Gly Lys Asn
    690                 695                 700
Trp Cys Glu Glu Asp Met Glu Asn Gly Gly Cys Glu Tyr Leu Cys Leu
705                 710                 715                 720
Pro Ala Pro Gln Ile Asn Asp His Ser Pro Lys Tyr Thr Cys Ser Cys
                725                 730                 735
Pro Ser Gly Tyr Asn Val Glu Glu Asn Gly Arg Asp Cys Gln Ser Thr
            740                 745                 750
Ala Thr Thr Val Thr Tyr Ser Glu Thr Lys Asp Thr Asn Thr Thr Glu
        755                 760                 765
Ile Ser Ala Thr Ser Gly Leu Val Pro Gly Gly Ile Asn Val Thr Thr
    770                 775                 780
Ala Val Ser Glu Val Ser Val Pro Pro Lys Gly Thr Ser Ala Ala Trp
785                 790                 795                 800
Ala Ile Leu Pro Leu Leu Leu Leu Val Met Ala Ala Val Gly Gly Tyr
                805                 810                 815
Leu Met Trp Arg Asn Trp Gln His Lys Asn Met Lys Ser Met Asn Phe
            820                 825                 830
Asp Asn Pro Val Tyr Leu Lys Thr Thr Glu Glu Asp Leu Ser Ile Asp
        835                 840                 845
Ile Gly Arg His Ser Ala Ser Val Gly His Thr Tyr Pro Ala Ile Ser
    850                 855                 860
Val Val Ser Thr Asp Asp Asp Leu Ala
865                 870

<210> 131
<211> 255

<212>  PRT

<213>  Homo sapiens

<220>

<221>  cathepsin G

<222>  (1)..(255)

<223>  LocusID: 1511; NM_001911

<400>  131

Met Gln Pro Leu Leu Leu Leu Leu Ala Phe Leu Leu Pro Thr Gly Ala
1               5                   10                  15

Glu Ala Gly Glu Ile Ile Gly Gly Arg Glu Ser Arg Pro His Ser Arg
            20                  25                  30

Pro Tyr Met Ala Tyr Leu Gln Ile Gln Ser Pro Ala Gly Gln Ser Arg
        35                  40                  45

Cys Gly Gly Phe Leu Val Arg Glu Asp Phe Val Leu Thr Ala Ala His
    50                  55                  60

Cys Trp Gly Ser Asn Ile Asn Val Thr Leu Gly Ala His Asn Ile Gln
65                  70                  75                  80

Arg Arg Glu Asn Thr Gln Gln His Ile Thr Ala Arg Arg Ala Ile Arg
                85                  90                  95

His Pro Gln Tyr Asn Gln Arg Thr Ile Gln Asn Asp Ile Met Leu Leu
            100                 105                 110

Gln Leu Ser Arg Arg Val Arg Arg Asn Arg Asn Val Asn Pro Val Ala
        115                 120                 125

Leu Pro Arg Ala Gln Glu Gly Leu Arg Pro Gly Thr Leu Cys Thr Val
    130                 135                 140

Ala Gly Trp Gly Arg Val Ser Met Arg Arg Gly Thr Asp Thr Leu Arg
145                 150                 155                 160

Glu Val Gln Leu Arg Val Gln Arg Asp Arg Gln Cys Leu Arg Ile Phe
                165                 170                 175

Gly Ser Tyr Asp Pro Arg Arg Gln Ile Cys Val Gly Asp Arg Arg Glu
            180                 185                 190

Arg Lys Ala Ala Phe Lys Gly Asp Ser Gly Gly Pro Leu Leu Cys Asn
        195                 200                 205

Asn Val Ala His Gly Ile Val Ser Tyr Gly Lys Ser Ser Gly Val Pro
    210                 215                 220

Pro Glu Val Phe Thr Arg Val Ser Ser Phe Leu Pro Trp Ile Arg Thr
225                 230                 235                 240

Thr Met Arg Ser Phe Lys Leu Leu Asp Gln Met Glu Thr Pro Leu
                245                 250                 255

<210>  132

<211>  447

<212>  PRT

<213>  Homo sapiens

<220>

<221>  galactokinase 2 variant 2

<222>  (1)..(447)

<223>  LocusID: 2585; NM_001001556

<400>  132

Met Pro Val Leu Tyr Asp Arg Leu Leu Lys Leu Lys Glu Met Phe Asn
1               5                   10                  15

Ser Lys Phe Gly Ser Ile Pro Lys Phe Tyr Val Arg Ala Pro Gly Arg
            20                  25                  30

Val Asn Ile Ile Gly Glu His Ile Asp Tyr Cys Gly Tyr Ser Val Leu
        35                  40                  45

Pro Met Ala Val Glu Gln Asp Val Leu Ile Ala Val Glu Pro Val Lys
    50                  55                  60

Thr Tyr Ala Leu Gln Leu Ala Asn Thr Asn Pro Leu Tyr Pro Asp Phe
65                  70                  75                  80

Ser Thr Ser Ala Asn Asn Ile Gln Ile Asp Lys Thr Lys Pro Leu Trp
                85                  90                  95

His Asn Tyr Phe Leu Cys Gly Leu Lys Gly Ile Gln Glu His Phe Gly
            100                 105                 110
Leu Ser Asn Leu Thr Gly Met Asn Cys Leu Val Asp Gly Asn Ile Pro
        115                 120                 125
Pro Ser Ser Gly Leu Ser Ser Ser Ser Ala Leu Val Cys Cys Ala Gly
    130                 135                 140
Leu Val Thr Leu Thr Val Leu Gly Arg Asn Leu Ser Lys Val Glu Leu
145                 150                 155                 160
Ala Glu Ile Cys Ala Lys Ser Glu Arg Tyr Ile Gly Thr Glu Gly Gly
                165                 170                 175
Gly Met Asp Gln Ser Ile Ser Phe Leu Ala Glu Glu Gly Thr Ala Lys
            180                 185                 190
Leu Ile Glu Phe Ser Pro Leu Arg Ala Thr Asp Val Lys Leu Pro Ser
        195                 200                 205
Gly Ala Val Phe Val Ile Ala Asn Ser Cys Val Glu Met Asn Lys Ala
    210                 215                 220
Ala Thr Ser His Phe Asn Ile Arg Val Met Glu Cys Arg Leu Ala Ala
225                 230                 235                 240
Lys Leu Leu Ala Lys Tyr Lys Ser Leu Gln Trp Asp Lys Val Leu Arg
                245                 250                 255
Leu Glu Glu Val Gln Ala Lys Leu Gly Ile Ser Leu Glu Glu Met Leu
            260                 265                 270
Leu Val Thr Glu Asp Ala Leu His Pro Glu Pro Tyr Asn Pro Glu Glu
        275                 280                 285
Ile Cys Arg Cys Leu Gly Ile Ser Leu Glu Glu Leu Arg Thr Gln Ile
    290                 295                 300
Leu Ser Pro Asn Thr Gln Asp Val Leu Ile Phe Lys Leu Tyr Gln Arg
305                 310                 315                 320
Ala Lys His Val Tyr Ser Glu Ala Ala Arg Val Leu Gln Phe Lys Lys
                325                 330                 335
Ile Cys Glu Glu Ala Pro Glu Asn Met Val Gln Leu Leu Gly Glu Leu
            340                 345                 350

Met Asn Gln Ser His Met Ser Cys Arg Asp Met Tyr Glu Cys Ser Cys
        355                 360                 365
Pro Glu Leu Asp Gln Leu Val Asp Ile Cys Arg Lys Phe Gly Ala Gln
    370                 375                 380
Gly Ser Arg Leu Thr Gly Ala Gly Trp Gly Gly Cys Thr Val Ser Met
385                 390                 395                 400
Val Pro Ala Asp Lys Leu Pro Ser Phe Leu Ala Asn Val His Lys Ala
                405                 410                 415
Tyr Tyr Gln Arg Ser Asp Gly Ser Leu Ala Pro Glu Lys Gln Ser Leu
            420                 425                 430
Phe Ala Thr Lys Pro Gly Gly Gly Ala Leu Val Leu Leu Glu Ala
        435                 440                 445

<210> 133
<211> 458
<212> PRT
<213> Homo sapiens
<220>
<221> galactokinase 2 variant 1
<222> (1)..(458)
<223> LocusID: 2585; NM_002044
<400> 133

Met Ala Thr Glu Ser Pro Ala Thr Arg Arg Val Gln Val Ala Glu His
1               5                   10                  15
Pro Arg Leu Leu Lys Leu Lys Glu Met Phe Asn Ser Lys Phe Gly Ser
            20                  25                  30
Ile Pro Lys Phe Tyr Val Arg Ala Pro Gly Arg Val Asn Ile Ile Gly
        35                  40                  45
Glu His Ile Asp Tyr Cys Gly Tyr Ser Val Leu Pro Met Ala Val Glu
    50                  55                  60

Gln Asp Val Leu Ile Ala Val Glu Pro Val Lys Thr Tyr Ala Leu Gln
65                  70                  75                  80
Leu Ala Asn Thr Asn Pro Leu Tyr Pro Asp Phe Ser Thr Ser Ala Asn
                85                  90                  95
Asn Ile Gln Ile Asp Lys Thr Lys Pro Leu Trp His Asn Tyr Phe Leu
            100                 105                 110
Cys Gly Leu Lys Gly Ile Gln Glu His Phe Gly Leu Ser Asn Leu Thr
        115                 120                 125
Gly Met Asn Cys Leu Val Asp Gly Asn Ile Pro Pro Ser Ser Gly Leu
    130                 135                 140
Ser Ser Ser Ser Ala Leu Val Cys Cys Ala Gly Leu Val Thr Leu Thr
145                 150                 155                 160
Val Leu Gly Arg Asn Leu Ser Lys Val Glu Leu Ala Glu Ile Cys Ala
                165                 170                 175
Lys Ser Glu Arg Tyr Ile Gly Thr Glu Gly Gly Gly Met Asp Gln Ser
            180                 185                 190
Ile Ser Phe Leu Ala Glu Glu Gly Thr Ala Lys Leu Ile Glu Phe Ser
        195                 200                 205
Pro Leu Arg Ala Thr Asp Val Lys Leu Pro Ser Gly Ala Val Phe Val
    210                 215                 220
Ile Ala Asn Ser Cys Val Glu Met Asn Lys Ala Ala Thr Ser His Phe
225                 230                 235                 240
Asn Ile Arg Val Met Glu Cys Arg Leu Ala Ala Lys Leu Leu Ala Lys
                245                 250                 255
Tyr Lys Ser Leu Gln Trp Asp Lys Val Leu Arg Leu Glu Glu Val Gln
            260                 265                 270
Ala Lys Leu Gly Ile Ser Leu Glu Glu Met Leu Leu Val Thr Glu Asp
        275                 280                 285
Ala Leu His Pro Glu Pro Tyr Asn Pro Glu Glu Ile Cys Arg Cys Leu
    290                 295                 300
Gly Ile Ser Leu Glu Glu Leu Arg Thr Gln Ile Leu Ser Pro Asn Thr
305                 310                 315                 320

Gln Asp Val Leu Ile Phe Lys Leu Tyr Gln Arg Ala Lys His Val Tyr
                325                 330                 335
Ser Glu Ala Ala Arg Val Leu Gln Phe Lys Lys Ile Cys Glu Glu Ala
            340                 345                 350
Pro Glu Asn Met Val Gln Leu Leu Gly Glu Leu Met Asn Gln Ser His
        355                 360                 365
Met Ser Cys Arg Asp Met Tyr Glu Cys Ser Cys Pro Glu Leu Asp Gln
    370                 375                 380
Leu Val Asp Ile Cys Arg Lys Phe Gly Ala Gln Gly Ser Arg Leu Thr
385                 390                 395                 400
Gly Ala Gly Trp Gly Gly Cys Thr Val Ser Met Val Pro Ala Asp Lys
                405                 410                 415
Leu Pro Ser Phe Leu Ala Asn Val His Lys Ala Tyr Tyr Gln Arg Ser
            420                 425                 430
Asp Gly Ser Leu Ala Pro Glu Lys Gln Ser Leu Phe Ala Thr Lys Pro
        435                 440                 445
Gly Gly Gly Ala Leu Val Leu Leu Glu Ala
    450                 455

<210> 134
<211> 190
<212> PRT
<213> Homo sapiens
<220>
<221> prostaglandin D2 synthase
<222> (1)..(190)
<223> LocusID: 5730; NM_000954
<400> 134

Met Ala Thr His His Thr Leu Trp Met Gly Leu Ala Leu Leu Gly Val
1               5                   10                  15

Leu Gly Asp Leu Gln Ala Ala Pro Glu Ala Gln Val Ser Val Gln Pro
            20                  25                  30
Asn Phe Gln Gln Asp Lys Phe Leu Gly Arg Trp Phe Ser Ala Gly Leu
        35                  40                  45
Ala Ser Asn Ser Ser Trp Leu Arg Glu Lys Lys Ala Ala Leu Ser Met
    50                  55                  60
Cys Lys Ser Val Val Ala Pro Ala Thr Asp Gly Gly Leu Asn Leu Thr
65                  70                  75                  80
Ser Thr Phe Leu Arg Lys Asn Gln Cys Glu Thr Arg Thr Met Leu Leu
                85                  90                  95
Gln Pro Ala Gly Ser Leu Gly Ser Tyr Ser Tyr Arg Ser Pro His Trp
            100                 105                 110
Gly Ser Thr Tyr Ser Val Ser Val Val Glu Thr Asp Tyr Asp Gln Tyr
        115                 120                 125
Ala Leu Leu Tyr Ser Gln Gly Ser Lys Gly Pro Gly Glu Asp Phe Arg
    130                 135                 140
Met Ala Thr Leu Tyr Ser Arg Thr Gln Thr Pro Arg Ala Glu Leu Lys
145                 150                 155                 160
Glu Lys Phe Thr Ala Phe Cys Lys Ala Gln Gly Phe Thr Glu Asp Thr
                165                 170                 175
Ile Val Phe Leu Pro Gln Thr Asp Lys Cys Met Thr Glu Gln
            180                 185                 190

<210> 135
<211> 424
<212> PRT
<213> Homo sapiens
<220>
<221> mitogen-activated protein kinase 9 variant 1
<222> (1)..(424)
<223> LocusID: 5601; NM_002752

<400> 135

Met Ser Asp Ser Lys Cys Asp Ser Gln Phe Tyr Ser Val Gln Val Ala
1               5                   10                  15

Asp Ser Thr Phe Thr Val Leu Lys Arg Tyr Gln Gln Leu Lys Pro Ile
            20                  25                  30

Gly Ser Gly Ala Gln Gly Ile Val Cys Ala Ala Phe Asp Thr Val Leu
        35                  40                  45

Gly Ile Asn Val Ala Val Lys Lys Leu Ser Arg Pro Phe Gln Asn Gln
    50                  55                  60

Thr His Ala Lys Arg Ala Tyr Arg Glu Leu Val Leu Leu Lys Cys Val
65                  70                  75                  80

Asn His Lys Asn Ile Ile Ser Leu Leu Asn Val Phe Thr Pro Gln Lys
                85                  90                  95

Thr Leu Glu Glu Phe Gln Asp Val Tyr Leu Val Met Glu Leu Met Asp
            100                 105                 110

Ala Asn Leu Cys Gln Val Ile His Met Glu Leu Asp His Glu Arg Met
        115                 120                 125

Ser Tyr Leu Leu Tyr Gln Met Leu Cys Gly Ile Lys His Leu His Ser
    130                 135                 140

Ala Gly Ile Ile His Arg Asp Leu Lys Pro Ser Asn Ile Val Val Lys
145                 150                 155                 160

Ser Asp Cys Thr Leu Lys Ile Leu Asp Phe Gly Leu Ala Arg Thr Ala
                165                 170                 175

Cys Thr Asn Phe Met Met Thr Pro Tyr Val Val Thr Arg Tyr Tyr Arg
            180                 185                 190

Ala Pro Glu Val Ile Leu Gly Met Gly Tyr Lys Glu Asn Val Asp Ile
        195                 200                 205

Trp Ser Val Gly Cys Ile Met Gly Glu Leu Val Lys Gly Cys Val Ile
    210                 215                 220

Phe Gln Gly Thr Asp His Ile Asp Gln Trp Asn Lys Val Ile Glu Gln
225                 230                 235                 240

Leu Gly Thr Pro Ser Ala Glu Phe Met Lys Lys Leu Gln Pro Thr Val
                245                 250                 255
Arg Asn Tyr Val Glu Asn Arg Pro Lys Tyr Pro Gly Ile Lys Phe Glu
            260                 265                 270
Glu Leu Phe Pro Asp Trp Ile Phe Pro Ser Glu Ser Glu Arg Asp Lys
        275                 280                 285
Ile Lys Thr Ser Gln Ala Arg Asp Leu Leu Ser Lys Met Leu Val Ile
    290                 295                 300
Asp Pro Asp Lys Arg Ile Ser Val Asp Glu Ala Leu Arg His Pro Tyr
305                 310                 315                 320
Ile Thr Val Trp Tyr Asp Pro Ala Glu Ala Glu Ala Pro Pro Pro Gln
                325                 330                 335
Ile Tyr Asp Ala Gln Leu Glu Glu Arg Glu His Ala Ile Glu Glu Trp
            340                 345                 350
Lys Glu Leu Ile Tyr Lys Glu Val Met Asp Trp Glu Glu Arg Ser Lys
        355                 360                 365
Asn Gly Val Val Lys Asp Gln Pro Ser Asp Ala Ala Val Ser Ser Asn
    370                 375                 380
Ala Thr Pro Ser Gln Ser Ser Ser Ile Asn Asp Ile Ser Ser Met Ser
385                 390                 395                 400
Thr Glu Gln Thr Leu Ala Ser Asp Thr Asp Ser Ser Leu Asp Ala Ser
                405                 410                 415
Thr Gly Pro Leu Glu Gly Cys Arg
            420

<210>  136
<211>  382
<212>  PRT
<213>  Homo sapiens
<220>
<221>  mitogen-activated protein kinase 9 variant 2

<222>  (1)..(382)

<223>  LocusID: 5601; NM_139068

<400>  136

Met Ser Asp Ser Lys Cys Asp Ser Gln Phe Tyr Ser Val Gln Val Ala
1               5                   10                  15

Asp Ser Thr Phe Thr Val Leu Lys Arg Tyr Gln Gln Leu Lys Pro Ile
            20                  25                  30

Gly Ser Gly Ala Gln Gly Ile Val Cys Ala Ala Phe Asp Thr Val Leu
        35                  40                  45

Gly Ile Asn Val Ala Val Lys Lys Leu Ser Arg Pro Phe Gln Asn Gln
    50                  55                  60

Thr His Ala Lys Arg Ala Tyr Arg Glu Leu Val Leu Leu Lys Cys Val
65                  70                  75                  80

Asn His Lys Asn Ile Ile Ser Leu Leu Asn Val Phe Thr Pro Gln Lys
                85                  90                  95

Thr Leu Glu Glu Phe Gln Asp Val Tyr Leu Val Met Glu Leu Met Asp
            100                 105                 110

Ala Asn Leu Cys Gln Val Ile His Met Glu Leu Asp His Glu Arg Met
        115                 120                 125

Ser Tyr Leu Leu Tyr Gln Met Leu Cys Gly Ile Lys His Leu His Ser
    130                 135                 140

Ala Gly Ile Ile His Arg Asp Leu Lys Pro Ser Asn Ile Val Val Lys
145                 150                 155                 160

Ser Asp Cys Thr Leu Lys Ile Leu Asp Phe Gly Leu Ala Arg Thr Ala
                165                 170                 175

Cys Thr Asn Phe Met Met Thr Pro Tyr Val Val Thr Arg Tyr Tyr Arg
            180                 185                 190

Ala Pro Glu Val Ile Leu Gly Met Gly Tyr Lys Glu Asn Val Asp Ile
        195                 200                 205

Trp Ser Val Gly Cys Ile Met Gly Glu Leu Val Lys Gly Cys Val Ile
    210                 215                 220

Phe Gln Gly Thr Asp His Ile Asp Gln Trp Asn Lys Val Ile Glu Gln
225                 230                 235                 240

Leu Gly Thr Pro Ser Ala Glu Phe Met Lys Lys Leu Gln Pro Thr Val
                245                 250                 255

Arg Asn Tyr Val Glu Asn Arg Pro Lys Tyr Pro Gly Ile Lys Phe Glu
            260                 265                 270

Glu Leu Phe Pro Asp Trp Ile Phe Pro Ser Glu Ser Glu Arg Asp Lys
        275                 280                 285

Ile Lys Thr Ser Gln Ala Arg Asp Leu Leu Ser Lys Met Leu Val Ile
    290                 295                 300

Asp Pro Asp Lys Arg Ile Ser Val Asp Glu Ala Leu Arg His Pro Tyr
305                 310                 315                 320

Ile Thr Val Trp Tyr Asp Pro Ala Glu Ala Glu Ala Pro Pro Pro Gln
                325                 330                 335

Ile Tyr Asp Ala Gln Leu Glu Glu Arg Glu His Ala Ile Glu Glu Trp
            340                 345                 350

Lys Glu Leu Ile Tyr Lys Glu Val Met Asp Trp Glu Glu Arg Ser Lys
        355                 360                 365

Asn Gly Val Val Lys Asp Gln Pro Ser Ala Gln Met Gln Gln
    370                 375                 380

<210>  137

<211>  382

<212>  PRT

<213>  Homo sapiens

<220>

<221>  mitogen-activated protein kinase 9 variant 3

<222>  (1)..(382)

<223>  LocusID: 5601; NM_139069

<400>  137

Met Ser Asp Ser Lys Cys Asp Ser Gln Phe Tyr Ser Val Gln Val Ala
1               5                   10                  15
Asp Ser Thr Phe Thr Val Leu Lys Arg Tyr Gln Gln Leu Lys Pro Ile
            20                  25                  30
Gly Ser Gly Ala Gln Gly Ile Val Cys Ala Ala Phe Asp Thr Val Leu
        35                  40                  45
Gly Ile Asn Val Ala Val Lys Lys Leu Ser Arg Pro Phe Gln Asn Gln
    50                  55                  60
Thr His Ala Lys Arg Ala Tyr Arg Glu Leu Val Leu Leu Lys Cys Val
65                  70                  75                  80
Asn His Lys Asn Ile Ile Ser Leu Leu Asn Val Phe Thr Pro Gln Lys
                85                  90                  95
Thr Leu Glu Glu Phe Gln Asp Val Tyr Leu Val Met Glu Leu Met Asp
            100                 105                 110
Ala Asn Leu Cys Gln Val Ile His Met Glu Leu Asp His Glu Arg Met
        115                 120                 125
Ser Tyr Leu Leu Tyr Gln Met Leu Cys Gly Ile Lys His Leu His Ser
    130                 135                 140
Ala Gly Ile Ile His Arg Asp Leu Lys Pro Ser Asn Ile Val Val Lys
145                 150                 155                 160
Ser Asp Cys Thr Leu Lys Ile Leu Asp Phe Gly Leu Ala Arg Thr Ala
                165                 170                 175
Cys Thr Asn Phe Met Met Thr Pro Tyr Val Val Thr Arg Tyr Tyr Arg
            180                 185                 190
Ala Pro Glu Val Ile Leu Gly Met Gly Tyr Lys Glu Asn Val Asp Ile
        195                 200                 205
Trp Ser Val Gly Cys Ile Met Ala Glu Met Val Leu His Lys Val Leu
    210                 215                 220
Phe Pro Gly Arg Asp Tyr Ile Asp Gln Trp Asn Lys Val Ile Glu Gln
225                 230                 235                 240
Leu Gly Thr Pro Ser Ala Glu Phe Met Lys Lys Leu Gln Pro Thr Val
                245                 250                 255

Arg Asn Tyr Val Glu Asn Arg Pro Lys Tyr Pro Gly Ile Lys Phe Glu
            260                 265                 270
Glu Leu Phe Pro Asp Trp Ile Phe Pro Ser Glu Ser Glu Arg Asp Lys
        275                 280                 285
Ile Lys Thr Ser Gln Ala Arg Asp Leu Leu Ser Lys Met Leu Val Ile
    290                 295                 300
Asp Pro Asp Lys Arg Ile Ser Val Asp Glu Ala Leu Arg His Pro Tyr
305                 310                 315                 320
Ile Thr Val Trp Tyr Asp Pro Ala Glu Ala Glu Ala Pro Pro Pro Gln
                325                 330                 335
Ile Tyr Asp Ala Gln Leu Glu Glu Arg Glu His Ala Ile Glu Glu Trp
            340                 345                 350
Lys Glu Leu Ile Tyr Lys Glu Val Met Asp Trp Glu Glu Arg Ser Lys
        355                 360                 365
Asn Gly Val Val Lys Asp Gln Pro Ser Ala Gln Met Gln Gln
    370                 375                 380

<210> 138
<211> 424
<212> PRT
<213> Homo sapiens
<220>
<221> mitogen-activated protein kinase 9 variant 4
<222> (1)..(424)
<223> LocusID: 5601; NM_139070
<400> 138

Met Ser Asp Ser Lys Cys Asp Ser Gln Phe Tyr Ser Val Gln Val Ala
1               5                   10                  15
Asp Ser Thr Phe Thr Val Leu Lys Arg Tyr Gln Gln Leu Lys Pro Ile
            20                  25                  30

Gly Ser Gly Ala Gln Gly Ile Val Cys Ala Ala Phe Asp Thr Val Leu
        35                  40                  45
Gly Ile Asn Val Ala Val Lys Lys Leu Ser Arg Pro Phe Gln Asn Gln
    50                  55                  60
Thr His Ala Lys Arg Ala Tyr Arg Glu Leu Val Leu Leu Lys Cys Val
65                  70                  75                  80
Asn His Lys Asn Ile Ile Ser Leu Leu Asn Val Phe Thr Pro Gln Lys
                85                  90                  95
Thr Leu Glu Glu Phe Gln Asp Val Tyr Leu Val Met Glu Leu Met Asp
            100                 105                 110
Ala Asn Leu Cys Gln Val Ile His Met Glu Leu Asp His Glu Arg Met
        115                 120                 125
Ser Tyr Leu Leu Tyr Gln Met Leu Cys Gly Ile Lys His Leu His Ser
    130                 135                 140
Ala Gly Ile Ile His Arg Asp Leu Lys Pro Ser Asn Ile Val Val Lys
145                 150                 155                 160
Ser Asp Cys Thr Leu Lys Ile Leu Asp Phe Gly Leu Ala Arg Thr Ala
                165                 170                 175
Cys Thr Asn Phe Met Met Thr Pro Tyr Val Val Thr Arg Tyr Tyr Arg
            180                 185                 190
Ala Pro Glu Val Ile Leu Gly Met Gly Tyr Lys Glu Asn Val Asp Ile
        195                 200                 205
Trp Ser Val Gly Cys Ile Met Ala Glu Met Val Leu His Lys Val Leu
    210                 215                 220
Phe Pro Gly Arg Asp Tyr Ile Asp Gln Trp Asn Lys Val Ile Glu Gln
225                 230                 235                 240
Leu Gly Thr Pro Ser Ala Glu Phe Met Lys Lys Leu Gln Pro Thr Val
                245                 250                 255
Arg Asn Tyr Val Glu Asn Arg Pro Lys Tyr Pro Gly Ile Lys Phe Glu
            260                 265                 270
Glu Leu Phe Pro Asp Trp Ile Phe Pro Ser Glu Ser Glu Arg Asp Lys
        275                 280                 285

Ile Lys Thr Ser Gln Ala Arg Asp Leu Leu Ser Lys Met Leu Val Ile
    290                 295                 300
Asp Pro Asp Lys Arg Ile Ser Val Asp Glu Ala Leu Arg His Pro Tyr
305                 310                 315                 320
Ile Thr Val Trp Tyr Asp Pro Ala Glu Ala Glu Ala Pro Pro Pro Gln
                325                 330                 335
Ile Tyr Asp Ala Gln Leu Glu Glu Arg Glu His Ala Ile Glu Glu Trp
            340                 345                 350
Lys Glu Leu Ile Tyr Lys Glu Val Met Asp Trp Glu Glu Arg Ser Lys
        355                 360                 365
Asn Gly Val Val Lys Asp Gln Pro Ser Asp Ala Ala Val Ser Ser Asn
    370                 375                 380
Ala Thr Pro Ser Gln Ser Ser Ser Ile Asn Asp Ile Ser Ser Met Ser
385                 390                 395                 400
Thr Glu Gln Thr Leu Ala Ser Asp Thr Asp Ser Ser Leu Asp Ala Ser
                405                 410                 415
Thr Gly Pro Leu Glu Gly Cys Arg
            420

<210> 139
<211> 162
<212> PRT
<213> Homo sapiens
<220>
<221> interleukin 15 variant 3
<222> (1)..(162)
<223> LocusID: 3600; NM_000585
<400> 139

Met Arg Ile Ser Lys Pro His Leu Arg Ser Ile Ser Ile Gln Cys Tyr
1               5                   10                  15

Leu Cys Leu Leu Leu Asn Ser His Phe Leu Thr Glu Ala Gly Ile His
            20                  25                  30
Val Phe Ile Leu Gly Cys Phe Ser Ala Gly Leu Pro Lys Thr Glu Ala
        35                  40                  45
Asn Trp Val Asn Val Ile Ser Asp Leu Lys Lys Ile Glu Asp Leu Ile
    50                  55                  60
Gln Ser Met His Ile Asp Ala Thr Leu Tyr Thr Glu Ser Asp Val His
65                  70                  75                  80
Pro Ser Cys Lys Val Thr Ala Met Lys Cys Phe Leu Leu Glu Leu Gln
                85                  90                  95
Val Ile Ser Leu Glu Ser Gly Asp Ala Ser Ile His Asp Thr Val Glu
            100                 105                 110
Asn Leu Ile Ile Leu Ala Asn Asn Ser Leu Ser Ser Asn Gly Asn Val
        115                 120                 125
Thr Glu Ser Gly Cys Lys Glu Cys Glu Glu Leu Glu Glu Lys Asn Ile
    130                 135                 140
Lys Glu Phe Leu Gln Ser Phe Val His Ile Val Gln Met Phe Ile Asn
145                 150                 155                 160
Thr Ser

<210> 140
<211> 162
<212> PRT
<213> Homo sapiens
<220>
<221> interleukin 15 variant 1
<222> (1)..(162)
<223> LocusID: 3600; NM_172174
<400> 140

Met Arg Ile Ser Lys Pro His Leu Arg Ser Ile Ser Ile Gln Cys Tyr
1               5                   10                  15

Leu Cys Leu Leu Leu Asn Ser His Phe Leu Thr Glu Ala Gly Ile His
            20                  25                  30

Val Phe Ile Leu Gly Cys Phe Ser Ala Gly Leu Pro Lys Thr Glu Ala
        35                  40                  45

Asn Trp Val Asn Val Ile Ser Asp Leu Lys Lys Ile Glu Asp Leu Ile
    50                  55                  60

Gln Ser Met His Ile Asp Ala Thr Leu Tyr Thr Glu Ser Asp Val His
65                  70                  75                  80

Pro Ser Cys Lys Val Thr Ala Met Lys Cys Phe Leu Leu Glu Leu Gln
                85                  90                  95

Val Ile Ser Leu Glu Ser Gly Asp Ala Ser Ile His Asp Thr Val Glu
            100                 105                 110

Asn Leu Ile Ile Leu Ala Asn Asn Ser Leu Ser Ser Asn Gly Asn Val
        115                 120                 125

Thr Glu Ser Gly Cys Lys Glu Cys Glu Glu Leu Glu Glu Lys Asn Ile
    130                 135                 140

Lys Glu Phe Leu Gln Ser Phe Val His Ile Val Gln Met Phe Ile Asn
145                 150                 155                 160

Thr Ser

<210>  141

<211>  135

<212>  PRT

<213>  Homo sapiens

<220>

<221>  interleukin 15 variant 2

<222>  (1)..(135)

<223>  LocusID: 3600; NM_172175

<400>  141

Met Val Leu Gly Thr Ile Asp Leu Cys Ser Cys Phe Ser Ala Gly Leu
1               5                   10                  15

Pro Lys Thr Glu Ala Asn Trp Val Asn Val Ile Ser Asp Leu Lys Lys
            20                  25                  30

Ile Glu Asp Leu Ile Gln Ser Met His Ile Asp Ala Thr Leu Tyr Thr
        35                  40                  45

Glu Ser Asp Val His Pro Ser Cys Lys Val Thr Ala Met Lys Cys Phe
    50                  55                  60

Leu Leu Glu Leu Gln Val Ile Ser Leu Glu Ser Gly Asp Ala Ser Ile
65                  70                  75                  80

His Asp Thr Val Glu Asn Leu Ile Ile Leu Ala Asn Asn Ser Leu Ser
                85                  90                  95

Ser Asn Gly Asn Val Thr Glu Ser Gly Cys Lys Glu Cys Glu Glu Leu
            100                 105                 110

Glu Glu Lys Asn Ile Lys Glu Phe Leu Gln Ser Phe Val His Ile Val
        115                 120                 125

Gln Met Phe Ile Asn Thr Ser
    130                 135

<210>  142
<211>  824
<212>  PRT
<213>  Homo sapiens
<220>
<221>  mucosa associated lymphoid tissue lymphoma translocation gene 1
       variant 1
<222>  (1)..(824)
<223>  LocusID: 10892; NM_006785
<400>  142

Met Ser Leu Leu Gly Asp Pro Leu Gln Ala Leu Pro Pro Ser Ala Ala
1               5                   10                  15
Pro Thr Gly Pro Leu Leu Ala Pro Pro Ala Gly Ala Thr Leu Asn Arg
            20                  25                  30
Leu Arg Glu Pro Leu Leu Arg Arg Leu Ser Glu Leu Leu Asp Gln Ala
        35                  40                  45
Pro Glu Gly Arg Gly Trp Arg Arg Leu Ala Glu Leu Ala Gly Ser Arg
    50                  55                  60
Gly Arg Leu Arg Leu Ser Cys Leu Asp Leu Glu Gln Cys Ser Leu Lys
65                  70                  75                  80
Val Leu Glu Pro Glu Gly Ser Pro Ser Leu Cys Leu Leu Lys Leu Met
                85                  90                  95
Gly Glu Lys Gly Cys Thr Val Thr Glu Leu Ser Asp Phe Leu Gln Ala
            100                 105                 110
Met Glu His Thr Glu Val Leu Gln Leu Leu Ser Pro Pro Gly Ile Lys
        115                 120                 125
Ile Thr Val Asn Pro Glu Ser Lys Ala Val Leu Ala Gly Gln Phe Val
    130                 135                 140
Lys Leu Cys Cys Arg Ala Thr Gly His Pro Phe Val Gln Tyr Gln Trp
145                 150                 155                 160
Phe Lys Met Asn Lys Glu Ile Pro Asn Gly Asn Thr Ser Glu Leu Ile
                165                 170                 175
Phe Asn Ala Val His Val Lys Asp Ala Gly Phe Tyr Val Cys Arg Val
            180                 185                 190
Asn Asn Asn Phe Thr Phe Glu Phe Ser Gln Trp Ser Gln Leu Asp Val
        195                 200                 205
Cys Asp Ile Pro Glu Ser Phe Gln Arg Ser Val Asp Gly Val Ser Glu
    210                 215                 220
Ser Lys Leu Gln Ile Cys Val Glu Pro Thr Ser Gln Lys Leu Met Pro
225                 230                 235                 240
Gly Ser Thr Leu Val Leu Gln Cys Val Ala Val Gly Ser Pro Ile Pro

245                 250                 255

His Tyr Gln Trp Phe Lys Asn Glu Leu Pro Leu Thr His Glu Thr Lys

            260                 265                 270

Lys Leu Tyr Met Val Pro Tyr Val Asp Leu Glu His Gln Gly Thr Tyr

        275                 280                 285

Trp Cys His Val Tyr Asn Asp Arg Asp Ser Gln Asp Ser Lys Lys Val

    290                 295                 300

Glu Ile Ile Ile Gly Arg Thr Asp Glu Ala Val Glu Cys Thr Glu Asp

305                 310                 315                 320

Glu Leu Asn Asn Leu Gly His Pro Asp Asn Lys Glu Gln Thr Thr Asp

                325                 330                 335

Gln Pro Leu Ala Lys Asp Lys Val Ala Leu Leu Ile Gly Asn Met Asn

            340                 345                 350

Tyr Arg Glu His Pro Lys Leu Lys Ala Pro Leu Val Asp Val Tyr Glu

        355                 360                 365

Leu Thr Asn Leu Leu Arg Gln Leu Asp Phe Lys Val Val Ser Leu Leu

    370                 375                 380

Asp Leu Thr Glu Tyr Glu Met Arg Asn Ala Val Asp Glu Phe Leu Leu

385                 390                 395                 400

Leu Leu Asp Lys Gly Val Tyr Gly Leu Leu Tyr Tyr Ala Gly His Gly

                405                 410                 415

Tyr Glu Asn Phe Gly Asn Ser Phe Met Val Pro Val Asp Ala Pro Asn

            420                 425                 430

Pro Tyr Arg Ser Glu Asn Cys Leu Cys Val Gln Asn Ile Leu Lys Leu

        435                 440                 445

Met Gln Glu Lys Glu Thr Gly Leu Asn Val Phe Leu Leu Asp Met Cys

    450                 455                 460

Arg Lys Arg Asn Asp Tyr Asp Asp Thr Ile Pro Ile Leu Asp Ala Leu

465                 470                 475                 480

Lys Val Thr Ala Asn Ile Val Phe Gly Tyr Ala Thr Cys Gln Gly Ala

                485                 490                 495

Glu Ala Phe Glu Ile Gln His Ser Gly Leu Ala Asn Gly Ile Phe Met

            500                 505                 510
Lys Phe Leu Lys Asp Arg Leu Leu Glu Asp Lys Lys Ile Thr Val Leu
        515                 520                 525
Leu Asp Glu Val Ala Glu Asp Met Gly Lys Cys His Leu Thr Lys Gly
    530                 535                 540
Lys Gln Ala Leu Glu Ile Arg Ser Ser Leu Ser Glu Lys Arg Ala Leu
545                 550                 555                 560
Thr Asp Pro Ile Gln Gly Thr Glu Tyr Ser Ala Glu Ser Leu Val Arg
                565                 570                 575
Asn Leu Gln Trp Ala Lys Ala His Glu Leu Pro Glu Ser Met Cys Leu
            580                 585                 590
Lys Phe Asp Cys Gly Val Gln Ile Gln Leu Gly Phe Ala Ala Glu Phe
        595                 600                 605
Ser Asn Val Met Ile Ile Tyr Thr Ser Ile Val Tyr Lys Pro Pro Glu
    610                 615                 620
Ile Ile Met Cys Asp Ala Tyr Val Thr Asp Phe Pro Leu Asp Leu Asp
625                 630                 635                 640
Ile Asp Pro Lys Asp Ala Asn Lys Gly Thr Pro Glu Glu Thr Gly Ser
                645                 650                 655
Tyr Leu Val Ser Lys Asp Leu Pro Lys His Cys Leu Tyr Thr Arg Leu
            660                 665                 670
Ser Ser Leu Gln Lys Leu Lys Glu His Leu Val Phe Thr Val Cys Leu
        675                 680                 685
Ser Tyr Gln Tyr Ser Gly Leu Glu Asp Thr Val Glu Asp Lys Gln Glu
    690                 695                 700
Val Asn Val Gly Lys Pro Leu Ile Ala Lys Leu Asp Met His Arg Gly
705                 710                 715                 720
Leu Gly Arg Lys Thr Cys Phe Gln Thr Cys Leu Met Ser Asn Gly Pro
                725                 730                 735
Tyr Gln Ser Ser Ala Ala Thr Ser Gly Gly Ala Gly His Tyr His Ser
            740                 745                 750
Leu Gln Asp Pro Phe His Gly Val Tyr His Ser His Pro Gly Asn Pro

755                 760                 765

Ser Asn Val Thr Pro Ala Asp Ser Cys His Cys Ser Arg Thr Pro Asp

    770                 775                 780

Ala Phe Ile Ser Ser Phe Ala His His Ala Ser Cys His Phe Ser Arg

785                 790                 795                 800

Ser Asn Val Pro Val Glu Thr Thr Asp Glu Ile Pro Phe Ser Phe Ser

                805                 810                 815

Asp Arg Leu Arg Ile Ser Glu Lys

            820

<210>  143

<211>  813

<212>  PRT

<213>  Homo sapiens

<220>

<221>  mucosa associated lymphoid tissue lymphoma translocation gene 1
       variant 2

<222>  (1)..(813)

<223>  LocusID: 10892; NM_173844

<400>  143

Met Ser Leu Leu Gly Asp Pro Leu Gln Ala Leu Pro Pro Ser Ala Ala

1               5                   10                  15

Pro Thr Gly Pro Leu Leu Ala Pro Pro Ala Gly Ala Thr Leu Asn Arg

            20                  25                  30

Leu Arg Glu Pro Leu Leu Arg Arg Leu Ser Glu Leu Leu Asp Gln Ala

        35                  40                  45

Pro Glu Gly Arg Gly Trp Arg Arg Leu Ala Glu Leu Ala Gly Ser Arg

    50                  55                  60

Gly Arg Leu Arg Leu Ser Cys Leu Asp Leu Glu Gln Cys Ser Leu Lys

65                  70                  75                  80

Val Leu Glu Pro Glu Gly Ser Pro Ser Leu Cys Leu Leu Lys Leu Met
                85                  90                  95
Gly Glu Lys Gly Cys Thr Val Thr Glu Leu Ser Asp Phe Leu Gln Ala
            100                 105                 110
Met Glu His Thr Glu Val Leu Gln Leu Leu Ser Pro Pro Gly Ile Lys
        115                 120                 125
Ile Thr Val Asn Pro Glu Ser Lys Ala Val Leu Ala Gly Gln Phe Val
    130                 135                 140
Lys Leu Cys Cys Arg Ala Thr Gly His Pro Phe Val Gln Tyr Gln Trp
145                 150                 155                 160
Phe Lys Met Asn Lys Glu Ile Pro Asn Gly Asn Thr Ser Glu Leu Ile
                165                 170                 175
Phe Asn Ala Val His Val Lys Asp Ala Gly Phe Tyr Val Cys Arg Val
            180                 185                 190
Asn Asn Asn Phe Thr Phe Glu Phe Ser Gln Trp Ser Gln Leu Asp Val
        195                 200                 205
Cys Asp Ile Pro Glu Ser Phe Gln Arg Ser Val Asp Gly Val Ser Glu
    210                 215                 220
Ser Lys Leu Gln Ile Cys Val Glu Pro Thr Ser Gln Lys Leu Met Pro
225                 230                 235                 240
Gly Ser Thr Leu Val Leu Gln Cys Val Ala Val Gly Ser Pro Ile Pro
                245                 250                 255
His Tyr Gln Trp Phe Lys Asn Glu Leu Pro Leu Thr His Glu Thr Lys
            260                 265                 270
Lys Leu Tyr Met Val Pro Tyr Val Asp Leu Glu His Gln Gly Thr Tyr
        275                 280                 285
Trp Cys His Val Tyr Asn Asp Arg Asp Ser Gln Asp Ser Lys Lys Val
    290                 295                 300
Glu Ile Ile Ile Asp Glu Leu Asn Asn Leu Gly His Pro Asp Asn Lys
305                 310                 315                 320
Glu Gln Thr Thr Asp Gln Pro Leu Ala Lys Asp Lys Val Ala Leu Leu
                325                 330                 335

Ile Gly Asn Met Asn Tyr Arg Glu His Pro Lys Leu Lys Ala Pro Leu
            340                 345                 350
Val Asp Val Tyr Glu Leu Thr Asn Leu Leu Arg Gln Leu Asp Phe Lys
        355                 360                 365
Val Val Ser Leu Leu Asp Leu Thr Glu Tyr Glu Met Arg Asn Ala Val
    370                 375                 380
Asp Glu Phe Leu Leu Leu Leu Asp Lys Gly Val Tyr Gly Leu Leu Tyr
385                 390                 395                 400
Tyr Ala Gly His Gly Tyr Glu Asn Phe Gly Asn Ser Phe Met Val Pro
                405                 410                 415
Val Asp Ala Pro Asn Pro Tyr Arg Ser Glu Asn Cys Leu Cys Val Gln
            420                 425                 430
Asn Ile Leu Lys Leu Met Gln Glu Lys Glu Thr Gly Leu Asn Val Phe
        435                 440                 445
Leu Leu Asp Met Cys Arg Lys Arg Asn Asp Tyr Asp Asp Thr Ile Pro
    450                 455                 460
Ile Leu Asp Ala Leu Lys Val Thr Ala Asn Ile Val Phe Gly Tyr Ala
465                 470                 475                 480
Thr Cys Gln Gly Ala Glu Ala Phe Glu Ile Gln His Ser Gly Leu Ala
                485                 490                 495
Asn Gly Ile Phe Met Lys Phe Leu Lys Asp Arg Leu Leu Glu Asp Lys
            500                 505                 510
Lys Ile Thr Val Leu Leu Asp Glu Val Ala Glu Asp Met Gly Lys Cys
        515                 520                 525
His Leu Thr Lys Gly Lys Gln Ala Leu Glu Ile Arg Ser Ser Leu Ser
    530                 535                 540
Glu Lys Arg Ala Leu Thr Asp Pro Ile Gln Gly Thr Glu Tyr Ser Ala
545                 550                 555                 560
Glu Ser Leu Val Arg Asn Leu Gln Trp Ala Lys Ala His Glu Leu Pro
                565                 570                 575
Glu Ser Met Cys Leu Lys Phe Asp Cys Gly Val Gln Ile Gln Leu Gly
            580                 585                 590

Phe Ala Ala Glu Phe Ser Asn Val Met Ile Ile Tyr Thr Ser Ile Val
        595                 600                 605
Tyr Lys Pro Pro Glu Ile Ile Met Cys Asp Ala Tyr Val Thr Asp Phe
    610                 615                 620
Pro Leu Asp Leu Asp Ile Asp Pro Lys Asp Ala Asn Lys Gly Thr Pro
625                 630                 635                 640
Glu Glu Thr Gly Ser Tyr Leu Val Ser Lys Asp Leu Pro Lys His Cys
                645                 650                 655
Leu Tyr Thr Arg Leu Ser Ser Leu Gln Lys Leu Lys Glu His Leu Val
            660                 665                 670
Phe Thr Val Cys Leu Ser Tyr Gln Tyr Ser Gly Leu Glu Asp Thr Val
        675                 680                 685
Glu Asp Lys Gln Glu Val Asn Val Gly Lys Pro Leu Ile Ala Lys Leu
    690                 695                 700
Asp Met His Arg Gly Leu Gly Arg Lys Thr Cys Phe Gln Thr Cys Leu
705                 710                 715                 720
Met Ser Asn Gly Pro Tyr Gln Ser Ser Ala Ala Thr Ser Gly Gly Ala
                725                 730                 735
Gly His Tyr His Ser Leu Gln Asp Pro Phe His Gly Val Tyr His Ser
            740                 745                 750
His Pro Gly Asn Pro Ser Asn Val Thr Pro Ala Asp Ser Cys His Cys
        755                 760                 765
Ser Arg Thr Pro Asp Ala Phe Ile Ser Ser Phe Ala His His Ala Ser
    770                 775                 780
Cys His Phe Ser Arg Ser Asn Val Pro Val Glu Thr Thr Asp Glu Ile
785                 790                 795                 800
Pro Phe Ser Phe Ser Asp Arg Leu Arg Ile Ser Glu Lys
                805                 810

<210>  144
<211>  417

<212>  PRT

<213>  Homo sapiens

<220>

<221>  lysyl oxidase

<222>  (1)..(417)

<223>  LocusID: 4015; NM_002317

<400>  144

<210> 145

<211> 799

<212> PRT

<213> Homo sapiens

<220>

<221> integrin, beta 5

<222> (1)..(799)

<223> LocusID: 3693; NM_002213

<400> 145

Met Pro Arg Ala Pro Ala Pro Leu Tyr Ala Cys Leu Leu Gly Leu Cys
1               5                   10                  15

Ala Leu Leu Pro Arg Leu Ala Gly Leu Asn Ile Cys Thr Ser Gly Ser
            20                  25                  30

Ala Thr Ser Cys Glu Glu Cys Leu Leu Ile His Pro Lys Cys Ala Trp
        35                  40                  45

Cys Ser Lys Glu Asp Phe Gly Ser Pro Arg Ser Ile Thr Ser Arg Cys
    50                  55                  60

Asp Leu Arg Ala Asn Leu Val Lys Asn Gly Cys Gly Gly Glu Ile Glu
65                  70                  75                  80

Ser Pro Ala Ser Ser Phe His Val Leu Arg Ser Leu Pro Leu Ser Ser
                85                  90                  95

Lys Gly Ser Gly Ser Ala Gly Trp Asp Val Ile Gln Met Thr Pro Gln
            100                 105                 110

Glu Ile Ala Val Asn Leu Arg Pro Gly Asp Lys Thr Thr Phe Gln Leu
        115                 120                 125

Gln Val Arg Gln Val Glu Asp Tyr Pro Val Asp Leu Tyr Tyr Leu Met
    130                 135                 140

Asp Leu Ser Leu Ser Met Lys Asp Asp Leu Asp Asn Ile Arg Ser Leu
145                 150                 155                 160

Gly Thr Lys Leu Ala Glu Glu Met Arg Lys Leu Thr Ser Asn Phe Arg
                165                 170                 175

Leu Gly Phe Gly Ser Phe Val Asp Lys Asp Ile Ser Pro Phe Ser Tyr
            180                 185                 190
Thr Ala Pro Arg Tyr Gln Thr Asn Pro Cys Ile Gly Tyr Lys Leu Phe
        195                 200                 205
Pro Asn Cys Val Pro Ser Phe Gly Phe Arg His Leu Leu Pro Leu Thr
    210                 215                 220
Asp Arg Val Asp Ser Phe Asn Glu Glu Val Arg Lys Gln Arg Val Ser
225                 230                 235                 240
Arg Asn Arg Asp Ala Pro Glu Gly Gly Phe Asp Ala Val Leu Gln Ala
                245                 250                 255
Ala Val Cys Lys Glu Lys Ile Gly Trp Arg Lys Asp Ala Leu His Leu
            260                 265                 270
Leu Val Phe Thr Thr Asp Asp Val Pro His Ile Ala Leu Asp Gly Lys
        275                 280                 285
Leu Gly Gly Leu Val Gln Pro His Asp Gly Gln Cys His Leu Asn Glu
    290                 295                 300
Ala Asn Glu Tyr Thr Ala Ser Asn Gln Met Asp Tyr Pro Ser Leu Ala
305                 310                 315                 320
Leu Leu Gly Glu Lys Leu Ala Glu Asn Asn Ile Asn Leu Ile Phe Ala
                325                 330                 335
Val Thr Lys Asn His Tyr Met Leu Tyr Lys Asn Phe Thr Ala Leu Ile
            340                 345                 350
Pro Gly Thr Thr Val Glu Ile Leu Asp Gly Asp Ser Lys Asn Ile Ile
        355                 360                 365
Gln Leu Ile Ile Asn Ala Tyr Asn Ser Ile Arg Ser Lys Val Glu Leu
    370                 375                 380
Ser Val Trp Asp Gln Pro Glu Asp Leu Asn Leu Phe Phe Thr Ala Thr
385                 390                 395                 400
Cys Gln Asp Gly Val Ser Tyr Pro Gly Gln Arg Lys Cys Glu Gly Leu
                405                 410                 415
Lys Ile Gly Asp Thr Ala Ser Phe Glu Val Ser Leu Glu Ala Arg Ser
            420                 425                 430

Cys Pro Ser Arg His Thr Glu His Val Phe Ala Leu Arg Pro Val Gly
        435                 440                 445
Phe Arg Asp Ser Leu Glu Val Gly Val Thr Tyr Asn Cys Thr Cys Gly
    450                 455                 460
Cys Ser Val Gly Leu Glu Pro Asn Ser Ala Arg Cys Asn Gly Ser Gly
465                 470                 475                 480
Thr Tyr Val Cys Gly Leu Cys Glu Cys Ser Pro Gly Tyr Leu Gly Thr
                485                 490                 495
Arg Cys Glu Cys Gln Asp Gly Glu Asn Gln Ser Val Tyr Gln Asn Leu
            500                 505                 510
Cys Arg Glu Ala Glu Gly Lys Pro Leu Cys Ser Gly Arg Gly Asp Cys
        515                 520                 525
Ser Cys Asn Gln Cys Ser Cys Phe Glu Ser Glu Phe Gly Lys Ile Tyr
    530                 535                 540
Gly Pro Phe Cys Glu Cys Asp Asn Phe Ser Cys Ala Arg Asn Lys Gly
545                 550                 555                 560
Val Leu Cys Ser Gly His Gly Glu Cys His Cys Gly Glu Cys Lys Cys
                565                 570                 575
His Ala Gly Tyr Ile Gly Asp Asn Cys Asn Cys Ser Thr Asp Ile Ser
            580                 585                 590
Thr Cys Arg Gly Arg Asp Gly Gln Ile Cys Ser Glu Arg Gly His Cys
        595                 600                 605
Leu Cys Gly Gln Cys Gln Cys Thr Glu Pro Gly Ala Phe Gly Glu Met
    610                 615                 620
Cys Glu Lys Cys Pro Thr Cys Pro Asp Ala Cys Ser Thr Lys Arg Asp
625                 630                 635                 640
Cys Val Glu Cys Leu Leu Leu His Ser Gly Lys Pro Asp Asn Gln Thr
                645                 650                 655
Cys His Ser Leu Cys Arg Asp Glu Val Ile Thr Trp Val Asp Thr Ile
            660                 665                 670
Val Lys Asp Asp Gln Glu Ala Val Leu Cys Phe Tyr Lys Thr Ala Lys
        675                 680                 685

Asp Cys Val Met Met Phe Thr Tyr Val Glu Leu Pro Ser Gly Lys Ser
    690                 695                 700
Asn Leu Thr Val Leu Arg Glu Pro Glu Cys Gly Asn Thr Pro Asn Ala
705                 710                 715                 720
Met Thr Ile Leu Leu Ala Val Val Gly Ser Ile Leu Leu Val Gly Leu
                725                 730                 735
Ala Leu Leu Ala Ile Trp Lys Leu Leu Val Thr Ile His Asp Arg Arg
            740                 745                 750
Glu Phe Ala Lys Phe Gln Ser Glu Arg Ser Arg Ala Arg Tyr Glu Met
        755                 760                 765
Ala Ser Asn Pro Leu Tyr Arg Lys Pro Ile Ser Thr His Thr Val Asp
    770                 775                 780
Phe Thr Phe Asn Lys Phe Asn Lys Ser Tyr Asn Gly Thr Val Asp
785                 790                 795

<210> 146
<211> 485
<212> PRT
<213> Homo sapiens
<220>
<221> aldehyde dehydrogenase 3 family, member A2
<222> (1)..(485)
<223> LocusID: 224; NM_000382
<400> 146

## Description

Multifactorial diseases such as obesity are caused by mutations in more than one gene with a large contribution from environmental factors. There has been spectacular success in identifying the genes responsible for Mendelian disorders, whereas finding the susceptibility genes involved in multifactorial diseases has so far been difficult. The evidence suggests that humans inherit a genetic predisposition to gain weight on a high fat diet. Therefore optimizing patient sampling for the collection of tissues on the bases of clinical and physiological parameters is critical.

There is clearly an unmet medical need for novel therapeutic solutions to this health problem, in particular in the light of the fact that current medication that promote weight loss are transient as the lost excess of weight is gained back within 1 to 5 years. Therefore, there is a need to identify new targets for the development of new treatments.

### Description of the invention

The invention provides methods (also referred to herein as "screening assays") for identifying compounds which can be used for the modulation of body weight, e.g., for the treatment of a body weight disorder.

A set of 8000 patients, enrolled in a Diabetes/Obesity Prevention Program with the Stockholm Prevention Program, was monitored for a number of clinical parameters and vital signs. From this large pool of patients, a clinically well annotated series of tissue biopsies from 10 obese, non diabetic, and 10 matched control patients were analyzed for gene expression profiling. The following matched clinical parameters and vital signs were, among others, used to recruit these patients: BMI (control mean=22.2 sd+/-1.3 and case mean=32.8 sd+/-2.1), age (control mean=54.6 years and case mean=56.3 years), male gender, VO2 ratio, total fat versus truncal fat, waist-hip ratio, energy expediture, blood pressure, FA oxidation. CHO oxydation, OGTT negative, birth weight, no diabetes in the family, no smoking, sedentary and no alcohol habits.

**Table 1**

| Obese | Control |
|---|---|
| BMI 30-35 | BMI 20-23 |
| without impaired OGTT | without impaired OGTT |
| without type 2 diabetes | without type 2 diabetes |

Other parameters used for patient selection:
* family history of diabetes
* birth weight
* blood pressure
* medication (if any)
* food intake
* physical activity education
* bodyweight history
* chronic illness
* tobacco and alcohol use
* housing conditions
* socio-economical factors

The methods provided by this invention entail identifying candidate or test compounds or agents (e.g., peptides, peptidomimetics, small molecules or other drugs) which bind a polypeptide selected from the group consisting of the polypeptides of Seq ID No. 13 to 24, or a polypeptide selected from the group consisting of the polypeptides of Seq ID No.86 to 146 and/or have a stimulatory or inhibitory effect on the activity or the expression of a polypeptide selected from the group consisting of the polypeptides of Seq ID No. 13 to 24, or a polypeptide selected from the group consisting of the polypeptides of Seq ID No. 86 to 146 and then determining which of the compounds that bind a polypeptide selected from the group consisting of the polypeptides of Seq ID No. 13 to 24 or a polypeptide selected from the group consisting of the polypeptides of Seq ID No.86 to 146 or have a stimulatory or inhibitory effect on the activity or the expression of a polypeptide selected from the group consisting of the polypeptides of Seq ID No. 13 to 24 or a polypeptide selected from the group consisting of the polypeptides of Seq ID No.86 to 146 have an effect on the feeding behavior, body weight, or metabolic rate of a mammal (e.g., a mouse or a rat) in an in vivo assay.

The present invention pertains to a method of screening for compounds that reduce and/or prevent obesity comprising: a) contacting a cell expressing a gene listed in table 2 with a compound; and b) measuring the expression of said gene, or a polypeptide encoded by said gene; wherein a compound which up-regulates expression is a compound which causes an increase of expression of said gene or of the polypeptide encoded by said gene.

The term "up-regulation of expression" as used herein refers to an increase in expression of mRNA levels of a nucleic acid, or to an increase in expression of polypeptide levels. This term may also relate to increased post-translational modifications that are necessary for the activity and/or function of a polypeptide, e.g. addition of sugar moieties, phosphorylation etc.

A cell used in the method hereinbefore described, or in any of the methods hereinafter described may be an adipocyte, or a host or host cell as defined hereinafter.

Preferably, said gene is Seq ID. No.1. In another preferred embodiment, said gene is Seq ID No. 2. In another preferred embodiment, said gene is Seq ID No. 3. In another preferred embodiment, said gene is Seq ID No. 4. In another preferred embodiment, said gene is Seq ID No. 5. In another preferred embodiment, said gene is Seq ID No. 6.

The present invention further provides a method of screening for compounds that reduce and/or prevent obesity comprising: a) contacting a cell expressing a gene selected from the group consisting of Seq ID No. 25-37 with a compound; and b) measuring the expression of said gene, or a polypeptide encoded by said gene; wherein a compound which up-regulates gene expression is a compound which causes an increase of expression of said gene or of the polypeptide encoded by said gene.

Preferably, said polypeptide is Seq ID No. 13. In another preferred embodiment, said polypeptide is Seq ID No. 14. In another preferred embodiment, said polypeptide is Seq ID No. 15. In another preferred embodiment, said polypeptide is Seq ID No. 16. In another preferred embodiment, said polypeptide is Seq ID No. 17. In another preferred embodiment, said polypeptide is Seq ID No. 18.

The present invention also pertains to a method of screening for compounds that reduce and/or prevent obesity comprising a) contacting a cell expressing a gene listed in table 3 with a compound; and b) measuring the expression of said gene, or a polypeptide encoded by said gene;
wherein a compound which down-regulates gene expression is a compound which causes a decrease of said gene or a polypeptide encoded by said gene.

The term "down-regulation of expression" as used herein refers to a decrease in expression of mRNA levels of a nucleic acid, or to a decrease in expression of polypeptide levels. This term may also relate to decreased post-translational modifications that are necessary for the activity and/or function of a polypeptide, e.g. addition of sugar moieties, phosphorylation etc.

Preferably, said gene is Seq ID. No.7. In another preferred embodiment, said gene is Seq ID No. 8. In another preferred embodiment, said gene is Seq ID No. 9. In another preferred embodiment, said gene is Seq ID No. 10. In another preferred embodiment, said gene is Seq ID No. 11. In another preferred embodiment, said gene is Seq ID No. 12.

Preferably, said polypeptide is Seq ID. No.19. In another preferred embodiment, said polypeptide is Seq ID No. 20. In another preferred embodiment, said polypeptide is Seq ID No. 21. In another preferred embodiment, said polypeptide is Seq ID No. 22. In another preferred embodiment, said polypeptide is Seq ID No. 23. In another preferred embodiment, said polypeptide is Seq ID No. 24.

The present invention further provides a method of screening for compounds that reduce and/or prevent obesity comprising: a) contacting a cell expressing a gene selected from the group consisting of Seq ID No. 38 to 85 with a compound; and b) measuring the expression of said gene, or a polypeptide encoded by said gene; wherein a compound which down-regulates gene expression is a compound which causes a decrease of said gene or a polypeptide encoded by said gene.

The present invention provides a method of screening for compounds that reduce and/or prevent obesity comprising: a) contacting a polypeptide selected from the group consisting of Seq ID No. 13 to 18 with a compound; and b) determining and/or measuring the activity and/or function of said polypeptide; wherein a compound which reduces and/or prevents obesity by agonizing said polypeptide is a compound which causes an increase in activity and/or function of said polypeptide.

The present invention also pertains to a method for screening of compounds that reduce and/or prevent obesity comprising: a) contacting a cell expressing a nucleic acid encoding a polypeptide selected from the group consisting of Seq ID No. 13 to 18 with a compound; and b) determining and/or measuring the activity and/or function of said polypeptide; wherein a compound which reduces and/or prevents obesity by agonizing said polypeptide is a compound which causes an increase in activity and/or function of said polypeptide.

Preferably, said polypeptide is Seq ID. No.13. In another preferred embodiment, said polypeptide is Seq ID No. 14. In another preferred embodiment, said polypeptide is Seq ID No. 15. In another preferred embodiment, said polypeptide is Seq ID No. 16. In another preferred embodiment, said polypeptide is Seq ID No. 17. In another preferred embodiment, said polypeptide is Seq ID No. 18.

The terms "activity and/or function" as used with respect to the polypeptide encoded by Seq ID No. 1, AZGP1, refers to the lipolytic and lipid mobilizing activity of AZGP1. Assays to determine these activities are well known in the art and are e.g. described in WO99/62939.

The terms "activity and/or function" as used with respect to the polypeptide encoded by Seq ID No. 2, IRS1, refers to modifications of IRS1, eg. by phosphorylation, preferably by tyrosine phosphorylation, and/or binding to downstream effectors following activation, eg. binding to PI3-kinase, Syp or Grb2, and/or phosphorylation or activation of downstream effectors of IRS1 activation. Assays to measure the activity and/or function of IRS1 are well known in the art, and are, for example, described in Ridderstrale et al., J. Biol. Chem. 270, 3471, 1995; or Kuhne et al., J. Biol. Chem. 268, 11479-81, 1993.

The terms "activity and/or function" as used with respect to the polypeptide encoded by Seq ID No. 3, ApoAI, refers to the binding of ApoAI and/or lipid vesicles containing ApoAI to target cells and/or the ability of ApoAI and/or lipid vesicles containing ApoAI to induce efflux of cellular cholesterol and phospholipids. Assays to determine these activities are well known in the art and are e.g. described in Hauser et al., Biochemistry 1998, 37, 17843-17850; Yancey et al., Biochemistry 1995, 34, 7955-7965.

The terms "activity and/or function" as used with respect to the polypeptide encoded by Seq ID No. 4, ARNT, refers to the transcriptional and/or co-activating activity of ARNT. Assays to determine these activities are well known in the art and are e.g. described in Whitelaw et al., Mol. Cell Biol. 12, 1994, 8343-8355 and/or Brunnberg et al., Proc. Natl. Acad. Sci. USA 100, 2003, 6517-6522.

The terms "activity and/or function" as used with respect to the polypeptide encoded by Seq ID No. 5, creatine kinase from brain (CKB), refers to the kinase activity of CKB. Assays to determine these activities are well known in the art and are e.g. described in O'Gorman et al., Biochem. Biophys. Acta 1276, 1996, 161-170; and/or Durany et al., Mol. Pathol. 55, 2002, 242-249.

The terms "activity and/or function" as used with respect to the polypeptide encoded by Seq ID No. 6, 6 sterol-c5-desaturase, refers to the ability of 6 sterol-c5-desaturase to desaturate n-3 and n-6 fatty acids. Assays to determine these activities are well known in the art and are e.g. described in de Antueno et al., FEBS Letters 509, 2001, 77-80; de Antueno et al., FEBS Letters 491, 2001, 247-251; Taton et al., Biochemistry 39, 2000,701-711.

The present invention also pertains to a method of screening for compounds that reduce and/or prevent obesity comprising: a) contacting a polypeptide selected from the group consisting of Seq ID No. 86 to 98 with a compound; and b) determining and/or measuring the activity and/or function of said polypeptide; wherein a compound which reduces and/or prevents obesity by agonizing said polypeptide is a compound which causes an increase in activity and/or function of said polypeptide.

A method for screening of compounds that reduce and/or prevent obesity comprising: a) contacting a cell expressing a nucleic acid encoding a polypeptide selected from the group consisting of Seq ID No. 86 to 98 with a compound; and b) determining and/or measuring the activity and/or function of said polypeptide; wherein a compound which reduces and/or prevents obesity by agonizing said polypeptide is a compound which causes an increase in activity and/or function of said polypeptide.

The present invention pertains to a method for screening of compounds that reduce and/or prevent obesity comprising: a) contacting a cell expressing a polypeptide selected from the group consisting of Seq ID No. 19 to 24 with a compound; and b) determining and/or measuring the activity and/or function of said gene, or a polypeptide encoded by said gene; wherein a compound which reduces and/or prevents obesity by antagonizing said polypeptide is a compound which causes a decrease in activity and/or function of said polypeptide.

The present invention provides a method for screening of compounds that reduce and/or prevent obesity comprising: a) contacting a cell expressing a nucleic acid encoding a polypeptide selected from the group consisting of Seq ID No. 19 to 24 with a compound; and b) determining and/or measuring the activity and/or function of said polypeptide; wherein a compound which reduces and/or prevents obesity by antagonizing said polypeptide is a compound which causes a decrease in activity and/or function of said polypeptide.

Preferably, said polypeptide is Seq ID. No.19. In another preferred embodiment, said polypeptide is Seq ID No. 20. In another preferred embodiment, said polypeptide is Seq ID No. 21. In another preferred embodiment, said polypeptide is Seq ID No. 22. In another preferred embodiment, said polypeptide is Seq ID No. 23. In another preferred embodiment, said polypeptide is Seq ID No. 24.

The terms "activity and/or function" as used with respect to the polypeptide encoded by Seq ID No. 7, osteomodulin (osteoadherin), refers to the ability of osteomodulin to mediate cell adhesion and/or attachment. Assays to determine these activities are well known in the art and are e.g. described in Wendel et al., J. Cell Biol. 141,1998,839-847.

The terms "activity and/or function" as used with respect to the polypeptide encoded by Seq ID No. 8, VLDLR (Very Low Density Lipoprotein Receptor), refers to the ability of VLDLR to e.g. bind bind to ligands such as proteinases, apolipoproteins, extracellular proteins. Assays to determine these activities are well known in the art and are e.g. described in Rettenberger et al., J. Biol. Chem. 274, 1999, 8973-8980, which also lists references for binding assays for ligands including serine proteinase-serpin complexes, the pro-enzyme form of uPA, ApoE containing lipoproteins, apo(a), lipoprotein lipase and thrombospondin-1.

The terms "activity and/or function" as used with respect to the polypeptide encoded by Seq ID No. 9, 11-beta-hydroxysteroid dehydrogenase type 1 (HSD11B1), refers to dehydrogenase and/or reductase activities of HSD11B1. Assays to determine these activities are well known in the art and are e.g. described in Bujalska, I.J. et al., J. Clin. Endocrinol. Metab. 2002, 87, 1205-1210.

The terms "activity and/or function" as used with respect to the polypeptide encoded by Seq ID No. 10, reelin, refers to the ability of reelin to bind to receptors, e.g. VLDLR or ApoER2, or to induce phosphorylation of downstream signaling molecules, e.g. Disabled 1. Assays to determine these activities are well known in the art and are e.g. described in D'Arcangelo, G. et al., Neuron 1999, 24, 471-479, and Howell, B.W. et al., Genes Dev. 1999, 13, 643-648.

The terms "activity and/or function" as used with respect to the polypeptide encoded by Seq ID No. 11, Multidrug resistance protein 7 (MRP7, ABCC10), refers to the transporter activities of ABCC10. Assays to determine these activities are well known in the art and are e.g. described in Mol. Pharmacol. 2003, 63, 351-358.

The terms "activity and/or function" as used with respect to the polypeptide encoded by Seq ID No. 12, cyclophilin 40 (CyP40), refers to the ability of CyP40 to bind to other chaperones, e.g. Hsp90, and to its chaperone activity. Assays to determine these activities are well known in the art and are e.g. described in Ward, B.K., et al., J. Biol. Chem. 2002, 277, 40799-40809, and Freeman B.C. et al., Science 1996, 274, 1718-1720.

The present invention provides a method of screening for compounds that reduce and/or prevent obesity comprising: a) contacting a polypeptide selected from the group consisting of Seq ID No. 99 to 146 with a compound; and b) determining and/or measuring the activity and/or function of said polypeptide;
wherein a compound which reduces and/or prevents obesity by antagonizing said polypeptide is a compound which causes a decrease in activity and/or function of said polypeptide.

The present invention further provides a method for screening of compounds that reduce and/or prevent obesity comprising: a) contacting a cell expressing a nucleic acid encoding a polypeptide selected from the group consisting of Seq ID No. 99 to 146 with a compound; and b) determining and/or measuring the activity and/or function of said polypeptide;
wherein a compound which reduces and/or prevents obesity by antagonizing said polypeptide is a compound which causes a decrease in activity and/or function of said polypeptide.

The present invention also provides a method of screening for compounds that bind to a polypeptide selected from the group consisting of the polypeptides of Seq ID No. 13 to 24, comprising the steps of a) contacting a compound with said polypeptide; and b) determining the ability of said compound to bind to said polypeptide.

The present invention provides a method of screening for compounds that bind to a polypeptide selected from the group consisting of the polypeptides of Seq ID No. 86 to 146, comprising the steps of a) contacting a compound with said polypeptide; and b) determining the ability of said compound to bind to said polypeptide.

Candidate or test compounds or agents which bind a polypeptide selected from the group consisting of Seq ID No 13 to 24 or a polypeptide selected from the group consisting of Seq ID No 86 to 146 and/or have a stimulatory or inhibitory effect on the activity or the expression of said polypeptide are identified in assays that employ either cells which express a form of said polypeptide (cell-based assays) or isolated polypeptide (cell-free assays). The various assays can employ a variety of forms of said polypeptide (e.g., full-length polypeptide, a biologically active fragment of a polypeptide, or a fusion protein which includes all or a portion of said polypeptide). Moreover, the polypeptide can be derived from any suitable mammalian species. The assay can be a binding assay entailing direct or indirect measurement of the binding of a test compound or the polypeptide to a known ligand or receptor, as defined above. The assay can also be an activity assay entailing direct or indirect measurement of the activity of said polypeptide. The assay can also be an expression assay entailing direct or indirect measurement of the expression of said polypeptide (e.g., polypeptide- encoding mRNA or the polypeptide). The various screening assays are combined with an in vivo assay entailing measuring the effect of the test compound on the feeding behavior, body weight, or metabolic rate of a mammal (e.g., a mouse or a rat).

In another embodiment, the assay is a cell-based assay comprising contacting a cell expressing a polypeptide (e.g., full-length polypeptide, a biologically active fragment of said polypeptide, or a fusion protein which includes all or a portion of said polypeptide) with a test compound and determining the ability of the test compound to modulate (e.g., stimulate or inhibit) the activity of the polypeptide. Determining the ability of the test compound to modulate the activity of the said polypeptide can be accomplished by any method suitable for measuring the activity of said polypeptide.

The present invention also includes cell-free assays. Such assays involve contacting a form of a polypeptide selected from the group consisting of Seq ID No 13 to 24 or a polypeptide selected from the group consisting of Seq ID No 86 to 146 (e.g., full-length polypeptide, a biologically active fragment of said polypeptide, or a fusion protein comprising all or a portion of said polypeptide) with a test compound and determining the ability of the test compound to bind to said polypeptide. Binding of the test compound to said polypeptide can be determined either directly or indirectly as described above. In one embodiment, the assay includes contacting the said polypeptide with a known compound which binds said polypeptide to form an assay mixture, contacting the assay mixture with a test compound, and determining the ability of the test compound to interact with said polypeptide, wherein determining the ability of the test compound to interact with said polypeptide comprises determining the ability of the test compound to preferentially bind to the said polypeptide as compared to the known compound.

The cell-free assays of the present invention are amenable to use of either a membrane-bound form of a polypeptide or a soluble fragment thereof. In the case of cell-free assays comprising the membrane-bound form of the polypeptide, it may be desirable to utilize a solubilizing agent such that the membrane-bound form of the polypeptide is maintained in solution. Examples of such solubilizing agents include nonionic detergents such as n-octylglucoside, n-dodecylglucoside, n-dodecylmaltoside, octanoyl-N-mcthylglucamidc, decanoyl-Nmethylglucamide, Triton X-100, Triton X-114, Thesit, Isotridecypoly(ethylene glycol ether)n, 3-[(3-cholamidopropyl)dimethylamminio]-1-propane sulfonate (CHAPS), 3-[(3-cholamidopropyl)dimethylamminio]-2-hydroxy-1-propane sulfonate (CHAPSO), or N-dodecyl-N, N-dimethyl-3-ammonio-1 -propane sulfonate.

In various embodiments of the above assay methods of the present invention, it may be desirable to immobilize a polypeptide to facilitate separation of complexed from uncomplexed forms of the polypeptide with a binding molecule, as well as to accommodate automation of the assay. Binding of a test compound to a polypeptide, or interaction of a polypeptide with a binding molecule in the presence and absence of a candidate compound, can be accomplished in any vessel suitable for containing the reactants. Examples of such vessels include microtitre plates, test tubes, and microcentrifuge tubes. In one embodiment, a fusion protein can be provided which adds a domain that allows one or both of the proteins to be bound to a matrix. For example, glutathione-S-transferase fusion proteins can be adsorbed onto glutathione sepharose beads (Sigma Chemical; St. Louis, Mo.) or glutathione derivatized microtitre plates, which are then combined with the test compound or the test compound and either the non-adsorbed binding protein or polypeptide, and the mixture incubated under conditions conducive to complex formation (e.g., at physiological conditions for salt and pH). Following incubation, the beads or microtitre plate wells are washed to remove any unbound components and complex formation is measured either directly or indirectly, for example, as described above. Alternatively, the complexes can be dissociated from the matrix, and the level of binding or activity of a polypeptide hereinbefore described can be determined using standard techniques.

Other techniques for immobilizing proteins on matrices can also be used in the screening assays of the invention. For example, either a polypeptide hereinbefore described or its binding molecule can be immobilized utilizing conjugation of biotin and streptavidin. Biotinylated polypeptide of the invention or target molecules can be prepared from biotin-NHS (N-hydroxy-succinimide) using techniques well known in the art (e.g., biotinylation kit, Pierce Chemicals; Rockford, Ill.), and immobilized in the wells of streptavidin-coated 96 well plates (Pierce Chemical). Alternatively, antibodies reactive with a polypeptide or binding molecules, but which do not interfere with binding of the polypeptide of the invention to its binding molecule, can be derivatized to the wells of the plate. Unbound binding protein or polypeptide of the invention is trapped in the wells by antibody conjugation. Methods for detecting such complexes, in addition to those described above for the GST-immobilized complexes, include immunodetection of complexes using antibodies reactive with a polypeptide hereinbefore described or binding molecule, as well as enzyme-linked assays which rely on detecting an enzymatic activity associated with a polypeptide or binding molecule.

### II. Test Compounds

Suitable test compounds for use in the screening assays of the invention can be obtained from any suitable source, e.g., conventional compound libraries. The test compounds can also be obtained using any of the numerous approaches in combinatorial library methods known in the art, including biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds (Lam (1997) *Anticancer Drug Des. 12:145)*.

Examples of methods for the synthesis of molecular libraries can be found in the art, for example in: DeWitt et al. (1993) *Proc. Natl. Acad. Sci. USA* 90:6909; Erb Ct al. (1994) *Proc. Natl.Acad. Sci. USA* 91:11422; Zuckermann et al. (1994). *J. Med. Chem*. 37:2678; Cho et al. (1993) *Science* 261:1303; Carrell et al. *(1994) Angew Chem. Int. Ed. Engl*. 33:2059; Carell et al. (1994) *Angew Chem. Int. Ed. Engi*.33:2061; and Gallop et al. (1994) *J. Med. Chem*. 37:1233.

Libraries of compounds may be presented in solution (e.g., Houghten (1992) *BioTechniques* 13.412-421), or on beads (Lam (1991) *Nature* 354:82-84), chips (Fodor *(1993) Nature 364:555-556),* bacteria (U.S. Pat. No.5,223,409), spores (U.S. Pat. Nos. *5,571,698;* 5,403,484; and 5,223, 409), plasmids (Cull et al. (1992) *Proc. Natl. Acad. Sci. USA* 89.1865-1869) or phage (Scott and Smith (1990) Science249:386-390; Devlin (1990) *Science* 249:404-406; Cwirla et al. (1990) *Proc. Natl. Acad. Sci. USA* 87:6378-6382; and Felici (1991) *J. Mol. Biol*. 222:301-310).

The present invention provides a compound identified by any of the methods hereinbefore described.

### III. Isolated Nucleic Acid Molecules

One aspect of the invention pertains to isolated nucleic acid molecules that encode a polypeptide selected from the group consisting of Seq ID No. 13 to 24 a polypeptide selected from the group consisting of Seq ID No 86 to 146 or a biologically active portion thereof, as well as nucleic acid molecules sufficient for use as hybridization probes to identify nucleic acid molecules encoding a gene selected from the group consisting of Seq ID No. 1 to 12 or a gene selected from the group consisting of Seq ID No 25 to 85 and fragments of such nucleic acid molecules suitable for use as PCR primers for the amplification or mutation of nucleic acid molecules. As used herein, the term "nucleic acid molecule" is intended to include DNA molecules (e.g., cDNA or genomic DNA) and RNA molecules (e.g., mRNA) and analogs of the DNA or RNA generated using nucleotide analogs. The nucleic acid molecule can be single-stranded or double-stranded, but preferably is double-stranded DNA. This section describes the nucleic acids hereinbefore described and methods for making and using such nucleic acids.

An "isolated" nucleic acid molecule is one which is separated from other nucleic acid molecules which are present in the natural source of the nucleic acid molecule. Preferably, an "isolated" nucleic acid molecule is free of sequences (preferably protein encoding sequences) which naturally flank the nucleic acid (i.e., sequences located at the 5' and 3' ends of the nucleic acid) in the genomic DNA of the organism from which the nucleic acid is derived. For example, in various embodiments, the isolated nucleic acid molecule can contain less than about 5 kb, 4 kb, 3 kb, 2 kb, 1 kb, 0.5 kb or 0.1 kb of nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material, or culture medium when produced by recombinant techniques, or substantially free of chemical precursors or other chemicals when chemically synthesized.

A nucleic acid molecule of the present invention can be isolated using standard molecular biology techniques and the sequence information provided herein. Using all or a portion of the nucleic acid sequences selected from the group consisting of Seq ID No. 1 to 12 or a gene selected from the group consisting of Seq ID No 25 to 85 as a hybridization probe, nucleic acid molecules of the invention can be isolated using standard hybridization and cloning techniques (e.g., as described in Sambrook et al., eds., *Molecular Cloning: A Laboratory Manual, 2nd ed.,* Cold Spring Harbor Laboratory, Cold Spring Harbor Laboratory Press, Cold Spring Harbor, N.Y, 1989).

A nucleic acid molecule of the invention can be amplified using cDNA, mRNA or genomic DNA as a template and appropriate oligonucleotide primers according to standard PCR amplification techniques. The nucleic acid so amplified can be cloned into an appropriate vector and characterized by DNA sequence analysis. Furthermore, oligonucleotides corresponding to all or a portion of a nucleic acid molecule of the invention can be prepared by standard synthetic techniques, e.g., using an automated DNA synthesizer.

Moreover, a nucleic acid molecule of the invention can comprise only a portion of a nucleic acid sequence encoding a polypeptide selected from the group consisting of Seq ID No. 13 to 24 or a polypeptide selected from the group consisting of Seq ID No 86 to 146, for example, a fragment which can be used as a probe or primer or a fragment encoding a biologically active portion of a polypeptide selected from the group consisting of Seq ID No. 13 to 24 or a polypeptide selected from the group consisting of Seq ID No 86 to 146. The nucleotide sequence determined from the cloning of any one of the genes listed in tables 2 and 3 or Seq ID No. 25 to 85 for the generation of probes and primers designed for use in identifying and/or cloning allelic variants and other variants of any one of the genes listed in tables 2 and 3 or Seq ID No. 25 to 85. The probe/primer typically comprises substantially purified oligonucleotide. The oligonucleotide typically comprises a region of nucleotide sequence that hybridizes under stringent conditions to at least about 12, preferably about 25, more preferably about 50, 75, 100, 125, 150, 175, 200, 250, 300, 350 or 400 consecutive nucleotides of the sense or antisense sequence of any one of the genes listed in tables 2 and 3 or Seq ID No. 25 to 85 or naturally occurring mutant or allelic variant thereof.

Probes based on the sequence of a nucleic acid molecule of the invention can be used to detect transcripts or genomic sequences encoding the same protein molecule encoded by a selected nucleic acid molecule. The probe comprises a label group attached thereto, e.g., a radioisotope, a fluorescent compound, an enzyme, or an enzyme cofactor. Such probes can be used as part of a diagnostic test kit for identifying cells or tissues which miss-express the protein, such as by measuring levels of a nucleic acid molecule encoding the protein in a sample of cells from a subject, e.g., detecting mRNA levels or determining whether a gene encoding the protein has been mutated or deleted.

A nucleic acid fragment encoding a "biologically active portion" of a polypeptide selected from the group consisting of Seq ID No. 13 to 24 or a polypeptide selected from the group consisting of Seq ID No 86 to 146 can be prepared by isolating a portion of nucleic acid which encodes a polypeptide having a biological activity, expressing the encoded portion of the polypeptide protein (e.g., by recombinant expression in vitro) and assessing the activity of the encoded portion of the polypeptide.

The invention further encompasses nucleic acid molecules that differ from the nucleotide sequence of a nucleic acid selected from the group consisting of Seq ID No. 1 to 12 or a gene selected from the group consisting of Seq ID No 25 to 85 due to degeneracy of the genetic code and thus encode the same protein as that encoded by the said nucleotide sequence.

In addition to the nucleotide sequence of any one of Seq ID No. 1 to 12 or a gene selected from the group consisting of Seq ID No 25 to 85, it will be appreciated by those skilled in the art that DNA sequence polymorphisms that lead to changes in the amino acid sequence may exist within a population. Such genetic polymorphisms may exist among individuals within a population due to natural allelic variation. An allele is one of a group of genes which occur alternatively at a given genetic locus. As used herein, the phrase "allelic variant" refers to a nucleotide sequence which occurs at a given locus or to a polypeptide encoded by the nucleotide sequence. Such natural allelic variations can typically result in *1-5%* variance in the nucleotide sequence of a given gene. Alternative alleles can be identified by sequencing the gene of interest in a number of different individuals. This can be readily carried out by using hybridization probes to identify the same genetic locus in a variety of individuals. Any and all such nucleotide variations and resulting amino acid polymorphisms or variations that are the result of natural allelic variation and that do not alter the functional activity are intended to be within the scope of the invention.

Accordingly, in another embodiment, an isolated nucleic acid molecule of the invention is at least 300 (325, 350, 375, 400, 425, 450, 500, 550, 600, 650, 700, 800, 900, 1000, or 1290) nucleotides in length and hybridizes under stringent conditions to the nucleic acid molecule comprising the nucleotide sequence, preferably the coding sequence of any one of the genes listed in table 2 and/or 3 or Seq ID No. 25 to 85 and encodes an allelic variant or mutant of said gene.

As used herein, the term "hybridizes under stringent conditions" is intended to describe conditions for hybridization and washing under which nucleotide sequences at least 60% (65%, 70%, preferably 75%) identical to each other typically remain hybridized to each other. Such stringent conditions are known to those skilled in the art and can be found in *Current Protocols in Molecular Biology,* John Wiley & Sons, N.Y (1989), 6.3.1-6.3.6. A preferred, non-limiting example of stringent hybridization conditions are hybridization in 6xsodium chloride/sodium citrate (SSC) at about 45 degrees C., followed by one or more washes in 0.2xSSC, 0.1% SDS at 50-65 degrees C. Preferably, an isolated nucleic acid molecule of the invention that hybridizes under stringent conditions to the sequence selected from the group consisting of Seq ID No. 1 to 12 or a gene selected from the group consisting of Seq ID No 25 to 85, corresponds to a naturally-occurring nucleic acid molecule. As used herein, a "naturally-occurring" nucleic acid molecule refers to an RNA or DNA molecule having a nucleotide sequence that occurs in nature (e.g., encodes a natural protein).

In addition to naturally occurring allelic variants of any one of the genes listed in tables 2 and 3 or Seq ID No. 25 to 85, the skilled artisan will further appreciate that changes can be introduced by mutation thereby leading to changes in the amino acid sequence of the encoded protein, without altering the biological activity of the protein. For example, one can make nucleotide substitutions leading to amino acid substitutions at "non-essential" amino acid residues. A "non-essential" amino acid residue is a residue that can be altered from the wild-type sequence without altering the biological activity, whereas an "essential" amino acid residue is required for biological activity. For example, amino acid residues that are not conserved or only semi-conserved among homologues of various species may be non-essential for activity and thus would be likely targets for alteration. Alternatively, amino acid residues that are conserved among the homologues of various species (e.g., murine and human) may be essential for activity and thus would not be likely targets for alteration.

Accordingly, another aspect of the invention pertains to nucleic acid molecules encoding a polypeptide selected from the group consisting of Seq ID No. 13 to 24 or a polypeptide selected from the group consisting of Seq ID No 86 to 146 that contain changes in amino acid residues that are not essential for activity. Such polypeptides differ in amino acid sequence from said polypeptide yet retain biological activity. In one embodiment, the isolated nucleic acid molecule includes a nucleotide sequence encoding a protein that includes an amino acid sequence that is at least about 85%, 95%, 96%, 97%, 98%, or 99% identical to the amino acid sequence of any one of Seq ID No. 13 to 24 or of Seq ID No. 86 to 146.

An isolated nucleic acid molecule encoding a variant protein can be created by introducing one or more nucleotide substitutions, additions or deletions into the nucleotide sequence of any one of the genes listed in tables 2 and 3 or Seq ID No. 25 to 85 such that one or more amino acid substitutions, additions or deletions are introduced into the encoded protein. Mutations can be introduced by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Alternatively, mutations can be introduced randomly along all or part of the coding sequence, such as by saturation mutagenesis, and the resultant mutants can be screened for biological activity to identify mutants that retain activity. Following mutagenesis, the encoded protein can be expressed recombinantly and the activity of the protein can be determined.

In one embodiment, a mutant polypeptide that is a variant of a polypeptide selected from the group consisting of Seq ID No. 13 to 24 or a polypeptide selected from the group consisting of Seq ID No 86 to 146 can be assayed for (1) the ability to form protein-protein interactions with proteins in a signaling pathway; (2) the ability to bind a ligand of said polypeptide; or (3) the ability to bind to an intracellular binding protein for said polypeptide. In another embodiment, the mutant polypeptide can be assayed for the ability to mediate changes in feeding behavior, body weight, or metabolism.

The present invention encompasses antisense nucleic acid molecules, i.e., molecules which are complementary to a sense nucleic acid encoding a polypeptide selected from the group consisting of Seq ID No. 19 to 24 or a polypeptide selected from the group consisting of Seq ID No 99 to 146, e.g., complementary to the coding strand of a double-stranded cDNA molecule or complementary to an mRNA sequence. Accordingly, an antisense nucleic acid can hydrogen bond to a sense nucleic acid. The antisense nucleic acid can be complementary to an entire coding strand, or to only a portion thereof, e.g., all or part of the protein coding region (or open reading frame). An antisense nucleic acid molecule can be antisense to all or part of a noncoding region of the coding strand of a nucleotide sequence encoding a polypeptide selected from the group consisting of Seq ID No. 19 to 24 or a polypeptide selected from the group consisting of Seq ID No 99 to 146. The noncoding regions ("5' and 3' untranslated regions") are the 5' and 3' sequences which flank the coding region and are not translated into amino acids.

An antisense oligonucleotide can be, for example, about 5, 10, 15, 20, 25, 30, 35, 40, 45 or 50 nucleotides in length. An antisense nucleic acid of the invention can be constructed using chemical synthesis and enzymatic ligation reactions using procedures known in the art. For example, an antisense nucleic acid (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. Examples of modified nucleotides which can be used to generate the antisense nucleic acid include 5-fluorouracil, 5 -bromouracil, 5-chlorouracil, 5 -iodouracil, hypoxanthine xanthine, 4-acetylcytosine, 5-(carboxyhydroxylmethyl) uracil, 5 -carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine 2-methyladenine, 2-methylguanine, 3-methylcytosine, 5-methylcytosine, N6-adenine, 7-methylguanine, 5 -methylaminomethyluracil, 5-methoxyaminomethyl-2-thiouracil, beta-D-mannosylqueosine, 5'-methoxycarboxymethyluracil, 5-methoxyuracil, uracil-5-oxyacetic acid (v), wybutoxosine, pseudouracil, queosine, 2-thiocytosine, 5 -methyl-2-thiouracil, 2-thiouracil, 4-thiouracil, 5-methyluracil, uracil-5-oxyacetic acid methylester, uracil-5-oxyacetic acid (v), 5-methyl-2-thiotiracil, 3-(3-amino-3-N-2-carboxypropyl) tiracil, (acp3) w, and 2,6-diaminopurine. Alternatively, the antisense nucleic acid can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

The antisense nucleic acid molecules of the invention are typically administered to a subject or generated in situ such that they hybridize with or bind to cellular mRNA and/or genomic DNA encoding uracil to thereby inhibit expression, e.g., by inhibiting transcription and/or translation. The hybridization can be by conventional nucleotide complementarity to form a stable duplex, or, for example, in the case of an antisense nucleic acid molecule which binds to DNA duplexes, through specific interactions in the major groove of the double helix. An example of a route of administration of antisense nucleic acid molecules of the invention includes direct injection at a tissue site. Alternatively, antisense nucleic acid molecules can be modified to target selected cells and then administered systemically. For example, for systemic administration, antisense molecules can be modified such that they specifically bind to receptors or antigens expressed on a selected cell surface, e.g., by linking the antisense nucleic acid molecules to peptides or antibodies which bind to cell surface receptors or antigens. The antisense nucleic acid molecules can also be delivered to cells using the vectors described herein. To achieve sufficient intracellular concentrations of the anti-sense molecules, vector constructs in which the antisense nucleic acid molecule is placed under the control of a strong pol II or pol III promoter are preferred.

An antisense nucleic acid molecule of the invention can be an α-anomeric nucleic acid molecule. An α-anomeric nucleic acid molecule forms specific double-stranded hybrids with complementary RNA in which, contrary to the usual α-units, the strands run parallel to each other (Gaultier et al. (1987) *Nucleic Acids Res.* 15:6625-6641). The anti-sense nucleic acid molecule can also comprise a 2'-o-methylribonucleotide (Inolle C et al. (1987) *Nucleic Acids Res.* 15:6131-6148) or a chimeric RNA-DNA analogue (Inoue et al. (1987) *FEBS Lett.* 215:327-330).

The invention also encompasses ribozymes. Ribozymes are catalytic RNA molecules with ribonuclease activity which are capable of cleaving a single-stranded nucleic acid, such as a mRNA, to which they have a complementary region. Thus, ribozymes (e.g., hammerhead ribozymes (described in Haselhoff and Gerlach (1988) *Nature* 334:585-591)) can be used to catalytically cleave mRNA transcripts to thereby inhibit translation of the protein encoded by the mRNA. A ribozyme having specificity for a nucleic acid molecule encoding a polypeptide selected from the group consisting of Seq ID No. 13 to 24 a polypeptide selected from the group consisting of Seq ID No 86 to 146 can be designed based upon the nucleotide sequence of a cDNA disclosed herein. For example, a derivative of a Tetrahymena L-19 JVS RNA can be constructed in which the nucleotide sequence of the active site is complementary to the nudeotide sequence to be cleaved. Cech et al. U.S. Pat. No.4,987,071; and Cech et al. U.S. Pat. No. 5,116,742. Alternatively, an mRNA encoding any one of the genes listed in tables 1 and 2 can be used to select a catalytic RNA having a specific ribonuclease activity from a pool of RNA molecules. See, e.g., Bartel and Szostak (1993) *Science* 261.1411-1418.

The invention also encompasses nucleic acid molecules which form triple helical structures. For example, expression of a polypeptide selected from the group consisting of Seq ID No. 19 to 24 or a polypeptide selected from the group consisting of Seq ID No 99 to 146, can be inhibited by targeting nucleotide sequences complementary to the regulatory region of the gene encoding the polypeptide (e.g., the promoter and/or enhancer) to form triple helical structures that prevent transcription of the gene in target cells. See generally Helene (1991) *Anticancer Drug Des. 6(6):569-84;* Helene (1992) *Ann. N.Y Acad. Sci.* 660:27-36; and Maher (1992) *Bioassays* 14(12):807-15.

In certain embodiments, the nucleic acid molecules of the invention can be modified at the base moiety, sugar moiety or phosphate backbone to improve, e.g., the stability, hybridization, or solubility of the molecule. For example, the deoxyribose phosphate backbone of the nucleic acids can be modified to generate peptide nucleic acids (see Hyrup et al. (1996) *Bioorganic & Medicinal Chemistry* 4(1): *5-23).* As used herein, the terms "peptide nucleic acids" or "PNAs" refer to nucleic acid mimics, e.g., DNA mimics, in which the deoxyribose phosphate backbone is replaced by a pseudopeptide backbone and only the four natural nucleobases are retained. The neutral backbone of PNAs has been shown to allow for specific hvbridization to DNA and RNA under conditions of low ionic strength. The synthesis of PNA oligomers can be performed using standard solid phase peptide synthesis protocols as described in Hyrup et al. (1996), supra; Perry-O'Keefe et al. (1996) *Proc. Natl. Acad. Sci. USA* 93: 14670-675. PNAs can be used in therapeutic and diagnostic applications. For example, PNAs can be used as antisense or antigene agents for sequence-specific modulation of gene expression by, e.g., inducing transcription or translation arrest or inhibiting replication. PNAs can also be used, e.g., in the analysis of single base pair mutations in a gene by, e.g., PNA directed PCR clamping; as artificial restriction enzymes when used in combination with other enzymes, e.g., 51 nucleases (Hyrup (1996), supra; or as probes or primers for DNA sequence and hybridization (Hyrup (1996), supra; Perry-O'Keefe et al. (1996) *Proc. Natl. Acad. Sci. USA 93: 1467∼675)*.

In another embodiment, PNAs can be modified, e.g., to enhance their stability or cellular uptake, by attaching lipophilic or other helper groups to PNA, by the formation of PNA-DNA chimeras, or by the use of liposomes or other techniques of drug delivery known in the art. For example, PNA-DNA chimeras can be generated which may combine the advantageous properties of PNA and DNA. Such chimeras allow DNA recognition enzymes, e.g., RNAse H and DNA polymerases, to interact with the DNA portion while the PNA portion would provide high binding affinity and specificity. PNA-DNA chimeras can be linked using linkers of appropriate lengths selected in terms of base stacking, number of bonds between the nucleobases, and orientation (Hyrup (1996), supra). The synthesis of PNA-DNA chimeras can be performed as described in Hyrup (1996), supra, and Finn et al. (1996) *Nucleic Acids Res. 24(17):3357-63.* For example, a DNA chain can be synthesized on a solid support using standard phosphoramidite coupling chemistry and modified nucleoside analogs. Compounds such as 5'-(4-methoxytrityl)amino-5'-deoxy-thymidine phosphoramidite can be used as a link between the PNA and the 5' end of DNA (Mag et al. (1989) *Nucleic Acids Res.* 17:5973-88). PNA monomers are then coupled in a stepwise manner to produce a chimeric molecule with a 5' PNA segment and a 3' DNA segment (Finn et al. (1996) *Nucleic Acids Res. 24(17):3357-63).* Alternatively, chimeric molecules can be synthesized with a 5' DNA segment and a 3' PNA segment (Petersen et al. (1975) *Bioorganic Med. Chem. Lett.* 5:1119-11124).

In other embodiments, the oligonucleotide may include other appended groups such as peptides (e.g., for targeting host cell receptors in vivo), or agents facilitating transport across the cell membrane (see, e.g., Letsinger et al. (1989) *Proc. Natl. Acad. Sci. USA 86:6553-6556;* Lemaitre et al. (1987) *Proc. Natl. Acad. Sci. USA* 84:648-652; PCT Publication No. WO 88/09810) or the blood-brain barrier (see, e.g., PCT Publication No. WO 89110134). In addition, oligonucleotides can be modified with hybridization-triggered cleavage agents (see, e.g., Krol et al. (1988) *BiolTechniques 6:958-976)* or intercalating agents (see, e.g., Zon (1988) *Pharm. Res.* 5:539-549). To this end, the oligonucleotide may be conjugated to another molecule, e.g., a peptide, hybridization triggered cross-linking agent, transport agent, hybridization-triggered cleavage agent, etc.

The present invention provides a use of a gene or a polypeptide encoded by a gene listed in tables 2 and/or 3 or Seq ID No. 25 to 85 as a target for screening of compounds that reduce and/or prevent obesity.

The present invention also provides a use of a nucleic acid encoding a polypeptide selected from the group consisting of Seq ID No. 13-24 a polypeptide selected from the group consisting of Seq ID No 86 to 146, or of mutants or fragments thereof as a target for screening of compounds that reduce and/or prevent obesity.

### V. Isolated Proteins and Antibodies

One aspect of the invention pertains to isolated proteins, and biologically active portions thereof, as well as polypeptide fragments suitable for use as immunogen to raise antibodies directed against a polypeptide selected from the group consisting of Seq ID No. 13 to 24 or a polypeptide selected from the group consisting of Seq ID No 86 to 146. In one embodiment, native polypeptide can be isolated from cells or tissue sources by an appropriate purification scheme using standard protein purification techniques. In another embodiment, polypeptides of the invention are produced by recombinant DNA techniques. Alternative to recombinant expression, polypeptides can be synthesized chemically using standard peptide synthesis techniques. This section describes polypeptides of any one of Seq ID No. 13 to 24 or SeqID No. 86 to 146, antibodies directed against said polypeptides, and methods for making and using such polypeptides and antibodies.

An "isolated" or "purified" protein or biologically active portion thereof is substantially free of cellular material or other contaminating proteins from the cell or tissue source from which the protein is derived, or substantially free of chemical precursors or other chemicals when chemically synthesized. The language "substantially free of cellular material" includes preparations of protein in which the protein is separated from cellular components of the cells from which it is isolated or recombinantly produced. Thus, protein that is substantially free of cellular material includes preparations of protein having less than about 30%, 20%, 10%, or 5% (by dry weight) of heterologous protein (also referred to herein as a "contaminating protein"). When the protein or biologically active portion thereof is recombinantly produced, it is also preferably substantially free of culture medium, i.e., culture medium represents less than about 20%, 10%, or 5% of the volume of the protein preparation. When the protein is produced by chemical synthesis, it is preferably substantially free of chemical precursors or other chemicals, i.e., it is separated from chemical precursors or other chemicals which are involved in the synthesis of the protein. Accordingly such preparations of the protein have less than about 30%, 20%, 10%, 5% (by dry weight) of chemical precursors or unrelated chemicals.

Biologically active portions of a polypeptide selected from the group consisting of Seq ID No. 13 to 24 or a polypeptide selected from the group consisting of Seq ID No 86 to 146 include polypeptides comprising amino acid sequences sufficiently identical to or derived from the amino acid sequence of the protein which include fewer amino acids than the full length protein, and exhibit at least one activity of the corresponding full-length protein. Typically, biologically active portions comprise a domain or motif with at least one activity of the corresponding portion. A biologically active portion of the invention can be a polypeptide which is, for example, 10, 25, 50, 100 or more amino acids in length. Moreover, other biologically active portions, in which other regions of the protein are deleted, can be prepared by recombinant techniques and evaluated for one or more of the functional activities of the native form of said polypeptide.

Among the useful polypeptides are those having the amino acid sequence of a polypeptide selected from the group consisting of Seq ID No. 13 to 24 or a polypeptide selected from the group consisting of Seq ID No 86 to 146. Other useful proteins are substantially identical (e.g., at least about 96%, 97%, 98%, 99%, or 99.5%) to any of said polypeptides and retain the functional activity of the protein of the corresponding naturally-occurring protein yet differ in amino acid sequence due to natural allelic variation or mutagenesis. To determine the percent identity of two amino acid sequences or of two nucleic acid sequences, the sequences are aligned for optimal comparison purposes (e.g., gaps can be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (i.e., % identity=# of identical positions/total # of positions (e.g., overlapping positions)x100). Preferably, the two sequences are the same length.

The invention also provides chimeric or fusion proteins. As used herein, a "chimeric protein" or "fusion protein" comprises all or part (e.g., biologically active fragment) of a polypeptide selected from the group consisting of one Seq ID No. 13 to 24 or a polypeptide selected from the group consisting of Seq ID No 86 to 146 operably linked to a heterologous polypeptide (i.e., a polypeptide other than the same polypeptide of the invention). Within the fusion protein, the term "operably linked" is intended to indicate that the polypeptide of the invention and the heterologous polypeptide are fused in-frame to each other. The heterologous polypeptide can be fused to the N-terminus or C-terminus of said polypeptide.

One useful fusion protein is a GST fusion protein in which all or a portion of a polypeptide selected from the group consisting of Seq ID No. 13 to 24 or a polypeptide selected from the group consisting of Seq ID No 86 to 146 is fused to the C-terminus of GST sequences. Such fusion proteins can facilitate the purification of a recombinant polypeptide. Other useful fusion proteins include fusions to FLAGTM, a portion lacZ, GST, calmodulin-binding peptide, His⁶, or HA. Vectors for preparing such fusions proteins are available from Clontech, Inc. (Palo Alto, Calif.) and Stratagene, Inc. (La Jolla, Calif.).

In another embodiment, the fusion protein contains a heterologous signal sequence at its N-terminus. For example, the native signal sequence of any one of the polypeptides of Seq ID No. 13 to 24 or Seq ID No. 86 o 146 can be removed and replaced with a signal sequence from another protein. For example, the gp67 secretory sequence of the baculovirus envelope protein can be used as a heterologous signal sequence *(Current Protocols in Molecular Biology,* Ausubel et al., eds., John Wiley & Sons, 1992). Other examples of eukaryotic heterologous signal sequences include the secretory sequences of melittin and human placental alkaline phosphatase (Stratagene; La Jolla, Calif.). In yet another example, useful prokaryotic heterologous signal sequences include the phoA secretory signal (Sambrook Ct al., supra) and the protein A secretory signal (Pharmacia Biotech; Piscataway, N.J.).

In yet another embodiment, the fusion protein is an immunoglobulin fusion protein in which all or part of the sequence of a polypeptide of Seq ID No. 13 to 24 or Seq ID No. 86 to 146 is fused to sequences derived from a member of the immunoglobulin protein family. The immunoglobulin fusion proteins of the invention can be incorporated into pharmaceutical compositions and administered to a subject to inhibit an interaction between a ligand (soluble or membrane-bound) and a protein on the surface of a cell (receptor), to thereby suppress signal transduction in vivo. The immunoglobulin fusion protein can be used to affect the bioavailability of a cognate ligand of a polypeptide of Seq ID No. 13 to 24 or Seq ID No. 86 to 146. Inhibition of ligand/receptor interaction may be useful therapeutically for modulating feeding behavior, body weight, and/or metabolic rate. Moreover, the immunoglobulin fusion proteins of the invention can be used as immunogen to produce antibodies directed against a polypeptide hereinbefore described in a subject, to purify ligands and in screening assays to identify molecules which inhibit the interaction of receptors with ligands.

Chimeric and fusion protein of the invention can be produced by standard recombinant DNA techniques. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence (see, e.g., Ausubel et al., supra). Moreover, many expression vectors are commercially available that already encode a fusion moiety (e.g., a GSJ polypeptide). A nucleic acid encoding any one of the polypeptides of Seq ID No. 13 to 24 or Seq ID No. 86 to 146 can be cloned into such an expression vector such that the fusion moiety is linked in-frame to the polypeptide of the invention.

The present invention also pertains to variants of any one of the polypeptides of Seq ID No. 13 to 24 or Seq ID No. 86 to 146. Such variants have an altered amino acid sequence which can function as either agonists (mimetics) or as antagonist. Variants can be generated by mutagenesis, e.g., discrete point mutation or truncation. An agonist can retain substantially the same, or a subset, of the biological activities of the naturally occurring form of the protein. An antagonist of a protein can inhibit one or more of the activities of the naturally occurring form of the protein by, for example, competitively binding to a downstream or upstream member of a cellular signaling cascade which includes the protein of interest. Thus, specific biological effects can be elicited by treatment with a variant of limited function. Treatment of a subject with a variant having a subset of the biological activities of the naturally occurring form of the protein can have fewer side effects in a subject relative to treatment with the naturally occurring form of the protein.

Variants of a protein of the invention which function as either agonists (mimetics) or as antagonists can be identified by screening combinatorial libraries of mutants, e.g., truncation mutants, of the protein of the invention for agonist or antagonist activity. In one embodiment, a variegated library of variants is generated by combinatorial mutagenesis at the nucleic acid level and is encoded by a variegated gene library. A variegated library of variants can be produced by, for example, enzymatically ligating a mixture of synthetic oligonucleotides into gene sequences such that a degenerate set of potential protein sequences is expressible as individual polypeptides, or alternatively, as a set of larger fusion proteins (e.g., for phage display). There are a variety of methods which can be used to produce libraries of potential variants of the polyepeptides of the invention from a degenerate oligonucleotide sequence. Methods for synthesizing degenerate oligonucleotides are known in the art (see, e.g., Narang (1983) *Tetrahedron 39:3;* Itakura et al. (1984)Arniu. *Rev. Biochem. 53:323;* Itakura et al. (1984) *Science* 198:1056; Ike et al. (1983) *NucleicAcid Res.* 11:477).

In addition, libraries of fragments of a polypeptide selected from the group consisting of Seq ID No. 13 to 24or a polypeptide selected from the group consisting of Seq ID No 86 to 146 can be used to generate a variegated population of polypeptides for screening and subsequent selection of variants. For example, a library of coding sequence fragments can be generated by treating a double stranded PCR fragment of the coding sequence of interest with a nuclease under conditions wherein nicking occurs only about once per molecule, denaturing the double stranded DNA, renaturing the DNA to form double stranded DNA which can include sense/antisense pairs from different nicked products, removing single stranded portions from reformed duplexes by treatment with SI nuclease, and ligating the resulting fragment library into an expression vector. By this method, an expression library can be derived which encodes N-terminal and internal fragments of various sizes of the protein of interest.

Several techniques are known in the art for screening gene products of combinatorial libraries made by point mutations or truncation, and for screening cDNA libraries for gene products having a selected property. The most widely used techniques, which are amenable to high through-put analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected.

Recursive ensemble mutagenesis (REM), a technique which enhances the frequency of functional mutants in the libraries, can be used in combination with the screening assays to identify variants of a protein of the invention (Arkin and YollrvaIl (1992) *Proc. Natl Acad USA* 89:7811-7815; Delgrave et al. (1993) *Protein Engineering* 6(3):327-331).

### VI. Recombinant Expression Vectors and Host Cells

Another aspect of the invention pertains to vectors (e.g., expression vectors) containing a nucleic acid encoding a polypeptide selected from the group consisting of Seq ID No. 13 to 24 or a polypeptide selected from the group consisting of Seq ID No 86 to 146 (or a portion thereof). As used herein, the "vector" refers to a nucleic acid molecule capable of transporting another nucleic acid to which it has been linked. One type of vector is a "plasmid," which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors, expression vectors, are capable of directing the expression of genes to which they are operably linked. In general, expression vectors of utility in recombinant DNA techniques are often in the form of plasmids (vectors). However, the invention is intended to include such other forms of expression vectors, such as viral vectors (e.g., replication defective retroviruses, adenoviruses and adeno-associated viruses), which serve equivalent functions. This section describes vectors and host cells harboring nucleic acids selected from the group consisting of Seq ID No. 1 to 12 a gene selected from the group consisting of Seq ID No 25 to 85 and variants thereof and methods for their production and use.

The recombinant expression vectors of the invention comprise a nucleic acid of the invention in a form suitable for expression of the nucleic acid in a host cell. This means that the recombinant expression vectors include one or more regulatory sequences, selected on the basis of the host cells to be used for expression, which is operably linked to the nucleic acid sequence to be expressed. Within a recombinant expression vector, "operably linked" is intended to mean that the nucleotide sequence of interest is linked to the regulatory sequence(s) in a manner which allows for expression of the nucleotide sequence (e.g., in an in vitro transcription/translation system or in a host cell when the vector is introduced into the host cell). The term "regulatory sequence" is intended to include promoters, enhancers and other expression control elements (e.g., polyadenylation signals). Such regulatory sequences are described, for example, in Goeddel, *Gene Expression Technology: Method in Enzymology* 185, Academic Press, San Diego, Calif. (1990). Regulatory sequences include those which direct constitutive expression of a nucleotide sequence in many types of host cell and those which direct expression of the nucleotide sequence only in certain host cells (e.g., tissue-specific regulatory sequences). It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein.

The recombinant expression vectors of the present invention can be designed for expression of a gene listed in tables 2 or 3 or Seq ID No. 25 to 85 in prokaryotic or eukaryotic cells, e.g., bacterial cells such as *E. coli,* insect cells (using baculovirus exprcssion vectors), yeast cells or mammalian cells. Suitable host cells are discussed further in Goeddel, supra. Alternatively, the recombinant expression vector can be transcribed and translated in vitro, for example using T7 promoter regulatory sequences and T7 polymerase.

Expression of proteins in prokaryotes is most often carried out in *E. coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include PGEX (Pharmacia Biotech Inc; Smith and Johnson (1988) *Gene 67*:31-40), pMAL (New England Biolabs, Beverly, Mass.) and pRIT5 (Pharmacia Piscataway, N.J.) which fuse glutathione 5-transferase (GST), maltose E binding protein, or protein A, respectively, to the target recombinant protein.

Examples of suitable inducible non-fusion *E. coli* expression vectors include pTrc (Amann et al., (1988) *Gene 69*:301-315) and pET lid (Studier et al., *Gene Expression Technology: Methods in Enzymology* 185, Academic Press San Diego, Calif. (1990) 60∼9). Target gene expression from the pTrc vector relies on host RNA polymerase transcription from a hybrid trp-lac fusion promoter. Target gene expression from the pET lid vector relies on transcription from a T7 gn10-lac fusion promoter mediated by a coexpressed viral RNA polymerase (T7 gn1). The viral polymerase is supplied by host strains BL21(DE3) or HM5174 (DE3) from a resident prophage harboring a T7 gn1 gene under the transcriptional control of the lacUV 5 promoter.

One strategy to maximize recombinant protein expression in *E. coli* is to express the protein in a host bacteria with an impaired capacity to proteolytically cleave the recombinant protein (Gottesman, *Gene Expression Technology: Methods in Enzymology* 185, Academic Press, San Diego, Calif. (1990) 119-128). Another strategy is to alter the nucleic acid sequence of the nucleic acid to be inserted into an expression vector so that the individual codons for each amino acid are those preferentially utilized in *E. coli* (Wada et al. (1992) *Nucleic Acids Res.* 20:2111-2118). Such alteration of nucleic acid sequences of the invention can be carried out by standard DNA synthesis techniques.

In another embodiment, the expression vector is a yeast expression vector. Examples of vectors for expression in yeast S. *cerivisae* include pYepSecl (Baldari et al. (1987) m*EMBO J.* 6:229-234), pMFa (Kurjan and Herskowitz, (1982) *Cell* 30:933∼943), pJRY88 (Schultz et al. (1987) *Gene 54:113-123)*, pYES2 (Invitrogen Corporation, SanDiego, Calif.), and pPicZ (InVitrogen Corp, San Diego, 15 Calif.).

Alternatively, the expression vector is a baculovirus expression vector. Baculovirus vectors available for expression of proteins in cultured insect cells (e.g., Sf 9 cells) include the pAc series (Smith et al. (1983) *Mol. Cell Biol*.20 *3:2156-2165)* and the pVL series (Lucklow and Summers (1989) *virology* 170:31-39).

In yet another embodiment, a nucleic acid of the invention is expressed in mammalian cells using a mammalian expression vector. Examples of mammalian expression vectors include pCDM8 (Seed (1987) *Nature* 329:840) and pMT2PC (Kaufman et al. (1987) *EMBO J. 6:187-195)*. When used in mammalian cells, the expression vector's control functions are often provided by viral regulatory elements. For example, commonly used promoters are derived from polyoma, Adenovirus 2, cytomegalovirus and Simian Virus 40. For other suitable expression systems for both prokaryotic and eukaryotic cells see chapters 16 and 17 of Sambrook et al., supra.

In another embodiment, the recombinant mammalian expression vector is capable of directing expression of the nucleic acid preferentially in a particular cell type (e.g., tissue-specific regulatory elemen[s are ilsed to express the nucleic acid). Tissue-specific regulatory elements are known in the art. Non-limiting examples of suitable tissue-specific promoters include the albumin promoter (liver-specific; Pinkert et al. (1987) *Genes Dev.* 1:268-277), lymphoid-specific promoters (Calame and Eaton *(1988)Adv Immunol. 43:235-275)*, in particular promoters of T cell receptors (Winoto and Baltimore (1989) *EMBO J.* 8:729-733) and immunoglobulins (Banerji et al. (1983) *Cell* 33:729-740; Queen and Baltimore (1983) *Cell* 33:741-748), neuron-specific promoters (e.g., the neurofilament promoter; Byrne and Ruddle (1989) *Proc. Natl. Acad. Sci. USA 86:5473-5477)*, pancreas-specific promoters (Edlund et al. *(1985) Science* 230:912-916), and mammary gland-specific promoters (e.g., milk whey promoter; U.S. Pat. No.4,873, 316 and European Application Publication No.264,166). Developmentally-regulated promoters are also encompassed, for example the murine hox promoters (Kessel and Gruss (1990) *Science* 249:374-379) and the CL-fetoprotein promoter (Campes and Tilghman (1989) *Genes Dev. 3:537-546).*

The invention further provides a recombinant expression vector comprising a DNA molecule of the invention cloned into the expression vector in an antisense orientation. That is, the DNA molecule is operably linked to a regulatory sequence in a manner which allows for expression (by transcription of the DNA molecule) of an RNA molecule which is antisense to the mRNA encoding a polypeptide selected from the group consisting of Seq ID No. 13 to 24 or a polypeptide selected from the group consisting of Seq ID No 86 to 146. Regulatory sequences operably linked to a nucleic acid cloned in the antisense orientation can be chosen which direct the continuous expression of the antisense RNA molecule in a variety of cell types, for instance viral promoters and/or enhancers, or regulatory sequences can be chosen which direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activitvity of which can be determined by the cell type into which the vector is introduced. For a discussion of the regulation of gene expression using anti-sense genes see Weintraub et al. *(Reviews-Trends in Genetics,* Vol. 1(1) 1986).

Another aspect of the invention pertains to host cells into which a recombinant expression vector of the invention has been introduced. It is understood that this termsrefer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell (e.g., *E. coli*, insect cells, yeast or mammalian cells). Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid into a host cell, including calcium phosphate or calcium chloride co-precipitation, DEAE-dextran-mediated transfection, lipofection, or electroporation. Suitable methods for transforming or transfecting host cells can be found in Sambrook, et al. (supra), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (e.g., for resistance to antibiotics) is generally introduced into the host cells along with the gene of interest. Useful selectable markers include those which confer resistance to drugs, such as G418, hygromycin and methotrexate. Cells stably transfected with the introduced nucleic acid can be identified by drug selection (e.g., cells that have incorporated the selectable marker gene will survive, while the other cells die).

A host cell of the invention, such as a prokaryotic or eukaryotic host cell in culture, can be used to produce a polypeptide selected from the group consisting of Seq ID No. 13 to 24 or a polypeptide selected from the group consisting of Seq ID No 86 to 146. Accordingly, the invention further provides methods for producing a polypeptide selected from the group consisting of Seq ID No. 13 to 24 or a polypeptide selected from the group consisting of Seq ID No 86 to 146 using the host cells of the invention. In one embodiment, the method comprises culturing the host cell of invention (into which a recombinant expression vector encoding a polypeptide selected from the group consisting of Seq ID No. 13 to 24 or a polypeptide selected from the group consisting of Seq ID No 86 to 146 has been introduced) in a suitable medium such that the polypeptide is produced. In another embodiment, the method further comprises isolating the polypeptide from the medium or the host cell.

### VII. Methods of Treatment

The present invention provides for both prophylactic and therapeutic methods for modulating body weight, e.g., by altering feeding behavior or metabolic rate.

In one aspect, the present invention provides a method for modulating body weight by administering an agent which modulates an activity of any one of the polypeptides of Seq ID No. 13 to 24 or Seq ID No. 86 to 146. Such methods are useful for modulating body weight both in patients having aberrant expression or activity of said polypeptide or other patients which would benefit from administration of an agent which modulates activity of said polypeptide. Depending on the needs of the patient a polypeptide agonist or antagonist can be used for treating the subject.

Agonists of the activity of any one of the polypeptides of Seq ID No. 13-18 or Seq ID No. 86 to 146, or compounds which increase expression of said polypeptide are useful for treatment of high body weight, e.g., obesity, because they can be used to reduce body weight. Similarly, compounds which increase the activity or expression of a protein in the signalling pathway of said polypeptide are useful for treatment of high body weight. Conversely, antagonists of the activity of said polypeptide or compounds which reduce the expression or activity of said polypeptide are useful for treatment of low body weight, e.g., cachexia, because they can be used to increase body weight. Compounds which reduce the activity or expression of a protein in the signalling pathway of a polypeptide of any one of Seq ID NO. 13 to 18 or Seq ID No. 86 to 146 are useful for treatment of low body weight.

Antagonists of the activity of any one of the polypeptides of Seq ID No. 19-24 or Seq ID No. 99 to 146, or compounds which decrease expression of said polypeptide are useful for treatment of high body weight, e.g., obesity, because they can be used to reduce body weight. Similarly, compounds which decrease the activity or expression of a protein in the signalling pathway of said polypeptide are useful for treatment of high body weight. Conversely, agonists of the activity of said polypeptide or compounds which increase the expression or activity of said polypeptide are useful for treatment of low body weight, e.g., cachexia, because they can be used to increase body weight. Compounds which icrease the activity or expression of a protein in the signalling pathway of a polypeptide of any one of Seq ID NO. 19 to 24 or Seq ID No. 99 to 146 are useful for treatment of low body weight.

The present invention also provides a use of an agonist, or a compound which increases the expression of a polypeptide selected from the group consisting of Seq ID No. 13 to 18 or a polypeptide selected from the group consisting of Seq ID No 86 to 98 for the preparation of a medicament for the treatment of obesity. Futhermore, the present invention provides a use of an antagonist, or a compound that decreases the expression of a polypeptide selected from the group consisting of Seq ID No. 19 to 24 or a polypeptide selected from the group consisting of Seq ID No 99 to 146 for the preparation of a medicament for the treatment of obesity.

### VIII. Pharmaceutical Compositions

The present invention further pertains to novel agents identified by the above-described screening assays and uses thereof for treatments as described herein. The nucleic acid molecules, polypeptides, and antibodies (also referred to herein as "active compounds") of the invention can be incorporated into pharmaceutical compositions suitable for administration. Such compositions typically comprise the nucleic acid molecule, protein, or anti-body and a pharmaceutically acceptable carrier. As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

The invention includes pharmaceutical compositions comprising a modulator of expression or activity of any one of the polypeptides of Seq ID No. 13 to 24 or Seq ID No. 86 to 146 (and/or a modulator of the activity or expression of a protein in the signalling pathway of said polypeptide) as a well as methods for preparing such compositions by combining one or more such modulators and a pharmaceutically acceptable carrier. Also within the scope of the present invention are pharmaceutical compositions comprising a modulator identified using the screening assays of the invention packaged with instructions for use. For modulators that are antagonists of the activity of any one of the polypeptides of Seq ID No. 13 to 24 or Seq ID No. 86 to 146 or which reduce the expression of said polypeptide, the instructions would specify use of the pharmaceutical composition for treatment of low body weight (e.g., increase of body weight). For modulators that are agonists of the activity of said polypeptide or increase the expression of said polypeptide, the instructions would specify use of the pharmaceutical composition for treatment of high body weight (i.e., reduction of body weight).

The present invention provides a pharmaceutical formulation for the modulation of body weight, comprising a compound that modulates the activity of a polypeptide selected from the group consisting of Seq ID No. 86 to 146, mixed with a pharmaceutically acceptable carrier.

A pharmaceutical composition of the invention is formulated to be compatible with its intended route of administration. Examples of routes of administration include parenteral, e.g., intravenous, intradermal, subcutaneous, oral (e. g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersions are provided. For intravenous administration, suitable carriers include physiological saline, bacteriostatic water, Cremophor ELTM (BASF; Parsippany, N.J.) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (e.g., a polypeptide or antibody) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from a pressurized container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Pat. No.4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to t)e achieved, and the limitations inherent in the art of compounding such an active compound for the treatment of individuals.

The nucleic acid molecules of the invention can be inserted into vectors and used as gene therapy vectors. Gene therapy vectors can be delivered to a subject by, for example, intravenous injection, local administration (U.S. Pat. No. 5,328,470) or by stereotactic injection (see, e.g., Chen et al. (1994) *Proc. Natl Acad. Sci. USA 91:3054-3057).* The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g. retroviral vectors, the pharmaceutical preparation can include one or more cells which produce the gene delivery system.

The pharmaceutical compositions can be included in a container, pack, or dispenser together with instructions for administration.

The present invention provides a pharmaceutical formulation for the modulation of body weight, comprising a compound that modulates the activity of a polypeptide selected from the group consisting of Seq ID No. 13 to 24 or a polypeptide selected from the group consisting of Seq ID No 86 to 146, mixed with a pharmaceutically acceptable carrier.

The present invention also refers to a package comprising the pharmaceutical formulation hereinbefore described and instructions for administering the pharmaceutical formulation for the purpose of modulating body weight.

The present invention pertains to a method for preparing a pharmaceutical composition useful for modulating body weight, the method comprising: a) contacting a test compound with a polypeptide selected from the group consisting of Seq ID No. 13 to 24 or a polypeptide selected from the group consisting of Seq ID No 86 to 146; b) determining whether the test compound binds to the polypeptide selected from the group consisting of Seq ID No. 13 to 24 or a polypeptide selected from the group consisting of Seq ID No 86 to 146; and c) combining the test compound that binds to the polypeptide selected from the group consisting of Seq ID No. 13 to 24 or a polypeptide selected from the group consisting of Seq ID No 86 to 146 with a pharmaceutically acceptable carrier to create a pharmaceutical composition useful for modulating body weight.

The present invention provides a method for preparing a pharmaceutical composition useful for modulating body weight, the method comprising: a) contacting a ligand of a polypeptide selected from the group consisting of Seq ID No. 13 to 24 or a polypeptide selected from the group consisting of Seq ID No 86 to 146 with a polypeptide selected from the group consisting of Seq ID No. 13 to 24 or a polypeptide selected from the group consisting of Seq ID No 86 to 146 in the presence and absence of a test compound; b) determining whether the test compound alters the binding of the ligand of the polypeptide selected from the group consisting of Seq ID No. 13 to 24 or a polypeptide selected from the group consisting of Seq ID No 86 to 146 to the polypeptide selected from the group consisting of Seq ID No. 13 to 24 or a polypeptide selected from the group consisting of Seq ID No 86 to 146; and c) combining the test compound that alters the binding of said ligand to said polypeptide with a pharmaceutically acceptable carrier to create a pharmaceutical composition useful for modulating body weight.

The present invention also provides a use of a gene selected from the group consisting of Seq ID No. 1 to 12 or a gene selected from the group consisting of Seq ID No 25 to 85, or of a polypeptide selected from the group consisting of Seq ID No 13 to 24 as a target for screening of compounds that reduce and/or prevent obesity.

The present invention further provides a use of a gene or a polypeptide encoded by a selected from the group consisting of Seq ID No. 25 to 146 as a target for screening of compounds that reduce and/or prevent obesity.

Further to this, the present invention also pertains to a use of a nucleic acid encoding a polypeptide selected from the group consisting of Seq ID No. 13-24, or of mutants or fragments thereof, as a target for screening of compounds that reduce and/or prevent obesity.

Furthermore, the present invention provides a use of a nucleic acid encoding a polypeptide selected from the group consisting of Seq ID No. 86 to 146, or of mutants or fragments thereof as a target for screening of compounds that reduce and/or prevent obesity.

The present invention also provides a kit for screening for compounds that reduce and/or prevent obesity comprising a polypeptide selected from the group consisting of Seq ID No. 13 to 24.

The present invention additionally provides a kit for screening for compounds that reduce and/or prevent obesity comprising a polypeptide selected from the group consisting of Seq ID No. 86 to 146.

### Examples:

### Example 1. RNA preparation

Total RNA from 500 mg subcutaneous fat tissue was isolated using the TriZol reagent (Life Technologies) and the Fast RNA green (BIO101) kit according to the manufacturer's protocols. Total DNA was purified from contaminating DNA the RNeasy kit (Qiagen).

### Example 2. Gene expression measurement by DNA chips

Synthesis of first and second strand cDNA were performed using the SuperScript Choice Gene Chip Kit (Life Technologies) and reagents from Gibco. The double stranded cDNA, containing an incorporated T7 RNA polymerase binding site, was purified by extraction with a mix of phenol:chloroform:isoamylalcohol (Life Technologies). The organic and aqueous phases were separated by Phase Lock Gel (Eppendorf) and double-stranded cDNA was recovered by precipitation according to the manufacturer's protocol and then resuspended in water.

The double-stranded cDNA was converted to biotin-labeled cRNA by *in vitro* transcription (IVT) using a T7 kit (Ambion) and biotin-containing ribonucleotides (Enzo - LOXO GmbH). The IVT-material was purified from unincorporated ribonucleotides using RNeasy spin columns (Qiagen). Following cleanup, the single-stranded biotin-labeled cRNA were chemically hydrolyzed to smaller fragments in 500 mM calcium acetate, 150 mM magnesium acetate, pH 8.1 for 35 min at 95°C. The reaction was terminated by chilling samples on ice.

Probes were hybridized to the U95 A GeneChip Microarray (Affymetrix) which contains features representing ∼12,000 genes. All washing, hybridization, detection, and signal amplification steps were performed using a GeneChip Fluidics Station (Affymetrix). Fluorescence intensity data was collected from the hybridized GeneArrays using a GeneArray scanner (Affymetrix). The raw files containing the fluorescence intensity information were transformed into data files using the Affymetrix Microarray Suite (MAS) software. Differentially expressed genes were identified using the Roche Affymetrix Chip Experiment Analysis (RACE-A) software. Differences between control patients (n=10) vs. obese case patients (n=10) were evaluated by several statistical filters as change factor vs. control.

**Table 2**

| Genes down-regulated in fat tissue in obesity | | | | | | |
|---|---|---|---|---|---|---|
| Seq ID No. DNA (protein) | Description | CHCF | P value | Lean mean | Obese mean | Unigene No. Accession No. |
| 1 (13) | Alpha-2-glycoprotein, zinc | -10.57 | 0.01292 | 122.29 | 10.35 | His.512643 NM001185 |
| 2 (14) | Insulin receptor substrate 1 | -1.25 | 0.00211 | 21.64 | 8.51 | Hs.390242 NM005544 |
| 3 (15) | Apolipoprotein a-i | -418.62 | 0.05987 | 1678.49 | 621.08 | Hs.93194 M27875 |
| 4 (16) | Aryl hydrocarbon receptor nuclear translocator | -1.14 | 0.00032 | 30.9 | 13.49 | Hs.131494 NM001668 |
| 5 (17) | Creatine kinase, brain | -0.82 | 0.01454 | 395.52 | 223.65 | Hs.173724 NM001823 |
| 6 (18) | Sterol-c5-desaturase | -1.13 | 0.00011 | 48.56 | 27.03 | Hs.287749 AF187981 |

**Table 3**

| Genes up-regulated in fat tissue in obesity | | | | | | |
|---|---|---|---|---|---|---|
| Seq ID No. DNA (protein) | Description | CHCF | P value | Lean mean | Obese mean | Unigene No. Accession No. |
| 7 (19) | osteomodulin | 1.1 | 0.00008 | 2.96 | 7.77 | Hs.94070 NM005014 |
| 8 (20) | Very low density lipoprotein receptor | 1.65 | 0.0032 | 67.14 | 96.66 | Hs.370422 D16494 |
| 9 (21) | Hydroxysteroid (11-beta) dehydrogenase 1 | 1.87 | 0.00108 | 25.78 | 58.23 | Hs.275215 NM005525 |
| 10 (22) | reelin | 1.72 | 0.00003 | 4.79 | 11.83 | Hs.431010 U79716 |
| 11(23) | Atp-binding cassette, subfamily c (cftr/mrp), member 10 | 1.1 | 0.00193 | 17.71 | 38.51 | Hs.55879 AL133613 |
| 12 (24) | Homo sapiens dna for cyclophilin 40 | 2.63 | 0.00008 | 53.63 | 145.18 | D63861 |

**Table 4**

| Genes down-regulated in fat tissue in obesity | | | | | |
|---|---|---|---|---|---|
| Seq ID No. DNA (protein) | Description | CHCF | P value | Locus ID (Gene ID) | Accession No. |
| 25( 86) | Retinoid x receptor gamma | -0.63 | 0.00041 | 6258 | NM_006917 |
| 26 (87) | Vascular endothelial growth factor | -1.52 | 0.01468 | 7422 | NM_003376 |
| 27 (88) 28 (89) | Cannabinoid receptor 1 (brain) | -0.34 | 0.00788 | 1268 | NM_016083 Variant 1 NM_033181 Variant 2 |
| 29 (90) | Cathepsin O | -0.52 | 0.00260 | 1519 | NM_001334 |
| 30 (91) | Enoyl coenzyme a hydratase, short chain 1, mitochondrial | -0.45 | 0.00169 | 1892 | NM_004092 |
| 31 (92) | Vascular endothelial growth factor B | -0.24 | 0.00169 | 7423 | NM_003377 |
| 32 (93) 33 (94) | Pyruvate carboxylase | -0.77 | 0.00520 | 5091 | NM_000920 Variant A NM_022172 Variant 2 |
| 34 (95) | L-3-hydroxyacyl-Coenzyme A dehydrogenase, short chain | -0.83 | 0.00201 | 3033 | NM_005327 |
| 35 (96) | pyruvate dehydrogenase kinase, isoenzyme 2 | -0.47 | 0.00186 | 5164 | NM_002611 |
| 36 (97) 37 (98) | Citrate synthase | -0.66 | 0.02528 | 1431 | NM_004077 Variant 1 NM_198324 Variant 2 |

**Table 5**

| Genes up-regulated in fat tissue in obesity | | | | | |
|---|---|---|---|---|---|
| Seq ID No. DNA | Description | CHCF | P value | Locus ID (Gene ID) | Accession No. |
| (protein) | | | | | |
| 38 (99) 39 (100) 40(101) 41 (102) 42(103) 43 (104) 44 (105) 45 (106) 46 (107) 47 (108) 48 (109) 49 (110) 50 (111) | fibroblast growth factor receptor 2 | 1.12 | 0.00015 | 2263 | NM_000141 Variant 1 NM_022969 Variant 2 NM_022970 Variant 3 NM_022971 Variant 4 NM_022972 Variant 5 NM_022973 Variant 6 NM_022974 Variant 7 NM_022975 Variant 8 NM_022976 Variant 9 NM_023028 Variant 10 NM_023029 Variant 11 NM_023030 Variant 12 NM_023031 Variant 13 |
| 51 (112) | tumor necrosis factor receptor superfamily, member 1B | 0.32 | 0.00015 | 7133 | NM_001066 |
| 52 (113) | tumor necrosis factor (ligand) superfamily, member 10 | 0.75 | 0.00548 | 8743 | NM_003810 |
| 53 (114) 54 (115) | angiopoietin 1 | 1.52 | 0.00010 | 284 | NM_001146 Variant 1 NM_139290 Variant 2 |
| 55 (116) 56 (117) 57 (118) 58 (119) | retinoid X receptor, alpha degenerative spermatocyte homolog, lipid desaturase solute carrier family 7 member 7 | 0.42 1.19 1.15 | 0.00885 0.00011 0.00215 | 6256 8560 9056 | NM_002957 NM_003676 Variant 1 NM_144780 Variant2 NM_003982 |
| 59 (120) 60(121) | procollagen-lysine, 2-oxoglutarate 5-dioxygenase 2 | | | | NM_000935 Variant 2 NM_182943 Variant 1 |
| 61 (122) | UDP-glucose dehydrogenase | 0.64 | 0.00045 | 7358 | NM_003359 |
| 62 (123) 63 (124) | phosphatidylinositol-4-phosphate 5-kinase, type II beta | 0.43 | 0.00892 | 8396 | NM_003559 Variant 1 NM_138687 Variant 2 |
| 64 (125) 65 (126) | MAP kinase interacting serine/threonine kinase 2 | 0.64 | 0.00013 | 2872 | NM_017572 Sequence 1 NM_199054 Sequence 2 |
| 66 (127) | carboxypeptidase E | 1.52 | 0.00013 | 1363 | NM_001873 |
| 67 (128) | carboxypeptidase A3 (mast cell) | 0.8 | 0.00802 | 1359 | NM_001870 |
| 68 (129) | solute carrier family 38, member 6 | 1.45 | 0.00271 | 145389 | NM_153811 |
| 69 (130) | very low density lipoprotein receptor | 1.65 | 0.00320 | 7436 | NM_003383 |
| 70 (131) | cathepsin G | 1.61 | 0.00057 | 1511 | NM_001911 |
| 71 (132) 72 (133) | Galactokinase 2 | 1.02 | 0.00067 | 2585 | NM_001001556 Variant 2 NM_002044 Variant 1 |
| 73 (134) | prostaglandin D2 synthase | 1 | 0.00046 | 5730 | NM_000954 |
| 74 (135) 75 (136) 76 (137) 77 (138) | mitogen-activated protein kinase 9 | 0.86 | 0.00014 | 5601 | NM_002752 Variant 1 NM_139068 Variant 2 NM_139069 Variant 3 NM_139070 Variant 4 |
| 78 (139) 79 (140) 80 (141) | Interleukin 15 | 0.95 | 0.00007 | 3600 | NM_000585 Variant 3 NM_172174 Variant 1 NM_172175 Variant 2 |
| 81 (142) 82 (143) | mucosa associated lymphoid tissue lymphoma translocation gene 1 | 1.24 | 0.00380 | 10892 | NM_006785 Variant 1 NM_173844 Variant2 |
| 83 (144) | lysyl oxidase | 0.7 | 0.04940 | 4015 | NM_002317 |
| 84 (145) | Integrin beta 5 | 1.38 | 0.00030 | 3693 | NM_002213 |
| 85 (146) | aldehyde dehydrogenase 3 family, member A2 | 0.91 | 0.00003 | 224 | NM_000382 |

## Claims

1. A method of screening for compounds that reduce and/or prevent obesity comprising
a) contacting a cell expressing a gene listed in table 2 with a compound; and
b) measuring the expression of said gene, or a polypeptide encoded by said gene;
wherein a compound which up-regulates gene expression is a compound which causes an increase of expression of said gene or of the polypeptide encoded by said gene.

2. The method of claim 1, wherein the gene is Seq. ID No. 1.

3. The method of claim 1, wherein the gene is Seq. ID No. 2.

4. The method of claim 1, wherein the gene is Seq. ID No. 3.

5. The method of claim 1, wherein the gene is Seq. ID No. 4.

6. The method of claim 1, wherein the gene is Seq. ID No. 5.

7. The method of claim 1, wherein the gene is Seq. ID No. 6.

8. A method of screening for compounds that reduce and/or prevent obesity comprising
a) contacting a cell expressing a gene selected from the group consisting of Seq ID No. 25-37 with a compound; and
b) measuring the expression of said gene, or a polypeptide encoded by said gene;
wherein a compound which up-regulates gene expression is a compound which causes an increase of expression of said gene or of the polypeptide encoded by said gene.

9. A method of screening for compounds that reduce and/or prevent obesity comprising
a) contacting a cell expressing a gene listed in table 3 with a compound; and
b) measuring the expression of said gene, or a polypeptide encoded by said gene;
wherein a compound which down-regulates gene expression is a compound which causes a decrease of said gene or a polypeptide encoded by said gene.

10. The method of claim 8, wherein the gene is Seq. ID No. 7.

11. The method of claim 8, wherein the gene is Seq. ID No. 8.

12. The method of claim 8, wherein the gene is Seq. ID No. 9.

13. The method of claim 8, wherein the gene is Seq. ID No. 10.

14. The method of claim 8, wherein the gene is Seq. ID No. 11.

15. The method of claim 8, wherein the gene is Seq. ID No. 12.

16. A method of screening for compounds that reduce and/or prevent obesity comprising
a) contacting a cell expressing a gene selected from the group consisting of Seq ID No. 38 to 85 with a compound; and
b) measuring the expression of said gene, or a polypeptide encoded by said gene;
wherein a compound which down-regulates gene expression is a compound which causes a decrease of said gene or a polypeptide encoded by said gene.

17. A method of screening for compounds that reduce and/or prevent obesity comprising:
a) contacting a polypeptide selected from the group consisting of Seq ID No. 13 to 18 with a compound; and
b) determining and/or measuring the activity and/or function of said polypeptide;
wherein a compound which reduces and/or prevents obesity by agonizing said polypeptide is a compound which causes an increase in activity and/or function of said polypeptide.

18. A method of screening for compounds that reduce and/or prevent obesity comprising:
a) contacting a polypeptide selected from the group consisting of Seq ID No. 86 to 98 with a compound; and
b) determining and/or measuring the activity and/or function of said polypeptide;
wherein a compound which reduces and/or prevents obesity by agonizing said polypeptide is a compound which causes an increase in activity and/or function of said polypeptide.

19. A method of screening for compounds that reduce and/or prevent obesity comprising:
a) contacting a polypeptide selected from the group consisting of Seq ID No. 19 to 24 with a compound; and
b) determining and/or measuring the activity and/or function of said polypeptide;
wherein a compound which reduces and/or prevents obesity by antagonizing said polypeptide is a compound which causes a decrease in activity and/or function of said polypeptide.

20. A method of screening for compounds that reduce and/or prevent obesity comprising:
a) contacting a polypeptide selected from the group consisting of Seq ID No. 99 to 146 with a compound; and
b) determining and/or measuring the activity and/or function of said polypeptide;
wherein a compound which reduces and/or prevents obesity by antagonizing said polypeptide is a compound which causes a decrease in activity and/or function of said polypeptide.

21. A method for screening of compounds that reduce and/or prevent obesity comprising:
a) contacting a cell expressing a nucleic acid encoding a polypeptide selected from the group consisting of Seq ID No. 13 to 18 with a compound; and
b) determining and/or measuring the activity and/or function of said polypeptide;
wherein a compound which reduces and/or prevents obesity by agonizing said polypeptide is a compound which causes an increase in activity and/or function of said polypeptide.

22. A method for screening of compounds that reduce and/or prevent obesity comprising:
a) contacting a cell expressing a nucleic acid encoding a polypeptide selected from the group consisting of Seq ID No. 86 to 98 with a compound; and
b) determining and/or measuring the activity and/or function of said polypeptide;
wherein a compound which reduces and/or prevents obesity by agonizing said polypeptide is a compound which causes an increase in activity and/or function of said polypeptide.

23. A method for screening of compounds that reduce and/or prevent obesity comprising:
a) contacting a cell expressing a nucleic acid encoding a polypeptide selected from the group consisting of Seq ID No. 19 to 24 with a compound; and
b) determining and/or measuring the activity and/or function of said polypeptide;
wherein a compound which reduces and/or prevents obesity by antagonizing said polypeptide is a compound which causes a decrease in activity and/or function of said polypeptide.

24. A method for screening of compounds that reduce and/or prevent obesity comprising:
a) contacting a cell expressing a nucleic acid encoding a polypeptide selected from the group consisting of Seq ID No. 99 to 146 with a compound; and
b) determining and/or measuring the activity and/or function of said polypeptide;
wherein a compound which reduces and/or prevents obesity by antagonizing said polypeptide is a compound which causes a decrease in activity and/or function of said polypeptide.

25. A method of screening for compounds that bind to a polypeptide selected from the group consisting of the polypeptides of Seq ID No. 13 to 24, comprising the steps of
a) contacting a compound with said polypeptide; and
b) determining the ability of said compound to bind to said polypeptide.

26. A method of screening for compounds that bind to a polypeptide selected from the group consisting of the polypeptides of Seq ID No. 86 to 146, comprising the steps of
a) contacting a compound with said polypeptide; and
b) determining the ability of said compound to bind to said polypeptide.

27. Use of a gene or a polypeptide encoded by a gene listed in tables 2 and/or 3 as a target for screening of compounds that reduce and/or prevent obesity.

28. Use of a gene or a polypeptide encoded by a selected from the group consisting of Seq ID No. 25 to 146 as a target for screening of compounds that reduce and/or prevent obesity.

29. Use of a nucleic acid encoding a polypeptide selected from the group consisting of Seq ID No. 13-24, or of mutants or fragments thereof as a target for screening of compounds that reduce and/or prevent obesity.

30. Use of a nucleic acid encoding a polypeptide selected from the group consisting of Seq ID No. 86 to 146, or of mutants or fragments thereof as a target for screening of compounds that reduce and/or prevent obesity.

31. A kit for screening for compounds that reduce and/or prevent obesity comprising a polypeptide selected from the group consisting of Seq ID No. 13 to 24.

32. A kit for screening for compounds that reduce and/or prevent obesity comprising a polypeptide selected from the group consisting of Seq ID No. 86 to 146.

33. A compound identified by the method of any one of claims 1 to 26.

34. A pharmaceutical formulation for the modulation of body weight, comprising a compound that modulates the activity of a polypeptide selected from the group consisting of Seq ID No. 13 to 24, mixed with a pharmaceutically acceptable carrier.

35. A pharmaceutical formulation for the modulation of body weight, comprising a compound that modulates the activity of a polypeptide selected from the group consisting of Seq ID No. 86 to 146, mixed with a pharmaceutically acceptable carrier.

36. Use of a compound of claim 33 for the preparation of a medicament for the treatment of obesity.

37. Use of a compound of claim 33 for the preparation of a medicament for the treatment of cachexia.

38. The methods, compound, formulation and uses substantially as hereinbefore described, especially with reference to the foregoing examples.
